# EUROPEAN PATENT APPLICATION

(11) **EP 4 039 685 A1**
(43) Date of publication of application: **10.08.2022**
(21) Application number: 21155769.9
(22) Date of filing: 08.02.2021
(51) Int. Cl.: C07D 409/14, C07D 401/14, C07D 417/14, C07D 487/04, C07D 519/00, A61K 31/497, A61K 31/4985, A61P 35/00, A61P 37/00

(54) **AZABICYCLIC SHP2 INHIBITORS**

(71) Applicant: IRBM S.P.A., 00071 Pomezia (RM) (IT); C.N.C.C.S. S.c.a.r.l. Collezione Nazionale Dei Composti Chimici e Centro Screening, 00071 Pomezia (RM) (IT)
(72) Inventor: DI FABIO, Romano, 00071 Pomezia (IT); PETROCCHI, Alessia, 00071 Pomezia (IT); FERRANTE, Luca, 20132 Milano (IT); GRILLO, Alessandro, 20132 Milano (IT); SFERRAZZA, Alessio, 00071 Pomezia (IT); MONTALBETTI, Christian, 00071 Pomezia (IT); ROSSETTI, Ilaria, 00071 Pomezia (IT); TORRENTE, Esther, 00071 Pomezia (IT)
(74) Representative: Capasso, Olga

(57) **Abstract**

The present invention relates to new compounds capable of inhibiting the activity of SHP2 phosphatase, having the general Formula (I):

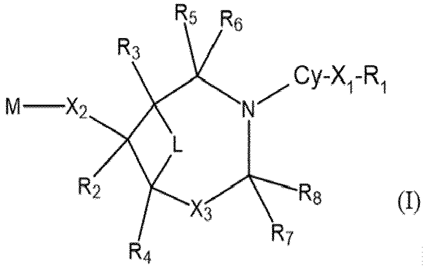

## Description

### FIELD OF THE INVENTION

The present invention relates to new compounds capable of inhibiting the activity of SHP2 phosphatase. Compounds of the invention can be used for the treatment of disorders associated with SHP2 deregulation. The present invention also relates to pharmaceutical compositions containing said compounds and to their method of manufacture.

### BACKGROUND OF THE INVENTION

Src homology phosphotyrosine phosphatase 2 (SHP2) encoded by *PTPN11* is a non-receptor protein tyrosine phosphatase (PTP) composed of a C-terminal domain, a PTP domain, and two N-terminal Src homology (N-SH2) domains, that contributes to multiple cellular functions including proliferation, differentiation, cell cycle maintenance and migration. SHP2 is a positive regulator of signalling downstream of several receptor tyrosine kynases through the Ras-mitogen-activated protein kinase, the JAK-STAT or the phosphoinositol-3-kinase-AKT pathways. The protein exists in an inactive, self-inhibited conformation, stabilized by a binding network involving residues from both the N-SH2 domains and the catalytic PTP domain. Recruitment of SHP2 to an activated receptor releases the self-inhibitory conformation and leads to catalytic activation of its phosphatase domain. In addition to its function as a phosphatase, SHP2 also serves as a docking protein to recruit other signalling intermediates through its two amino terminus N-SH2 domains. Since SHP2 is a positive regulator of cellular signalling leading to proliferation, differentiation, and survival, its constitutive activation is associated with oncogenesis.

SHP2 emerged as an attractive target for therapeutic targeting in the treatment of various diseases, such as Noonan Syndrome, Leopard Syndrome, juvenile myelocytic leukemias, neuroblastoma, melanoma, acute myeloid leukemia and cancers of the breast, lung and colon.

Both academic institutions and pharmaceutical companies have disclosed drug discovery programs exploiting SHP2 inhibitors based on different heterocyclic scaffolds.

WO2015/107493, WO2015/107494 and WO2015/107495 from Novartis disclose compounds of general formula (A) as indicated below:

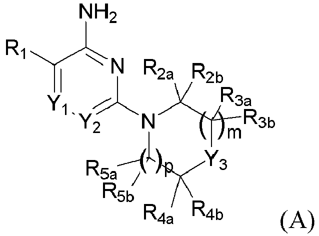

Still from Novartis the compounds of general structures (B), (C), (D) and (E) indicated below are disclosed in, respectively, WO2016/203404, WO2016/203405 and WO2017/216706:

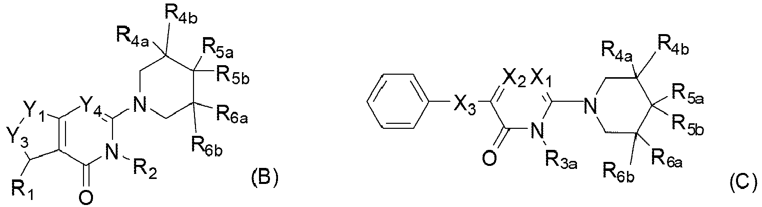

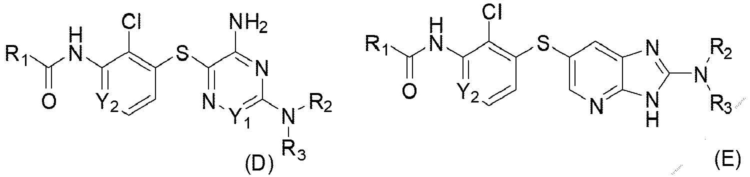

The general structure E disclosed in WO2017/216706 and the compounds therein identified are 2-amino-3H-imidazo[4,5-b]pyridine 5- or 6- thiol derivatives.

Jacobio Pharmaceuticals disclosed in WO2017/211303 and in WO2018/172984 pyrazine derivatives of structures (F) and (G) as indicated below:

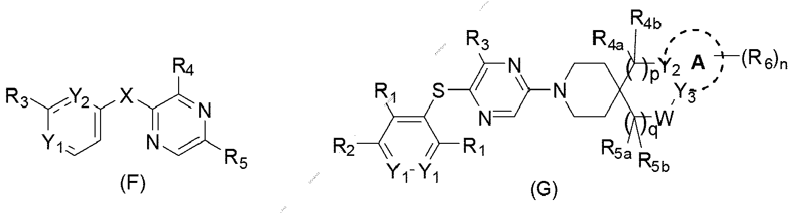

Futher pyridine, pyrazine and triazine compounds as allosteric SHP2 inhibitors have been recently disclosed by Revolution Medicines in WO2018/013597, WO2018/136264 and WO2019/075265.

WO2018/136265 and WO2019/118909 both relate to bicyclic heteroaromatic scaffolds comprising imidazopyrazines, triazolopyrazines, pyrazolopyridine, imidazopyrimidines of general structure (H):

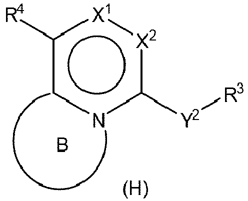

Pyrazolopyrazines and ring-fused pyrimidin-4-ones have been disclosed by the Board of Regents, University of Texas System, in WO2017/210134 and in WO2017/156397, respectively.

Pyrazolopyrazines were further disclosed by Relay Therapeutics in WO2018/081091, WO2018/218133, WO2018/057884 and WO2019/067843.

Imidazopyrimidine indicated below are disclosed by Gilead in WO2020/072656:

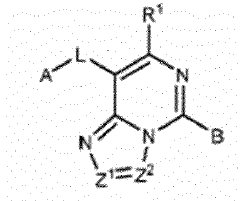

SHP2 therefore represents a highly attractive target for the development of novel therapies for the treatment of various diseases where it is involved. Therefore, there is the need to develop novel therapeutic agents that act as SHP2 inhibitors. The compounds of the present invention fulfil such need since they are small molecules capable of inhibiting the activity of SHP2.

### DESCRIPTION OF THE INVENTION

The present invention relates to heterocyclic compounds useful as SHP2 inhibitors and for the treatment of conditions mediated by SHP2.

The inventors have found that compounds having a specific general formula surprisingly act as potent SHP2 inhibitors, as evidenced by both enzymatic and cellular IC₅₀ values for SHP2 in the low nanomolar or micromolar range.

It is therefore an object of the present invention a compound of general formula (I): 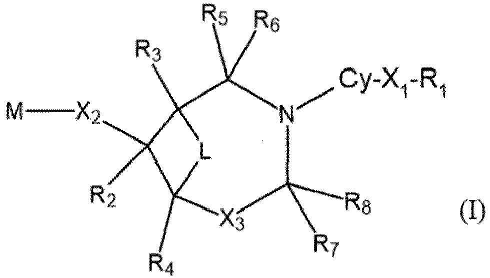 Wherein
X₁ is S, O, CR₁R_{1b}, NH, N-CH₃ or a single bond;
X₂ is (CR₂ₐR_{2b})ₘ;
X₃ is (CR₃ₐR_{3b})ₙ;
M is NH₂, NH-C₁₋₆alkyl, N(C₁₋₆alkyl)₂, NHC(=O)Rₓ or NHC(=O)ORₓ wherein Rₓ is C₁₋₆alkyl, aryl, heteroaryl or C₁₋₆alkyl-aryl;
L is a bond or C₁₋₃alkyl;
Cy is a ring of general formula (A): 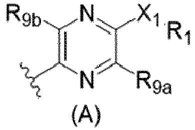 wherein
   - R₉ₐ is selected from H, NH₂, C₁₋₆alkyl, halogen, cyano, C₃₋₅cycloalkyl, haloC₁₋₆alkyl, hydroxy-C₁₋₆alkyl or aminoC₁₋₆alkyl;
   - R_{9b} is selected from H, C₁₋₆alkyl, halogen, haloC₁₋₆alkyl, hydroxy-C₁₋₆alkyl or aminoC₁₋₆alkyl;
or Cy is a bicyclic ring of general formula (B): 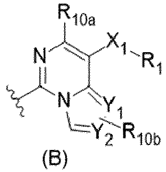 wherein
   - Y₁ and Y₂ are C or N;
   - R₁₀ₐ is H or C₁₋₆alkyl;
   - R_{10b} is at each occurrence independently H, C₁₋₆alkyl, NH₂, CN, OH, haloC₁₋₃alkyl, C₁₋₃alkoxy, haloC₁₋₃alkoxy, or halogen;
or Cy is a bicyclic ring of general formula (C): 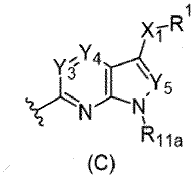 wherein
   - Y₃ is C-R_{11b} or N;
   - Y₄ is C-R_{11b} or N; or Y₃ is N-CH₃ and Y₄ is C(=O);
   - Y₅ is C-R_{11c} or N;
   - Y₃=Y₄ is a double bond when Y₃ is C-R_{11b} or N and when Y₄ is C-R_{11b} or N; Y₃=Y₄ is a single bond when Y₃ is N-CH₃ and Y₄ is C(=O);
   - R₁₁ₐ is H or methyl;
   - R_{11b} is H, C₁₋₆alkyl, hydroxyC₁₋₆alkyl;
   - R_{11c} is H, C₁₋₆alkyl, halogen;
or Cy is a bicyclic ring of general formula (D): 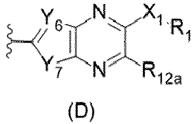 wherein
   - Y₆ is N, CH or C-CH₃;
   - Y₇ is N-R_{12b}, O or S;
   - R₁₂ₐ is H, C₁₋₆alkyl, halogen or cyano;
   - R_{12b} is H or C₁₋₆alkyl;
or Cy is a bicyclic ring of general formula (E): 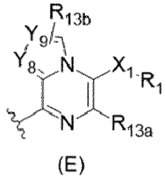 wherein
   - Y₈ and Y₉ are C or N;
   - R₁₃ₐ is H, C₁₋₆alkyl, halogen, NH₂ or cyano;
   - R_{13b} is at each occurency independently H, CH₃ or hydroxyC₁₋₆alkyl;
R₁ is an aryl, heteroaryl, partially unsaturated aryl, partially unsaturated heteroaryl or an heterocyclic ring, each of said aryl, heteroaryl, partially unsaturated aryl, partially unsaturated heteroaryl or an heterocyclic ring being optionally substituted with one or more substituents independently selected from C(O)CH₃, C(O)OCH₃, heteroaryl, aryl, OH, halogen, NH₂, NH-C₁₋₆alkyl, N(C₁₋₆alkyl)₂, C₁₋₆alkyl, C₃₋₅cycloalkyl, C₁₋₆alkyl-NH₂, C₁₋₆alkyl-NH(CH₃), C₁₋₆alkyl-NH(CH₃)₂, C₁₋₆alkyl-NHCOCH₃, C₁₋₃alkylaryl, C₁₋₃alkylheteroaryl, haloC₁₋₆alkyl, hydroxyC₁₋₆alkyl, CN, haloC₁₋₆alkoxy, C₁₋₆alkoxy, C₅₋₇heterocycloalkoxy, oxetane, OCF₃, CH₂OCH₃, OSO₂F, SO₂F and a cyclic amine selected from the group consisting of: pyrrolidine, piperidine, morpholine, thiomorpholine, piperazine, N-methylpyperazine and pyrrolidin-3-yloxy;
R₂ is H, aryl, heteroaryl, C₃₋₉cycloalkyl, partially unsaturated heteroaryl, C₁₋₃alkylaryl, C₁₋₃alkylheteroaryl, each of said aryl, heteroaryl, C₃₋₉cycloalkyl, partially unsaturated heteroaryl, C₁₋₃alkylaryl, C₁₋₃alkylheteroaryl being optionally substituted with one ore more substituents independently selected from the group consisting of: halogen, CN, C₁₋₃alkyl, C₃₋₈cycloalkyl, haloC₁₋₃alkyl, C₁₋₃alkoxy, haloC₁₋₃alkoxy, C₁₋₃alkyl-N(R_{y})₂, C(O)OC₁₋₃alkyl, C(O)C₁₋₃alkyl, OH, hydroxy-C₁₋₃alkyl, C₁₋₃alkyl-OC₁₋₃alkyl, CON(R_{y})₂, N(R_{y})₂ wherein each R_{y} is independently selected from H, methyl, ethyl and cyclopropyl;
R₁ₐ and R_{1b} are independently selected from H, C₁₋₆alkyl or R₁ₐ and R_{1b} are joined together to form together with the carbon atom to which they are linked a spiro-C₃₋₅cycloalkyl ring;
R₂ₐ and R_{2b} are independently selected from H, halogen, C₁₋₆alkyl, C₃₋₅cycloalkyl or R₃ₐ and R_{3b} are joined together to form together with the carbon atom to which they are linked a spiro-C₃₋₅cycloalkyl ring;
R₃, R₃ₐ, R_{3b}, R₄, R₅, R₆, R₇ and R₈ are at each occurrence independently selected from H, methyl or halogen;
m is 0, 1, 2 or 3;
n is 0, 1 or 2, with the proviso that if n is 0 then Cy has general structure (A), (C) (D) or (E);
or a pharmaceutically acceptable salt, tautomer, solvate, or stereoisomer thereof.

In a preferred embodiment compounds of the invention have general formula (II) or general formula (III) or general formula (IV) or general formula (V) or general formula (VI) or or general formula (VII) or general formula (VIII) or general formula (IX): 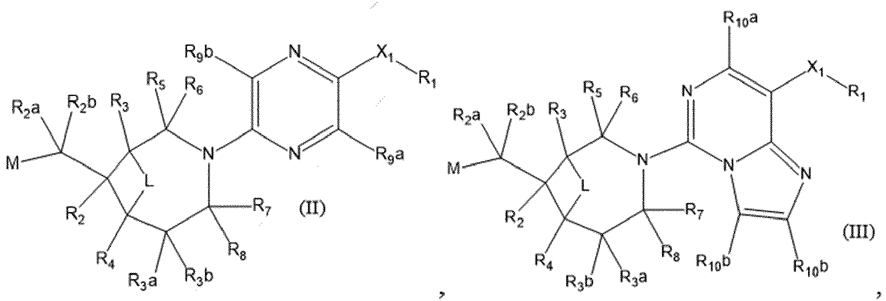 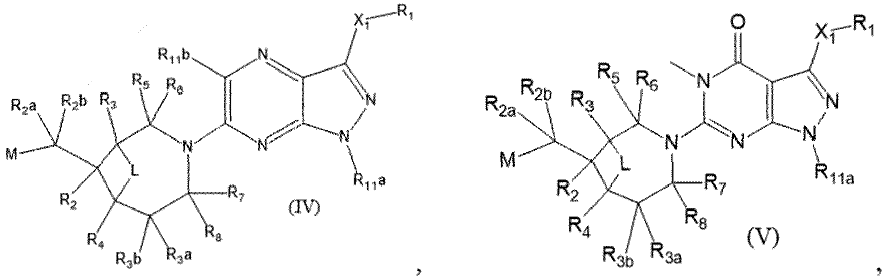 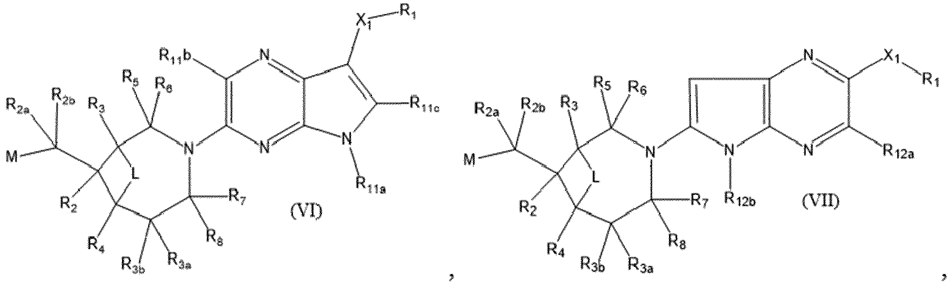 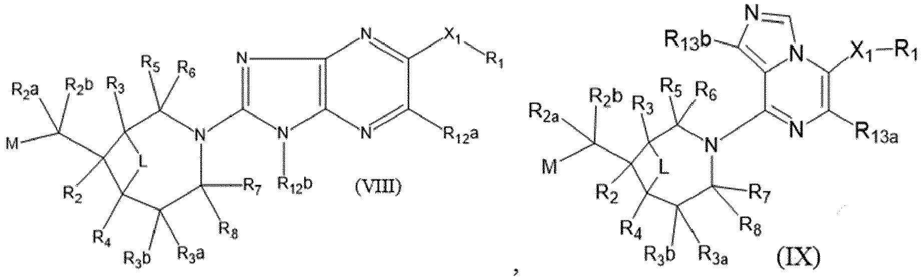 wherein: X₁, R₁, R₂, R₂ₐ, R_{2b}, R₃, R₃ₐ, R_{3b}, R₄, R₅, R₆, R₇, R₈, R₉ₐ, R_{9b}, R₁₀ₐ, R_{10b}, R₁₁ₐ, R_{11b}, R₁₂ₐ, R_{12b}, R₁₃ₐ, R_{13b}, M and L are as defined above.

In a further preferred embodiment compounds of the invention have general formula (X) or general formula (XI) or general formula (XII) or general formula (XIII) or general formula (XIV) or general formula (XV) or general formula (XVI): 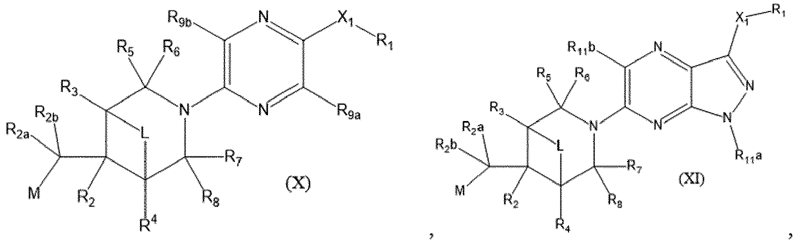 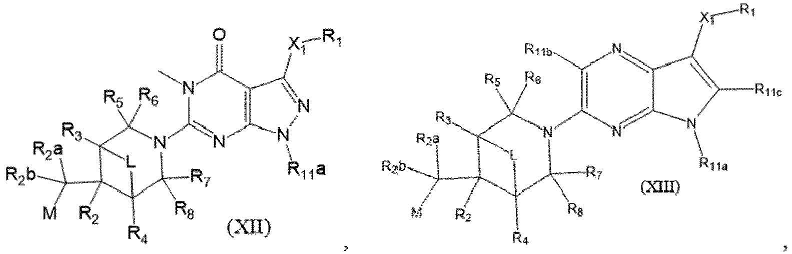 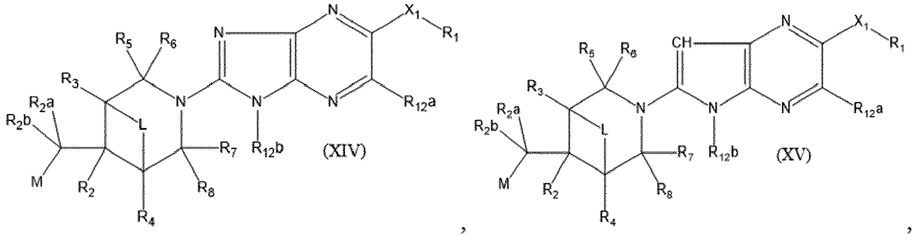 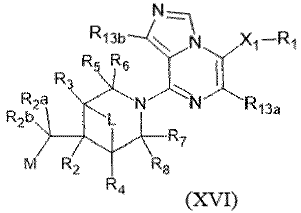 wherein: X₁, R₁, R₂, R₂ₐ, R_{2b}, R₃, R₄, R₅, R₆, R₇, R₈, R₉ₐ, R_{9b}, R₁₁ₐ, R_{11b}, R_{11c}, R₁₂ₐ, R_{12b}, R₁₃ₐ, R_{13b}, M and L are as defined above.

In a further preferred embodiment in the compounds of the invention:
- L is a single bond; and/or
- X₁ is a single bond or S.

Still preferably M is NH₂; even more preferably R₂ₐ, R_{2b}, R₃, R₃ₐ, R_{3b}, R₄, R₅, R₆, R₇, R₈ are H.

R₁ is preferably selected from: phenyl, napthyl, benzothiophene, quinoline, quinoxaline, isoquinoline, pyrazolo[3,4-b]pyrazine, pyridine, pyrimidine, pyrazine, thienopyridine, indole, indoline, 2,3-dihydroisoindol-1-one, indol-2-one, benzothiazole, 6a,7,8,9-tetrahydro-6H-pyrido[3,2-b]pyrrolo[1,2-d][1,4]oxazine, naphtyridine, 1,2,3,4-tetrahydro-1,5-naphthyridine, benzo[d][1,3]dioxole, 2,3-dihydrobenzo[b][1,4]dioxine, benzoxazole, thieno[3,2-b]pyridine, indazole, each being optionally substituted with one or more substituents independently selected form halogen, C₁₋₃alkyl, CN, CF₃, NH₂, OCH₃, OCF₃, CH₂OCH₃, OSO₂F, SO₂F, oxetane, C(CH₃)₂OH, CH(CH₃)OH.

R₂ is preferably selected from: phenyl, pyridine, pyrimidine, pyrazine, oxazole, isoxazole, pyrrole, pyrazole triazole, oxadiazole, thiophene, thiazole, thiadiazole, imidazole, each optionally substituted with one or more substituents independently selected form halogen, C₁₋₃alkyl, CN, CF₃, NH₂, OCH₃, OH, CH₂OH, CH₂OCH₃, CO₂CH₃, CONHC₁₋₆alkyl.

In a preferred embodiment of the invention, the compounds are selected from the following list:
- [3-[7-(aminomethyl)-7-thiophen-3-yl-3-azabicyclo[4.1.0]heptan-3-yl]-6-(2,3-dichlorophenyl)-5-methylpyrazin-2-yl]methanol;
- [3-[3-(2,3-dichlorophenyl)-1H-pyrazolo[3,4-b]pyrazin-6-yl]-7-pyridin-3-yl-3-azabicyclo[4.1.0]heptan-7-yl]methanamine;
- [3-[3-(2,3-dichlorophenyl)-1H-pyrazolo[3,4-b]pyrazin-6-yl]-7-(2-fluorophenyl)-3-azabicyclo[4.1.0]heptan-7-yl]methanamine;
- [3-[3-(1-benzothiophen-7-yl)-1H-pyrazolo[3,4-b]pyrazin-6-yl]-7-(2-fluorophenyl)-3-azabicyclo[4.1.0]heptan-7-yl]methanamine;
- [7-(2-fluorophenyl)-3-(3-quinolin-5-yl-1H-pyrazolo[3,4-b]pyrazin-6-yl)-3-azabicyclo[4.1.0]heptan-7-yl]methanamine;
- [3-[3-(2,3-dichlorophenyl)-1H-pyrazolo[3,4-b]pyrazin-6-yl]-7-(3-methyl-1,2-oxazol-5-yl)-3-azabicyclo[4.1.0]heptan-7-yl]methanamine;
- [3-[3-(2,3-dichlorophenyl)-1H-pyrazolo[3,4-b]pyrazin-6-yl]-7-(3-fluorophenyl)-3-azabicyclo[4.1.0]heptan-7-yl]methanamine;
- [3-[3-(2,3-dichlorophenyl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-(3-quinolin-5-yl-1H-pyrazolo[3,4-b]pyrazin-6-yl)-3-azabicyclo[4.1.0]heptan-7-yl]methanamine;
- [3-[3-(2,3-dichlorophenyl)-1H-pyrazolo[3,4-b]pyrazin-6-yl]-7-(4-fluorophenyl)-3-azabicyclo[4.1.0]heptan-7-yl]methanamine;
- [7-(4-fluorophenyl)-3-(3-quinoxalin-6-yl-1H-pyrazolo[3,4-b]pyrazin-6-yl)-3-azabicyclo[4.1.0]heptan-7-yl]methanamine;
- [7-(4-fluorophenyl)-3-(3-quinolin-8-yl-1H-pyrazolo[3,4-b]pyrazin-6-yl)-3-azabicyclo[4.1.0]heptan-7-yl]methanamine;
- [3-[3-(2,3-dichlorophenyl)-1H-pyrazolo[3,4-b]pyrazin-6-yl]-7-(1-methyltriazol-4-yl)-3-azabicyclo[4.1.0]heptan-7-yl]methanamine;
- [3-[3-(2,3-dichlorophenyl)-1H-pyrazolo[3,4-b]pyrazin-6-yl]-7-(4-methyl-1,2-oxazol-3-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(4-methyl-1,2-oxazol-3-yl)-3-azabicyclo[4.1.0]heptan-7-yl]methanamine;
- 4-[6-[7-(aminomethyl)-7-(2-fluorophenyl)-3-azabicyclo[4.1.0]heptan-3-yl]-1H-pyrazolo[3,4-b]pyrazin-3-yl]-3-chloro-∼{N}-methylpyridin-2-amine;
- 4-[6-[7-(aminomethyl)-7-(5-methyl-1,2-oxazol-3-yl)-3-azabicyclo[4.1,0]heptan-3-yl]-1H-pyrazolo [3,4-b]pyrazin-3-yl]-3-chloro-∼{N}-methylpyridin-2-amine;
- [7-(5-methyl-1,2-oxazol-3-yl)-3-(3-thieno[3,2-b]pyridin-7-yl-1H-pyrazolo[3,4-b]pyrazin-6-yl)-3-azabicyclo[4.1.0]heptan-7-yl]methanamine;
- 4-[6-[7-(aminomethyl)-7-(5-methyl-1,2-oxazol-3-yl)-3-azabicyclo[4.1.0]heptan-3-yl]-1H-pyrazolo [3,4-b]pyrazin-3-yl]-2,3-dihydroisoindol-1-one;
- [3-[3-(1,3-benzothiazol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-[3-(8-methylquinolin-5-yl)-1H-pyrazolo[3,4-b]pyrazin-6-yl]-3-azabicyclo[4.1.0]heptan-7-yl]methanamine;
- [3-[3-(8-methoxyquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-(3-isoquinolin-5-yl-1H-pyrazolo[3,4-b]pyrazin-6-yl)-7-(5-methyl-1,2-oxazol-3-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-[3-(2-methylquinolin-5-yl)-1H-pyrazolo[3,4-b]pyrazin-6-yl]-3-azabicyclo[4.1.0]heptan-7-yl]methanamine;
- [3-[3-(2-methylindazol-4-yl)-1H-pyrazolo[3,4-b]pyrazin-6-yl]-7-(5-methyl-1,2-oxazol-3-yl)-3-azabicyclo[4.1,0]heptan-7-yl]methanamine;
- [7-(5-methyl-1,2-oxazol-3-yl)-3-(3-quinolin-4-yl-1H-pyrazolo[3,4-b]pyrazin-6-yl)-3-azabicyclo[4.1.0]heptan-7-yl]methanamine;
- [3-[3-(2-methoxypyridin-3-yl)-1H-pyrazolo[3,4-b]pyrazin-6-yl]-7-(5-methyl-1,2-oxazol-3-yl)-3-azabicyclo[4.1.0]heptan-7-yl]methanamine;
- (7-(5-methylisoxazol-3-yl)-3-(3-((S)-6a,7,8,9-tetrahydro-6H-pyrido[3,2-b]pyrrolo[1,2-d][1,4]oxazin-4-yl)-1H-pyrazolo[3,4-b]pyrazin-6-yl)-3-azabicyclo[4.1.0]heptan-7-yl)methanamine;
- [3-[3-(8-fluoroquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-[3-(2,3-dichloropyridin-4-yl)-1H-pyrazolo[3,4-b]pyrazin-6-yl]-7-(5-methyl-1,2-oxazol-3-yl)-3-azabicyclo[4.1.0]heptan-7-yl]methanamme;
- 5-[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]quinoline-8-carbonitrile;
- [3-[3-(8-chloroquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-[3-(8-chloroisoquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-[3-(1,5-naphthyridin-4-yl)-1H-pyrazolo[3,4-b]pyrazin-6-yl]-3-azabicyclo[4.1.0]heptan-7-yl]methanamine;
- 4-[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]-3-chloropyridin-2-amine;
- [(1S,6R,7S)-3-[3-(2,2-difluoro-1,3-benzodioxol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-7-(5-methyl-1,2-oxazol-3-yl)-3-[3-(1,8-naphthyridin-4-yl)-1H-pyrazolo[3,4-b]pyrazin-6-yl]-3-azabicyclo[4.1.0]heptan-7-yl]methanamine;
- [(1S,6R,7S)-3-[3-(6-fluoroquinolin-4-yl)-1H-pyrazolo[3,4-b]pyrazin-6-yl]-7-(5-methyl-1,2-oxazol-3-yl)-3-azabicyclo[4.1,0]heptan-7-yl]methanamine;
- [(1S,6R,7S)-3-[3-(2,3-dihydro-1,4-benzodioxin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(5-fluoroquinolin-4-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(6-chloroquinolin-4-yl)-1H-pyrazolo[3,4-b]pyrazin-6-yl]-7-(5-methyl-1,2-oxazol-3-yl)-3-azabicyclo[4.1,0]heptan-7-yl]methanamine;
- [(1S,6R,7S)-7-(5-methyl-1,2-oxazol-3-yl)-3-[3-[8-(trifluoromethyl)quinolin-5-yl]-1H-pyrazolo[3,4-b]pyrazin-6-yl]-3-azabicyclo[4.1.0]heptan-7-yl]methanamine;
- [(1S,6R,7S)-3-[3-(3,4-dichloro-2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(7-fluoroquinolin-4-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(4-chloro-2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-[5-(methoxymethyl)-1,2-oxazol-3-yl]-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(5-cyclopropyl-1,2-oxazol-3-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(3-fluoropyridin-2-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(5-fluoropyridin-2-yl)-3-azabicyclo[4.1.0]heptan-7-yl]methanamine;
- 4-[6-[(1S,6R,7S)-7-(aminomethyl)-7-(5-methyl-1,2-oxazol-3-yl)-3-azabicyclo [4.1.0]heptan-3 -yl] -1H-pyrazolo [3,4-b]pyrazin-3-yl]-3,3-difluoro-1-methylindol-2-one;
- [3-[3-(3-methyl-1,2-benzoxazol-5-yl)-1H-pyrazolo[3,4-b]pyrazin-6-yl]-7-(4-methyl-1,2-oxazol-3-yl)-3-azabicyclo[4.1.0]heptan-7-yl]methanamine;
- 4-[[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]sulfanyl]-3-chloropyridin-2-amine;
- [7-(5-methyl-1,2-oxazol-3-yl)-3-(3-thieno[3,2-b]pyridin-7-ylsulfanyl-1H-pyrazolo[3,4-b]pyrazin-6-yl)-3-azabicyclo[4.1.0]heptan-7-yl]methanamine;
- [6-[7-(aminomethyl)-7-(3-fluorophenyl)-3-azabicyclo[4.1.0]heptan-3-yl]-3-(2,3-dichlorophenyl)-1H-pyrazolo[3,4-b]pyrazin-5-yl]methanol;
- [6-[7-(aminomethyl)-7-(3-fluorophenyl)-3-azabicyclo[4.1.0]heptan-3-yl]-3-phenyl-1H-pyrazolo[3,4-b]pyrazin-5-yl]methanol;
- [3-(2-amino-3-chloropyridin-4-yl)sulfanyl-6-[7-(aminomethyl)-7-(5-methyl-1,2-oxazol-3-yl)-3-azabicyclo[4.1.0]heptan-3-yl]-1H-pyrazolo[3,4-b]pyrazin-5-yl]methanol;
- [3-(2-amino-3-chloropyridin-4-yl)-6-[7-(aminomethyl)-7-(5-methyl-1,2-oxazol-3-yl)-3-azabicyclo[4.1.0]heptan-3-yl]-1H-pyrazolo[3,4-b]pyrazin-5-yl]methanol;
- 4-[[6-[7-(aminomethyl)-7-(2-fluorophenyl)-3-azabicyclo[4.1,0]heptan-3-yl]-5H-pyrrolo[2,3-b]pyrazin-2-yl]sulfanyl]-3-chloropyridin-2-amine;
- [3-[3-(3,4-dihydro-2H-1,5-naphthyridin-1-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-[7-(aminomethyl)-3-[3-(4-chloro-2-methylindazol-5-yl)-1H-pyrazolo[3,4-b]pyrazin-6-yl]-3-azabicyclo[4.1.0]heptan-7-yl]-1,2-oxazol-5-yl]methanol;
- 6-[7-(aminomethyl)-7-(5-methyl-1,2-oxazol-3-yl)-3-azabicyclo[4.1.0]heptan-3-yl]-3-(4-chloro-2-methylindazol-5 -yl)-5 -methyl-1H-pyrazolo [3,4-d]pyrimidin-4-one;
- [7-phenyl-3-[5-[2-(trifluoromethyl)pyridin-3-yl]sulfanyl-1H-imidazo[4,5-b]pyrazin-2-yl]-3-azabicyclo[4.1.0]heptan-7-yl]methanamine;
- [7-thiophen-3-yl-3-[5-[2-(trifluoromethyl)pyridin-3-yl]sulfanyl-1H-imidazo[4,5-b]pyrazin-2-yl]-3-azabicyclo[4.1,0]heptan-7-yl]methanamine;
- [7-pyridin-2-yl-3-[5-[2-(trifluoromethyl)pyridin-3-yl]thio-1H-imidazo[4,5-b]pyrazin-2-yl]-3-azabicyclo[4.1.0]heptan-7-yl]methanamine;
- [7-pyrimidin-2-yl-3-[5-[2-(trifluoromethyl)pyridin-3-yl]sulfanyl-1H-imidazo[4,5-b]pyrazin-2-yl]-3-azabicyclo[4.1.0]heptan-7-yl]methanamine;
- [7-(4-methyl-1,3-thiazol-2-yl)-3-[5-[2-(trifluoromethyl)pyridin-3-yl]thio-1H-imidazo[4,5-b]pyrazin-2-yl]-3-azabicyclo[4.1.0]heptan-7-yl]methanamine;
- [3-[5-[2-(triftuoromethyl)pyridin-3-yl]thio-1H-imidazo[4,5-b]pyrazin-2-yl]-3-azabicyclo[4.1.0]heptan-7-yl]methanamine;
- 4-[[2-[7-(aminomethyl)-7-phenyl-3-azabicyclo[4.1.0]heptan-3-yl]-1H-imidazo[4,5-b]pyrazin-5-yl]thio]-3-chloropyridin-2-amine;
- 4-[[2-[7-(aminomethyl)-7-(4-methyl-1,3-thiazol-2-yl)-3-azabicyclo[4.1.0]heptan-3-yl]-1H-imidazo[4,5-b]pyrazin-5-yl]thio]-3-chloropyridin-2-amine;
- 6-[7-(aminomethyl)-7-phenyl-3-azabicyclo[4.1,0]heptan-3-yl]-3-[(2-(trifluoromethyl)pyridin-3-yl)thio]pyrazin-2-amine;
- 6-[7-(aminomethyl)-7-pyridin-2-yl-3-azabicyclo[4.1,0]heptan-3-yl]-3-[(2-(trifluoromethyl)pyridin-3-yl)thio]pyrazin-2-amine;
- 6-(7-(aminomethyl)-7-(4-methylthiazol-2-yl)-3-azabicyclo[4.1,0]heptan-3-yl)-3-((2-(trifluoromethyl)pyridin-3-yl)thio)pyrazin-2-amine;
- 3-((2-amino-3-chloropyridin-4-yl)thio)-6-[7-(aminomethyl)-7-(4-methyl-1,3-thiazol-2-yl)-3-azabicyclo[4.1,0]heptan-3-yl]pyrazin-2-amine;
- 6-(7-(aminomethyl)-3-azabicyclo[4.1.0]heptan-3-yl)-3-((2-(trifluoromethyl)pyridin-3-yl)thio)pyrazin-2-amine;
- 4-((5-(7-(aminomethyl)-7-(4-methylthiazol-2-yl)-3-azabicyclo[4.1.0]heptan-3-yl)imidazo[1,2-c]pyrimidin-8-yl)thio)-3-chloropyridin-2-amine;
- [3-[3-(2,3-dichlorophenyl)-1H-pyrazolo[3,4-b]pyrazin-6-yl]-7-(4-methyl-1,3-thiazol-2-yl)-3-azabicyclo[4.1,0]heptan-7-yl]methanamine;
- [(1S,6R,7S)-3-[3-(2,3-dichlorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [(1S,6R,7S)-3-[3-(4-chloro-2-methylindazol-5-yl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(2,3-dichlorophenyl)-1H-pyrazolo[3,4-b]pyrazin-6-yl]-7-phenyl-3-azabicyclo[4.1.0]heptan-7-yl]methanamine;
- [3-[3-(4-chloro-2-methylindazol-5-yl)-1H-pyrazolo[3,4-b]pyrazin-6-yl]-7-phenyl-3-azabicyclo[4.1.0]heptan-7-yl]methanamine;
- [3-[3-(2,3-dichlorophenyl)-1H-pyrazolo[3,4-b]pyrazin-6-yl]-7-pyridin-2-yl-3-azabicyclo[4.1.0]heptan-7-yl]methanamine;
- [3-[3-(2,3-dichlorophenyl)-1H-pyrazolo[3,4-b]pyrazin-6-yl]-7-pyrimidin-2-yl-3-azabicyclo[4.1.0]heptan-7-yl]methanamine;
- [3-[3-(2-chlorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(3-chlorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(2,3-difluorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(2-chloro-3-methoxyphenyl)-1H-pyrazolo[3,4-b]pyrazin-6-yl]-7-(4-methyl-1,3-thiazol-2-yl)-3-azabicyclo[4.1.0]heptan-7-yl]methanamine;
- [7-(4-methyl-1,3-thiazol-2-yl)-3-(3-quinolin-5-yl-1H-pyrazolo[3,4-b]pyrazin-6-yl)-3-azabicyclo[4.1.0]heptan-7-yl]methanamine;
- [7-(4-methyl-1,3-thiazol-2-yl)-3-(3-naphthalen-1-yl-1H-pyrazolo[3,4-b]pyrazin-6-yl)-3-azabicyclo[4.1.0]heptan-7-yl]methanamine;
- [7-(4-methyl-1,3-thiazol-2-yl)-3-[3-[2-(trifluoromethyl)pyridin-3-yl]-1H-pyrazolo[3,4-b]pyrazin-6-yl]-3-azabicyclo[4.1.0]heptan-7-yl]methanamine;
- [3-[3-(1,3-benzodioxol-4-yl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(8-fluoroquinolin-5-yl)-1H-pyrazolo[3,4-b]pyrazin-6-yl]-7-(4-methyl-1,3-thiazol-2-yl)-3-azabicyclo[4.1.0]heptan-7-yl]methanamine;
- 4-[[6-[7-(aminomethyl)-7-(4-methyl-1,3-thiazol-2-yl)-3-azabicyclo[4.1,0]heptan-3-yl]-1H-pyrazolo[3,4-b]pyrazin-3-yl]sulfanyl]-3-chloropyridin-2-amine;
- [3-[3-(2,3-dichlorophenyl)-1H-pyrazolo[3,4-b]pyrazin-6-yl]-7-thiophen-3-yl-3-azabicyclo[4.1.0]heptan-7-yl]methanamine;
- [7-thiophen-3-yl-3-[3-[2-(trifluoromethyl)pyridin-4-yl]-1H-pyrazolo[3,4-b]pyrazin-6-yl]-3-azabicyclo[4.1.0]heptan-7-yl]methanamine;
- [7-thiophen-3-yl-3-[3-[2-(trifluoromethyl)pyridin-3-yl]-1H-pyrazolo[3,4-b]pyrazin-6-yl]-3-azabicyclo[4.1.0]heptan-7-yl]methanamine;
- [3-[3-(2,3-dichlorophenyl)-1H-pyrazolo[3,4-b]pyrazin-6-yl]-7-(2-methylpyrazol-3-yl)-3-azabicyclo[4.1.0]heptan-7-yl]methanamine;
- [3-[3-(2,3-dichlorophenyl)-1H-pyrazolo[3,4-b]pyrazin-6-yl]-7-(1-methylpyrazol-3-yl)-3-azabicyclo[4.1.0]heptan-7-yl]methanamine;
- [3-[3-(2,3-dichlorophenyl)-1H-pyrazolo[3,4-b]pyrazin-6-yl]-7-(1,2-oxazol-3-yl)-3-azabicyclo[4.1.0]heptan-7-yl]methanamine;
- [3-[3-(2,3-dichlorophenyl)-1H-pyrazolo[3,4-b]pyrazin-6-yl]-3-azabicyclo[4.1.0]heptan-7-yl]methanamine;
- [6-[7-(aminomethyl)-7-(4-methyl-1,3-thiazol-2-yl)-3-azabicyclo[4.1.0]heptan-3-yl]-3-quinolin-5-yl-1H-pyrazolo[3,4-b]pyrazin-5-yl]methanol;
- [6-[7-(aminomethyl)-7-(4-methyl-1,3-thiazol-2-yl)-3-azabicyclo[4.1.0]heptan-3-yl]-3-(2,3-dichlorophenyl)-1H-pyrazolo[3,4-b]pyrazin-5-yl]methanol;
- 2-[7-(aminomethyl)-3-[3-(4-fluorosulfonyloxynaphthalen-1-yl)-1H-pyrazolo[3,4-b]pyrazin-6-yl]-3-azabicyclo[4.1.0]heptan-7-yl]-4-methyl-1,3-thiazole;
- 6-(6-(aminomethyl)-6-phenyl-3-azabicyclo[3.1.0]hexan-3-yl)-3-((2-(trifluoromethyl)pyridin-3-yl)thio)pyrazin-2-amine;
- 4-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)-3-chloropyridin-2-amine;
- ((1S,6R,7R)-7-(2,6-difluorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine;
- (3-(3-(8-chloroquinolin-5-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7S)-3-(3-(7-chloro-2-methylbenzo[d]thiazol-6-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(4-chloro-2,3-dimethyl-2H-indazol-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(4-chloro-2-methyl-2H-indazol-5-yl)-1H-pyrazolo[3,4-b]pyrazin-6-yl)-7-(5-fluoropyridin-2-yl)-3-azabicyclo[4.1.0]heptan-7-yl)methanamine;
- 4-(6-(7-(aminomethyl)-7-(4-methylthiazol-2-yl)-3-azabicyclo[4.1.0]heptan-3-yl)-1H-pyrazolo[3,4-b]pyrazin-3-yl)naphthalene-1-sulfonyl fluoride;
- ((1S,6R,7S)-3-(3-(4-chloro-3-methoxy-2-methyl-2H-indazol-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(3,4-dichloro-2-methyl-2H-indazol-5-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7S)-3-(3-(7-chloro-2-methylbenzo[d]thiazol-6-yl)-1H-pyrazolo[3,4-b]pyrazin-6-yl)-7-(5-fluoropyridin-2-yl)-3-azabicyclo[4.1.0]heptan-7-yl)methanamine;
- (3-(3-(7-chloro-2-methylbenzo[d]thiazol-6-yl)-5-methyl-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(4-chloro-2-methyl-2H-indazol-5-yl)-1H-pyrazolo[3,4-b]pyrazin-6-yl)-7-(isoxazol-3-yl)-3-azabicyclo[4.1.0]heptan-7-yl)methanamine;
- ((1S,6R,7S)-3-(3-(7-chloro-2-methylbenzo[d]thiazol-6-yl)-1H-pyrazolo[3,4-b]pyrazin-6-yl)-7-(isoxazol-3-yl)-3-azabicyclo[4.1.0]heptan-7-yl)methanamine;
- ((1S,6R,7S)-3-(3-(2-chloro-3-(oxazol-2-yl)phenyl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(5-chloro-3-methoxyquinoxalin-6-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(thieno[2,3-b]pyridin-3-yl)-1H-pyrazolo[3,4-b]pyrazin-6-yl)-3-azabicyclo[4.1.0]heptan-7-yl)methanamine;
- ((1S,6R,7S)-3-(3-(4-chloropyrazolo[1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7S)-3-(3-(7-chloro-2-methylbenzo[d]thiazol-6-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7S)-3-(3-(8-fluoroquinolin-4-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(8-methoxyquinolin-4-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(8-(trifluoromethoxy)quinolin-5-yl)-1H-pyrazolo[3,4-b]pyrazin-6-yl)-3-azabicyclo[4.1.0]heptan-7-yl)methanamine;
- ((1S,6R,7S)-3-(3-(6-methoxyquinolin-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(2,4-dimethyl-2H-indazol-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(8-(methoxymethyl)quinolin-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(pyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7S)-3-(3-(7-fluoroquinolin-5-yl)-1H-pyrazolo[3,4-b]pyrazin-6-yl)-7-(5-methylisoxazol-3 -yl)-3 -azabicyclo [4.1.0]heptan-7-yl)methanamine;
- ((1S,6R,7R)-7-(2-fluorophenyl)-3-(3-(pyrazolo[1,5-a]pyridin-4-yl)-1H-pyrazolo[3,4-b]pyrazin-6-yl)-3-azabicyclo[4.1.0]heptan-7-yl)methanamine;
- 4-(6-(7-(aminomethyl)-7-(4-methylthiazol-2-yl)-3-azabicyclo[4.1.0]heptan-3-yl)-1H-pyrazolo[3,4-b]pyrazin-3-yl)-2,3-dichlorophenyl sulfurofluoridate;
- 5-(6-((1S,6R,7S)-7-(aminomethyl)-7-(4-methylthiazol-2-yl)-3-azabicyclo[4.1.0]heptan-3-yl)-1H-pyrazolo[3,4-b]pyrazin-3-yl)quinolin-8-ylsulfurofluoridate;
- ((1S,6R,7S)-3-(3-(1,7-naphthyridin-5-yl)-1H-pyrazolo[3,4-b]pyrazin-6-yl)-7-(5-methylisoxazol-3 -yl)-3 -azabicyclo [4.1.0]heptan-7-yl)methanamine;
- ((1S,6R,7S)-3-(3-(2-methoxyquinolin-6-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(2-(trifluoromethyl)quinolin-6-yl)-1H-pyrazolo[3,4-b]pyrazin-6-yl)-3-azabicyclo[4.1.0]heptan-7-yl)methanamine;
- ((1S,6R,7S)-3-(3-(4-chloro-2-methylpyrazolo[1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7R)-7-(2-fluorophenyl)-3-(3-(8-fluoroquinolin-4-yl)-1H-pyrazolo[3,4-b]pyrazin-6-yl)-3-azabicyclo[4.1.0]heptan-7-yl)methanamine;
- ((1S,6R,7S)-3-(3-(7-methoxyquinolin-3-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(7-(trifluoromethyl)quinolin-3-yl)-1H-pyrazolo[3,4-b]pyrazin-6-yl)-3-azabicyclo[4.1.0]heptan-7-yl)methanamine;
- ((1S,6R,7R)-7-(2,6-difluorophenyl)-3-(3-(pyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7R)-3-(3-(4-chloro-2-methyl-2H-indazol-5-yl)-1H-pyrazolo[3,4-b]pyrazin-6-yl)-7-(2,6-difluorophenyl)-3-azabicyclo[4.1.0]heptan-7-yl)methanamine;
- ((1S,6R,7S)-3-(3-(8-(difluoromethyl)quinolin-5-yl)-1H-pyrazolo[3,4-b]pyrazin-6-yl)-7-(5-methylisoxazol-3-yl)-3-azabicyclo[4.1.0]heptan-7-yl)methanamine;
- 1-(5-(6-((1S,6R,7S)-7-(aminomethyl)-7-(5-methylisoxazol-3-yl)-3-azabicyclo[4.1.0]heptan-3-yl)-1H-pyrazolo[3,4-b]pyrazin-3-yl)quinolin-8-yl)ethan-1-ol;
- ((1S,6R,7R)-7-(2-fluorophenyl)-3-(3-(8-fluoroquinolin-5-yl)-1H-pyrazolo[3,4-b]pyrazin-6-yl)-3-azabicyclo[4.1.0]heptan-7-yl)methanamine;
- 4-((8-((1S,6R,7R)-7-(aminomethyl)-7-(2-fluorophenyl)-3-azabicyclo[4.1.0]heptan-3-yl)imidazo[1 ,5-a]pyrazin-5-yl)thio)-3-chloropyridin-2-amine;
- ((1S,6R,7R)-7-(2-fluorophenyl)-3-(3-(pyrazolo[1,5-a]pyridin-5-yl)-1H-pyrazolo[3,4-b]pyrazin-6-yl)-3-azabicyclo[4.1.0]heptan-7-yl)methanamine;
- ((1S,6R,7S)-3-(3-(6-chloropyrazolo[1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine;
- (7-(4-chlorothiazol-2-yl)-3-(3-(8-fluoroquinolin-5-yl)-1H-pyrazolo[3,4-b]pyrazin-6-yl)-3-azabicyclo[4.1.0]heptan-7-yl)methanamine;
- 2-(5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl)propan-2-ol;
- ((1S,6R,7S)-7-(3-fluoropyridin-2-yl)-3-(3-(pyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7R)-7-(2-fluorophenyl)-3-(3-(quinolin-6-yl)-1H-pyrazolo[3,4-b]pyrazin-6-yl)-3-azabicyclo[4.1.0]heptan-7-yl)methanamine;
- ((1S,6R,7S)-7-(3-fluoropyridin-2-yl)-3-(3-(8-(trifluoromethoxy)quinolin-5-yl)-1H-pyrazolo[3,4-b]pyrazin-6-yl)-3-azabicyclo[4.1.0]heptan-7-yl)methanamine;
- ((1S,6R,7R)-3-(3-(4-chloropyrazolo[1,5-a]pyridin-5-yl)-1H-pyrazolo[3,4-b]pyrazin-6-yl)-7-(2-fluorophenyl)-3-azabicyclo[4.1.0]heptan-7-yl)methanamine;
- 1-(5-(6-((1S,6R,7R)-7-(aminomethyl)-7-(2-fluorophenyl)-3-azabicyclo[4.1,0]heptan-3-yl)-1H-pyrazolo[3,4-b]pyrazin-3-yl)quinolin-8-yl)ethan-1-ol;
- ((1S,6R,7S)-3-(7-(8-fluoroquinolin-5-yl)-5H-pyrrolo[2,3-b]pyrazin-3-yl)-7-(4-methylthiazol-2-yl)-3-azabicyclo[4.1.0]heptan-7-yl)methanamine;
- ((1S,6R,7R)-7-(2-fluorophenyl)-3-(3-(8-isopropoxyquinolin-5-yl)-1H-pyrazolo [3,4-b]pyrazin-6-yl)-3-azabicyclo[4.1,0]heptan-7-yl)methanamine;
- ((1S,6R,7S)-3-(7-(8-fluoroquinolin-5-yl)-5H-pyrrolo[2,3-b]pyrazin-3-yl)-7-(5-methylisoxazol-3-yl)-3-azabicyclo[4.1.0]heptan-7-yl)methanamine;
- ((1S,6R,7R)-3-(3-(benzo[d]thiazol-6-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(7-(2,4-dimethyl-2H-indazol-5-yl)-5H-pyrrolo[2,3-b]pyrazin-3-yl)-7-(2-fluorophenyl)-3-azabicyclo[4.1.0]heptan-7-yl)methanamine;
- ((1S,6R,7R)-3-(3-(2,4-dimethyl-2H-indazol-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(3-(8-methoxyquinolin-5-yl)-1H-pyrazolo[3,4-b]pyrazin-6-yl)-3-azabicyclo[4.1.0]heptan-7-yl)methanamine;
- ((1S,6R,7R)-3-(3-(5-chloro-3-methoxyquinoxalin-6-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(8-cyclopropylquinolin-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(3-(imidazo[1,2-a]pyridin-5-yl)-1H-pyrazolo[3,4-b]pyrazin-6-yl)-3-azabicyclo[4.1.0]heptan-7-yl)methanamine;
- ((1S,6R,7R)-3-(3-(2-chloro-3-(pyrazin-2-yl)phenyl)-1H-pyrazolo[3,4-b]pyrazin-6-yl)-7-(2-fluorophenyl)-3-azabicyclo[4.1.0]heptan-7-yl)methanamine;
- 4-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)indolin-2-one;
- ((1S,6R,7R)-3-(3-(2,3-dihydrofuro[2,3-b]pyridin-4-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(1,7-naphthyridin-4-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(7-(8-fluoroquinolin-5-yl)-5H-pyrrolo[2,3-b]pyrazin-3-yl)-3-azabicyclo[4.1.0]heptan-7-yl)methanamine;
- ((1S,6R,7R)-3-(3-(1,6-naphthyridin-5-yl)-1H-pyrazolo[3,4-b]pyrazin-6-yl)-7-(2-fluorophenyl)-3-azabicyclo[4.1,0]heptan-7-yl)methanamine;
- ((1S,6R,7R)-7-(2-fluorophenyl)-3-(3-(6-methoxypyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7R)-7-(2,3-difluorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine;
- 4-(6-((1S,6R,7R)-7-(aminomethyl)-7-(2,3-difluorophenyl)-3-azabicyclo[4.1.0]heptan-3-yl)-1H-pyrazolo [3,4-b]pyrazin-3 -yl)-3 -chloropyridin-2-amine;
- 5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinoline-8-sulfonyl fluoride;
- ((1S,6R,7R)-3-(3-(3-chloro-2-fluoropyridin-4-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(3-chloro-2-(1H-pyrazol-1-yl)pyridin-4-yl)-1H-pyrazolo[3,4-b]pyrazin-6-yl)-7-(2-fluorophenyl)-3-azabicyclo[4.1.0]heptan-7-yl)methanamine;
- (5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl)methanol;
- ((1S,6R,7R)-3-(3-(8-(difluoromethoxy)quinolin-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(3-(7-methylpyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7R)-7-(2-chlorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine;
- ((1S,6R,7S)-3-(3-(2,4-dimethyl-2H-indazol-5-yl)-1H-pyrazolo[3,4-b]pyrazin-6-yl)-7-(3-fluoropyridin-2-yl)-3-azabicyclo[4.1.0]heptan-7-yl)methanamine;
- 4-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)pyrimidin-2-amine;
- 4-(6-((1S,6R,7R)-7-(aminomethyl)-7-(2-chlorophenyl)-3-azabicyclo[4.1.0]heptan-3-yl)-1H-pyrazolo[3,4-b]pyrazin-3-yl)-3 -chloropyridin-2-amine;
- 5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl sulfurofluoridate;
- ((1S,6R,7R)-3-(3-(8-ethoxyquinolin-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(3-bromo-2-chlorophenyl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(2-chloro-3-(oxazol-2-yl)phenyl)-1H-pyrazolo[3,4-b]pyrazin-6-yl)-7-(2-fluorophenyl)-3-azabicyclo[4.1,0]heptan-7-yl)methanamine;
- (3-(3-(8-chloroquinolin-5-yl)-1H-pyrazolo[3,4-b]pyrazin-6-yl)-7-(5-methylisoxazol-3-yl)-3-azabicyclo[4.1.0]heptan-7-yl)methanamine;
- (3-(3-(4-chloro-2-methyl-2H-indazol-5-yl)-1H-pyrazolo[3,4-b]pyrazin-6-yl)-7-phenyl-3-azabicyclo[4.1,0]heptan-7-yl)methanamine;
or a pharmaceutically acceptable salt, tautomer, solvate, or stereoisomer thereof.

Compounds of the invention may be used in the form of prodrugs. A prodrug may be a pharmacologically inactive derivative of a biologically active substance (the "parent drug" or "parent molecule", i.e. the compound of the invention) that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The invention also includes all suitable isotopic variations of a compound of the invention. Examples of isotopes that can be incorporated into compounds of the disclosure include isotopes such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the disclosure, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the invention can generally be prepared by conventional procedures such as by the illustrative methods and the preparations described in the Descriptions and in the Examples hereafter using appropriate isotopic variations of suitable reagents.

The present invention includes within its scope solvates of the compounds of Formula (I) or of the relative salts, for example, hydrates, alcoholates and the like.

In addition, the compounds disclosed herein may exist as tautomers and all tautomeric forms are intended to be encompassed by the scope of the invention, even though only one tautomeric structure is depicted. For example, a reference to 2-hydroxypyridine also includes pyridin-2-one as its tautomeric form and a reference to 4-hydroxypyridine also includes pyridin-4-one as its tautomeric form. Similarly, a reference to a hydroxypyrimidine derivative also includes the corresponding pyrimidinone tautomer, a reference to 2,4-dihydro-3H-1,2,4-triazol-3-one also includes the corresponding 1H-1,2,4-triazol-5-ol, and so on. A particular embodiment of Formula (I) encompasses compounds of structures indicated below: 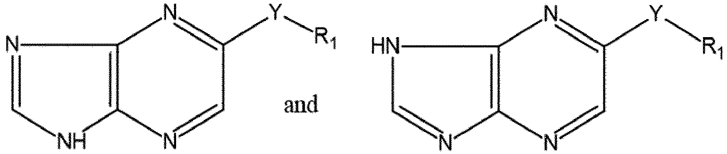 wherein R₁ and Y are as defined hereinabove.

The compounds disclosed herein may exist in different isomeric forms, all of which are encompassed by the present invention. In particular, any reference to the compound of the present invention is intended to include all its possible resonance forms.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention.

Pure stereoisomeric forms of the compounds and intermediates of this invention may be obtained by the application of art-known procedures and are intended to be encompassed by the scope of the invention. In particular, "pure stereoisomeric form" or "stereoisomerically pure" indicate a compound having stereoisomeric excess of at least 80%, preferably of at least 85%. For instance, enantiomers may be separated from each other by the selective crystallization of their diastereomeric salts or by chromatographic techniques using chiral stationary phases. Pure stereochemically isomeric forms may also be derived from the corresponding pure stereochemically isomeric forms of the appropriate starting materials, provided that the reaction occurs stereospecifically. The term "enantiomerically pure" shall be interpreted in a similar way, having regard to the enantiomeric ratio.

When any variable (e.g. R₁ and R₂, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable carbon atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents.

According to the invention, "-̅-̅-̅-̅-̅" represent a single or a double bond.

The expression "one or more substituents" refers to in particular to 1, 2, 3, 4 or more substituents, in particular to 1, 2, 3 or 4 substituents, more in particular 1, 2 or 3 substituents.

As used herein "X₁ is a single bond" indicates that, in the general Formula (I), Cy is directly linked via a single bond to R₁; if "L is a single bond", the carbon atoms substituted, respectively, with R₃ and R₄ are linked together forming a fused cyclopropyl ring, as in the structure below:

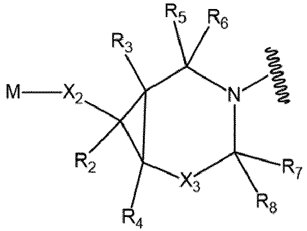

As used herein, if two residues taken together represent a single bond, this means that the two atoms to which they are attached are connected by a single bond or by an additional bond thus forming a multiple bond.

As used herein, "alkyl" is intended to include both branched and straight-chain saturated aliphatic hydrocarbon groups having the specified number of carbon atoms. For example, "C₁₋₆alkyl" is defined to include groups having 1, 2, 3, 4, 5 or 6 carbons in a linear or branched arrangement and specifically includes methyl, ethyl, *n*-propyl, *i*-propyl, *n*-butyl, *t*-butyl, *i*-butyl, pentyl, hexyl, and so on. Preferably, "C₁-₆alkyl" refers to "C₁-₄alkyl" or "C₁-₃alkyl". "C₁-₄alkyl" is defined to include groups having 1, 2, 3 or 4 carbons in a linear or branched arrangement. For example, "C₁-₄ alkyl" specifically includes methyl, ethyl, *n*-propyl, *i*-propyl, *n*-butyl, *t*-butyl, *i*-butyl, and so on. "C₁₋₃alkyl" is defined to include groups having 1, 2, or 3 carbons in a linear or branched arrangement. For example, "C₁-₃ alkyl" specifically includes methyl, ethyl, *n*-propyl, *i*-propyl, and so on. Preferred alkyl groups are methyl, ethyl, *i*-propyl, *t*-butyl or *i*-butyl.

As used herein, "alkoxy" represents an alkyl group of indicated number of carbon atoms attached through an oxygen bridge. "Alkoxy" therefore encompasses the definitions of alkyl above. C₁-₆ alkoxy group is preferably a linear or branched C₁-₄alkoxy group, more preferably a C₁-₃alkoxy group, still more preferably a C₁-₂ alkoxy group. Examples of suitable alkoxy groups include, but are not limited to methoxy, ethoxy, *n*-propoxy, *i*-propoxy, *n*-butoxy, *s*-butoxy or *t*-butoxy. Preferred alkoxy groups include methoxy, ethoxy and *t*-butoxy.

As used herein, the terms "haloC₁-₆alkyl", "haloC₁-₆alkoxy" and variants thereof such as "C₁-₆haloalkyl" mean a C₁₋₆alkyl or C₁-₆alkoxy group in which one or more (in particular, 1 to 3) hydrogen atoms have been replaced by halogen atoms, especially fluorine or chlorine atoms. HaloC₁-₆alkoxy group is preferably a linear or branched haloC₁-₄alkoxy group, more preferably a haloC₁-₃alkoxy group, still more preferably a haloC₁-₂alkoxy group, for example OCF₃, OCHF₂, OCH₂F, OCH₂CH₂F, OCH₂CHF₂ or OCH₂CF₃, and most especially OCF₃ or OCHF₂. HaloC₁-₆alkyl group is preferably a linear or branched haloC₁-₃alkyl group, more preferably a haloC₁-₂alkyl group for example, CF₃, CHF₂, CH₂F, CH₂CH₂F, CH₂CHF₂, CH₂CF₃ or CH(CH₃)CF₃, and most especially CF₃, CHF₂ or CH(CH₃)CF₃.

As used herein, the term "hydroxy-C₁₋₆alkyl" means a C₁-₆alkyl group in which one or more (in particular, 1 to 3) hydrogen atoms have been replaced by hydroxy groups. Preferably, the hydroxyC₁₋₆alkyl is a hydroxyC₁₋₄alkyl, meaning a C₁₋₄alkyl group in which one or more (in particular, 1 to 2) hydrogen atoms have been replaced by hydroxy groups. Illustrative examples include, but are not limited to CH₂OH, CH₂CH₂OH, CH(CH₃)OH, C(CH₃)₂OH and CHOHCH₂OH.

As used herein, the terms "heteroaryl-C₁₋₆alkoxy" mean a C₁₋₆alkoxy group in which one hydrogen atom is replaced by an heteroaryl group, wherein said aryl or heteroaryl can be further substituted by, for example, methyl, halogen, hydroxyl or amine.

As used herein, the term "aminoC₁₋₆alkyl" means a C₁₋₆alkyl group in which one or more (in particular, 1 to 3) hydrogen atoms have been replaced by small amino groups, such as NH₂, NHCH₃, N(CH₃)₂ and the like.

As used herein, the term "aryl" or "aromatic ring" means a monocyclic or polycyclic aromatic ring comprising carbon atoms and hydrogen atoms. If indicated, such aromatic ring may include one or more heteroatoms, then also referred to as "heteroaryl" or "heteroaromatic ring", preferably, 1 to 3 heteroatoms, independently selected from nitrogen, oxygen, and sulfur, preferably nitrogen. As is well known to those skilled in the art, heteroaryl rings have less aromatic character than their all-carbon counter parts. Thus, for the purposes of the present invention, a heteroaryl group need only have some degree of aromatic character. Preferably, the ring component of aryl or heteroaryl groups comprises 5 or 6 members (i.e. atoms). Still preferably, aryl or heteroaryl groups are polycylic aromatic rings. Illustrative examples of aryl groups are optionally substituted phenyls. Illustrative examples of heteroaryl groups according to the invention include optionally substituted thiophene, oxazole, thiazole, thiadiazole, imidazole, pyrazole, pyrimidine, pyrazine, pyridine and pyridine N-oxide. Thus, examples of monocyclic aryl optionally containing one or more heteroatoms, for example one or two heteroatoms, are a 5- or 6-membered aryl or heteroaryl group such as, but not limited to, phenyl, pyridyl, pyrimidinyl, pyrazinyl, pyridazinyl, pyrrolyl, thienyl, thiazolyl, thiadiazolyl, pyrazolyl, imidazolyl, triazolyl, tetrazolyl, furyl, isoxazolyl, oxadiazolyl and oxazolyl. Examples of polycyclic aromatic ring, optionally containing one or more heteroatoms, for example one or two heteroatoms, are a 8-10 membered aryl or heteroaryl group such as, but not limited to, benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzoxadiazolyl, benzoisoxazolyl, benzoisothiazolyl, indolyl, indolinyl, indolizinyl, indazolyl, isobenzofuranyl, isoindolyl, isoindolinyl, isoquinolyl, quinazolinyl, quinolyl, quinoxalinyl, quinolizinyl, naphtyl, naphthyridinyl and phthalazinyl. Other examples of polycyclic heteroaromatic rings according to the invention are 2H-pyrazolo[3,4-b]pyridine, indazole, 2H-pyrazolo[3,4-c]pyridine, 6H-pyrrolo[3,4-b]pyridine, 6H-pyrrolo[3,4-b]pyrazine, 6H-pyrrolo[3,4-d]pyrimidine, 2H-pyrazolo[3,4-d]pyrimidine, 1,5-naphthyridine, imidazo[1,2-a]pyridine. A preferred aryl according to the present invention is phenyl. A preferred heteroaryl according to the present invention is pyridyl.

The expressions "optionally substituted aryl", "optionaly substituted heteroaryl", "optionally substituted aryloxy", "optionally substituted heteroaryl-C₁₋₆alkyl", "optionally substituted heteroaryl-C₁₋₆alkoxy" generically refer to aryl, heteroaryl or aryloxy groups wherein the aromatic or heteroaromatic ring may be substituted with one or more substituents. Examples of said substituents include alkyl, alkoxy, amino, trifluoromethyl, aryl, heteroaryl, hydroxyl, carboxyalkyl and the like.

As used herein, heterocycle, heterocyclic compound or ring structure, heterocycloalkyl and variants thereof refer to a saturated monocyclic or polycyclic compound that has atoms of at least two different elements as members of its ring(s).

Aryl or heteroaryl rings can also have a partially unsaturated structure and can thus be derived from the partially hydrogenated analogues of the before-listed aryl or heteroaryl groups but also from an aryl or heteroaryl ring fused with a cycloalkyl or heterocycloalkyl ring. Said rings might also contain a group selected from SO, SO₂ and C=O. Examples of said partially unsaturated aryl or heteroaryl derivatives include 2,3-dihydro-1H-indene, 2,3-dihydro-1H-inden-1-one, 2,3-dihydroisoindol-1-one, indoline, 1,2,3,4-tetrahydroquinoline, 1,2,3,4-tetrahydroisoquinoline, isoindoline, 1-methyl-indol-2-one, dihydroquinazoline, dihydroquinoxaline, 2,3-dihydrobenzofuran, benzo[d][1,3]dioxole, 1,3-dihydroisobenzofuran, 3,4-dihydro-2H-benzo[b][1,4]oxazine, 2,3,4,5-tetrahydrobenzo[f][1,4]oxazepine, quinazolin-4(3H)-one, 4,5,6,7-tetrahydro-1H-indazole, 4,5,6,7-tetrahydropyrazolo[1,5-a]pyrazine, 2,3,4,5-tetrahydro-1H-benzo[d]azepine, 6',7'-dihydrospiro[azetidine-3,5'-pyrrolo[1,2-a]imidazole], 2,3-dihydrobenzo[b][1,4]dioxine,benzo[d]oxazol-2(3H)-one, 2H-benzo[b][1,4]oxazin-3(4H)-one, indolin-2-one, 1,2,3,4-tetrahydro-1,5-naphthyridine, 3',4'-dihydro-2'H-spiro[azetidine-3,1'-pyrrolo[1,2-a]pyrazine], 3,4-dihydroquinolin-2(1H)-one, 4-methyl-2H-benzo[b][1,4]oxazin-3(4H)-one, quinoxalin-2(1H)-one, 4H-pyrido[1,2-a]pyrimidin-4-one, (6aS)-6a,7,8,9-tetrahydro-6H-pyrido[3,2-b]pyrrolo[1,2-d][1,4]oxazine, 3,4-dihydro-2H-1,5-naphthyridin-1-yl, dihydrofuro[2,3-b]pyridinyl and the like.

As used herein, the term "aryloxy" represents an aryl group as defined above attached through an oxygen bridge. Aryloxy therefore encompasses the definitions of aryl and heteroaryl above. Illustrative examples include phenoxy, naphtyloxy, pyridinyloxy and so on.

It should be noted that different isomers of the various heterocycles may exist within the definitions as used throughout the specification. For example, pyrrolyl may be 1H-pyrrolyl or 2H-pyrrolyl.

It should also be noted that the radical positions on any molecular moiety used in the definitions may be anywhere on such moiety as long as it is chemically stable. For example, pyridyl includes 2-pyridyl, 3-pyridyl, 4-pyridyl.

As used herein, the term "halogen" refers to fluorine, chlorine, bromine and iodine, of which fluorine, chlorine and bromine are preferred.

The term "heteroatom" refers to an atom other than carbon or hydrogen in a ring structure or a saturated backbone as defined herein. Typical heteroatoms include N(H), O, S.

As used herein, "cycloalkoxy" represents a cycloalkyl group of the indicated number of carbons attached through an oxygen bridge. "Cycloalkoxy" therefore encompasses the definitions of cycloalkyl above and is preferably a C₁₋₆alkoxy as cyclopropyloxy, cyclobutyloxy, cyclopentyloxy, cyclohexyloxy.

As used herein, the expression "C₁₋₆alkyl-NH₂", "C₁₋₆alkyl-NH(CH₃)", "C₁₋₆alkyl-N(CH₃)₂", "C₁₋₆alkyl-NHCOCH₃" refers to a C₁-₆alkyl as defined above wherein any one hydrogen is substituted respectively by a group of formula NH₂, NH(CH₃), N(CH₃)₂ or NHCOCH₃. Preferably, said "C₁-₆alkyl-" is a "C₁₋₃alkyl-", thus encompasses an alkyl of 1, 2 or 3 carbon atoms.

Included in the instant invention is the free base of compounds of Formula (I), and any of Formula (II) - (XVI) as well as the pharmaceutically acceptable salts and stereoisomers thereof. Some of the specific compounds exemplified herein are the protonated salts of amine compounds. Compounds containing one or more N atoms may be protonated on any one, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of Formula (I), and any of Formula (II) - (XVI). The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base. In a preferred embodiment, the compounds of the invention have at least one acidic proton and the corresponding sodium or potassium salt can be formed, for example, by reaction with the appropriate base.

Thus, pharmaceutically acceptable salts of the compounds of this invention include the conventional non-toxic salts of the compounds of this invention as formed by reacting a basic instant compound with an inorganic or organic acid or an acid compound with an inorganic or organic base. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Convential non-toxic salts further include those derived from an inorganic base, such as potassium, sodium hydroxide, magnesium or calcium hydroxide, as well as salts prepared from organic bases, such as ethylene diamine, lysine, tromethamine, meglumine and the like. Preferably, a pharmaceutically acceptable salt of this invention contains one equivalent of a compound of Formula (I) or Formula (II), Formula (III), Formula (IV), Formula (V), Formula (VI), Formula (VII), Formula (VIII), Formula (IX), Formula (X), Formula (XI), Formula (XII), Formula (XIII), Formula (XIV), Formula (XV) or Formula (XVI) and 1, 2 or 3 equivalent of an inorganic or organic acid or base. More particularly, pharmaceutically acceptable salts of this invention are the tartrate, trifluoroacetate or the chloride salts.

When the compound of the present invention is acidic, suitable "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

Preferably, compounds of the present invention, including salts, tautomers, stereoisomers and solvates thereof, are SHP2 inhibitors, meaning that for example they can inhibit the activity or function of SHP2. Then, the present invention relates to compounds for use as inhibitors of at least one SHP2 function and to a method of inhibiting at least one SHP2 function comprising the step of contacting SHP2 with a compound as described herein.

"SHP2" means "Src Homology-2-phosphatase" and is also known as SH-PTP2, SH-PTP3, Syp, PTPID, PTP2C, SAP-2 or PTPN11.

The functions of SHP2 are varied as SHP2 is involved in multiple signaling processes, such as RAS-ERK, JAK-STAT, PI3K-AKT, NF-κB, and mTOR pathways. SHP2 regulates cancer cell survival and proliferation primarily by activating the RAS-ERK signaling pathway (T. Matozaki, Y. Murata, Y. Saito, H. Okazawa, H. Ohnishi, Cancer Sci, 100 (2009), pp. 1786-1793). In the RAS-ERK pathway, SHP2 acts as a positive regulator at upstream to promote RAS-RAF-ERK kinase cascade signaling transduction. Therefore, SHP2 inhibition leads to dephosphorylation of ERK and suppression of the pro-oncogenic function of RAS-RAF-ERK pathway, resulting in cell growth inhibition and apoptosis induction in cancer cells. Recently, Chen et al. (Y.N. Chen, M.J. LaMarche, H.M. Chan, P. Fekkes, J. Garcia-Fortanet, M.G. Acker et al., Nature, 535 (2016), pp. 148-152) found that cancer cell lines sensitive to SHP2 depletion were also sensitive to EGFR depletion, which validated reports that RTK-driven cancer cells depend on SHP2 for survival. Furthermore, recent studies have shown that SHP2 is required for the growth of mutant KRAS-driven cancers while wild-type KRAS-amplified gastroesophageal cancer can be controlled through combined SHP2 and MEK inhibition (S. Mainardi, A. Mulero-Sanchez, A. Prahallad, G. Germano, A. Bosma, P. Krimpenfort, et al. Nat Med, 24 (2018), pp. 961-9; D.A. Ruess, G.J. Heynen, K.J. Ciecielski, J. Ai, A. Berninger, D. Kabacaoglu, et al. Nat Med, 24 (2018), pp. 954-960; G.S. Wong, J. Zhou, J.B. Liu, Z. Wu, X. Xu, T. Li, et al. Nat Med, 24 (2018), pp. 968-977). As a downstream target of several receptors, SHP2 is also involved in signaling in T-cells (M. Tajan, A. de Rocca Serra, P. Valet, T. Edouard, A. Yart, Eur J Med Genet, 58 (2015), pp. 509-525; R.J. Salmond, D.R. Alexander, Trends Immunol, 27 (2006), pp. 154-160). It is a downstream molecule in the PD-1 signaling pathway which not only suppresses T-cell activation but also causes T-cell anergy. SHP2-deficiency in T-cells triggered an anti-tumor immune response against colitis-associated cancer in mice (W. Liu, W. Guo, L. Shen, Z. Chen, Q. Luo, X. Luo, et al. Oncotarget, 8 (2017), pp. 7586-7597). Therefore, targeting SHP2 may restore or even enhance T-cell functions.

SHP2 inhibition may be assessed or measured by: the cell phenotype (as for example the phenotypes of proliferation and resistance to EGFR and c-MET co-inhibition, the mesenchymal phenotype in BTBC cells), cell proliferation, activity of SHP2, change in biochemical output produced by active SHP2, expression of SHP2, or binding of SHP2 with a natural binding partner may be monitored as a measure of SHP2 inhibition. In particular, inhibition of SHP2 activity or function can be measured by the IC₅₀ (concentration of inhibitor which reduces the activity of SHP2 to half-maximal level), as described in the assays hereinbelow or in the biochemical assays for SHP2 inhibition reported for example by Chen et al., Nature (535) 2016 or by Bagdanoff et al., J. Med. Chem. 2019, 62, 1781-1792. Preferably, compounds of the invention exhibit an IC₅₀ towards SHP2 lower than or equal to 10 µM. Preferred compounds exhibit an enzymatic IC₅₀ towards SHP2, as defined hereinbelow, lower than or equal to 3 µM (preferably lower than or equal to 0.5 µM or between 0.5 µM and 3 µM) and/or inhibition of SHP2 in cell based assays, as defined hereinbelow, with IC₅₀ lower than or equal to 5 µM (preferably lower than or equal to 1 µM or between 1 µM and 5 µM). Then, the compounds of the present invention, including salts, tautomers, stereoisomers and solvates thereof, may be for use in a method of inhibiting SHP2 activity. In other words, they may be for use in the prevention and/or treatment of any condition that would be ameliorated by SHP2 inhibition.

In a preferred embodiment, the compound or a pharmaceutically acceptable salt, tautomer, solvate, or stereoisomer thereof as defined above is for use in inhibiting SHP2 activity. Inhibition of SHP2 activity may be measured with respect to a proper control, such as a subject affected by a disease or disorder mediated by the activity of SHP2 or a subject throughout the course of a therapy for a disease or disorder mediated by the activity of SHP2. Preferably, compounds of the invention inhibit SHP2 activity by at least 10%, 20%, 30%, 40%, 50%, 60%, 70%, 80%, 90% in respect to a proper control. More preferably, compounds of the invention inhibit SHP2 activity by approximately 10%, 20%, 30%, 40%, 50%, 60%, 70%, 80%, 90% in respect to a proper control.

Yet more preferably, compounds of the invention inhibit SHP2 activity by more than 90%, for instance by approximately 92%, 94%, 95%, 98%, 99% or 100% in respect to a proper control.

Thereofore, the compounds of the invention can be used for the treatment of diseases and for carrying out biological assays, cellular assays, biochemical assays or the like.

It is an object of the invention a compound or a pharmaceutically acceptable salt, tautomer, solvate, or stereoisomer thereof as defined above for medical use.

Preferably, the compound or the pharmaceutically acceptable salt, tautomer, solvate, or stereoisomer thereof as defined above is for use in inhibiting SHP2 activity. Inhibition of SHP2 activity further leads to dephosphorylation of ERK and suppression of the pro-oncogenic function of RAS-RAF-ERK pathway. Then, inhibition of SHP2 activity may be measured by ERK dephosphorylation, wherein ERK phosphorylation may be evaluated by any method known in the art, for instance as described in the Examples below. Dephosphorylation of ERK may be measured with reference to any proper control.

More preferably, the compound or the pharmaceutically acceptable salt, tautomer, solvate, or stereoisomer thereof as defined above is for use in the treatment and/or prevention of a disease or disorder mediated by the activity of SHP2. Preferably, the disease or disorder mediated by the activity of SHP2 is selected from the group consisting of: cancer, cardiovascular disease, immunological disorder, fibrosis, an ocular disorder, systemic lupus erythematosus, diabetes, neutropenia, and combinations thereof. Yet preferably, the disease or disorder mediated by the activity of SHP2 is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof. Preferably any one of said cancers is a primary cancer or a cancer metastasis.

A disease or disorder mediated by the activity of SHP2 indicates a condition in a subject in which modulation, in particular inhibition, of SHP2 activity can prevent, inhibit, ameliorate, slow down or eradicate the condition and/or the symptomology thereof. Treatment of said disease or disorder might comprise administering to the subject in need thereof a therapeutically effective amount of a compound of Formula (I) according to the invention.

In diseases or disorders mediated by the activity of SHP2, mutations are often observed at the N-SH2/PTP interface (e.g. E76D/E76K), resulting in constitutively active protein and abnormal proliferation. Cancers harboring "PTPN11 mutations" include but are not limited to: N58Y; D61Y, V; E69K; A72V, T, D; E76G, Q, K (ALL); G60A; D61Y; E69V; F71K; A72V; T73I; E76G, K; R289G; G503V (AML); G60R, D61Y, V, N; Y62D; E69K; A72T, V; T73I; E76K, V, G, A, Q; E139D; G503A, R; Q506P (JMML); G60V; D61V; E69K; F71L; A72V; E76A (MDS); Y63C (CMML); Y62C; E69K; T507K (neuroblastoma); V46L; N58S; E76V (Lung cancer); R138Q (melanoma); E76G (colon cancer). The compounds of the invention can exhibit affinity at low concentrations for wild type SHP2 and can also be active against mutant forms of the protein.

Another aspect of the present invention relates to a compound of the invention, including any pharmaceutically acceptable salt, tautomer, solvate or stereoisomer thereof, as defined hereinabove for use in a method of preventing/treating an SHP2-mediated disorder and/or disorders mediated by the pro-oncogenic function of RAS-RAF-ERK pathway.

Another aspect of the present invention relates to a method of preventing/treating an SHP2-mediated disorder comprising the step of administering to a patient in need thereof a therapeutically effective amount of a compound of the invention, including any pharmaceutically acceptable salt, tautomer, solvate or stereoisomer thereof, as defined hereinabove. In another aspect, the present invention relates to a method of preventing/treating an SHP2-mediated disorder comprising the step of administering to a patient in need thereof a therapeutically effective amount of a chemotherapeutic agent, as further defined below, in combination with a therapeutically effective amount of a compound of the invention.

Another aspect of the present invention relates to the use of compounds of the invention, including any pharmaceutically acceptable salts tautomer, solvate or stereoisomer thereof, as defined hereinabove in preventing/treating an SHP2-mediated disorder.

In yet another aspect of the present invention, there are provided the compound, salt, solvate, stereoisomer or tautomer as defined above for use in the treatment and/or prevention of a disease or disorder selected from the group consisting of: cancer, cardiovascular disease, immunological disorder, fibrosis, an ocular disorder, systemic lupus erythematosus, diabetes, neutropenia and combinations thereof. Preferably, the disease or disorder is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof. Preferably, the cancer is a primary cancer or a cancer metastasis.

In certain embodiments the present invention relates to the aforementioned use/method, wherein said disorder is selected from Noonan Syndrome (NS) and Leopard Syndrome (LS).

In further embodiments, the present invention relates to the aforementioned use/method, wherein said SHP2-mediated disorders are those due to dysregulated cellular proliferation, including cancer. The cancer may be hormone-dependent or hormone-resistant, such as in the case of breast cancers. Preferably, the cancer is RTK-driven or KRAS-driven, such as KRAS amplified gastroesophageal cancer. In certain embodiments, the cancer is a solid tumor. In other embodiments, the cancer is a lymphoma or leukemia or a glioma. In certain embodiments, the cancer is a drug resistant phenotype of a cancer disclosed herein or known in the art. The cancer may be primary or metastatic. Tumor invasion, tumor growth, tumor metastasis, and angiogenesis may also be treated using the compositions and methods disclosed herein. Precancerous neoplasias may also be treated using the compositions and methods disclosed herein.

Compounds of the invention can be used for the treatment of cancers selected from, but not limited to: Juvenile Myelomonocytic Leukemias (JMML); Acute Myeloid Leukemia (AML); Myelodysplastic Syndrome (MDS); B cell acute lymphoblastic leukemia (B-ALL); neuroblastoma; esophageal; breast cancer; lung cancer; colon cancer; gastric cancer, head and neck cancer; ovarian cancer; prostate cancer; cancers of the oral cavity and pharynx (lip, tongue, mouth, larynx, pharynx), stomach, small intestine, large intestine, colon, rectum, liver and biliary passages; pancreas, bone, connective tissue, skin, cervix, uterus, corpus endometrium, testis, bladder, kidney and other urinary tissues, including renal cell carcinoma (RCC); gastroesophageal cancer (preferably KRAS-amplified gastroesophageal cancer), cancers of the eye, brain, spinal cord, and other components of the central and peripheral nervous systems, as well as associated structures such as the meninges; thyroid and other endocrine glands, Hodgkin's disease, non-Hodgkin's lymphomas, multiple myeloma and hematopoietic malignancies including leukemias Chronic Lymphocytic Leukemia (CLL), Acute Lymphocytic Leukemia (ALL), Chronic Myelogenous Leukemia (CML), Acute Myelogenous Leukemia (AML), Chronic Myelomonocytic Leukemia (CMML), and lymphomas including lymphocytic, granulocytic and monocytic. Additional types of cancers which may be treated using the compounds and methods of the invention include, but are not limited to, adenocarcinoma, angiosarcoma, astrocytoma, acoustic neuroma, anaplastic astrocytoma, basal cell carcinoma, blastoglioma, chondrosarcoma, choriocarcinoma, chordoma, craniopharyngioma, cutaneous melanoma, cystadenocarcinoma, endotheliosarcoma, embryonal carcinoma, ependymoma, Ewing's tumor, epithelial carcinoma, fibrosarcoma, gastric cancer, genitourinary tract cancers, glioblastoma multiforme, hemangioblastoma, hepatocellular carcinoma, hepatoma, Kaposi's sarcoma, large cell carcinoma, leiomyosarcoma, leukemias, liposarcoma, lymphatic system cancer, lymphomas, lymphangiosarcoma, lymphangioendotheliosarcoma, medullary thyroid carcinoma, medulloblastoma, meningioma mesothelioma, myelomas, myxosarcoma neuroblastoma, neurofibrosarcoma, oligodendroglioma, osteogenic sarcoma, epithelial ovarian cancer, papillary carcinoma, papillary adenocarcinomas, paraganglioma, parathyroid tumours, pheochromocytoma, pinealoma, plasmacytomas, retinoblastoma, rhabdomyosarcoma, sebaceous gland carcinoma, seminoma, skin cancers, melanoma, small cell lung carcinoma, non-small cell lung carcinoma, squamous cell carcinoma, sweat gland carcinoma, synovioma, thyroid cancer, uveal melanoma, Wilm's tumor, anaplastic large-cell lymphoma, colitis associated cancer.

The compounds of the present invention may be useful in the treatment of any other disease or condition related to the aberrant activity of SHP2. Thus, as a further aspect, the invention relates to a method of treatment of a disorder selected from: NS; LS; JMML; AML; MDS; B-ALL; neuroblastoma; esophageal; breast cancer; lung cancer; colon cancer; gastric cancer; head and neck cancer.

A compound of the present invention, including any pharmaceutically acceptable salt, tautomer, solvate, or stereoisomer thereof, may be usefully combined with any another known therapy that is useful for the prevention/treatment of a disease or disorder mediated by the activity of SHP2. Such therapy may include radiotherapy. Such therapy may also comprise the administration of another pharmacologically active compound, or of two or more other pharmacologically active compounds, particularly compound(s) active in the prevention/treatment of cancer, also referred to as "anti-cancer drug(s)" or "chemotherapy agents". For example, a compound of the invention, including any pharmaceutically acceptable salt, tautomer, solvate, or stereoisomer thereof as defined above, may be administered simultaneously, sequentially or separately in combination with any one or more other pharmacologically active compound. For simultaneous administration, the compound of the present invention and the other one or more pharmacologically active compound may be formulated in the same composition.

Classes of anti-cancer drugs that may be combined with the compounds of the invention include, but are not limited to: alkylating agents, anti-metabolites, antimitotics, checkpoint inhibitors, plant alkaloids and terpenoids, topoisomerase inhibitors, cytotoxic antibiotics, aromatase inhibitors, angiogenesis inhibitors, anti-steroids and anti-androgens, mTOR inhibitors, tyrosine kinase inhibitors, and others. Chemotherapy agents include, for example, mitotic inhibitors such as a taxane, a vinca alkaloid, paclitaxel, docetaxel, vincristine, vinblastine, vinorelbine or vinflunine, and other anticancer agents, e.g. cisplatin, 5-fluorouracil or 5-fluoro-2-4(1H,3H)-pyrimidinedione (5FU), flutamide or gemcitabine. Such combinations may offer significant advantages, including synergistic activity, in therapy.

Alkylating agents are compounds that work by adding an alkyl group to the guanine base of the DNA molecule, preventing the strands of the double helix from linking as they should thus causing breakage of the DNA strands and affecting the ability of the cancer cells to multiply. Antimetabolites are drugs that interfere with one or more enzymes or their reactions that are necessary for DNA synthesis. An antimitotic agent is a type of drug that blocks cell growth by stopping mitosis. Checkpoint inhibitors are a type of immunotherapy which block proteins that stop the immune system from attacking the cancer cells. Topoisomerase inhibitors are chemical compounds that block the action of topoisomerase (topoisomerase I and II), which is a type of enzyme that controls the changes in DNA structure by catalyzing the breaking and rejoining of the phosphodiester backbone of DNA strands during the normal cell cycle. Aromatase inhibitors are a class of drugs that work by inhibiting the action of the enzyme aromatase, which converts androgens into estrogens by a process called aromatization. Angiogenesis inhibitors are substances that inhibit the growth of new blod vessels and are used to treat cancers and other diseases that involve a proliferation of blood vessels. mTOR inhibitors are a class of drugs that inhibit the mammalian target of rapamycin (mTOR), which is a serine/threonine-specific protein kinase that belongs to the family of phosphatidylinositol-3 kinase (PI3K) related kinases (PIKKs). mTOR regulates cellular metabolism, growth, and proliferation by forming and signaling through two protein complexes, mTORC1 and mTORC2. The most established mTOR inhibitors are so-called rapalogs (rapamycin and its analogs), which have shown tumor responses in clinical trials against various tumor types.

It is thus a preferred object of the invention a compound as defined above or the pharmaceutically acceptable salt, tautomer, solvate, or stereoisomer thereof, for use in combination with at least one further therapeutic agent.

In any case, the multiple therapeutic agents (at least one of which is a compound disclosed herein) may be administered in any order or even simultaneously.

Preferably, said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1H,3H)-pyrimidinedione and combinations thereof.

The invention further provides pharmaceutical preparations comprising the compounds of the invention. In particular, it is a further object of the invention a pharmaceutical composition comprising the compound or the pharmaceutically acceptable salt, tautomer, solvate, or stereoisomer thereof as defined above, alone or in combination with at least one further therapeutic agent, and at least one pharmaceutically acceptable excipient. Preferably, said at least one further therapeutic agent in the pharmaceutical composition is selected among those indicated above.

In a preferred embodiment, the pharmaceutical combination or the composition of the invention is for use in the treatment and/or prevention of a disease or disorder as herein defined, in particular a disease or disorder mediated by the activity of SHP2 and/or a disease or disorder selected from the group consisting of: cancer, cardiovascular disease, immunological disorder, fibrosis, an ocular disorder, systemic lupus erythematosus, diabetes, neutropenia and combinations thereof.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions of the invention may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water-soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate.

The emulsions may also contain sweetening, flavoring agents, preservatives and antioxidants. Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulsion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS^{™} model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound(s) of the invention are employed. For purposes of this application, topical application shall include mouth washes and gargles.

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

The compounds of the invention may be presented in a liposome or other micro particulate or other nanoparticle designed to target the compound. Acceptable liposomes can be neutral, negatively, or positively charged, the charge being a function of the charge of the liposome components and pH of the liposome solution. Liposomes can be normally prepared using a mixture of Phospholipids and cholesterol. Suitable phospholipids include phosphatidylcholine, phosphatidylethanolamine, phosphatidic acid, phosphotidylglycerol, phosphatidylinositol. Polyethylene glycol can be added to improve the blood circulation time of liposomes. Acceptable nanoparticles include albumin nanoparticles and gold nanoparticles.

When a compound according to this invention is administered into a human subject, the daily dosage will normally be determined by the prescribing physician with the dosage generally varying according to the age, weight, sex and response of the individual patient, as well as the severity of the patient's symptoms. Generally, dosage levels on the order of from about 0.01 mg/kg to about 150 mg/kg of body weight are useful in the treatment of the above indicated conditions.

As used herein, the term "composition" is intended to encompass a product comprising the specified ingredients in the specified amounts, as well as any product which results, directly or indirectly, from combination of the specified ingredients in the specified amounts.

Another object of the present invention relates to an *in vitro* method of inhibiting SHP2 with the compound of the present invention. This may be useful, for instance, to evaluate whether any given compound is an inhibitor/activator of SHP2 and therefore acts also on the ERK pathway.

A further object of the present invention concerns a kit comprising at least one pharmaceutically acceptable vial or container of other type, containing one or more doses of a compound of the invention, including any pharmaceutically acceptable salt, tautomer, solvate or stereoisomer thereof, or of a pharmaceutical composition of the invention and optionally a) instructions for use thereof in mammals and/or b) an infusion bag or container containing a pharmaceutically acceptable diluent.

In certain embodiments, the compound or the composition of the invention is administered parenterally, intramuscularly, intravenously, subcutaneously, orally, pulmonary, intrathecally, topically, intranasally, or systemically.

In certain embodiments, the patient who is administered the compound or the composition of the invention is a mammal, preferably a primate, more preferably a human.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

As used herein, the term "prevention" means no disorder or disease development if none had occurred, or no further disorder or disease development if there had already been development of the disorder or disease. Also considered is the ability of one to prevent some or all of the symptoms associated with the disorder or disease. As used herein, any reference to "treatment"/"treating" includes the amelioration of at least one symptom of the disease/disorder to be treated. Such amelioration is to be evaluated in comparison to the same symptom prior to administration of the compound or composition of the invention.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

The present invention will be described by means of the following non-limiting examples and biological data.

### MATERIALS AND METHODS

### Chemistry

As used herein, the following abbreviations have the following meanings. If an abbreviation is not defined, it has its generally accepted meaning.

### Abbreviations

AcOH: Acetic acid; (Boc)₂O: Di-t*ert*-butyl dicarbonate; 15-crown-5: 1,4,7,10,13-Pentaoxacyclopentadecane; t-BuOH; *t*-butanol; tBuOK: Potassium tert-butoxide; CDI: 1,1'-Carbonyldiimidazole; CDCl₃: Deuterated chloroform; CHCl3: Chloroform; CoCl2: Cobalt(II) chloride; DBU: 1,8-Diazabicyclo[5.4.0]undec-7-ene; DCM: Dichloromethane; DIAD: Diisopropyl azodicarboxylate; DIBAL-H: Diisobutylaluminium hydride; DIPEA: *N*,*N-*Diisopropylethylamine; DMA: *N*,*N*-Dimethylacetamide; DMAP:4-(Dimethylamino)pyridine; DME: Dimethoxyethane; DMSO: Dimethylsulfoxide; ES⁺: Electrospray Positive Ionisation; Et₂O: Diethylether; EtOAc: Ethyl acetate; EtOH: Ethanol; HBF₄: Tetrafluoroboric acid; HCl: hydrochloric acid; h: Hour; H₂: Hydrogen; H₂O: Water; H₂SO₄: Sulfuric acid HPLC:High Performance Liquid Chromatography; KOAc: Potassium acetate; K₃PO₄: Tripotassium phosphate; K₂CO₃: potassium carbonate; LCMS: Liquid Chromatography Mass Spectrometry; LiOH: Lithium hydroxide; *m*CPBA: *meta*-Chloroperoxybenzoic acid; MeCN: Acetonitrile; MeI: Iodomethane; MeOH: Methanol; min: Minutes; MW: Microwave; N₂: nitrogen; NaNO₂: Sodium nitrite; NaOH: sodium hydroxide; NaBH4: sodium borohydride; Na₂CO₃: Sodium carbonate; NaHCO₃: Sodium bicarbonate; Na₂SO₄: sodium sulfate; PPh₃: Triphenylphosphine; Pd₂(dba)₃: Tris(dibenzylideneacetone)dipalladium(0); POCl3: Phosphoryl chloride; Pd(PPh₃)₄: Tetrakis(triphenylphosphine)palladium(0); RP: Reverse Phase; RT: Retention time; rt: Room temperature; SEM: trimethylsilylethoxymethyl; TBAF: Tetrabutylammonium fluoride; TBDMS-Cl: tert-Butyldimethylsilyl chloride; TCDI: 1-1'-Thiocarbonyldiimidazole; TEA: Triethylamine; [(t-Bu)₃PH]BF₄: Tri-*tert*-butylphosphonium tetrafluoroborate; TFA: Trifluoroacetic acid; THF: Tetrahydrofuran; THP: Tetrahydropyranyl; Sat.: Saturated; Sol.: Solution; UPLC: Ultra High Performance Liquid Chromatography; Xantphos: 4,5-Bis(diphenylphosphino)-9,9-dimethylxanthene.

### General

Solvents and reagents were obtained from commercial suppliers and were used without further purification. Flash chromatography purifications were performed on prepacked cartridges on a Biotage system. Purity of final compounds was determined using MS and UPLC. UPLC-MS analyses were performed on a Waters Acquity UPLC^{™}, equipped with a diode array and a ZQ mass spectrometer, using an X-Terra C18 column (5 µm, 4.6 x 50 mm) or a BEH C18 column (1.7 mm, 2.1 x 50 mm). Mobile phase comprised a linear gradient of binary mixtures of H₂O containing 0.1% formic acid (A), and MeCN containing 0.1% formic acid (B). The linear gradient used is: (A): 90% (0.1 min), 90%-0% (2.6 min), 0% (0.3 min), 0%-90% (0.1 min) with a 0.5 mL/min flow. Purity of final compounds was ≥95%. All ¹H spectra were recorded on Bruker AV400 spectrometer at 400 MHz except where indicated. Chemical shift (δ) are reported in parts per million relative to TMS using CDCl₃ as a solvent or relative to the residual solvent signal using DMSO-d₆. Coupling costants (*J*) are reported in Hertz (Hz). Multiplicities are reported as singlet (s), broad (br), doublet (d), doublet of doublet (dd), doublet of doublet of doublet (ddd), triplet (t), doublet of triplet (dt), doublet of doublet of triplet (ddt), triplet of triplet (tt), quartet (q), doublet of quartets (dq) or multiplet (m). Unless indicated, spectra were acquired at 300 K. Temperatures are expressed in degrees Celsius (°C) and are uncorrected.

### Processes for Making the Compounds of the Invention

The present invention also includes processes for the preparation of compounds of the invention. The following schemes are examples of synthetic routes that may be adopted to prepare compounds of the invention.
In the reaction schemes described below, it can be useful to protect reactive functional groups, for example amino, imino, hydroxyl, thio or carboxy groups, to avoid their unwanted participations to reactions. Conventional protecting groups can be used in accordance with standard practice, for example see T. Greene and P.G. M. Wuts in "Protective Groups in Organic Chemistry", John Wiley and Sons, 1991.
Those skilled in the art will readily appreciate that certain compounds of the invention can be converted into other compounds of the invention according to standard chemical methods (e.g. by salification/desalification).

The following examples were synthesized using according to the Schemes and the procedures described infra or modifications thereof using the appropriate starting materials (Table 1).

**Table 1 - Compounds prepared according to the Examples and experimental data (MS).**

| **Example #** | **Chemical Structure** | **IUPAC Name** | **MS [M+H]+** |
|---|---|---|---|
| 1 | 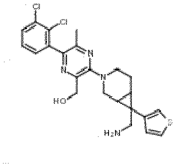 | [3-[7-(aminomethyl)-7-thiophen-3-yl-3-azabicyclo[4.1.0]heptan-3-yl]-6-(2,3-dichlorophenyl)-5-methylpyrazin-2-yl]methanol | 475.13 |
| 2 | 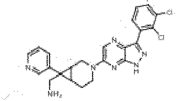 | [3-[3-(2,3-dichlorophenyl)-1H-pyrazolo[3,4-b]pyrazin-6-yl]-7-pyridin-3-yl-3-azabicyclo[4.1.0]heptan-7-yl]methanamine | 466.13 |
| 3 | 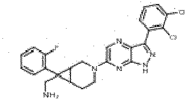 | [3-[3-(2,3-dichlorophenyl)-1H-pyrazolo[3,4-b]pyrazin-6-yl]-7-(2-fluorophenyl)-3-azabicyclo[4.1.0]heptan-7-yl]methanamine | 483.12 |
| 4 | 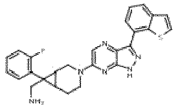 | [3-[3-(1-benzothiophen-7-yl)-1H-pyrazolo[3,4-b]pyrazin-6-yl]-7-(2-fluorophenyl)-3-azabicyclo[4.1.0]heptan-7-yl]methanamine | 471.17 |
| 5 | 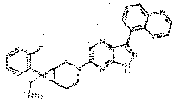 | [7-(2-fluorophenyl)-3-(3-qumolm-5-yl-1H-pyrazolo[3,4-b]pyrazin-6-yl)-3-azabicyclo[4.1.0]heptan-7-yl]methanamine | 466.21 |
| 6 | 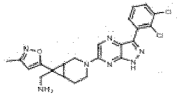 | [3-[3-(2,3-dichlorophenyl)-1H-pyrazolo[3,4-b]pyrazin-6-yl]-7-(3-methyl-1,2-oxazol-5-yl)-3-azabicyclo[4.1.0]heptan-7-yl]methanamine | 470.12 |
| 7 | 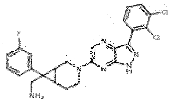 | [3-[3-(2,3-dichlorophenyl)-1 H -pyrazolo[3,4-b]pyrazin-6-yl]-7-(3-fluorophenyl)-3-azabicyclo[4.1.0]heptan-7-yl]methanamine | 483.18 |
| 8 | 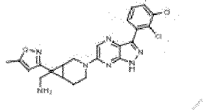 | [3-[3-(2,3-dichlorophenyl)-1H -pyrazolo[3,4-b]pyrazin-6-yl]-7-(5-methyl-1,2-oxazol-3-yl)-3-azabicyclo[4.1.0]heptan-7-yl]methanamine | 470.12 |
| 9 | 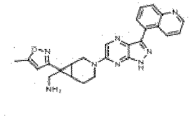 | [7-(5-methyl-1,2-oxazol-3-yl)-3-(3-quinolin-5-yl-1H-pyrazolo[3,4-b]pyrazin-6-yl)-3-azabicyclo[4.1.0]heptan-7-yl]methanamine | 453.21 |
| 10 | 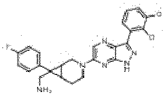 | [3-[3-(2,3-dichlorophenyl)-1H-pyrazolo[3,4-b]pyrazin-6-yl]-7-(4-fluorophenyl)-3-azabicyclo[4.1.0]heptan-7-yl]methanamine | 483.12 |
| 11 | 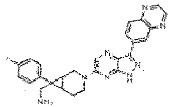 | [7-(4-fluorophenyl)-3-(3-quinoxalin-6-yl-1H-pyrazolo[3,4-b]pyrazin-6-yl)-3-azabicyclo[4.1.0]heptan-7-yl]methanamine | 467.21 |
| 12 | 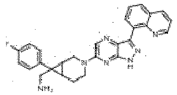 | [7-(4-fluorophenyl)-3-(3-quinolin-8-yl-1H-pyrazolo[3,4-b]pyrazin-6-yl)-3-azabicyclo[4.1.0]heptan-7-yl]methanamine | 466.21 |
| 13 | 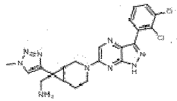 | [3-[3-(2,3-dichlorophenyl)-1H-pyrazolo[3,4-b]pyrazin-6-yl]-7-(1-methyltriazol-4-yl)-3-azabicyclo[4.1.0]heptan-7-yl]methanamine | 470.13 |
| 14 | 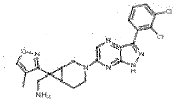 | [3-[3-(2,3-dichlorophenyl)-1H-pyrazolo[3,4-b]pyrazin-6-yl]-7-(4-methyl-l,2-oxazol-3-yl)-3-azabicyclo[4.1.0]heptan-7-yl]methanamine | 470.12 |
| 15 | 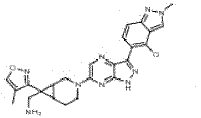 | [3-[3-(4-chloro-2-methylindazol-5-yl)-1H-pyrazolo[3,4-b]pyrazin-6-yl]-7-(4-methyl-1,2-oxazol-3-yl)-3-azabicyclo[4.1.0]heptan-7-yl]methanamine | 490.18 |
| 16 | 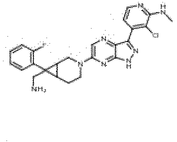 | 4-[6-[7-(aminomethyl)-7-(2-fluorophenyl)-3-azabicyclo[4.1,0]heptan-3-yl]-1H-pyrazolo[3,4-b]pyrazin-3-yl]-3-chloro-~ {N}-methylpyridin-2-amine | 479.18 |
| 17 | 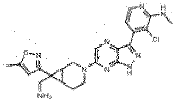 | 4-[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]-3-chloro-~{N}-methylpyridin-2-amine | 466.18 |
| 18 | 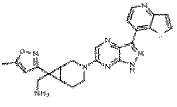 | [7-(5-methyl-1,2-oxazol-3-yl)-3-(3-thieno[3,2-b]pyridm-7-yl-1H-pyrazolo[3,4-b]pyrazin-6-yl)-3-azabicyclo[4.1.0]heptan-7-yl]methanamine | 459.17 |
| 19 | 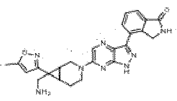 | 4-[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]-2,3-dihydroisoindol-1-one | 457.20 |
| 20 | 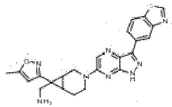 | [3-[3-(1,3-benzothiazol-5-yl)-1H-pyrazol0[3,4-b]pyrazin-6-yl]-7-(5-methyl-1,2-oxazol-3-yl)-3-azabicyclo[4.1.0]heptan-7-yl]methanamine | 459.17 |
| 21 | 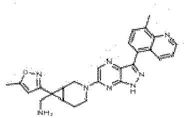 | [7-(5-methyl-1,2-oxazol-3-yl)-3-[3-(8-methylquinolin-5-yl)-1H-pyrazolo[3,4-b]pyrazin-6-yl]-3-azabicyclo[4.1.0]heptan-7-yl]methanamine | 467.23 |
| 22 | 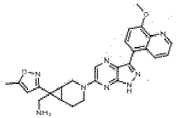 | [3-[3-(8-methoxyquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine | 483.22 |
| 23 | 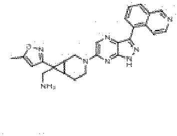 | [3-(3-isoquinolin-5-yl-1H-pyrazolo[3,4-b]pyrazin-6-yl)-7-(5-methyl-1,2-oxazol-3-yl)-3-azabicyclo[4.1.0]heptan-7-yl]methanamine | 453.21 |
| 24 | 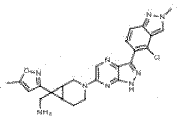 | [3-[3-(4-chloro-2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine | 490.18 |
| 25 | 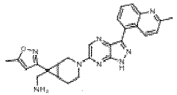 | [7-(5-methyl-1,2-oxazol-3-yl)-3-[3-(2-methylquinolin-5-yl)-1H-pyrazolo[3,4-b]pyrazin-6-yl]-3-azabicyclo[4.1,0]heptan-7-yl]methanamine | 467.23 |
| 26 | 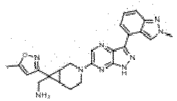 | [3-[3-(2-methylindazol-4-yl)-1H-pyrazolo[3,4-b]pyrazin-6-yl]-7-(5-methyl-1,2-oxazol-3-yl)-3-azabicyclo[4.1.0]heptan-7-yl]methanamine | 456.22 |
| 27 | 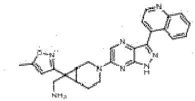 | [7-(5-methyl-1,2-oxazol-3-yl)-3-(3-quinolin-4-yl-1H-pyrazolo[3,4-b]pyrazin-6-yl)-3-azabicyclo[4.1.0]heptan-7-yl]methanamine | 453.21 |
| 28 | 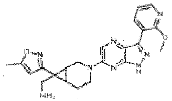 | [3-[3-(2-methoxypyridin-3-yl)-1H-pyrazolo[3,4-b]pyrazin-6-yl]-7-(5-methyl-1,2-oxazol-3-yl)-3-azabicyclo[4.1.0]heptan-7-yl]methanamine | 433.20 |
| 29 | 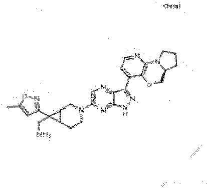 | [(7-(5-methylisoxazol-3-yl)-3-(3-((S)-6a,7,8,9-tetrahydro-6H-pyrido[3,2-b]pyrrolo[1,2-d][1,4]oxazin-4-yl)-1H-pyrazolo[3,4-b]pyrazin-6-yl)-3-azabicyclo[4.1.0]heptan-7-yl)methanamine | 500.25 |
| 30 | 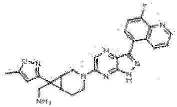 | [3-[3-(8-fluoroqumolm-5-yl)-1H-pyrazolo[3,4-b]pyrazin-6-yl]-7-(5-methyl-1,2-oxazol-3-yl)-3-azabicyclo[4.1.0]heptan-7-yl]methanamine | 471.20 |
| 31 | 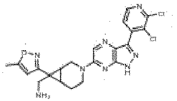 | [3-[3-(2,3-dichloropyridin-4-yl)-1H-pyrazolo[3,4-b]pyrazin-6-yl]-7-(5-methyl-1,2-oxazol-3-yl)-3-azabicyclo[4.1.0]heptan-7-yl]methanamine | 471.12 |
| 32 | 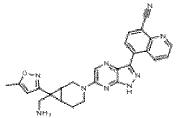 | 5-[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]quinoline-8-carbonitrile | 478.21 |
| 33 | 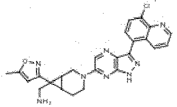 | [3-[3-(8-chloroquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine | 487.17 |
| 34 | 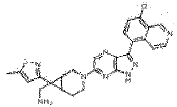 | [3-[3-(8-chloroisoquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine | 487.17 |
| 35 | 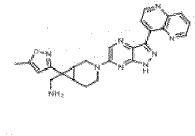 | [7-(5-methyl-1,2-oxazol-3-yl)-3-[3-(1,5-naphthyridin-4-yl)-1H-pyrazolo[3,4-b]pyrazin-6-yl]-3-azabicyclo[4.1.0]heptan-7-yl]methanamine | 454.21 |
| 36 | 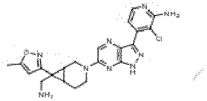 | 4-[6-[7-(aminomethyl)-7-(5-methyl-1,2-oxazol-3-yl)-3-azabicyclo[ 4.1.0]heptan-3-yl]-1H-pyrazolo[3,4-b]pyrazin-3-yl]-3-chloropyridin-2-amine | 452.17 |
| 37 | 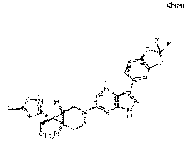 | [(1S,6R,7S)-3-[3-(2,2-difluoro-1,3-benzodioxol-5-yl)-1H-pyrazolo[3,4-b]pyrazin-6-yl]-7-(5-methyl-1,2-oxazol-3-yl)-3-azabicyclo[4.1.0]heptan-7-yl]methanamine | 482.17 |
| 38 | 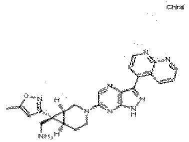 | [(1S,6R,7S)-7-(5-methyl-1,2-oxazol-3-yl)-3-[3-(1,8-naphthyridin-4-yl)-1H-pyrazolo[3,4-b]pyrazin-6-yl]-3-azabicyclo[4.1,0]heptan-7-yl]methanamine | 454.14 |
| 39 | 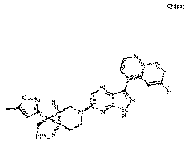 | [(1S,6R,7S)-3-[3-(6-Huoroquinolm-4-yl)-1H-pyrazolo[3,4-b]pyrazin-6-yl]-7-(5-methyl-1,2-oxazol-3-yl)-3-azabicyclo[4.1.0]heptan-7-yl]methanamine | 471.19 |
| 40 | 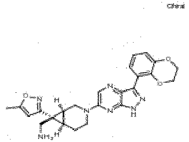 | [(1S,6R,7S)-3-[3-(2,3-dihydro-1,4-benzodioxin-5-yl)-1H-pyrazolo[3,4-b]pyrazin-6-yl]-7-(5-methyl-1,2-oxazol-3-yl)-3-azabicyclo[4.1.0]heptan-7-yl]methanamine | 460.20 |
| 41 | 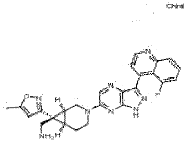 | [(1S,6R,7S)-3-[3-(5-auoroqumolm-4-yl)-1H-pyrazolo[3,4-b]pyrazin-6-yl]-7-(5-methyl-1,2-oxazol-3-yl)-3-azabicyclo[4.1.0]heptan-7-yl]methanamine | 471.20 |
| 42 | 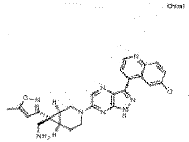 | [(1S,6R,7S)-3-[3-(6-chloroquinolin-4-yl)-1H-pyrazolo[3,4-b]pyrazin-6-yl]-7-(5-methyl-1,2-oxazol-3-yl)-3-azabicyclo[4.1.0]heptan-7-yl]methanamine | 487.19 |
| 43 | 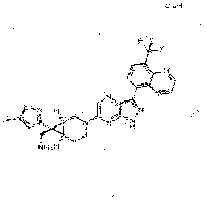 | [(1S,6R,7S)-7-(5-methyl-1,2-oxazol-3-yl)-3-[3-[8-(trifluoromethyl)quinolin-5-yl]-1H-pyrazolo[3,4-b]pyrazin-6-yl]-3-azabicyclo[4.1.0]heptan-7-yl]methanamine | 521.21 |
| 44 | 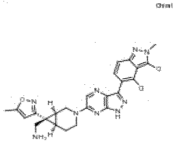 | [(1S,6R, 7S)- 3-[3-(3, 4-dichloro- 2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine | 524.14 |
| 45 | 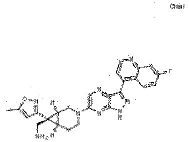 | [(1S,6R,7S)-3-[3-(7-fluoroquinolin-4-yl)-1H-pyrazolo[3,4-b]pyrazin-6-yl]-7-(5-methyl-1,2-oxazol-3-yl)-3-azabicyclo[4.1.0]heptan-7-yl]methanamine | 471.20 |
| 46 | 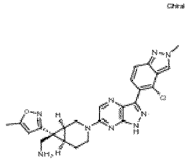 | [(1 S,6R,7S)-3-[3-(4-chloro-2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine | 490.18 |
| 47 | 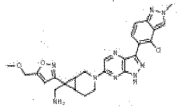 | [3-[3-(4-chloro-2-methylindazol-5-yl)-1H-pyrazolo[3,4-b]pyrazin-6-yl]-7-[5-(methoxymethyl)-1,2-oxazol-3-yl]-3-azabicyclo[4.1.0]heptan-7-yl]methanamine | 520.19 |
| 48 | 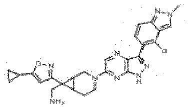 | [3-[3-(4-chloro-2-methylindazol-5-yl)-1H-pyrazolo[3,4-b]pyrazin-6-yl]-7-(5-cyclopropyl-1,2-oxazol-3-yl)-3-azabicyclo[4.1.0]heptan-7-yl]methanamine | 516.20 |
| 49 | 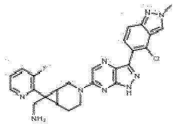 | [3-[3-(4-chloro-2-methylindazol-5-yl)-1H-pyrazolo[3,4-b]pyrazin-6-yl]-7-(3-fluoropyridin-2-yl)-3-azabicyclo[4.1.0]heptan-7-yl]methanamine | 504.18 |
| 50 | 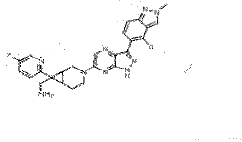 | [3-[3-(4-chloro-2-methylindazol-5-yl)-1H-pyrazolo[3,4-b]pyrazin-6-yl]-7-(5-fluoropyridin-2-yl)-3-azabicyclo[4.1.0]heptan-7-yl]methanamine | 504.18 |
| 51 | 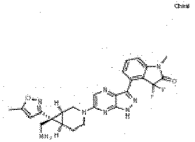 | 4-[6-[(1S,6R,7S)-7-(aminomethyl)-7-(5-methyl-1,2-oxazol-3-yl)-3-azabicyclo[4.1,0]heptan-3-yl]-1H-pyrazolo[3,4-b]pyrazin-3-yl]-3,3-difluoro-1-methylindol-2-one | 507.20 |
| 52 | 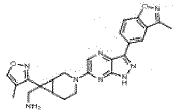 | [3-[3-(3-methyl-1,2-benzoxazol-5-yl)-1H-pyrazolo[3,4-b]pyrazin-6-yl]-7-(4-methyl-1,2-oxazol-3-yl)-3-azabicyclo[4.1.0]heptan-7-yl]methanamine | 457.18 |
| 53 | 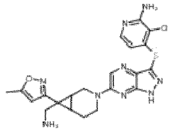 | 4-[[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]sulfanyl]-3-chloropyridin- 2-amine | 484.14 |
| 54 | 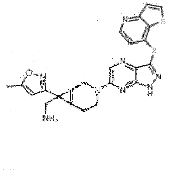 | [7-(5-methyl-1,2-oxazol-3-yl)-3-(3-thieno[3,2-b]pyridm-7-ylsulfanyl-1H-pyrazolo[3,4-b]pyrazm-6-yl)-3-azabicyclo[4.1.0]heptan-7-yl]methanamine | 491.28 |
| 55 | 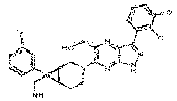 | [6-[7-(aminomethyl)-7-(3-fluorophenyl)-3-azabicyclo[4.1,0]heptan-3-yl]-3-(2,3-dichlorophenyl)-1H-pyrazolo[3,4-b]pyrazin-5-yl]methanol | 513.32 |
| 56 | 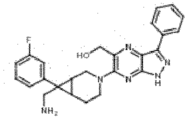 | [6-[7-(aminomethyl)-7-(3-fluorophenyl)-3-azabicyclo[4.1.0]heptan-3-yl]-3-phenyl-1H-pyrazolo[3,4-b]pyrazin-5-yl]methanol | 445.21 |
| 57 | 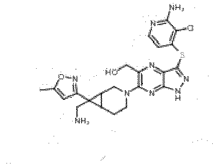 | [3-(2-amino-3-chloropyridin-4-yl)sulfanyl-6-[7-(aminomethyl)-7-(5-methyl-1,2-oxazol-3-yl)-3-azabicyclo[4.1,0]heptan-3-yl]-1H-pyrazolo[3,4-b]pyrazin-5-yl]methanol | 514.15 |
| 58 | 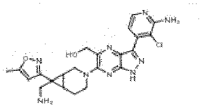 | [3-(2-amino-3-chloropyridin-4-yl)-6-[7-(ammomethyl)-7-(5-methyl-1,2-oxazol-3-yl)-3-azabicyclo[4.1.0]heptan-3-yl]-1H-pyrazolo[3,4-b]pyrazin-5-yl]methanol | 482.18 |
| 59 | 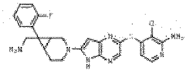 | 4-[[6-[7-(aminomethyl)-7-(2-fluorophenyl)-3-azabicyclo[4.1.0]heptan-3-yl]-5H-pyrrolo[2,3-b]pyrazin-2-yl]sulfanyl]-3-chloropyridin-2-amine | 496.14 |
| 60 | 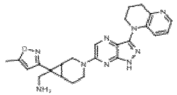 | [3-[3-(3,4-dihydro-2H-1,5-naphthyridin-1-yl)-1H-pyrazolo[3,4-b]pyrazin-6-yl]-7-(5-methyl-1,2-oxazol-3-yl)-3-azabicyclo[4.1,0]heptan-7-yl]methanamine | 458.24 |
| 61 | 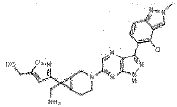 | [3-[7-(aminomethyl)-3-[3-(4-chloro-2-methylindazol-5-yl)-1H-pyrazolo[3,4-b]pyrazin-6-yl]-3-azabicyclo[4.1.0]heptan-7-yl]-1,2-oxazol-5-yl]methanol | 506.18 |
| 62 | 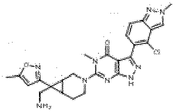 | 6-[7-(aminomethyl)-7-(5-methyl-1,2-oxazol-3-yl)-3-azabicyclo[4.1.0]heptan-3-yl]-3-(4-chloro-2-methylindazol-5-yl)-5-methyl-1H-pyrazolo[3,4-d]pyrimidin-4-one | 520.19 |
| 63 | 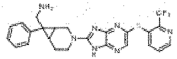 | [7-phenyl-3-[5-[2-(trifluoromethyl)pyridin-3-yl]sulfanyl-1H-imidazo[4,5-b]pyrazin-2-yl]-3-azabicyclo[4.1.0]heptan-7-yl]methanamine | 498.46 |
| 64 | 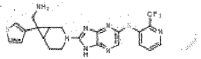 | [7-thiophen-3-yl-3-[5-[2-(trifluoromethyl)pyridin-3-yl]sulfanyl-1H-imidazo[4,5-b]pyrazin-2-yl]-3-azabicyclo[4.1.0]heptan-7-yl]methanamine | 504.20 |
| 65 | 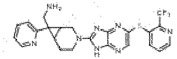 | [7 -pyridin- 2-yl- 3-[5-[2-(trifluoromethyl)pyridin-3-yl]thio-1H-imidazo[4,5-b]pyrazin-2-yl]-3-azabicyclo[4.1.0]heptan-7-yl]methanamine | 499.27 |
| 66 | 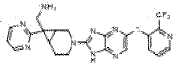 | [7-pyrimidin-2-yl-3-[5-[2-(trifluoromethyl)pyridin-3-yl]sulfanyl-1H-imidazo[4,5-b]pyrazin-2-yl]-3-azabicyclo[4.1.0]heptan-7-yl]methanamine | 500.40 |
| 67 | 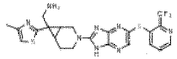 | [7-(4-methyl-1,3-thiazol-2-yl)-3-[5-[2-(trifluoromethyl)pyndm-3-yl]thio-1H-imidazo[4,5-b]pyrazin-2-yl]-3-azabicyclo[4.1.0]heptan-7-yl]methanamine | 519.26 |
| 68 | 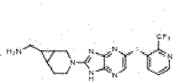 | [3-[5-[2-(trifluoromethyl)pyridin-3-yl]thio-1H-imidazo[4,5-b]pyrazin-2-yl]-3-azabicyclo[4.1.0]heptan-7-yl]methanamine | 422.27 |
| 69 | 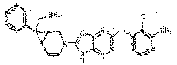 | 4-[[2-[7-(aminomethyl)-7-phenyl-3-azabicyclo[4.1.0]heptan-3-yl]-1H-imidazo[4,5-b]pyrazin-5-yl]thio]-3-chloropyridin-2-amine | 479.33 |
| 70 | 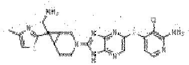 | 4-[[2-[7-(aminomethyl)-7-(4-methyl-1,3-thiazol-2-yl)-3-azabicyclo[4.1.0]heptan-3-yl]-1H-imidazo[4,5-b]pyrazin-5-yl]thio]-3-chloropyridin- 2-amine | 500.13 |
| 71 | 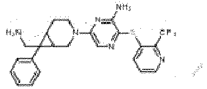 | 6-[7-(aminomethyl)-7-phenyl-3-azabicyclo[4.1.0]heptan-3-yl]-3-[(2-(trifluoromethyl)pyridin-3-yl)thio]pyrazin-2-amine | 473.27 |
| 72 | 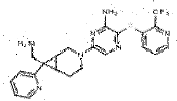 | 6-[7-(aminomethyl)-7-pyridin-2-yl-3-azabicyclo[4.1.0]heptan-3-yl]-3-[(2-(trifluoromethyl)pyridin-3-yl)thio]pyrazin-2-amine | 474.28 |
| 73 | 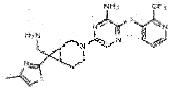 | 6-(7-(aminomethyl)-7-(4-methylthiazol-2-yl)-3-azabicyclo[4.1.0]heptan-3-yl)-3-((2-(trifluoromethyl)pyridin-3-yl)thio)pyrazin-2-amine | 494.25 |
| 74 | 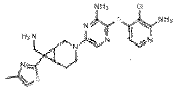 | 3-((2-amino-3-chloropyridin-4-yl)thio)-6-[7-(aminomethyl)-7-(4-methyl-1,3-thiazol-2-yl)-3-azabicyclo[4.1.0]heptan-3-yl]pyrazin-2-amine | 475.21 |
| 75 | 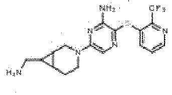 | 6-(7-(aminomethyl)-3-azabicyclo[4.1.0]heptan-3-yl)-3-((2-(trifluoromethyl)pyridin-3-yl)thio)pyrazin-2-amine | 397.28 |
| 76 | 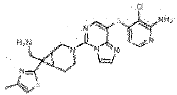 | 4-((5-(7-(aminomethyl)-7-(4-methylthiazol-2-yl)-3-azabicyclo[4.1.0]heptan-3-yl)imidazo[1,2-c]pyrimidin-8-yl)thio)-3-chloropyridin-2-amine | 499.25 |
| 77 | 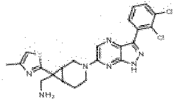 | [3-[3-(2,3-dichlorophenyl)-1H-pyrazolo[3,4-b]pyrazin-6-yl]-7-(4-methyl-1,3-thiazol-2-yl)-3-azabicyclo[4.1.0]heptan-7-yl]methanamine | 486,42 |
| 78 | 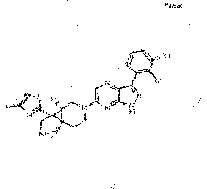 | [(1S,6R,7S)-3-[3-(2,3-dichlorophenyl)-1H-pyrazolo[3,4-b]pyrazin-6-yl]-7-(4-methyl-1,3-thiazol-2-yl)-3-azabicyclo[4.1.0]heptan-7-yl]methanamine | 486.35 |
| 79 | 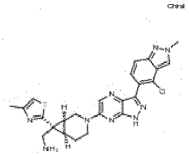 | [(1S,6R,7S)-3-[3-(4-chloro-2-methylindazol-5-yl)-1H-pyrazolo[3,4-b]pyrazin-6-yl]-7-(4-methyl-1,3-thiazol-2-yl)-3-azabicyclo[4.1.0]heptan-7-yl]methanamine | 506.48 |
| 80 | 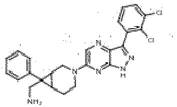 | [3-[3-(2,3-dichlorophenyl)-1H-pyrazolo[3,4-b]pyrazin-6-yl]-7-phenyl-3-azabicyclo[4.1.0]heptan-7-yl]methanamine | 465.29 |
| 81 | 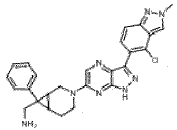 | [3-[3-(4-chloro- 2-methylindazol-5-yl)-1H-pyrazolo[3,4-b]pyrazin-6-yl]-7-phenyl-3-azabicyclo[4.1.0]heptan-7-yl]methanamine | 485.38 |
| 82 | 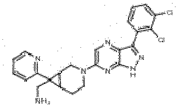 | [3-[3-(2,3-dichlorophenyl)-1H-pyrazolo[3,4-b]pyrazin-6-yl]-7-pyridin-2-yl-3-azabicyclo[4.1.0]heptan-7-yl]methanamine | 466.16 |
| 83 | 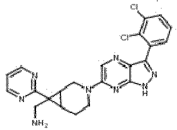 | [3-[3-(2,3-dichlorophenyl)-1H-pyrazolo[3,4-b]pyrazin-6-yl]-7-pyrimidin-2-yl-3-azabicyclo[4.1.0]heptan-7-yl]methanamine | 467.24 |
| 84 | 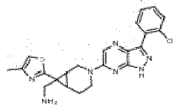 | [3-[3-(2-chlorophenyl)-1H-pyrazolo[3,4-b]pyrazin-6-yl]-7-(4-methyl-1,3-thiazol-2-yl)-3-azabicyclo[4.1.0]heptan-7-yl]methanamine | 452.47 |
| 85 | 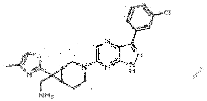 | [3-[3-(3-chlorophenyl)-1H-pyrazolo[3,4-b]pyrazin-6-yl]-7-(4-methyl-1,3-thiazol-2-yl)-3-azabicyclo[4.1.0]heptan-7-yl]methanamine | 452.40 |
| 86 | 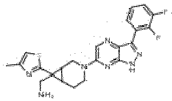 | [3-[3-(2,3-difluorophenyl)-1H-pyrazolo[3,4-b]pyrazin-6-yl]-7-(4-methyl-1,3-thiazol-2-yl)-3-azabicyclo[4.1.0]heptan-7-yl]methanamine | 454.47 |
| 87 | 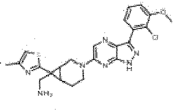 | [3-[3-(2-chloro-3-methoxyphenyl)-1H-pyrazolo[3,4-b]pyrazin-6-yl]-7-(4-methyl-1,3-thiazol-2-yl)-3-azabicyclo[4.1.0]heptan-7-yl]methanamine | 482.45 |
| 88 | 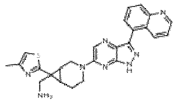 | [7-(4-methyl-1,3-thiazol-2-yl)-3-(3-quinolin-5-yl-1H-pyrazolo[3,4-b]pyrazin-6-yl)-3-azabicyclo[4.1.0]heptan-7-yl]methanamine | 469.43 |
| 89 | 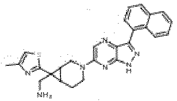 | [7-(4-methyl-1,3-thiazol-2-yl)-3-(3-naphthalen-1-yl-1H-pyrazolo[3,4-b]pyrazin-6-yl)-3-azabicyclo[4.1.0]heptan-7-yl]methanamine | 468.42 |
| 90 | 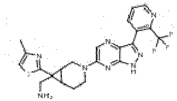 | [7-(4-methyl-1,3-thiazol-2-yl)-3-[3-[2-(trifluoromethyl)pyridin-3-yl]-1H-pyrazolo[3,4-b]pyrazin-6-yl]-3-azabicyclo[4.1.0]heptan-7-yl]methanamine | 487.30 |
| 91 | 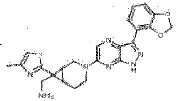 | [3-[3-(1,3-benzodioxol-4-yl)-1H-pyrazolo[3,4-b]pyrazin-6-yl]-7-(4-methyl-1,3-thiazol-2-yl)-3-azabicyclo[4.1.0]heptan-7-yl]methanamine | 462.38 |
| 92 | 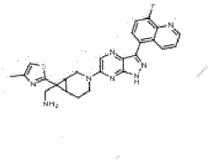 | [3-[3-(8-fluoroqumolm-5-yl)-1H-pyrazolo[3,4-b]pyrazin-6-yl]-7-(4-methyl-1,3-thiazol-2-yl)-3-azabicyclo[4.1.0]heptan-7-yl]methanamine | 487.32 |
| 93 | 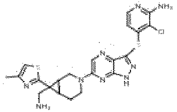 | 4-[[6-[7-(aminomethyl)-7-(4-methyl-1,3-thiazol-2-yl)-3-azabicyclo[4.1.0]heptan-3-yl]-1H-pyrazolo[3,4-b]pyrazin-3-yl]sulfanyl]-3-chloropyridin-2-amine | 500.19 |
| 94 | 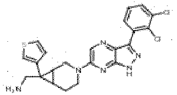 | [3-[3-(2,3-dichlorophenyl)-1H-pyrazolo[3,4-b]pyrazin-6-yl]-7-thiophen-3-yl-3-azabicyclo[4.1.0]heptan-7-yl]methanamine | 471.23 |
| 95 | 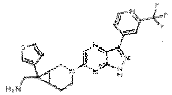 | [7-thiophen- 3-yl-3-[3-[2-(trifluoromethyl)pyridm-4-yl]-1H-pyrazolo[3,4-b]pyrazm-6-yl]-3-azabicyclo[4.1.0]heptan-7-yl]methanamine | 472.24 |
| 96 | 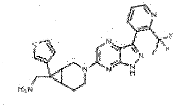 | [7-thiophen-3-yl-3-[3-[2-(trifluoromethyl)pyridin-3-yl]-1H-pyrazolo[3,4-b]pyrazin-6-yl]-3-azabicyclo[4.1.0]heptan-7-yl]methanamine | 472.24 |
| 97 | 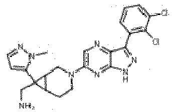 | [3-[3-(2,3-dichlorophenyl)-1H-pyrazolo[3,4-b]pyrazin-6-yl]-7-(2-methylpyrazol-3-yl)-3-azabicyclo[4.1.0]heptan-7-yl]methanamine | 469.47 |
| 98 | 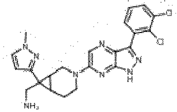 | [3-[3-(2,3-dichlorophenyl)-1H-pyrazolo[3,4-b]pyrazin-6-yl]-7-(1-methylpyrazol-3-yl)-3-azabicyclo[4.1.0]heptan-7-yl]methanamine | 469.47 |
| 99 | 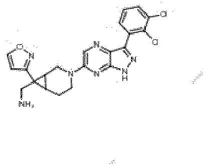 | [3-[3-(2,3-dichlorophenyl)-1H-pyrazolo[3,4-b]pyrazin-6-yl]-7-(1,2-oxazol-3-yl)-3-azabicyclo[4.1.0]heptan-7-yl]methanamine | 456.41 |
| 100 | 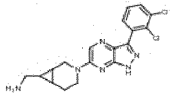 | [3-[3-(2,3-dichlorophenyl)-1H-pyrazolo[3,4-b]pyrazin-6-yl]-3-azabicyclo[4.1,0]heptan-7-yl]methanamine | 389.25 |
| 101 | 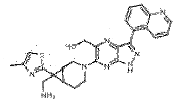 | [6-[7-(aminomethyl)-7-(4-methyl-1,3-thiazol-2-yl)-3-azabicyclo[4.1.0]heptan-3-yl]-3-quinolin-5-yl-1H-pyrazolo[3,4-b]pyrazin-5-yl]methanol | 499.47 |
| 102 | 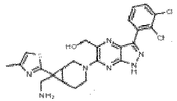 | [6-[7-(aminomethyl)-7-(4-methyl-1,3-thiazol-2-yl)-3-azabicyclo[4.1.0]heptan-3-yl]-3-(2,3-dichlorophenyl)-1H-pyrazolo[3,4-b]pyrazin-5-yl]methanol | 516.30 |
| 103 | 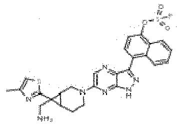 | 2-[7-(aminomethyl)- 3-[3-(4-fluorosulfonyloxynaphthalen-1-yl)-1H-pyrazolo[3,4-b]pyrazin-6-yl]-3-azabicyclo[4.1.0]heptan-7-yl]-4-methyl-1,3-thiazole | 566.38 |
| 104 | 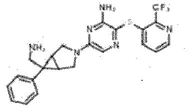 | 6-(6-(aminomethyl)-6-phenyl-3-azabicyclo[3.1.0]hexan-3-yl)-3-((2-(trifluoromethyl)pyridin-3-yl)thio)pyrazin-2-amine | 459.29 |
| 105 | 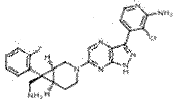 | 4-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)-3-chloropyridin- 2-amine | 465.71 |
| 106 | 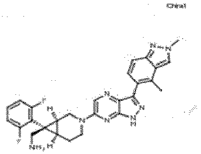 | ((1S,6R,7R)-7-(2,6-difluorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine | 501.23 |
| 107 | 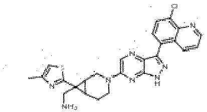 | (3-(3-(8-chloroquinolin-5-yl)-1H-pyrazolo[3,4-b]pyrazin-6-yl)-7-(4-methylthiazol-2-yl)-3-azabicyclo[4.1.0]heptan-7-yl)methanamine | 503.02 |
| 108 | 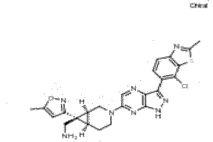 | ((1 S,6R,7S)-3-(3-(7-chloro-2-methylbenzo[d]thiazol-6-yl)-1H-pyrazolo[3,4-b]pyrazin-6-yl)-7-(5-methylisoxazol-3-yl)-3-azabicyclo[4.1.0]heptan-7-yl)methanamine | 507.01 |
| 109 | 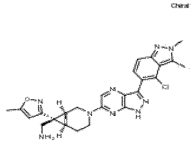 | ((1S,6R,7S)-3-(3-(4-chloro-2,3-dimethyl-2H-indazol-5-yl)-1H-pyrazolo[3,4-b]pyrazin-6-yl)-7-(5-methylisoxazol-3-yl)-3-azabicyclo[4.1.0]heptan-7-yl)methanamine | 504.20 |
| 110 | 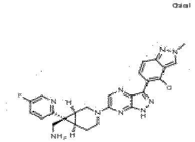 | ((1S,6R,7S)-3-(3-(4-chloro-2-methyl-2H-indazol-5-yl)-1H-pyrazolo[3,4-b]pyrazin-6-yl)-7-(5-fluoropyridin-2-yl)-3-azabicyclo[4.1.0]heptan-7-yl)methanamine | 504.18 |
| 111 | 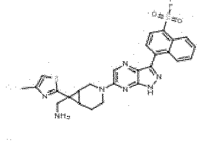 | 4-(6-(7-(aminomethyl)-7-(4-methylthiazol-2-yl)-3-azabicyclo[4.1.0]heptan-3-yl)-1H-pyrazolo[3,4-b]pyrazin-3-yl)naphthalene-1-sulfonyl fluoride | 550.35 |
| 112 | 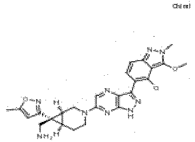 | ((1S,6R,7S)-3-(3-(4-chloro-3-methoxy-2-methyl-2H-indazol-5-yl)-1H-pyrazolo[3,4-b]pyrazin-6-yl)-7-(5-methylisoxazol-3-yl)-3-azabicyclo[4.1.0]heptan-7-yl)methanamine | 520.19 |
| 113 | 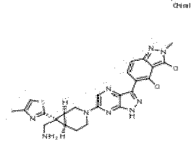 | ((1S,6R,7S)-3-(3-(3,4-dichloro-2-methyl-2H-indazol-5-yl)-1H-pyrazolo[3,4-b]pyrazin-6-yl)-7 -(4-methylthiazol-2-yl)-3-azabicyclo[4.1. 0]heptan-7 -yl)methanamine | 540.47 |
| 114 | 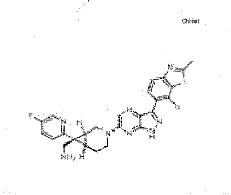 | ((1S,6R,7S)-3-(3-(7-chloro-2-methylbenzo[d]thiazol-6-yl)-1H-pyrazolo[3,4-b]pyrazin-6-yl)-7-(5-fluoropyridin-2-yl)-3-azabicyclo[4.1.0]heptan-7-yl)methanamine | 521.38 |
| 115 | 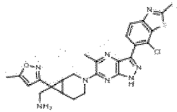 | (3-(3-(7-chloro-2-methylbenzo[d]thiazol-6-yl)-5-methyl-1H-pyrazolo[3,4-b]pyrazin-6-yl)-7-(5-methylisoxazol-3-yl)-3-azabicyclo[4.1.0]heptan-7-yl)methanamine | 521.16 |
| 116 | 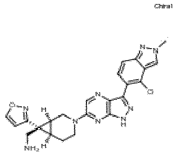 | ((1S,6R,7S)-3-(3-(4-chloro-2-methyl-2H-indazol-5-yl)-1H-pyrazolo[3,4-b]pyrazin-6-yl)-7-(isoxazol-3-yl)-3-azabicyclo[4.1.0]heptan-7-yl)methanamine | 476.17 |
| 117 | 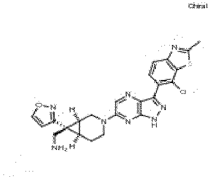 | ((1S,6R,7S)-3-(3-(7-chloro-2-methylbenzo[d]thiazol-6-yl)-1H-pyrazolo[3,4-b]pyrazin-6-yl)-7-(isoxazol-3-yl)-3-azabicyclo[4.1.0]heptan-7-yl)methanamine | 493.13 |
| 118 | 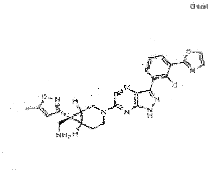 | ((1S,6R,7S)-3-(3-(2-chloro-3-(oxazol-2-yl)phenyl)-1H-pyrazolo[3,4-b]pyrazin-6-yl)-7-(5-methylisoxazol-3-yl)-3-azabicyclo[4.1.0]heptan-7-yl)methanamine | 503.17 |
| 119 | 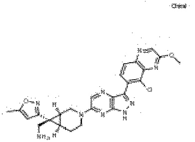 | ((1S,6R,7S)-3-(3-(5-chloro-3-methoxyqumoxalm-6-yl)-1H-pyrazolo[3,4-b]pyrazin-6-yl)-7-(5-methylisoxazol-3-yl)-3-azabicyclo[4.1.0]heptan-7-yl)methanamine | 518.18 |
| 120 | 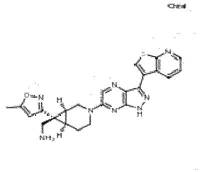 | ((1S,6R,7S)-7-(5-methylisoxazol-3-yl)-3-(3-(thieno[2,3-b]pyridin-3-yl)-1H-pyrazolo[3,4-b]pyrazin-6-yl)-3-azabicyclo[4.1.0]heptan-7-yl)methanamine | 459.17 |
| 121 | 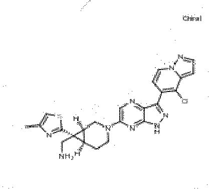 | ((1S,6R,7S)-3-(3-(4-chloropyrazolo[1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine | 492.40 |
| 122 | 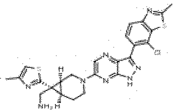 | ((1S,6R,7S)-3-(3-(7-chloro-2-methylbenzo[d]thiazol-6-yl)-1H-pyrazolo[3,4-b]pyrazin-6-yl)-7-(4-methylthiazol-2-yl)-3-azabicyclo[4.1.0]heptan-7-yl)methanamine | 523.38 |
| 123 | 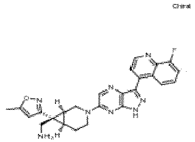 | ((1S,6R,7S)-3-(3-(8-fluoroquinolin-4-yl)-1H-pyrazolo[3,4-b]pyrazin-6-yl)-7-(5-methylisoxazol-3-yl)-3-azabicyclo[4.1.0]heptan-7-yl)methanamine | 471.20 |
| 124 | 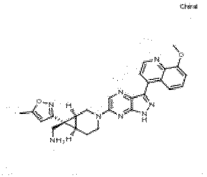 | ((1S,6R,7S)-3-(3-(8-methoxyquinolin-4-yl)-1H-pyrazolo[3,4-b]pyrazin-6-yl)-7-(5-methylisoxazol-3-yl)-3-azabicyclo[4.1.0]heptan-7-yl)methanamine | 483.25 |
| 125 | 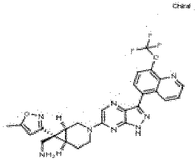 | ((1S,6R,7S)-7-(5-methylisoxazol-3-yl)-3-(3-(8-(trifluoromethoxy)quinolin-5-yl)-1H-pyrazolo[3,4-b]pyrazin-6-yl)-3-azabicyclo[4.1.0]heptan-7-yl)methanamine | 537.37 |
| 126 | 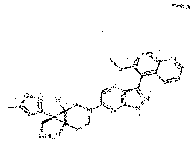 | ((1S,6R,7S)-3-(3-(6-methoxyqumolm-5-yl)-1H-pyrazolo[3,4-b]pyrazin-6-yl)-7-(5-methylisoxazol-3-yl)-3-azabicyclo[4.1.0]heptan-7-yl)methanamine | 483.22 |
| 127 | 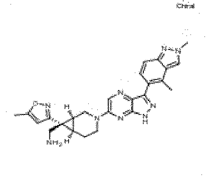 | ((1S,6R,7S)-3-(3-(2,4-dimethyl-2H-mdazol-5-yl)-1H-pyrazolo[3,4-b]pyrazin-6-yl)-7-(5-methylisoxazol-3-yl)-3-azabicyclo[4.1. 0]heptan-7-yl)methanamine | 470.47 |
| 128 | 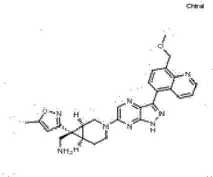 | ((1S,6R,7S)-3-(3-(8-(methoxymethyl)qumolm-5-yl)-1H-pyrazolo[3,4-b]pyrazin-6-yl)-7-(5-methylisoxazol-3-yl)-3-azabicyclo[ 4.1.0]heptan-7-yl)methanamine | 497.45 |
| 129 | 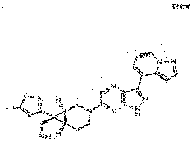 | ((1S,6R,7S)-7-(5-methylisoxazol-3-yl)-3-(3-(pyrazolo[1,5-a]pyridm-4-yl)-1H-pyrazolo[3,4-b]pyrazin-6-yl)-3-azabicyclo[4.1.0]heptan-7-yl)methanamine | 442.21 |
| 130 | 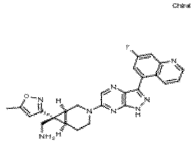 | ((1S,6R,7S)-3-(3-(7-fluoroquinolin-5-yl)-1H-pyrazolo[3,4-b]pyrazin-6-yl)-7-(5-methylisoxazol-3-yl)-3-azabicyclo[4.1.0]heptan-7-yl)methanamine | 471.2 |
| 131 | 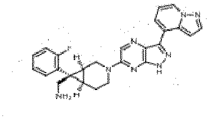 | ((1S,6R,7R)-7-(2-fluorophenyl)-3-(3-(pyrazolo[1,5-a]pyridin-4-yl)-1H-pyrazolo[3,4-b]pyrazin-6-yl)-3-azabicyclo[4.1.0]heptan-7-yl)methanamine | 455.21 |
| 132 | 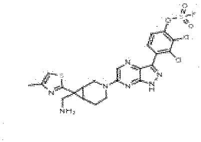 | 4-(6-(7-(aminomethyl)-7-(4-methylthiazol-2-yl)-3-azabicyclo[4.1.0]heptan-3-yl)-1H-pyrazolo[3,4-b]pyrazin-3-yl)-2,3-dichlorophenyl sulfurofluoridate | 584.27 |
| 133 | 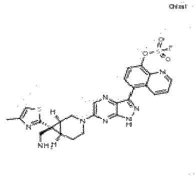 | 5-(6-((1S,6R,7S)-7-(aminomethyl)-7-(4-methylthiazol-2-yl)-3-azabicyclo[4.1.0]heptan-3-yl)-1H-pyrazolo[3,4-b]pyrazin-3-yl)quinolin-8-yl sulfurofluoridate | 567.31 |
| 134 | 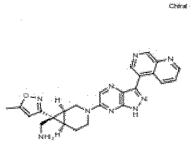 | ((1S,6R,7S)-3-(3-(1,7-naphthyridm-5-yl)-1H-pyrazolo[3,4-b]pyrazin-6-yl)-7-(5-methylisoxazol-3-yl)-3-azabicyclo[4.1.0]heptan-7-yl)methanamine | 454.24 |
| 135 | 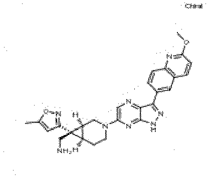 | ((1S,6R,7S)-3-(3-(2-methoxyquinolin-6-yl)-1H-pyrazolo[3,4-b]pyrazin-6-yl)-7-(5-methylisoxazol-3-yl)-3-azabicyclo[4.1.0]heptan-7-yl)methanamine | 483.22 |
| 136 | 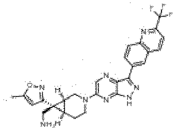 | ((1S,6R,7S)-7-(5-methylisoxazol-3-yl)-3-(3-(2-(trifluoromethyl)quinolin-6-yl)-1H-pyrazolo[3,4-b]pyrazin-6-yl)-3-azabicyclo[4.1.0]heptan-7-yl)methanamine | 521.20 |
| 137 | 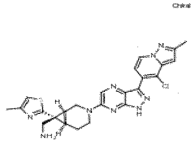 | ((1S,6R,7S)-3-(3-(4-chloro-2-methylpyrazolo[ 1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine | 506.42 |
| 138 | 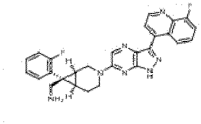 | ((1S,6R,7R)-7-(2-fluorophenyl)-3-(3-(8-fluoroquinolin-4-yl)-1H-pyrazolo[3,4-b]pyrazin-6-yl)-3-azabicyclo[4.1,0]heptan-7-yl)methanamine | 484.20 |
| 139 | 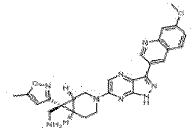 | ((1S,6R,7S)-3-(3-(7-methoxyquinolin-3-yl)-1H-pyrazolo[3,4-b]pyrazin-6-yl)-7-(5-methylisoxazol-3-yl)-3-azabicyclo[4.1.0]heptan-7 -yl)methanamine | 483.22 |
| 140 | 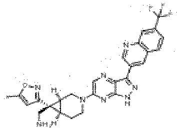 | ((1S,6R,7S)-7-(5-methylisoxazol-3-yl)-3-(3-(7-(trifluoromethyl)quinolin-3-yl)-1H-pyrazolo[3,4-b]pyrazin-6-yl)-3-azabicyclo[4.1.0]heptan-7-yl)methanamine | 521.16 |
| 141 | 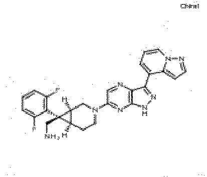 | ((1S,6R,7R)-7-(2,6-difluorophenyl)-3-(3-(pyrazolo[1,5-a]pyridin-4-yl)-1H-pyrazolo[3,4-b]pyrazin-6-yl)-3-azabicyclo[4.1.0]heptan-7-yl)methanamine | 473.20 |
| 142 | 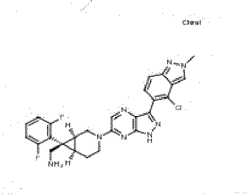 | ((1S,6R,7R)-3-(3-(4-chloro-2-methyl-2H-indazol-5-yl)-1H-pyrazolo[3,4-b]pyrazin-6-yl)-7-(2,6-difluorophenyl)-3-azabicyclo[4.1.0]heptan-7-yl)methanamine | 521.17 |
| 143 | 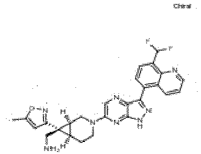 | ((1S,6R,7S)-3-(3-(8-(difluoromethyl)quinolin-5-yl)-1H-pyrazolo[3,4-b]pyrazin-6-yl)-7-(5-methylisoxazol- 3-yl)-3-azabicyclo[ 4.1.0]heptan-7-yl)methanamine | 503.30 |
| 144 | 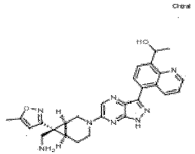 | 1-(5-(6-((1S,6R,7S)-7-(aminomethyl)-7-(5-methylisoxazol-3-yl)-3-azabicyclo[4.1.0]heptan-3-yl)-1H-pyrazolo[3,4-b]pyrazin-3-yl)quinolin-8-yl)ethan-1-ol | 497.33 |
| 145 | 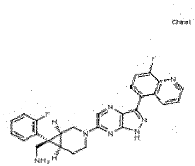 | ((1S,6R,7R)-7-(2-fluorophenyl)-3-(3-(8-fluoroquinolin-5-yl)-1H-pyrazolo[3,4-b]pyrazin-6-yl)-3-azabicyclo[4.1.0]heptan-7-yl)methanamine | 484.20 |
| 146 | 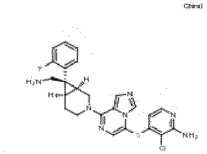 | 4-((8-((1S,6R,7R)-7-(aminomethyl)-7-(2-fluorophenyl)-3-azabicyclo[4.1.0]heptan-3-yl)imidazo[1,5-a]pyrazin-5-yl)thio)-3-chloropyridin- 2-amine | 496.22 |
| 147 | 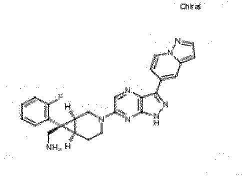 | ((1S,6R,7R)-7-(2-fluorophenyl)-3-(3-(pyrazolo[1,5-a]pyridin-5-yl)-1H-pyrazolo[3,4-b]pyrazin-6-yl)-3-azabicyclo[4.1.0]heptan-7-yl)methanamine | 455.21 |
| 148 | 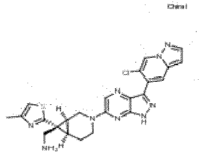 | ((1S,6R,7S)-3-(3-(6-chloropyrazolo[1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine | 492.26 |
| 149 | 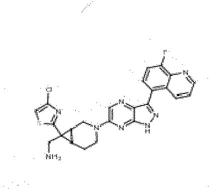 | (7-(4-chlorothiazol-2-yl)-3-(3-(8-fluoroquinolin-5-yl)-1H-pyrazolo[3,4-b]pyrazin-6-yl)-3-azabicyclo[4.1.0]heptan-7-yl)methanamine | 507.29 |
| 150 | 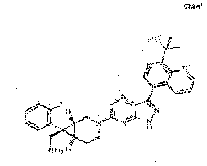 | 2-(5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl)propan-2-ol | 524.32 |
| 151 | 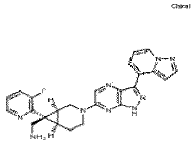 | ((1S,6R,7S)-7-(3-fluoropyridin-2-yl)-3-(3-(pyrazolo[1,5-a]pyridin-4-yl)-1H-pyrazolo[3,4-b]pyrazin-6-yl)-3-azabicyclo[4.1.0]heptan-7-yl)methanamine | 456.20 |
| 152 | 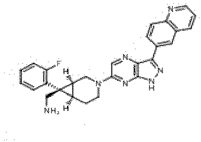 | ((1S,6R,7R)-7-(2-fluorophenyl)-3-(3-(quinolin-6-yl)-1H-pyrazolo[3,4-b]pyrazin-6-yl)-3-azabicyclo[4.1.0]heptan-7-yl)methanamine | 466.21 |
| 153 | 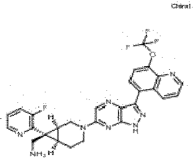 | ((1S,6R,7S)-7-(3-fluoropyridin-2-yl)-3-(3-(8-(trifluoromethoxy)quinolin-5-yl)-1H-pyrazolo[3,4-b]pyrazin-6-yl)-3-azabicyclo[4.1.0]heptan-7-yl)methanamine | 551.19 |
| 154 | 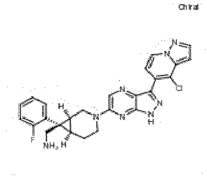 | ((1S,6R,7R)-3-(3-(4-chloropyrazolo[1,5-a]pyndm-5-yl)-1H-pyrazolo[3,4-b]pyrazm-6-yl)-7-(2-fluorophenyl)-3-azabicyclo[4.1.0]heptan-7-yl)methanamine | 489.32 |
| 155 | 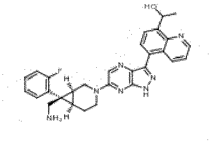 | 1-(5-(6-((1S,6R,7R)-7-(aminomethyl)-7-(2-flurophenyl)-3-azabicyclo[4.1.0]heptan-3-yl)-1H-pyrazolo[3,4-b]pyrazin-3-yl)quinolin-8-yl)ethan-1-ol | 510.24 |
| 156 | 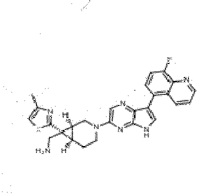 | ((1S,6R,7S)-3-(7-(8-fluoroquinolin-5-yl)-5H-pyrrolo[2,3-b]pyrazin-3-yl)-7-(4-methylthiazol-2-yl)-3-azabicyclo[4.1.0]heptan-7-yl)methanamine | 486.25 |
| 157 | 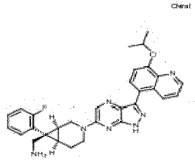 | ((1S,6R,7R)-7-(2-fluorophenyl)-3-(3-(8-isopropoxyquinolin-5-yl)-1H-pyrazolo[3,4-b]pyrazin-6-yl)-3-azabicyclo[4.1.0]heptan-7-yl)methanamine | 524.23 |
| 158 | 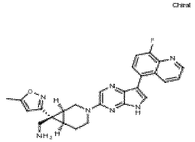 | ((1S,6R,7S)-3-(7-(8-fluoroquinolin-5-yl)-5H-pyrrolo[2,3-b]pyrazin-3-yl)-7-(5-methylisoxazol-3-yl)-3-azabicyclo[4.1.0]heptan-7-yl)methanamine | 470.21 |
| 159 | 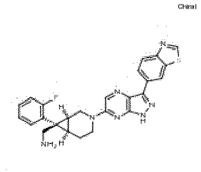 | ((1S,6R,7R)-3-(3-(benzo[d]thiazol-6-yl)-1H-pyrazolo[3,4-b]pyrazin-6-yl)-7-(2-fluorophenyl)-3-azabicyclo[4.1.0]heptan-7-yl)methanamine | 472.17 |
| 160 | 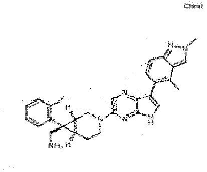 | ((1S,6R,7R)-3-(7-(2,4-dimethyl-2H-indazol-5-yl)-5H-pyrrolo[2,3-b]pyrazin-3-yl)-7-(2-fluorophenyl)-3-azabicyclo[4.1.0]heptan-7-yl)methanamine | 482.20 |
| 161 | 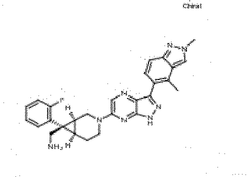 | ((1S,6R,7R)-3-(3-(2,4-dimethyl-2H-indazol-5-yl)-1H-pyrazolo[3,4-b]pyrazin-6-yl)-7-(2-fluorophenyl)-3-azabicyclo[4.1.0]heptan-7-yl)methanamine | 483.24 |
| 162 | 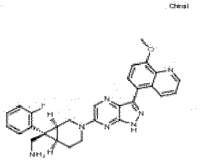 | ((1S,6R,7R)-7-(2-fluorophenyl)-3-(3-(8-methoxyquinolin-5-yl)-1H-pyrazolo[3,4-b]pyrazin-6-yl)-3-azabicyclo[4.1.0]heptan-7-yl)methanamine | 496.22 |
| 163 | 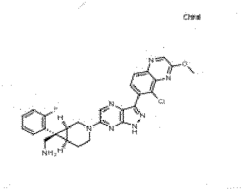 | ((1S,6R,7R)-3-(3-(5-chloro-3-methoxyquinoxalm-6-yl)-1H-pyrazolo[3,4-b]pyrazin-6-yl)-7-(2-fluorophenyl)-3-azabicyclo[4.1.0]heptan-7-yl)methanamine | 531.18 |
| 164 | 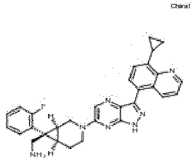 | ((1S,6R,7R)-3-(3-(8-cyclopropylquinolin-5-yl)-1H-pyrazolo[3,4-b]pyrazin-6-yl)-7-(2-fluorophenyl)-3-azabicyclo[4.1.0]heptan-7-yl)methanamine | 506.71 |
| 165 | 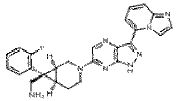 | ((1S,6R,7R)-7-(2-fluorophenyl)-3-(3-(imidazo[1,2-a]pyridin-5-yl)-1H-pyrazolo[3,4-b]pyrazm-6-yl)-3-azabicyclo[4.1.0]heptan-7-yl)methanamine | 455.24 |
| 166 | 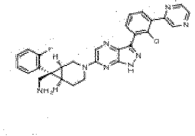 | ((1S,6R,7R)-3-(3-(2-chloro-3-(pyrazin-2-yl)phenyl)-1H-pyrazolo[3,4-b]pyrazin-6-yl)-7-(2-fluorophenyl)-3-azabicyclo[4.1.0]heptan-7-yl)methanamine | 527.12 |
| 167 | 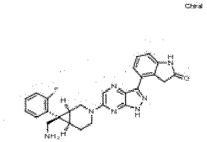 | 4-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)indolin-2-one | 470.21 |
| 168 | 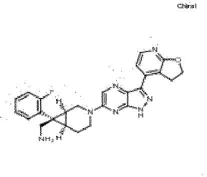 | ((1S,6R,7R)-3-(3-(2,3-dihydrofuro[2,3-b]pyridin-4-yl)-1H-pyrazolo[3,4-b]pyrazin-6-yl)-7-(2-fluorophenyl)-3-azabicyclo[4.1.0]heptan-7-yl)methanamine | 458.26 |
| 169 | 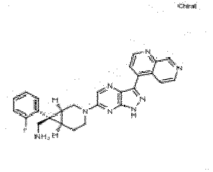 | ((1S,6R,7R)-3-(3-(1,7-naphthyridin-4-yl)-1H-pyrazolo[3,4-b]pyrazin-6-yl)-7-(2-fluorophenyl)-3-azabicyclo[4.1.0]heptan-7-yl)methanamine | 467.21 |
| 170 | 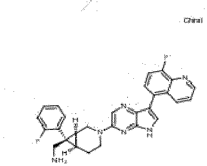 | ((1S,6R,7R)-7-(2-fluorophenyl)-3-(7-(8-fluoroquinolin-5-yl)-5H-pyrrolo[2,3-b]pyrazin-3-yl)-3-azabicyclo[4.1.0]heptan-7-yl)methanamine | 483.31 |
| 171 | 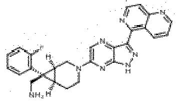 | ((1S,6R,7R)-3-(3-(1,6-naphthyridin-5-yl)-1H-pyrazolo[3,4-b]pyrazin-6-yl)-7-(2-fluorophenyl)-3-azabicyclo[4.1.0]heptan-7-yl)methanamine | 467.21 |
| 172 | 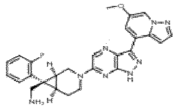 | ((1S,6R,7R)-7-(2-fluorophenyl)-3-(3-(6-methoxypyrazolo[1,5-a]pyridin-4-yl)-1H-pyrazolo[3,4-b]pyrazin-6-yl)-3-azabicyclo[4.1.0]heptan-7-yl)methanamine | 485.22 |
| 173 | 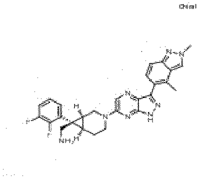 | ((1S,6R,7R)-7-(2,3-difluorophenyl)-3-(3-(2,4-dimethyl-2H-indazol-5-yl)-1H-pyrazolo[3,4-b]pyrazm-6-yl)-3-azabicyclo[4.1.0]heptan-7-yl)methanamine | 501.23 |
| 174 | 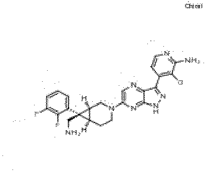 | 4-(6-((1S,6R,7R)-7-(aminomethyl)-7-(2,3-difluorophenyl)-3-azabicyclo[4.1.0]heptan-3-yl)-1H-pyrazolo[3,4-b]pyrazin-3-yl)-3-chloropyridin- 2-amine | 483.16 |
| 175 | 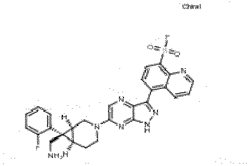 | 5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinoline-8-sulfonyl fluoride | 548.22 |
| 176 | 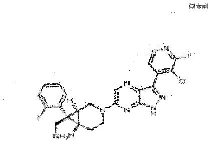 | ((1S,6R,7R)-3-(3-(3-chloro-2-fluoropyridin-4-yl)-1H-pyrazolo[3,4-b]pyrazin-6-yl)-7-(2-fluorophenyl)-3-azabicyclo[4.1.0]heptan-7-yl)methanamine | 468.22 |
| 177 | 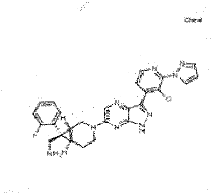 | ((1S,6R,7R)-3-(3-(3-chloro-2-(1H-pyrazol-1-yl)pyridin-4-yl)-1H-pyrazolo[3,4-b]pyrazin-6-yl)-7-(2-fluorophenyl)-3-azabicyclo[4.1.0]heptan-7-yl)methanamine | 516.23 |
| 178 | 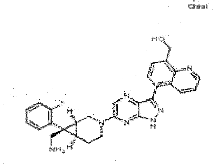 | (5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl)methanol | 496.18 |
| 179 | 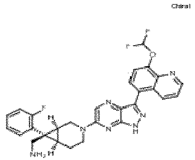 | ((1S,6R,7R)-3-(3-(8-(difluoromethoxy)quinolin-5-yl)-1H-pyrazolo[3,4-b]pyrazin-6-yl)-7-(2-fluorophenyl)-3-azabicyclo[4.1.0]heptan-7-yl)methanamine | 532.09 |
| 180 | 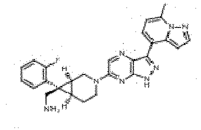 | ((1S,6R,7R)-7-(2-fluorophenyl)-3-(3-(7-methylpyrazolo[ 1,5-a]pyridin-4-yl)-1H-pyrazolo[3,4-b]pyrazin-6-yl)-3-azabicyclo[4.1.0]heptan-7-yl)methanamine | 469.22 |
| 181 | 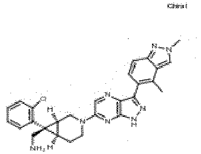 | ((1S,6R,7R)-7-(2-chlorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine | 499.12 |
| 182 | 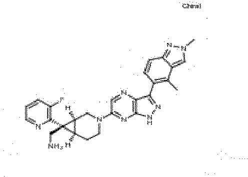 | ((1S,6R,7S)-3-(3-(2,4-dimethyl-2H-indazol-5-yl)-1H-pyrazolo[3,4-b]pyrazin-6-yl)-7-(3-luoropyridin-2-yl)-3-azabicyclo[4.1.0]heptan-7-yl)methanamine | 484.23 |
| 183 | 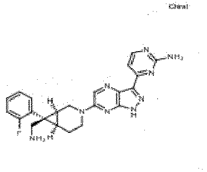 | 4-(6-((1S,6R,7R)-7-(aminomethyl)-7-(2-fluorophenyl)-3-azabicyclo[4.1,0]heptan-3-yl)-1H-pyrazolo[3,4-b]pyrazin-3-yl)pyrimidin-2-amine | 432.23 |
| 184 | 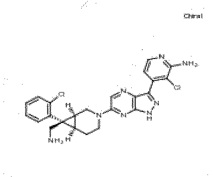 | 4-(6-((1S,6R,7R)-7-(aminomethyl)-7-(2-chlorophenyl)-3-azabicyclo[4.1.0]heptan-3-yl)-1H-pyrazolo[3,4-b]pyrazin-3-yl)-3-chloropyridin- 2-amine | 481.14 |
| 185 | 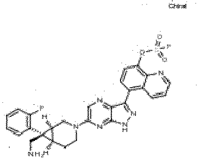 | 5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl sulfurofluoridate | 564.17 |
| 186 | 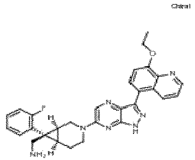 | ((1S,6R,7R)-3-(3-(8-ethoxyquinolin-5-yl)-1H-pyrazolo[3,4-b]pyrazin-6-yl)-7-(2-fluorophenyl)-3-azabicyclo[4.1.0]heptan-7-yl)methanamine | 510.27 |
| 187 | 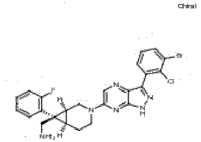 | ((1S,6R,7R)-3-(3-(3-bromo-2-chlorophenyl)-1H-pyrazolo[3,4-b]pyrazin-6-yl)-7-(2-fluorophenyl)-3-azabicyclo[4.1.0]heptan-7-yl)methanamine | 527.07 |
| 188 | 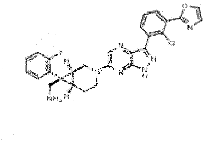 | ((1S,6R,7R)-3-(3-(2-chloro-3-(oxazol-2-yl)phenyl)-1H-pyrazolo[3,4-b]pyrazin-6-yl)-7-(2-fluorophenyl)-3-azabicyclo[4.1.0]heptan-7-yl)methanamine | 516.17 |
| 189 | 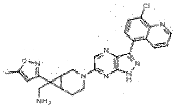 | (3-(3-(8-chloroquinolin-5-yl)-1H-pyrazolo[3,4-b]pyrazin-6-yl)-7-(5-methylisoxazol-3-yl)-3-azabicyclo[4.1.0]heptan-7-yl)methanamine | 487.17 |
| 190 | 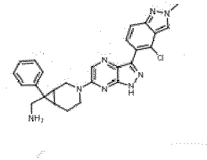 | (3-(3-(4-chloro-2-methyl-2H-indazol-5-yl)-1H-pyrazolo[3,4-b]pyrazin-6-yl)-7-phenyl-3-azabicyclo[4.1.0]heptan-7-yl)methanamine | 485.38 |

Exemplary and non-limiting processes for the synthesis of specific examples are reported hereinbelow.

### Synthesis of Intermediates

### Intermediate 1: tert-butyl tetrahydro-[1,3,2]dioxathiolo[4,5-c]pyridine-5(4H)-carboxylate 2,2-dioxide

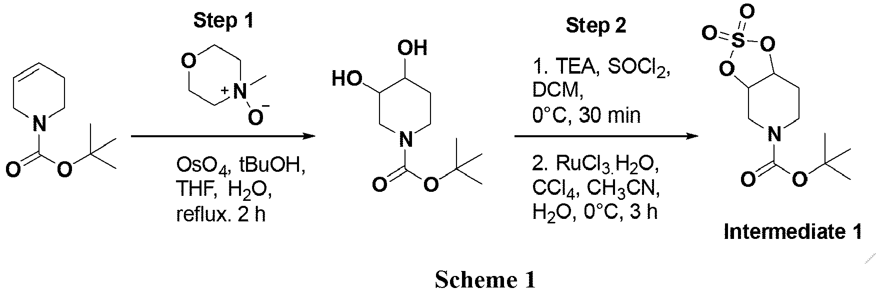

### Step 1: tert-butyl 3,4-dihydroxypiperidine-1-carboxylate

A solution of tert-butyl 3,6-dihydro-2H-pyridine-1-carboxylate (1.55 g, 8.46 mmol) and 4-methylmorpholine 4-oxide (1.49 g, 12.69 mmol) in a mixture of THF (8 mL), *t*-BuOH (5 mL) and H₂O (2 mL) at rt was treated with osmium(VIII) oxide (1.61 mL, 0.250 mmol; 4% in H₂O). The reaction mixture was heated at reflux for 2 h. After cooling the mixture was concentrated *in vacuo* and the residue dissolved in EtOAc. The organic phase was washed with brine, dried over Na₂SO₄, and concentrated under reduced pressure to give a residue which was purified by flash chromatography (gradient elution 0-100% EtOAc in petroleum ether) to afford the title compound as a colourless oil (1.68 g, 91%). LCMS (ES⁺) *m*/*z* 240 (M+Na)⁺, RT 0.95 min.

### Step 2: tert-butyl tetrahydro-[1,3,2]dioxathiolo[4,5-c]pyridine-5(4H)-carboxylate 2,2-dioxide

### (Intermediate 1)

A solution of tert-butyl 3,4-dihydroxypiperidine-1-carboxylate (1.68 g, 7.73 mmol) and TEA (4.31 mL, 30.93 mmol) in DCM (40 mL) at 0°C was treated with SOCl₂ (0.84 mL, 11.6 mmol). The mixture was stirred at this temperature for 30 min then the reaction was quenched with H₂O (1.5 mL). Et₂O and additional H₂O were added, and the two phases were separated. The organic layer was washed with H₂O, NaHCO₃ sat. sol., brine, dried over Na₂SO₄, and concentrated *in vacuo.* The residue was dissolved in a mixture of carbon tetrachloride (22 mL), MeCN (22 mL) and H₂O (33 mL) at 0°C and the solution was treated with trichlororuthenium hydrate (34.8 mg, 0.150 mmol) and sodium periodate (3.31 g, 15.47 mmol). The reaction mixture was stirred at 0°C for 3 h. Et₂O was added to the mixture and the phases were separated. The organic layer was then washed with H₂O, brine, dried over Na₂SO₄, and concentrated *in vacuo* to get a residue which was purified by flash chromatography (gradient elution 0-100% EtOAc in petroleum ether) to afford the title compound as a colourless oil (1.99 g, 92%). ¹H NMR (400 MHz, CDCl₃) δ 5.16-5.13 (m, 1H), 4.93 (br s, 1H), 3.95-3.90 (m, 1H), 3.88-3.82 (m, 1H), 3.62-3.57 (m, 1H), 3.46-3.42 (m, 1H), 2.33-2.27 (m, 1H), 2.09-2.05 (m, 1H), 1.48 (s, 9H). LCMS (ES⁺) *m*/*z* 280 (M+H)⁺, RT 1.55 min.

### Intermediate 2: 7-((((benzyloxy)carbonyl)amino)methyl)-7-(pyridin-3-yl)-3-azabicyclo [4.1.0] heptan-3-ium chloride

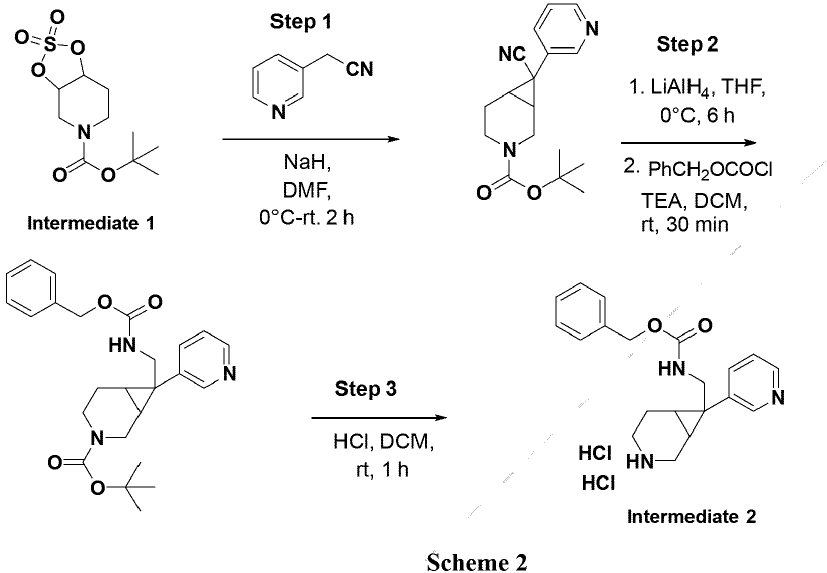

### Step 1: tert-butyl 7-cyano-7-(pyridin-3-yl)-3-azabicyclo[4.1.0]heptane-3-carboxylate

A suspension of sodium hydride (60% in mineral oil; 38.9 mg, 0.970 mmol) in DMF (2 mL) was treated with 2-(3-pyridyl)acetonitrile (69.0 mg, 0.580 mmol) dissolved in DMF (2 mL) at 0 °C. The orange suspension was stirred at 0 °C for 5 min and then treated with a solution of tert-butyl tetrahydro-[1,3,2]dioxathiolo[4,5-c]pyridine-5(4H)-carboxylate 2,2-dioxide (**Intermediate 1**; 136 mg, 0.490 mmol) in DMF (2 mL). The reaction mixture was warmed to rt and stirred for 2 h. Brine was added, and the mixture extracted with EtOAc (3x25 mL). Collected organics were washed with H₂O, brine, dried over Na₂SO₄, and concentrated *in vacuo* to get a residue which was purified by flash chromatography (gradient elution 0-100% EtOAc in petroleum ether) to afford the title compound as a yellow solid (50 mg, 34%). ¹H NMR (400 MHz, CDCl₃) δ 8.55-8.51 (m, 2H), 7.65 (d, *J* = 8.0 Hz, 1H), 7.31-7.28 (m, 1H), 4.10-3.78 (m, 2H), 3.05-2.80 (m, 2H), 2.27 (br s, 1H), 2.10-2.02 (m, 2H), 1.95 (br s, 1H), 1.49 (s, 9H). LCMS (ES⁺) *m*/*z* 300 (M+H)⁺, RT 1.08 min.

### Step 2: tert-butyl 7-((((benzyloxy)carbonyl)amino)methyl)-7-(pyridin-3-yl)-3-azabicyclo[4.1.0]heptane-3-carboxylate

A solution of tert-butyl 7-cyano-7-(pyridin-3-yl)-3-azabicyclo[4.1.0]heptane-3-carboxylate (25 mg, 0.084 mmol) in THF (2 mL) at 0 °C was treated with lithium aluminium hydride (1M in THF; 1.7 mL, 1.7 mmol) and the mixture was stirred at this temperature for 6 h. THF (5 mL) was added to the mixture followed by H₂O (80 uL) and NaOH (15%; 20 uL). The resulting slurry was stirred for 30 minutes at rt, filtered and the filtrate was washed with (DCM:EtOH = 1:1; 10 mL). The mixture was concentrated under reduced pressure and the crude was dissolved in DCM (11 mL) and cooled at 0 °C. TEA (0.12 mL, 0.850 mmol) and benzyl chloroformate (32 uL, 0.230 mmol) were added dropwise and mixture stirred for 30 min. Brine was added and the mixture was extracted with EtOAc (3x15 mL). The collected organics were washed with H₂O, brine, dried over Na₂SO₄, and concentrated *in vacuo* to obtain a residue which was purified by flash chromatography (gradient elution 0-100% EtOAc in petroleum ether) to afford the title compound as a yellow solid (10 mg, 27%). LCMS (ES⁺) *m*/*z* 438 (M+H)⁺, RT 1.44 min.

### Step 3: 7-((((benzyloxy)carbonyl)amino)methyl)-7-(pyridin-3-yl)-3-azabicyclo[4.1.0]heptan-3-ium chloride (Intermediate 2)

A solution of tert-butyl 7-((((benzyloxy)carbonyl)amino)methyl)-7-(pyridin-3-yl)-3-azabicyclo[4.1.0]heptane-3-carboxylate (10 mg, 0.020 mmol) in DCM (0.5 mL) was treated with HCl (371 uL, 1.49 mmol; 4M in 1,4-dioxane) and the solution was stirred at rt for 1 h. The mixture was concentrated under reduced pressure and the residue used as a crude. LCMS (ES⁺) *m*/*z* 338 (M+H)⁺, RT 0.26 min.

**Intermediate 2** was used for the synthesis of **Example 2.**

### Intermediate 3: 7-((((benzyloxy)carbonyl)amino)methyl)-7-(2-fluorophenyl)-3-azabicyclo [4.1.0] heptan-3-ium chloride

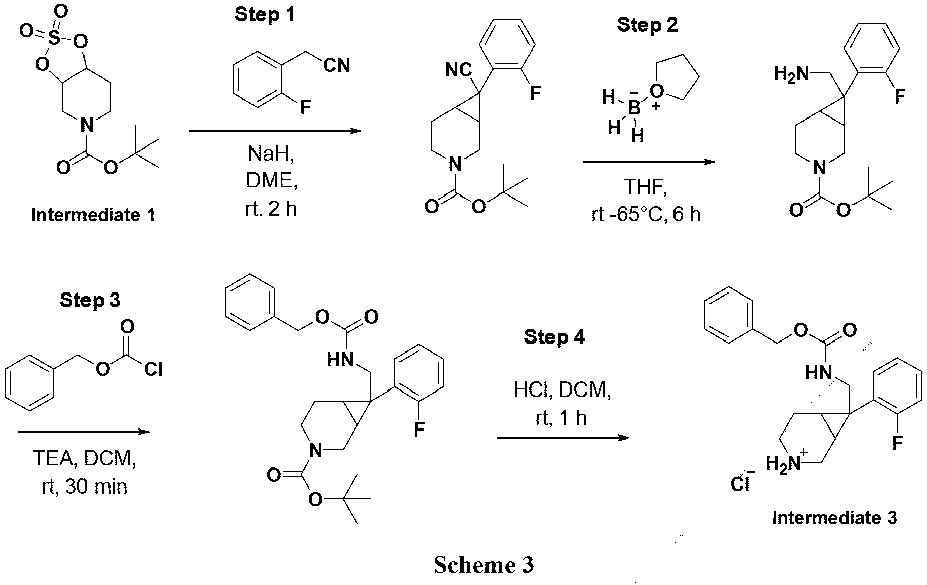

### Step 1: tert-butyl 7-cyano-7-(2-fluorophenyl)-3-azabicyclo[4.1.0]heptane-3-carboxylate

A suspension of sodium hydride (60% in mineral oil; 156.4 mg, 3.91 mmol) in DME (4.5 mL) was treated with a solution of 15-crown-5 (33.1 mg, 0.150 mmol), 2-(2-fluorophenyl)acetonitrile (223.5 mg, 1.65 mmol) and tert-butyl tetrahydro-[1,3,2]dioxathiolo[4,5-c]pyridine-5(4H)-carboxylate 2,2-dioxide (**Intermediate 1**; 420 mg, 1.5 mmol) in DME (4.5 mL) dropwise in 30 min. The resulting orange mixture was stirred at rt for 2 h. H₂O (2 mL) was added and the mixture was extracted with EtOAc (3x35 mL). The collected organics layers were washed with brine, dried over Na₂SO₄, and concentrated *in vacuo* to get a residue which was purified by flash chromatography (gradient elution 0-40% EtOAc in petroleum ether) to afford the title compound as a white solid (290 mg, 61%). ¹H NMR (400 MHz, CDCl₃) δ 7.35-7.26 (m, 2H), 7.15-7.06 (m, 2H), 4.20 (d, *J* = 16.0 Hz, 1H), 3.87-3.72 (m, 2H), 3.01-2.92 (m, 1H), 2.24 (br s, 1H), 2.13-2.09 (m, 1H), 1.98-1.79 (m, 2H), 1.49 (s, 9H). LCMS (ES⁺) *m*/*z* 339 (M+Na)⁺, RT 1.92 min.

### Step 2: tert-butyl 7-(aminomethyl)-7-(2-fluorophenyl)-3-azabicyclo[4.1.0]heptane-3-carboxylate

A solution of tert-butyl 7-cyano-7-(2-fluorophenyl)-3-azabicyclo[4.1.0]heptane-3-carboxylate (120 mg, 0.380 mmol) in THF (3 mL) was treated with borane tetrahydrofuran complex (2.84 mL, 2.84 mmol; 1M in THF) and the resulting mixture was stirred at 65°C for 6 h. After cooling, EtOH (4 mL) was added and mixture stirred at reflux for 4 h. The volatiles were removed by reduced pressure and the title compound was obtained as a crude and used without further purification. LCMS (ES⁺) *m*/*z* 321 (M+H)⁺, RT 1.33 min.

### Step 3: tert-butyl 7-((((benzyloxy)carbonyl)amino)methyl)-7-(2-fluorophenyl)-3-azabicyclo[4.1.0]heptane-3-carboxylate

A solution of tert-butyl 7-(aminomethyl)-7-(2-fluorophenyl)-3-azabicyclo[4.1.0]heptane-3-carboxylate (139 mg, 0.430 mmol) in DCM (5 mL) was treated at 0 °C with TEA (0.18 mL, 1.3 mmol) and benzyl chloroformate (55.7 uL, 0.390 mmol) and mixture was warmed to rt and stirred for 30 min. Brine was added and the mixture was extracted with EtOAc (2x35 mL). The collected organics were washed with H₂O, brine, dried over Na₂SO₄, and concentrated *in vacuo* to get a residue which was purified by flash chromatography (gradient elution 0-100% EtOAc in petroleum ether) to afford the title compound as a colourless oil (70 mg, 35%). LCMS (ES⁺) *m*/*z* 477 (M+Na)⁺, RT 2.28 min.

### Step 4: 7-((((benzyloxy)carbonyl)amino)methyl)-7-(2-fluorophenyl)-3-azabicyclo[4.1.0]heptan-3-ium chloride (Intermediate 3)

A solution of tert-butyl 7-((((benzyloxy)carbonyl)amino)methyl)-7-(2-fluorophenyl)-3-azabicyclo[4.1.0]heptane-3-carboxylate (25 mg, 0.060 mmol) in DCM (1 mL) was treated with HCl (0.89 mL, 3.58 mmol; 4M in 1,4-dioxane) and the solution was stirred at rt for 1 h. Mixture was concentrated under reduced pressure and the residue used as a crude. LCMS (ES⁺) *m*/*z* 355 (M+H)⁺, RT 1.28 min.

### Intermediate 3 was used for the synthesis of Example 3, Example 4, Example 5, Example 16, Example 59

### Intermediate 4: 7-((((benzyloxy)carbonyl)amino)methyl)-7-(3-methylisoxazol-5-yl)-3-azabicyclo[4.1.0] heptan-3-ium chloride

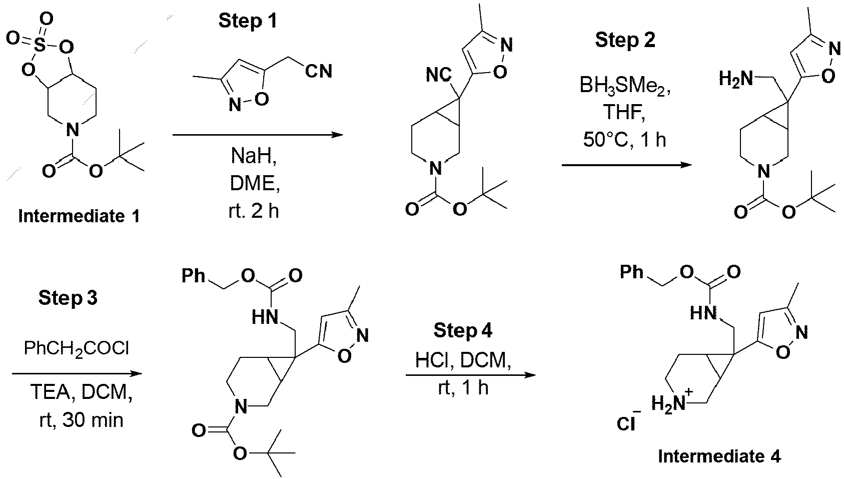

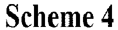

### Step 1: tert-butyl 7-cyano-7-(3-methylisoxazol-5-yl)-3-azabicyclo[4.1.0]heptane-3-carboxylate

A suspension of sodium hydride (60% in mineral oil; 111.7 mg, 2.79 mmol) in DME (3.5 mL) was treated with a solution of tert-butyl tetrahydro-[1,3,2]dioxathiolo[4,5-c]pyridine-5(4H)-carboxylate 2,2-dioxide (**Intermediate 1**; 300 mg, 1.07 mmol), 2-(3-methyl-1,2-oxazol-5-yl)ethanenitrile (144.3 mg, 1.18 mmol) and 15-crown-5 (23.7 mg, 0.110 mmol) in DME (3.5 mL) dropwise in 30 min. The resulting orange mixture was stirred at rt for 2 h. H₂O (2 mL) was added and the mixture was extracted with EtOAc (3x35 mL). The collected organics were washed with brine, dried over Na₂SO₄ and concentrated *in vacuo* to get a residue which was purified by flash chromatography (gradient elution 0-50% EtOAc in petroleum ether) to afford the title compound as a yellow oil (190 mg, 58%). ¹H NMR (400 MHz, CDCl₃) δ 6.21 (s, 1H), 4.26 (d, *J* = 16.0 Hz, 1H), 4.09-3.82 (m, 1H), 3.64 (dd, *J₁* = 4.0 Hz, *J₂* = 16.0 Hz, 1H), 2.91-2.75 (m, 1H), 2.38-2.35 (m, 1H), 2.29-2.15 (m, 2H), 2.28 (s, 3H), 2.05-1.98 (m, 1H), 1.48 (s, 9H). LCMS (ES⁺) *m*/*z* 326 (M+Na)⁺, RT 1.67 min.

### Step 2: tert-butyl 7-(aminomethyl)-7-(3-methylisoxazol-5-yl)-3-azabicyclo[4.1.0]heptane-3-carboxylate

A solution of *tert*-butyl 7-cyano-7-(3-methylisoxazol-5-yl)-3-azabicyclo[4.1.0]heptane-3-carboxylate (120 mg, 0.400 mmol) in THF (7 mL) was treated with borane methylsulfanylmethane (2 M in THF; 132 uL, 1.38 mmol) and the resulting mixture was stirred at 50 °C for 1 h. After cooling, EtOH (4 mL) was added and the mixture was stirred at reflux for 4 h. The volatiles were removed by reduced pressure and the title compound was obtained as a crude and used without further purification. LCMS (ES⁺) *m*/*z* 308 (M+H)⁺, RT 1.11 min.

### Step 3: tert-butyl 7-((((benzyloxy)carbonyl)amino)methyl)-7-(3-methylisoxazol-5-yl)-3-azabicyclo[4.1.0]heptane-3-carboxylate

A solution of tert-butyl 7-(aminomethyl)-7-(3-methylisoxazol-5-yl)-3-azabicyclo[4.1.0]heptane-3-carboxylate (120 mg, 0.390 mmol) in DCM (4 mL), cooled at 0 °C, was treated with TEA (0.16 mL, 1.17 mmol) and benzyl chloroformate (50 uL, 0.350 mmol) and the mixture was stirred at rt for 30 min. Brine was added and the mixture was extracted with EtOAc (2x35 mL). The collected organics were washed with H₂O, brine, dried over Na₂SO₄ and concentrated *in vacuo* to get a residue which was purified by flash chromatography (gradient elution 0-60% EtOAc in petroleum ether) to afford the title compound as a colourless oil (25 mg, 15%). LCMS (ES⁺) *m*/*z* 464 (M+Na)⁺, RT 1.99 min.

### Step 4: 7-((((benzyloxy)carbonyl)amino)methyl)-7-(3-methylisoxazol-5-yl)-3-azabicyclo[4.1.0]heptan-3-ium chloride (Intermediate 4)

A solution of tert-butyl 7-((((benzyloxy)carbonyl)amino)methyl)-7-(3-methylisoxazol-5-yl)-3-azabicyclo[4.1.0]heptane-3-carboxylate (25 mg, 0.060 mmol) in DCM (0.8 mL) was treated with HCl (4M in 1,4-dioxane; 0.92 mL, 3.68 mmol) and the solution was stirred at rt for 1 h. The mixture was concentrated under reduced pressure to afford the title compound as a white solid which was used as a crude. LCMS (ES⁺) *m*/*z* 342 (M+H)⁺, RT 1.12 min.

**Intermediate 4** was used for the synthesis of **Example 6.**

### Intermediate 5: 7-((((benzyloxy)carbonyl)amino)methyl)-7-(3-fluorophenyl)-3-azabicyclo[4.1.0] heptan-3-ium chloride

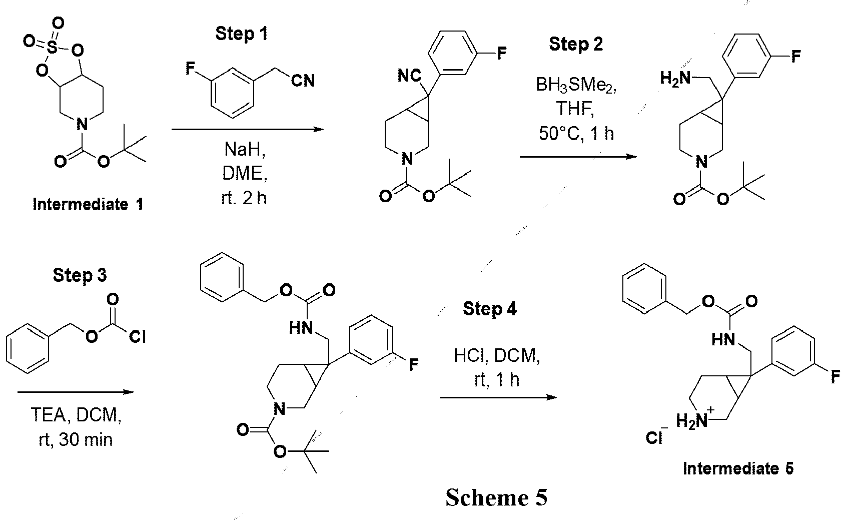

### Step 1: tert-butyl 7-cyano-7-(3-fluorophenyl)-3-azabicyclo[4.1.0]heptane-3-carboxylate

A solution of sodium hydride (60% in mineral oil; 111.7 mg, 2.79 mmol) in DME (3 mL) was treated with a solution of 2-(3-fluorophenyl)acetonitrile (145.2 mg, 1.07 mmol), 15-crown-5 (23.7 mg, 0.110 mmol) and tert-butyl tetrahydro-[1,3,2]dioxathiolo[4,5-c]pyridine-5(4H)-carboxylate 2,2-dioxide **(Intermediate 1;** 300 mg, 1.07 mmol) in DME (2 mL) and the reaction mixture was stirred at rt for 2 h. H₂O was added and the mixture was extracted with EtOAc. The organic phase was washed with brine, dried over Na₂SO₄, and concentrated *in vacuo* to get a residue which was purified by flash chromatography (gradient elution 0-50% EtOAc in petroleum ether) to afford the title compound as a colorless oil (190 mg, 56%). ¹ H NMR (400 MHz, CDCl₃) δ 7.36-7.30 (m, 1H), 7.10 (d, *J* = 8.0 Hz, 1H), 7.01-6.96 (m, 2H), 4.17 (d, *J* = 12.0 Hz, 1H), 4.01-3.74 (m, 2H), 3.00-2.85 (m, 1H), 2.29-2.22 (m, 1H), 2.10-1.89 (m, 2H), 1.66 (br s, 1H), 1.51 (s, 9H). LCMS (ES⁺) *m*/*z* 339 (M+Na)⁺, RT 2.0 min.

### Step 2: tert-butyl 7-(aminomethyl)-7-(3-fluorophenyl)-3-azabicyclo[4.1.0]heptane-3-carboxylate

A solution of tert-butyl 7-cyano-7-(3-fluorophenyl)-3-azabicyclo[4.1.0]heptane-3-carboxylate (190 mg, 0.600 mmol) in THF (12 mL) was treated with borane methylsulfanylmethane (2M in THF; 285 uL, 3 mmol) and the resulting mixture was stirred at 65 °C for 30 min. After cooling EtOH (4 mL) was added and the mixture stirred at reflux for 4 h. After cooling, the volatiles were removed by reduced pressure and the title compound was obtained as a colorless oil (192 mg, 99%) and used without further purification. LCMS (ES ⁺) *m*/*z* 321 (M+H)⁺, RT 1.15 min.

### Step 3: tert-butyl 7-((((benzyloxy)carbonyl)amino)methyl)-7-(3-fluorophenyl)-3-azabicyclo[4.1.0]heptane-3-carboxylate

A solution of tert-butyl 7-(aminomethyl)-7-(3-fluorophenyl)-3-azabicyclo[4.1.0]heptane-3-carboxylate (192 mg, 0.600 mmol) in DCM (5 mL) cooled at 0°C was treated with TEA (0.25 mL, 1.8 mmol) and benzyl chloroformate (68 uL, 0.480 mmol) and the mixture was stirred at rt for 45 min. Brine was added and the mixture was extracted with EtOAc (2x35 mL). The collected organics were washed with H₂O, brine, dried over Na₂SO₄, and concentrated *in vacuo* to get a residue which was purified by flash chromatography (gradient elution 0-100% EtOAc in petroleum ether) to afford the title compound as a colorless oil (112 mg, 41%). LCMS (ES⁺) *m*/*z* 477 (M+Na)⁺, RT 2.30 min.

### Step 4: 7-((((benzyloxy)carbonyl)amino)methyl)-7-(3-fluorophenyl)-3-azabicyclo[4.1.0]heptan-3-ium chloride (Intermediate 5)

A solution of tert-butyl 7-((((benzyloxy)carbonyl)amino)methyl)-7-(3-fluorophenyl)-3-azabicyclo[4.1.0]heptane-3-carboxylate (112 mg, 0.250 mmol) in DCM (4.0 mL) was treated with HCl (4M in 1,4-dioxane; 4.0 mL) and the solution stirred at rt for 2 h. The mixture was concentrated under reduced pressure to afford the title compound as a white solid which was used as a crude. LCMS (ES⁺) *m*/*z* 355 (M+H)⁺, RT 1.14 min.

**Intermediate 5** was used for the synthesis of **Example 7, Example 55** and **Example 56.**

### Intermediate 6: 7-((((benzyloxy)carbonyl)amino)methyl)-7-(5-methylisoxazol-3-yl)-3-azabicyclo[4.1.0]heptan-3-ium chloride

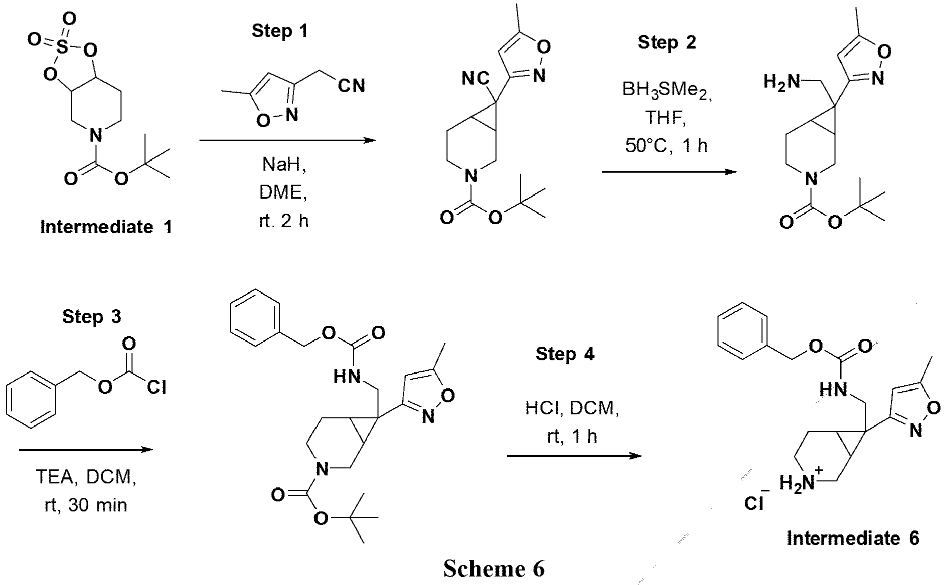

### Step 1: tert-butyl 7-cyano-7-(5-methylisoxazol-3-yl)-3-azabicyclo[4.1.0]heptane-3-carboxylate

A solution of sodium hydride (60% in mineral oil; 111.7 mg, 2.79 mmol) in DME (3.5 mL) was treated with a solution of *tert*-butyl tetrahydro-[1,3,2]dioxathiolo[4,5-c]pyridine-5(4H)-carboxylate 2,2-dioxide (**Intermediate 1;** 300 mg, 1.07 mmol), 2-(5-methyl-1,2-oxazol-3-yl)ethanenitrile (144.3 mg, 1.18 mmol) and 15-crown-5 (23.7 mg, 0.110 mmol) in DME (3.5 mL) and the reaction mixture was stirred at rt for 2 h. H₂O (2 mL) was added and the mixture was extracted with EtOAc (3x35 mL). The collected organics were washed with brine, dried over Na₂SO₄ and concentrated *in vacuo* to get a residue which was purified by flash chromatography (gradient elution 0-50% EtOAc in petroleum ether) to afford the title compound as a yellow oil (177 mg, 54%). ¹ H NMR (400 MHz, CDCl₃) δ 6.18 (s, 1H), 4.23 (d, *J* = 16.0 Hz, 1H), 3.98-3.80 (m, 1H), 3.66 (dd, *J₁* = 4.0 Hz, *J₂* = 16.0 Hz, 1H), 2.93-2.79 (m, 1H), 2.41 (s, 3H), 2.30-2.12 (m, 3H), 2.03-1.98 (m, 1H), 1.48 (s, 9H). LCMS (ES⁺) *m*/*z* 326 (M+Na)⁺, RT 1.71 min.

### Step 2: tert-butyl 7-(aminomethyl)-7-(5-methylisoxazol-3-yl)-3-azabicyclo[4.1.0]heptane-3-carboxylate

A solution of tert-butyl 7-cyano-7-(5-methylisoxazol-3-yl)-3-azabicyclo[4.1.0]heptane-3-carboxylate (177 mg, 0.580 mmol) in THF (7 mL) was treated with borane methylsulfanylmethane (2M in THF; 194 uL, 2.04 mmol) and the resulting mixture was stirred at 50 °C for 1 h. After cooling, EtOH (4 mL) was added and mixture stirred at reflux for 4 h. The volatiles were removed by reduced pressure and the title compound was used without further purification. LCMS (ES⁺) *m*/*z* 308 (M+H)⁺, RT 1.15 min.

### Step 3: tert-butyl 7-((((benzyloxy)carbonyl)amino)methyl)-7-(5-methylisoxazol-3-yl)-3-azabicyclo[4.1.0]heptane-3-carboxylate

A solution of tert-butyl 7-(aminomethyl)-7-(5-methylisoxazol-3-yl)-3-azabicyclo[4.1.0]heptane-3-carboxylate (188 mg, 0.610 mmol) in DCM (6 mL) cooled at 0°C was treated with TEA (0.26 mL, 1.83 mmol) and benzyl chloroformate (79 uL, 0.550 mmol) and mixture was stirred at rt for 30 min. Brine was added and mixture extracted with EtOAc (2x35 mL). The collected organics were washed with H₂O, brine, dried over Na₂SO₄, and concentrated *in vacuo* to get a residue which was purified by flash chromatography (gradient elution 0-60% EtOAc in petroleum ether) to afford the title compound as a colorless oil (57 mg, 21%). LCMS (ES⁺) *m*/*z* 464 (M+Na)⁺, RT 2.05 min.

### Step 4: 7-((((benzyloxy)carbonyl)amino)methyl)-7-(5-methylisoxazol-3-yl)-3-azabicyclo[4.1.0]heptan-3-ium chloride (Intermediate 6)

A solution of tert-butyl 7-((((benzyloxy)carbonyl)amino)methyl)-7-(5-methylisoxazol-3-yl)-3-azabicyclo[4.1.0]heptane-3-carboxylate (31 mg, 0.070 mmol) in DCM (1 mL) was treated with HCl (4M in 1,4-dioxane; 1.14 mL, 4.56 mmol) and the solution was stirred at rt for 1 h. The mixture was concentrated under reduced pressure to afford the title compound as a white solid which was used as a crude. LCMS (ES⁺) *m*/*z* 342 (M+H)⁺, RT 1.20 min.

### Intermediate 6 was used for the synthesis of Example 8, Example 9, Example 17, Example 25, Example 26, Example 27, Example 28, Example 29, Example 30, Example 31, Example 32, Example 33, Example 34, Example 35, Example 36, Example 53, Example 54, Example 60, Example 62.

### Intermediate 7: 7-((((benzyloxy)carbonyl)amino)methyl)-7-(4-fluorophenyl)-3-azabicyclo[4.1.0]heptan-3-ium chloride

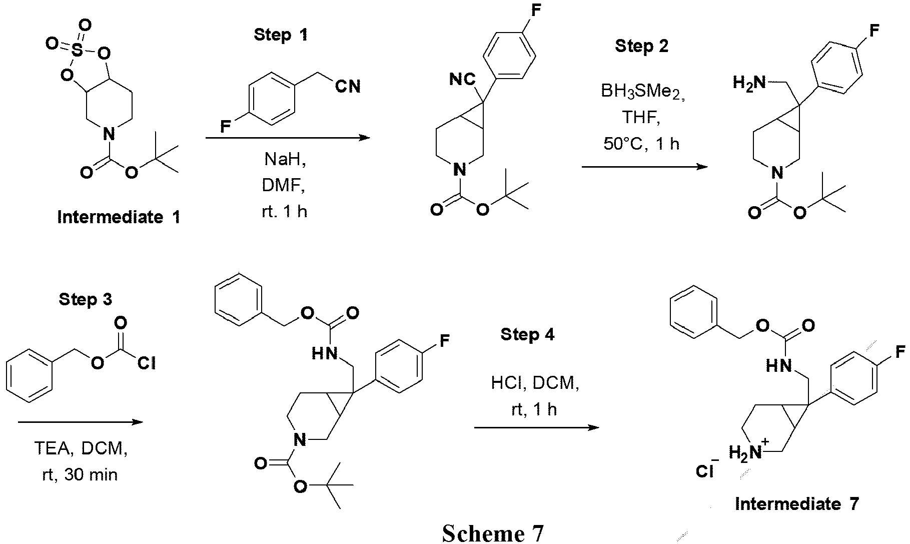

### Step 1: tert-butyl 7-cyano-7-(4-fluorophenyl)-3-azabicyclo[4.1.0]heptane-3-carboxylate

A suspension of sodium hydride (60% in mineral oil; 93 mg, 2.33 mmol) in DMF (2.5 mL) cooled at 0°C was treated with 2-(4-fluorophenyl)acetonitrile (118 uL, 0.980 mmol) in DMF (2.5 mL). A solution of tert-butyl tetrahydro-[1,3,2]dioxathiolo[4,5-c]pyridine-5(4H)-carboxylate 2,2-dioxide **(Intermediate 1;** 250 mg, 0.900 mmol) in DMF (2.5 mL) was then added to the suspension and the resulting orange mixture was stirred at rt for 1 h. H₂O (4 mL) was added and the mixture was extracted with EtOAc (3x35 mL). The collected organics were washed with brine, dried over Na₂SO₄, and concentrated *in vacuo* to get a residue which was purified by flash chromatography (gradient elution 0-40% EtOAc in petroleum ether) to afford the title compound as a yellow oil (135 mg, 48%). ¹ H NMR (400 MHz, CDCl₃) δ 7.27-7.25 (m, 2H), 7.06-7.02 (m, 2H), 4.17-4.12 (m, 1H), 3.95-3.72 (m, 2H), 3.00-2.86 (m, 1H), 2.24 (br s, 1H), 2.07-2.04 (m, 1H), 1.95-1.83 (m, 2H), 1.49 (s, 9H). LCMS (ES⁺) *m*/*z* 339 (M+Na)⁺, RT 2.05 min.

### Step 2: tert-butyl 7-(aminomethyl)-7-(4-fluorophenyl)-3-azabicyclo[4.1.0]heplane-3-carboxylate

A solution of tert-butyl 7-cyano-7-(4-fluorophenyl)-3-azabicyclo[4.1.0]heptane-3-carboxylate (135 mg, 0.430 mmol) in THF (5.5 mL) was treated with borane methylsulfanylmethane (2M in THF; 142 uL, 1.49 mmol) and the resulting mixture was stirred at 50°C for 1 h. After cooling, EtOH (4 mL) was added and the mixture was stirred at reflux for 4 h. The volatiles were removed by reduced pressure and the title compound was used without further purification. LCMS (ES ⁺) *m*/*z* 321 (M+H)⁺, RT 1.35 min.

### Step 3: tert-butyl 7-((((benzyloxy)carbonyl)amino)methyl)-7-(4-fluorophenyl)-3-azabicyclo[4.1.0]heptane-3-carboxylate

A solution of tert-butyl 7-(aminomethyl)-7-(4-fluorophenyl)-3-azabicyclo[4.1.0]heptane-3-carboxylate (145 mg, 0.450 mmol) in DCM (6 mL) cooled at 0°C was treated with TEA (189 uL, 1.36 mmol) and benzyl chloroformate (58 uL, 0.410 mmol) were added and the mixture was stirred at rt for 30 min. Brine was added and the mixture was extracted with EtOAc (2x35 mL). The collected organics were washed with H₂O, brine, dried over Na₂SO₄ and concentrated *in vacuo* to get a residue which was purified by flash chromatography (gradient elution 0-60% EtOAc in petroleum ether) to afford the title compound as a colorless oil (38 mg, 18%). LCMS (ES⁺) *m*/*z* 477 (M+Na)⁺, RT 2.27 min.

### Step 4: 7-((((benzyloxy)carbonyl)amino)methyl)-7-(4-fluorophenyl)-3-azabicyclo[4.1.0]heptan-3-ium chloride (Intermediate 7)

A solution of tert-butyl 7-((((benzyloxy)carbonyl)amino)methyl)-7-(4-fluorophenyl)-3-azabicyclo[4.1.0]heptane-3-carboxylate (38 mg, 0.090 mmol) in DCM (0.8 mL) was treated with HCl (4M in 1,4-dioxane; 1.39 mL, 5.58 mmol) and the solution was stirred at rt for 1 h. The mixture was concentrated under reduced pressure to afford the title compound as a white solid which was used as a crude. LCMS (ES⁺) *m*/*z* 355 (M+H)⁺, RT 1.37 min.

**Intermediate 7** was used for the synthesis of **Example 10, Example 11 and Example 12.**

### Intermediate 8: benzyl tetrahydro-[1,3,2]dioxathiolo[4,5-c]pyridine-5(4H)-carboxylate 2,2-dioxide

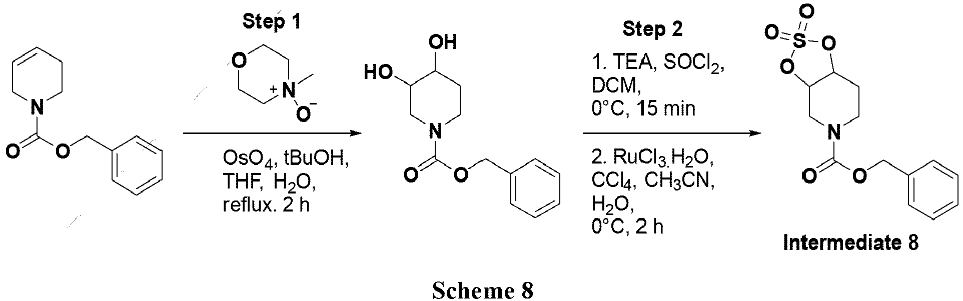

### Step 1: benzyl 3,4-dihydroxypiperidine-1-carboxylate

A solution of benzyl 3,6-dihydropyridine-1(2H)-carboxylate (2 g, 9.21 mmol) and 4-methylmorpholine 4-oxide (1.62 g, 13.81 mmol) in a mixture of THF (10 mL), tert-butanol (6 mL) and H₂O (2.5 mL) at rt was treated with osmium(VIII) oxide (4% in H₂O; 1.76 mL, 0.280 mmol). The reaction mixture was heated at reflux for 2 h and then concentrated *in vacuo* and the residue was taken up in EtOAc. The organic phase was washed with brine, dried over Na₂SO₄, and concentrated under reduced pressure to give a residue which was purified by flash chromatography (gradient elution 0-100% EtOAc in petroleum ether) to afford the title compound as a colorless oil (2.31 g, 100%). ¹H NMR (400 MHz, CDCl₃) δ 7.39-7.33 (m, 5H), 5.14 (s, 2H), 3.90-3.87 (m, 1H), 3.79 (br s, 1H), 3.69-3.63 (m, 2H), 3.52-3.49 (m, 1H), 3.37-3.33 (m, 1H), 2.21 (br s, 2H), 1.89-1.82 (m, 1H), 1.71 (br s, 1H). LCMS (ES⁺) *m*/*z* 274 (M+Na)⁺, RT 1.08 min.

### Step 2: benzyl tetrahydro-[1,3,2]dioxathiolo[4,5-c]pyridine-5(4H)-carboxylate 2,2-dioxide (Intermediate 8)

A solution of benzyl 3,4-dihydroxypiperidine-1-carboxylate (2.31 g, 9.35 mmol) and TEA (5.21 mL, 37.41 mmol) in DCM (48 mL) at 0°C was treated with thionyl chloride (1.02 mL, 14.03 mmol). The mixture was stirred at this temperature for 15 min then the reaction was quenched with H₂O (5 mL). Et₂O and additional H₂O were added, and the phases were separated. The organic layer was washed with H₂O, NaHCO₃ sat. sol., brine, dried over Na₂SO₄, and concentrated *in vacuo.* The residue was dissolved in a mixture of carbon tetrachloride (27 mL), MeCN (27 mL) and H₂O (45 mL) at 0°C and the solution was treated with trichlororuthenium hydrate (42.1 mg, 0.190 mmol) and sodium periodate (4 g, 18.7 mmol). The reaction mixture was stirred at 0°C for 2 h. Et₂O was added to the mixture and the two phases were separated. The organic layer was then washed with H₂O, brine, dried over Na₂SO₄ and concentrated *in vacuo* to get a residue which was purified by flash chromatography (gradient elution 0-60% EtOAc in petroleum ether) to afford the title compound as a pale yellow oil (2.7 g, 92%). ¹H NMR (400 MHz, CDCl₃) δ 7.41-7.35 (m, 5H), 5.20-5.13 (m, 1H), 5.16 (s, 2H), 4.94 (br s, 1H), 4.08-3.99 (m, 1H), 3.93-3.89 (m, 1H), 3.72-3.66 (m, 1H), 3.53-3.48 (m, 1H), 2.33-2.30 (m, 1H), 2.08 (br s, 1H). LCMS (ES⁺) *m*/*z* 314 (M+H)⁺, RT 1.60 min.

### Intermediate 9: tert-butyl ((7-(1-methyl-1H-1,2,3-triazol-4-yl)-3-azabicyclo[4.1.0]heptan-7-yl)methyl)carbamate

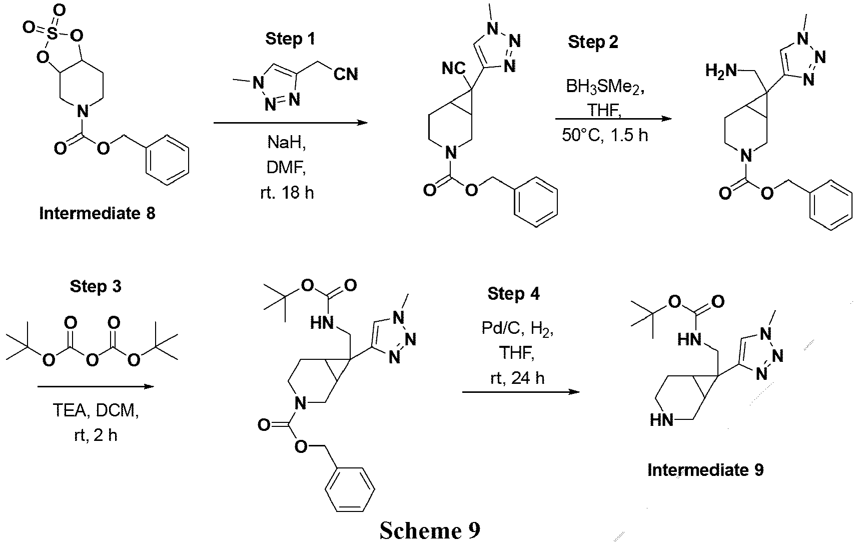

### Step 1: benzyl 7-cyano-7-(1-methyl-1H-1,2,3-triazol-4-yl)-3-azabicyclo[4.1.0]heptane-3-carboxylate

A suspension of sodium hydride (60% in mineral oil; 83 mg, 2.07 mmol) in DMF (2.0 mL) cooled at 0 °C was treated with 2-(1-methyl-1H-1,2,3-triazol-4-yl)acetonitrile (107.2 mg, 0.880 mmol) in DMF (2.0 mL). A solution of benzyl tetrahydro-[1,3,2]dioxathiolo[4,5-c]pyridine-5(4H)-carboxylate 2,2-dioxide **(Intermediate 8;** 250 mg, 0.800 mmol) in DMF (2.0 mL) was then added to the suspension and the resulting orange mixture was stirred at rt for 18 h. H₂O (4 mL) was added and the mixture extracted with EtOAc (3x35 mL). The collected organics were washed with brine, dried over Na₂SO₄ and concentrated *in vacuo* to get a residue which was purified by flash chromatography (gradient elution 0-100% EtOAc in petroleum ether) to afford the title compound as a yellow oil (75 mg, 28%). ¹ H NMR (400 MHz, CDCl₃) δ 7.65 (s, 1H), 7.39-7.33 (m, 5H), 5.21-5.10 (m, 2H), 4.32 (d, *J*=16.0 Hz, 1H), 4.08 (s, 3H), 4.08-4.00 (m, 1H), 3.75 (dd, *J₁*=4 Hz, *J₂*=16.0 Hz, 1H), 2.38-2.26 (m, 3H), 2.10-2.04 (m, 1H), 1.71 (br s, 1H). LCMS (ES⁺) *m*/*z* 360 (M+Na)⁺, RT 1.43 min.

### Step 2: benzyl 7-(aminomethyl)-7-(1-methyl-1H-1,2,3-triazol-4-yl)-3-azabicyclo[4.1.0]heptane-3-carboxylate

A solution of benzyl 7-cyano-7-(1-methyl-1H-1,2,3-triazol-4-yl)-3-azabicyclo[4.1.0]heptane-3-carboxylate (75 mg, 0.220 mmol) in THF (2.5 mL) was treated with borane methylsulfanylmethane (2M in THF; 389 uL, 0.780 mmol) and the resulting mixture was stirred at 50°C for 1.5 h. After cooling, EtOH (4 mL) was added and the mixture stirred at reflux for 2 h. After cooling, the solvent was then removed by reduced pressure and the title compound was used without further purification. LCMS (ES ⁺) *m*/*z* 342 (M+H)⁺, RT 1.07 min.

### Step 3: benzyl 7-(((tert-butoxycarbonyl)amino)methyl)-7-(1-methyl-1H-1,2,3-triazol-4-yl)-3-azabicyclo[4.1.0]heptane-3-carboxylate

A solution of benzyl 7-(aminomethyl)-7-(1-methyl-1H-1,2,3-triazol-4-yl)-3-azabicyclo[4.1.0]heptane-3-carboxylate (89 mg, 0.260 mmol) and TEA (50.9 uL, 0.360 mmol) in DCM (2.5 mL) was treated with tert-butoxycarbonyl tert-butyl carbonate (62.6 mg, 0.290 mmol) and the mixture was stirred at rt for 2 h. H₂O (3 mL) was added and the mixture extracted with EtOAc (2x35 mL). The collected organics were washed with H₂O, brine, dried over Na₂SO₄, and concentrated *in vacuo* to get a residue which was purified by flash chromatography (gradient elution 0-100% EtOAc in petroleum ether) to afford the title compound as a colorless oil (53 mg, 46%). LCMS (ES⁺) *m*/*z* 464 (M+Na)⁺, RT 1.72 min.

### Step 4: tert-butyl ((7-(1-methyl-1H-1,2,3-triazol-4-yl)-3-azabicyclo[4.1.0]heptan-7-yl)methyl)carbamate (Intermediate 9)

A solution of benzyl 7-(((tert-butoxycarbonyl)amino)methyl)-7-(1-methyl-1H-1,2,3-triazol-4-yl)-3-azabicyclo[4.1.0]heptane-3-carboxylate (53 mg, 0.120 mmol) in THF (3 mL) was treated with Pd/C (20 mg, 0.120 mmol) and the resulting suspension was stirred under H₂ atmosphere at rt for 24 h. After nitrogen fluxing to remove hydrogen, the catalyst was filtered on a pad of solka floc which was washed with MeOH. After volatiles removal under reduced pressure, the title compound was obtained as a colourless oil (26 mg, 70%) and used as crude. LCMS (ES⁺) *m*/*z* 308 (M+H)⁺, RT 0.70 min.

**Intermediate 9** was used for the synthesis of **Example 13.**

### Intermediate 10: tert-butyl ((7-(4-methylisoxazol-3-yl)-3-azabicyclo[4.1.0]heptan-7-yl)methyl)carbamate

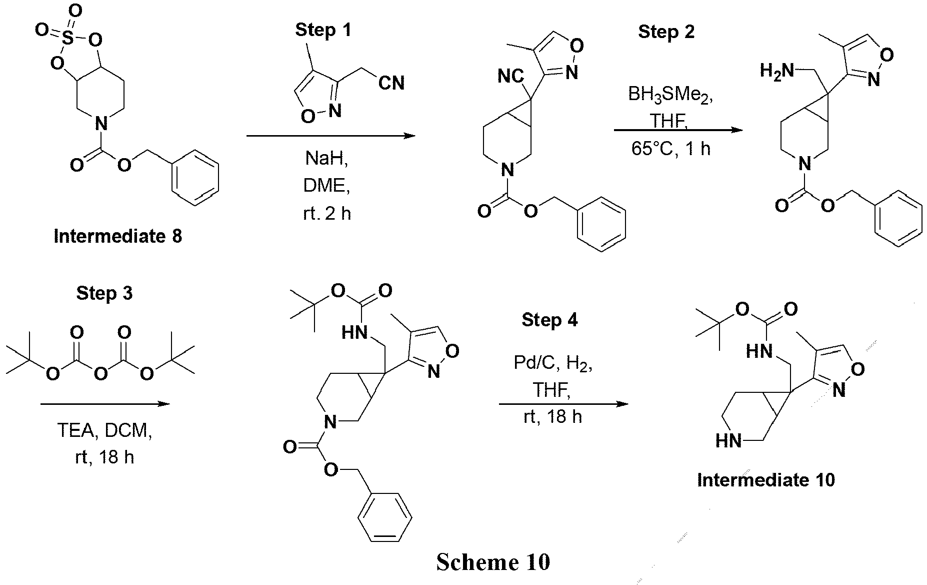

### Step 1: benzyl 7-cyano-7-(4-methylisoxazol-3-yl)-3-azabicyclo[4.1.0]heptane-3-carboxylate

A suspension of sodium hydride (60% in mineral oil; 99.6 mg, 2.49 mmol) in DME (3.2 mL) cooled at 0 °C was treated with 2-(4-methyl-1,2-oxazol-3-yl)ethanenitrile (128.6 mg, 1.05 mmol) followed by benzyl tetrahydro-[1,3,2]dioxathiolo[4,5-c]pyridine-5(4H)-carboxylate 2,2-dioxide **(Intermediate 8;** 300 mg, 0.960 mmol) and 15-crown-5(0.02 mL, 0.100 mmol) in DME (3.2 mL) and the resulting orange mixture was stirred at rt for 2 h. H₂O (2 mL) was added and the mixture extracted with EtOAc (3x35 mL). The collected organics were washed with brine, dried over Na₂SO₄ and concentrated *in vacuo* to get a residue which was purified by flash chromatography (gradient elution 0-100% EtOAc in petroleum ether) to afford the title compound as a yellow oil (120 mg, 37%). LCMS (ES⁺) *m*/*z* 338 (M+H)⁺, RT 1.76 min.

### Step 2: benzyl 7-(aminomethyl)-7-(4-methylisoxazol-3-yl)-3-azabicyclo[4.1.0]heptane-3-carboxylate

A solution of benzyl 7-cyano-7-(4-methylisoxazol-3-yl)-3-azabicyclo[4.1.0]heptane-3-carboxylate (120 mg, 0.360 mmol) in THF (9.0 mL) was treated with borane methylsulfanylmethane (2M in THF; 0.89 mL, 1.78 mmol) and the resulting mixture was stirred at 65°C for 1 h. After cooling, EtOH was added and mixture stirred at reflux for 4 h. The volatiles were removed under reduced pressure and the title compound was obtained as a colorless oil and used without further purification. LCMS (ES ⁺) *m*/*z* 342 (M+H)⁺, RT 1.07 min.

### Step 3: benzyl 7-(((tert-butoxycarbonyl)amino)methyl)-7-(4-methylisoxazol-3-yl)-3-azabicyclo[4.1.0]heptane-3-carboxylate

A solution of benzyl 7-(aminomethyl)-7-(4-methylisoxazol-3-yl)-3-azabicyclo[4.1.0]heptane-3-carboxylate (121 mg, 0.350 mmol) and TEA (0.1 mL, 0.710 mmol) in DCM (1.8 mL) was treated with tert-butoxycarbonyl tert-butyl carbonate (0.1 mL, 0.430 mmol) and the mixture was stirred at rt for 18 h. NaHCO₃ sat. sol. was added and mixture was extracted with EtOAc. The organic phase was washed with brine, dried over Na₂SO₄ and concentrated *in vacuo* to get a residue which was purified by flash chromatography (gradient elution 0-100% EtOAc in petroleum ether) to afford the title compound as a colorless oil (80 mg, 51%). LCMS (ES⁺) *m*/*z* 442 (M+H)⁺, RT 2.0 min.

### Step 4: tert-butyl ((7-(4-methylisoxazol-3-yl)-3-azabicyclo[4.1.0]heptan-7-yl)methyl)carbamate (Intermediate 10)

A solution of benzyl 7-(((tert-butoxycarbonyl)amino)methyl)-7-(4-methylisoxazol-3-yl)-3-azabicyclo[4.1.0]heptane-3-carboxylate (80 mg, 0.180 mmol) in THF (1.5 mL) was treated with Pd/C (30 mg) and the resulting suspension was stirred under H₂ atmosphere at rt for 18 h. After nitrogen fluxing to remove hydrogen, the catalyst was filtered on a pad of solka floc which was washed with MeOH. After volatiles removal under reduced pressure, the title compound was obtained as a colorless oil and used as crude. LCMS (ES⁺) *m*/*z* 308 (M+H)⁺, RT 0.91 min.

**Intermediate 10** was used for the synthesis of **Example 14, Example 15** and **Example 52.**

### Intermediate 11: tert-butyl (3-chloro-4-(4,4,5,5-tetramethyl-1,3,2-dioxaborolan-2-yl)pyridin-2-yl)(methyl)carbamate

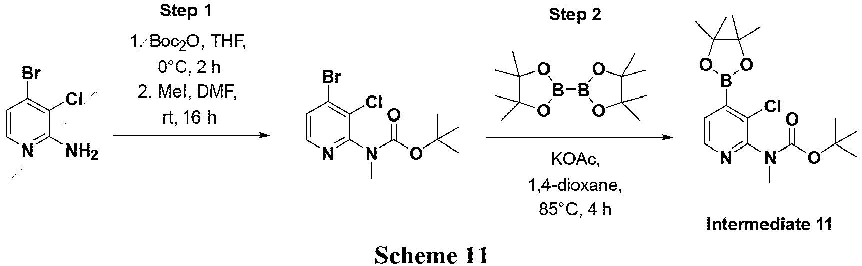

### Step 1: tert-butyl (4-bromo-3-chloropyridin-2-yl)(methyl)carbamate

A solution of 4-bromo-3-chloropyridin-2-amine (200 mg, 0.960 mmol) and tert-butoxycarbonyl tert-butyl carbonate (274 mg, 1.253 mmol) in THF (4 mL) at 0 °C was treated dropwise with [bis(trimethylsilyl)amino]sodium (2.41 mL, 2.41 mmol). The resulting mixture was stirred at rt for 2 h. DMF (5 mL) and iodomethane (90.0 uL, 1.45 mmol) were added and the mixture stirred at rt for 16 h. H₂O (5 mL) was added dropwise and the mixture was extracted with Et₂O (2x50 mL). The collected organics were washed with brine, dried over Na₂SO₄, and concentrated *in vacuo* to get a residue which was purified by flash chromatography (gradient elution 0-20% EtOAc in petroleum ether) to afford the title compound as a pale yellow oil (295 mg, 95%). ¹ H NMR (400 MHz, DMSO-*d₆*) δ 8.30 (d, *J*=4.1 Hz, 1H), 7.82 (d, *J*=4.0 Hz, 1H), 3.13 (s, 3H), 1.35 (s, 9H). LCMS (ES⁺) *m*/*z* 321 (M+H)⁺, RT 2.02 min.

### Step 2: tert-butyl (3-chloro-4-(4,4,5,5-tetramethyl-1,3,2-dioxaborolan-2-yl)pyridin-2-yl)(methyl)carbamate (Intermediate 11)

A solution of tert-butyl (4-bromo-3-chloropyridin-2-yl)(methyl)carbamate (79 mg, 0.800 mmol), [1,1'-Bis(diphenylphosphino)ferrocene]dichloropalladium(II), complex with dichloromethane (33.3 mg, 0.040 mmol), potassium acetate (79 mg, 0.805 mmol) and 4,4,4',4',5,5,5',5'-octamethyl-2,2'-bi(1,3,2-dioxaborolane) (112.4 mg, 0.440 mmol) in 1,4-dioxane (4 mL) was stirred at 85°C for 4 h. After cooling, the mixture was filtered on a pad of solka floc and the filtrate concentrated *in vacuo* to get a residue which was purified by flash chromatography (gradient elution 0-50% EtOAc in petroleum ether) to afford the title compound as a white powder (99 mg, 67%). ¹ H NMR (400 MHz, DMSO-*d₆*) δ 8.42 (d, *J*=4.0 Hz, 1H), 7.48 (d, *J*=4.0 Hz, 1H), 3.10 (s, 3H), 1.33 (s, 9H), 1.16 (s, 6H), 1.07 (s, 6H). LCMS (ES⁺) *m*/*z* 285 (M-H of the boronic acid), RT 1.20 min.

**Intermediate 11** was used for the synthesis of **Example 16** and **Example 17.**

### Intermediate 12: tert-butyl ((7-(5-methylisoxazol-3-yl)-3-azabicyclo[4.1.0]heptan-7-yl)methyl)carbamate

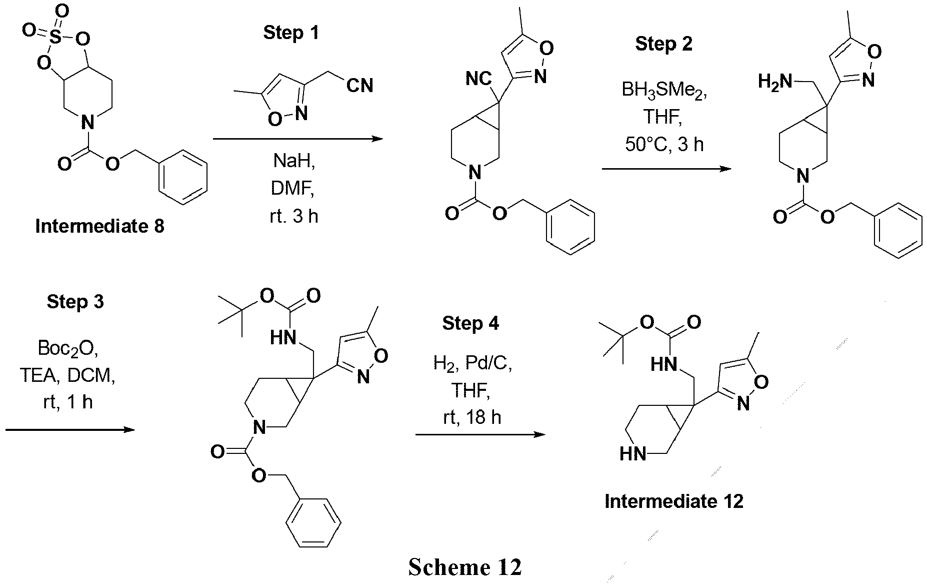

### Step 1: benzyl 7-cyano-7-(5-methylisoxazol-3-yl)-3-azabicyclo[4.1.0]heptane-3-carboxylate

A solution of sodium hydride (60% in mineral oil; 261 mg, 6.52 mmol) in DME (7.5 mL) was treated with a solution of benzyl tetrahydro-[1,3,2]dioxathiolo[4,5-c]pyridine-5(4H)-carboxylate 2,2-dioxide **(Intermediate 8;** 785 mg, 2.51 mmol), 2-(5-methyl-1,2-oxazol-3-yl)ethanenitrile (337 mg, 2.76 mmol) and 15-crown-5 (50 uL, 0.250 mmol) in DME (7.5 mL) and the reaction mixture was stirred at rt for 3 h. H₂O (4 mL) was added and mixture extracted with EtOAc (2x30 mL). The collected organics were washed with brine, dried over Na₂SO₄, and concentrated *in vacuo* to get a residue which was purified by flash chromatography (gradient elution 0-50% EtOAc in petroleum ether) to afford the title compound as a yellow oil (410 mg, 48%). ¹ H NMR (400 MHz, CDCl₃) δ 7.38-7.35 (m, 5H), 6.19 (s, 1H), 5.23-5.10 (m, 2H), 4.31 (d, *J* = 16.0 Hz, 1H), 4.05-3.70 (m, 2H), 3.02-2.87 (m, 1H), 2.42 (s, 3H), 2.31-2.15 (m, 3H), 2.11-2.07 (m, 1H). LCMS (ES⁺) *m*/*z* 360 (M+Na)⁺, RT 1.74 min.

### Step 2: benzyl 7-(aminomethyl)-7-(5-methylisoxazol-3-yl)-3-azabicyclo[4.1.0]heptane-3-carboxylate

A solution of benzyl 7-cyano-7-(5-methylisoxazol-3-yl)-3-azabicyclo[4.1.0]heptane-3-carboxylate (405 mg, 1.2 mmol) in THF (12 mL) was treated with borane methylsulfanylmethane (2M in THF; 2.1 mL, 4.2 mmol) and the resulting mixture was stirred at 50°C for 3 h. After cooling, EtOH (5 mL) was added and the mixture stirred at reflux for 15 h. The volatiles were removed under reduced pressure and the title compound was used without further purification. LCMS (ES ⁺) *m*/*z* 342 (M+H)⁺, RT 1.22 min.

### Step 3: benzyl 7-(((tert-butoxycarbonyl)amino)methyl)-7-(5-methylisoxazol-3-yl)-3-azabicyclo[4.1.0]heptane-3-carboxylate

A solution of benzyl 7-(aminomethyl)-7-(5-methylisoxazol-3-yl)-3-azabicyclo[4.1.0]heptane-3-carboxylate (430 mg, 1.26 mmol) and TEA (246 uL, 1.76 mmol) in DCM (12 mL) was treated with tert-butoxycarbonyl tert-butyl carbonate (302 mg, 1.39 mmol) and the mixture was stirred at rt for 1 h. H₂O (5 mL) was added and mixture extracted with EtOAc (2x55 mL). The collected organics were washed with brine, dried over Na₂SO₄, and concentrated *in vacuo* to get a residue which was purified by flash chromatography (gradient elution 0-50% EtOAc in petroleum ether) to afford the title compound as a colorless oil (330 mg, 59%). LCMS (ES⁺) *m*/*z* 464 (M+Na)⁺, RT 2.05 min.

### Step 4: tert-butyl ((7-(5-methylisoxazol-3-yl)-3-azabicyclo[4.1.0]heptan-7-yl)methyl)carbamate (Intermediate 12)

A solution of benzyl 7-(((tert-butoxycarbonyl)amino)methyl)-7-(5-methylisoxazol-3-yl)-3-azabicyclo[4.1.0]heptane-3-carboxylate (320 mg, 0.720 mmol) in THF (7.0 mL) was treated with Pd/C (150 mg) and the resulting suspension was stirred under H₂ atmosphere at rt for 18 h. After nitrogen fluxing, the catalyst was filtered on a pad of solka floc which was washed with MeOH. After solvent removal the title compound was obtained as a colorless oil and used as crude. LCMS (ES⁺) *m*/*z* 308 (M+H)⁺, RT 1.10 min.

### Intermediate 12 was used for the synthesis of Example 18, Example 19, Example 20, Example 21, Example 22, Example 23, Example 24, Example 57 and Example 58.

### Intermediate 13: thieno[3,2-b]pyridin-7-ylboronic acid

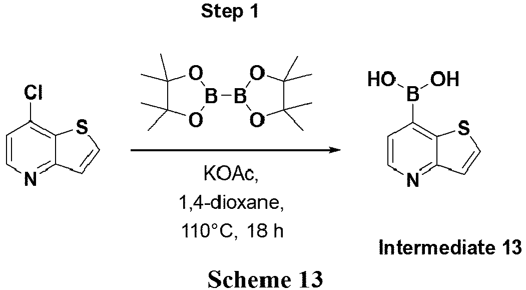

A solution of 4,4,4',4',5,5,5',5'-octamethyl-2,2'-bi(1,3,2-dioxaborolane) (599 mg, 2.36 mmol), 7-chlorothieno[3,2-b]pyridine (200 mg, 1.18 mmol), [1,1'-Bis(diphenylphosphino)ferrocene]dichloropalladium(II), complex with dichloromethane (48.7 mg, 0.060 mmol), and potassium acetate (347.1 mg, 3.54 mmol) in 1,4-dioxane (3.5 mL) was stirred at 110°C for 4 h. After cooling, the mixture was filtered on a pad of solka floc and the filtrate was washed with EtOAc. The organic phase was washed with brine, dried over Na₂SO₄ and concentrated *in vacuo* get the title compound which was used as crude. LCMS (ES⁺) *m*/*z* 180 (M+H)⁺, RT 0.36 min.

**Intermediate 13** was used for the synthesis of **Example 18.**

### Intermediate 14: potassium thieno[3,2-b]pyridine-7-thiolate

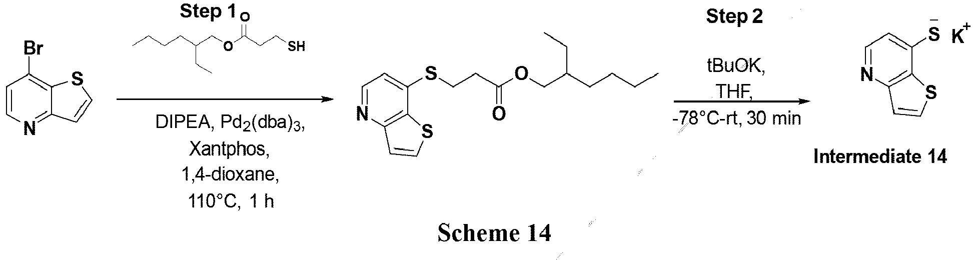

### Step 1: 2-ethylhexyl 3-(thieno[3,2-b]pyridin-7-ylthio)propanoate

A solution of 2-ethylhexyl 3-sulfanylpropanoate (76.5 mg, 0.350 mmol), DIPEA (90.6 mg, 0.700 mmol), Pd₂(dba)₃ (10.7 mg, 0.010 mmol) and Xantphos (6.8 mg, 0.010 mmol) in 1,4-dioxane (0.47 mL) was treated with 7-bromanylthieno[3,2-b]pyridine (50 mg, 0.230 mmol) and the mixture was stirred at 110°C for 1 h. After cooling, the solvent was concentrated *in vacuo* and the residue purified by flash chromatography (gradient elution 0-50% EtOAc in petroleum ether) to afford the title compound as a yellow oil (48 mg, 58%). LCMS (ES⁺) *m*/*z* 352 (M+H)⁺, RT 2.29 min.

### Step 2: potassium thieno[3,2-b]pyridine-7-thiolate

A solution of 2-ethylhexyl 3-(thieno[3,2-b]pyridin-7-ylthio)propanoate (48 mg, 0.140 mmol) in THF (0.47 mL) cooled to -78°C was treated with potassium 2-methylpropan-2-olate (1 M in THF; 0.27 mL, 0.270 mmol). The reaction mixture was stirred at this temperature for 30 min then the solvent was concentrated under reduced pressure to obtain the title compound as a yellow solid which was used without further purification. LCMS (ES⁺) *m*/*z* 167 (M+H)⁺, RT 0.61 min.

**Intermediate 14** was used for the synthesis of **Example 54.**

### Intermediate 15: 8-chloro-5-(4,4,5,5-tetramethyl-1,3,2-dioxaborolan-2-yl)quinoline

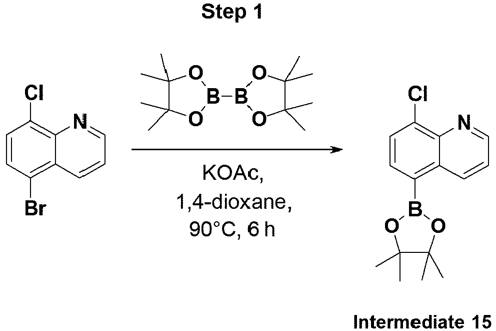

### Step 1: 8-chloro-5-(4,4,5,5-tetramethyl-1,3,2-dioxaborolan-2-yl)quinoline

A solution of [1,1'-Bis(diphenylphosphino)ferrocene]dichloropalladium(II) complex with dichloromethane (51 mg, 0.060 mmol), 5-bromanyl-8-chloranyl-quinoline (150 mg, 0.620 mmol), 4,4,4',4',5,5,5',5'-octamethyl-2,2'-bi(1,3,2-dioxaborolane) (346 mg, 1.36 mmol), and potassium acetate (182 mg, 1.86 mmol) in degassed 1,4-dioxane (2 mL) was stirred at 90°C for 6 h. After cooling the mixture was diluted with EtOAc and washed with H₂O and brine. The organic phase was dried over Na₂SO₄ and concentrated *in vacuo* to afford the title compound which was used as crude. LCMS (ES⁺) *m*/*z* 290 (M+H)⁺, RT 2.19 min.

**Intermediate 15** was used for the synthesis of Example **33**

### Intermediate 16: 8-chloro-5-(4,4,5,5-tetramethyl-1,3,2-dioxaborolan-2-yl)isoquinoline

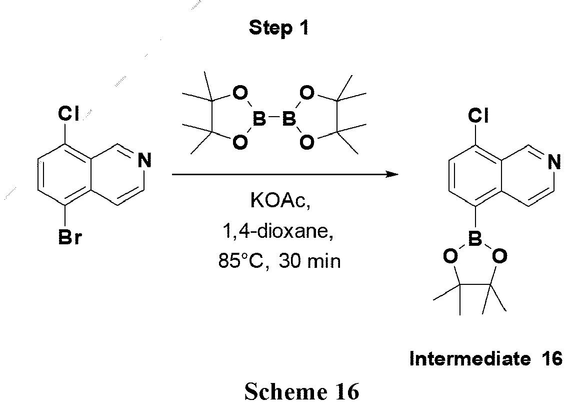

A solution of [1,1'-Bis(diphenylphosphino)ferrocene]dichloropalladium(II) complex with dichloromethane (34.1 mg, 0.040 mmol), 5-bromanyl-8-chloranyl-isoquinoline (100 mg, 0.410 mmol), 4,4,4',4',5,5,5',5'-octamethyl-2,2'-bi(1,3,2-dioxaborolane) (230.4 mg, 0.910 mmol), and potassium acetate (121.4 mg, 1.24 mmol) in degassed 1,4-dioxane (1.5 mL) was stirred at 85 °C for 30 min. After cooling, the mixture was diluted with EtOAc and washed with H₂O and brine.

The organic phase was dried over Na₂SO₄ and concentrated *in vacuo* to afford the title compound which was used as crude. LCMS (ES⁺) *m*/*z* 290 (M+H)⁺, RT 2.06 min.

**Intermediate 16** was used for the synthesis of **Example 34.**

### Intermediate 17: (2-(bis(tert-butoxycarbonyl)amino)-3-chloropyridin-4-yl)boronic acid

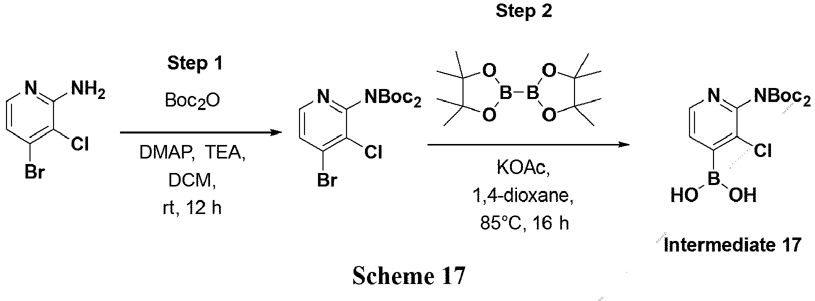

### Step 1: tert-butyl (4-bromo-3-chloropyridin-2-yl)(tert-butoxycarbonyl)carbamate

A solution of 4-bromo-3-chloropyridin-2-amine (106 mg, 0.510 mmol) and tert-butoxycarbonyl tert-butyl carbonate (557.6 mg, 2.55 mmol) in DCM (6 mL) was treated with DMAP (12.5 mg, 0.100 mmol) and TEA (0.21 mL, 1.53 mmol). The resulting mixture was stirred at rt for 12 h. H₂O (5 mL) was added dropwise and mixture was extracted with Et₂O (2x50 mL). Organic phase was washed with H₂O, brine, dried over Na₂SO₄ and concentrated *in vacuo* and the residue was purified by flash chromatography (gradient elution 0-50% EtOAc in petroleum ether) to afford the title compound as a white solid (184 mg, 88%). ¹ H NMR (400 MHz, DMSO-*d₆*) δ 8.35 (d, *J* = 4.0 Hz, 1H), 7.94 (d, *J* = 4.1 Hz, 1H), 1.36 (s, 18H). LCMS (ES⁺) *m*/*z* 407-409 (M+H)⁺, RT 2.26

### Step 2: (2-(bis(tert-butoxycarbonyl)amino)-3-chloropyridin-4-yl)boronic acid

A solution of tert-butyl (4-bromo-3-chloropyridin-2-yl)(tert-butoxycarbonyl)carbamate (100 mg, 0.250 mmol), potassium acetate (48.2 mg, 0.490 mmol), [1,1'-Bis(diphenylphosphino)ferrocene]dichloropalladium(II) complex with dichloromethane (20.3 mg, 0.020 mmol) and 4,4,4',4',5,5,5',5'-octamethyl-2,2'-bi(1,3,2-dioxaborolane) (68.5 mg, 0.270 mmol) in 1,4-dioxane (2.5 mL) was stirred at 85 °C for 16 h. After cooling, the mixture was filtered on a pad of solka floc and the filtrate was washed with EtOAc. The organic phase was concentrated *in vacuo* to afford the title compound which was used as crude. LCMS (ES⁺) *m*/*z* 373 (M+H)⁺, RT 1.55

**Intermediate 17** was used for the synthesis of **Example 36.**

### Intermediate 18: (lS,6R,7S)-7-((((benzyloxy)carbonyl)amino)methyl)-7-(5-methylisoxazol-3-yl)-3-azabicyclo [4.1.0] heptan-3-ium chloride

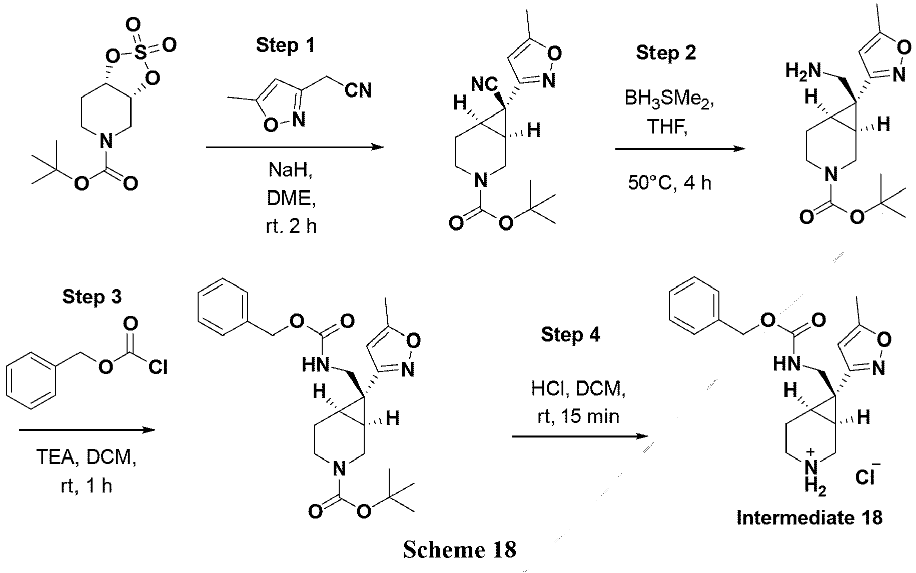

### Step 1: tert-butyl (1S,6R,7R)-7-cyano-7-(5-methylisoxazol-3-yl)-3-azabicyclo[4.1.0]heptane-3-carboxylate

A solution of sodium hydride (60% in mineral oil; 558.5 mg, 13.96 mmol) in DME (13 mL) was treated with a solution of tert-butyl (3aR,7aS)-2,2-bis(oxidanylidene)-4,6,7,7a-tetrahydro-3aH-[1,3,2]dioxathiolo[4,5-c]pyridine-5-carboxylate (prepared using the procedure described in Step 1 of **Intermediate 39**), 2-(5-methyl-1,2-oxazol-3-yl)ethanenitrile (721.4 mg, 5.91 mmol) and 15-crown-5 (107 uL, 0.540 mmol) in DME (13 mL) and the reaction mixture was stirred at rt for 2 h. H₂O (12 mL) was added and the mixture was extracted with EtOAc (2x90 mL). The collected organics were washed with brine, dried over Na₂SO₄ and concentrated *in vacuo* to get a residue which was purified by flash chromatography (gradient elution 0-40% EtOAc in petroleum ether) to afford the title compound as a yellow oil (1.1 g, 68%). LCMS (ES⁺) *m*/*z* 326 (M+Na)⁺, RT 1.70 min.

### Step 2: tert-butyl (1S,6R,7S)-7-(aminomethyl)-7-(5-methylisoxazol-3-yl)-3-azabicyclo[4.1.0]heptane-3-carboxylate

A solution of tert-butyl (1S,6R,7R)-7-cyano-7-(5-methylisoxazol-3-yl)-3-azabicyclo[4.1.0]heptane-3-carboxylate (1.1 g, 3.63 mmol) in THF (45 mL) was treated with borane methylsulfanylmethane (2M in THF; 6.35 mL, 12.69 mmol) and the resulting mixture was stirred at 50°C for 4 h. After cooling, EtOH (15 mL) was added and mixture stirred at reflux for 16 h. The volatiles were removed by reduced pressure and the title compound was used without further purification. LCMS (ES ⁺) *m*/*z* 308 (M+H)⁺, RT 1.15 min.

### Step 3: tert-butyl (1S,6R,7S)-7-((((benzyloxy)carbonyl)amino)methyl)-7-(5-methylisoxazo1-3-yl)-3-azabicyclo[4.1.0]heptane-3-carboxylate

A solution of tert-butyl (1S,6R,7S)-7-(aminomethyl)-7-(5-methylisoxazol-3-yl)-3-azabicyclo[4.1.0]heptane-3-carboxylate (1.2 g, 3.9 mmol) in DCM (38 mL) cooled at 0°C was treated with TEA (1.63 mL, 11.71 mmol), benzyl chloroformate (502 uL, 3.51 mmol) and the mixture was stirred at rt for 1 h. Brine was added and the mixture was extracted with EtOAc (2x100 mL). The collected organics were washed with H₂O, brine, dried over Na₂SO₄, and concentrated *in vacuo* to get a residue which was purified by flash chromatography (gradient elution 0-50% EtOAc in petroleum ether) to afford the title compound as a colorless oil (480 mg, 28%). LCMS (ES⁺) *m*/*z* 464 (M+Na)⁺, RT 2.02 min.

### Step 4: (1S,6R,7S)-7-((((benzyloxy)carbonyl)amino)methyl)-7-(5-methylisoxazol-3-yl)-3-azabicyclo[4.1.0]heptan-3-ium chloride (Intermediate 18)

A solution of tert-butyl (1S,6R,7S)-7-((((benzyloxy)carbonyl)amino)methyl)-7-(5-methylisoxazol-3-yl)-3-azabicyclo[4.1.0]heptane-3-carboxylate (480 mg, 1.09 mmol) in DCM (10 mL) was treated with HCl (4M in 1,4-dioxane; 10.87 mL, 43.49 mmol) and the solution stirred at rt for 15 min. The mixture was concentrated under reduced pressure to afford the title compound as a white solid which was used as a crude (410 mg, 100%). LCMS (ES⁺) *m*/*z* 342 (M+H)⁺, RT 1.18 min.

**Intermediate 18** was used for the synthesis of **Example 37, Example 38, Example 39, Example 40, Example 41, Example 42, Example 43, Example 44, Example 45, Example 46** and **Example 51.**

### Intermediate 19: (5-Fluoroquinolin-4-yl)boronic acid

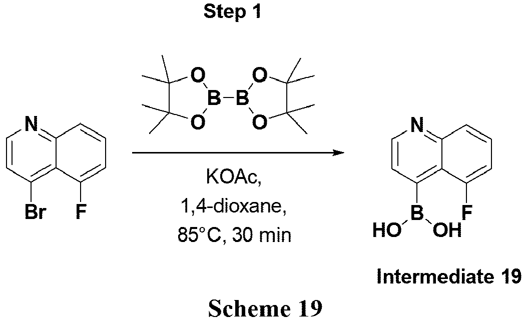

A solution of [1,1'-Bis(diphenylphosphino)ferrocene]dichloropalladium(II) complex with dichloromethane (36.6 mg, 0.040 mmol), 4-bromo-5-fluoroquinoline (100 mg, 0.440 mmol), 4,4,4',4',5,5,5',5'-octamethyl-2,2'-bi(1,3,2-dioxaborolane) (247.1 mg, 0.970 mmol), and potassium acetate (130.3 mg, 1.33 mmol) in degassed 1,4-dioxane (2 mL) was stirred at 85°C for 30 min. After cooling, the mixture was filtered on a pad of solka floc and the filtrate was washed with EtOAc. The organic phase was concentrated *in vacuo* to get the title compound which was used as crude. LCMS (ES⁺) *m*/*z* 192 (M+H)⁺, RT 0.27 min

**Intermediate 19** was used for the synthesis of **Example 41.**

### Intermediate 20: (6-chloroquinolin-4-yl)boronic acid

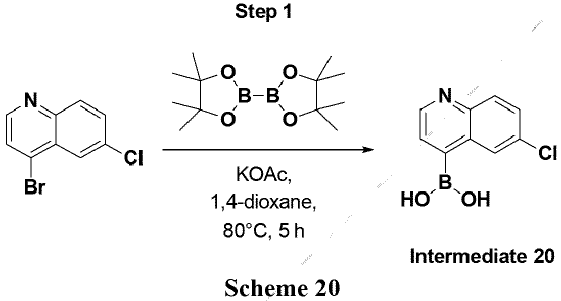

A solution of [1,1'-Bis(diphenylphosphino)ferrocene]dichloropalladium(II) complex with dichloromethane (34.1 mg, 0.040 mmol), 4-bromo-6-chloroquinoline (100 mg, 0.410 mmol), 4,4,4',4',5,5,5',5'-octamethyl-2,2'-bi(1,3,2-dioxaborolane) (125.7 mg, 0.490 mmol), and potassium acetate (121.4 mg, 1.24 mmol) in degassed 1,4-dioxane (0.8 mL) was stirred at 80°C for 5 h. After cooling, the mixture was filtered on a pad of solka floc and the filtrate was washed with EtOAc. The organic phase was concentrated *in vacuo* to get the title compound which was used as crude (85 mg, 99%). LCMS (ES⁺) *m*/*z* 208 (M+H)⁺, RT 0.74 min

**Intermediate 20** was used for the synthesis of **Example 42.**

### Intermediate 21: 5-(4,4,5,5-tetramethyl-1,3,2-dioxaborolan-2-yl)-8-(trifluoromethyl)quinoline

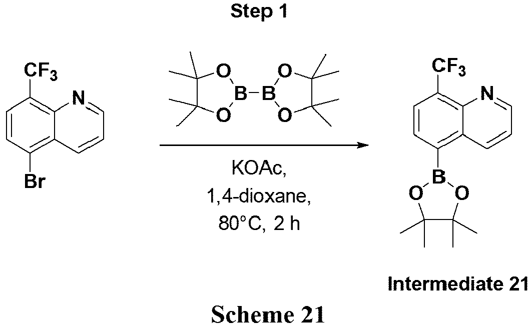

A solution of [1,1'-Bis(diphenylphosphino)ferrocene]dichloropalladium(II) complex with dichloromethane (30.0 mg, 0.040 mmol), 5-bromo-8-(trifluoromethyl)quinoline (100 mg, 0.360 mmol), 4,4,4',4',5,5,5',5'-octamethyl-2,2'-bi(1,3,2-dioxaborolane) (110.4 mg, 0.430 mmol), and potassium acetate (106.7 mg, 1.09 mmol) in degassed 1,4-dioxane (0.7 mL) was stirred at 80°C for 2 h. After cooling, the mixture was filtered on a pad of solka floc and the filtrate was washed with EtOAc. The organic phase was concentrated *in vacuo* to get the title compound which was used as crude (117 mg, 99%). LCMS (ES⁺) *m*/*z* 324 (M+H)⁺, RT 2.38 min.

**Intermediate 21** was used for the synthesis of **Example 43.**

### Intermediate 22: (7-fluoroquinolin-4-yl)boronic acid

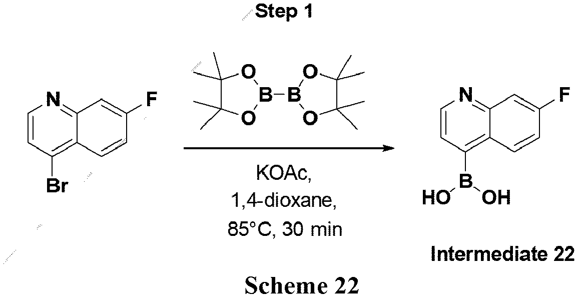

A solution of [1,1'-Bis(diphenylphosphino)ferrocene]dichloropalladium(II) complex with dichloromethane (73.1 mg, 0.090 mmol), 4-bromo-7-fluoroquinoline (200 mg, 0.880 mmol), 4,4,4',4',5,5,5',5'-octamethyl-2,2'-bi(1,3,2-dioxaborolane) (494.3 mg, 1.95 mmol), potassium acetate (260.5 mg, 2.65 mmol) in degassed 1,4-dioxane (3.5 mL) was stirred at 85°C for 30 min. After cooling, the mixture was filtered on a pad of solka floc and the filtrate was washed with EtOAc. The organic phase was concentrated *in vacuo* to get the title compound which was used as crude. LCMS (ES⁺) *m*/*z* 192 (M+H)⁺, RT 0.27 min

**Intermediate 22** was used for the synthesis of **Example 45.**

### Intermediate 23: 7-((((benzyloxy)carbonyl)amino)methyl)-7-(5-(methoxymethyl)isoxazol-3-yl)-3-azabicyclo[4.1.0]heptan-3-ium chloride

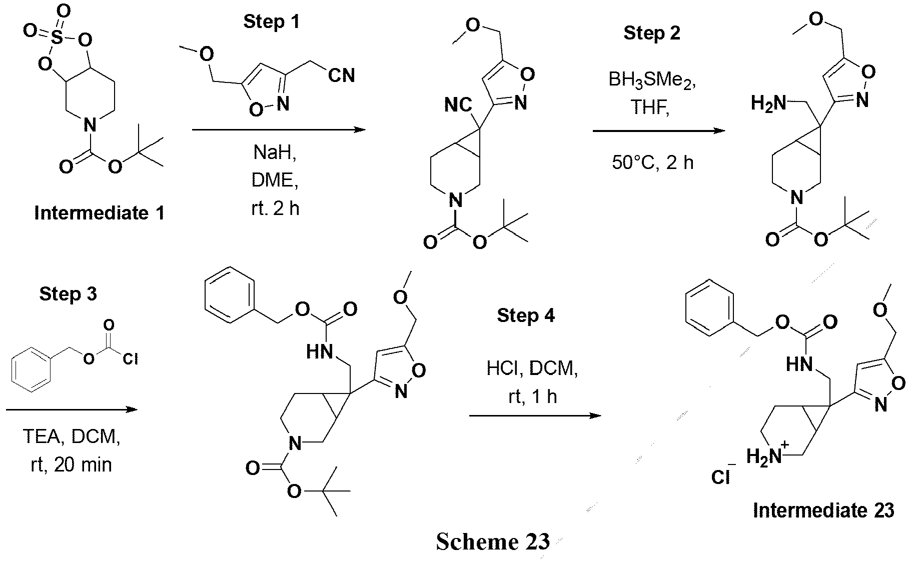

### Step 1: tert-butyl 7-cyano-7-(5-(methoxymethyl)isoxazol-3-yl)-3-azabicyclo[4.1.0]heptane-3-carboxylate

A solution of sodium hydride (60% in mineral oil; 93.1 mg, 2.33 mmol) in DME (5.0 mL) was treated with a solution of tert-butyl tetrahydro-[1,3,2]dioxathiolo[4,5-c]pyridine-5(4H)-carboxylate 2,2-dioxide **(Intermediate 1;** 250 mg, 0.900 mmol), 2-(5-(methoxymethyl)isoxazol-3-yl)acetonitrile (150 mg, 0.980 mmol) and 15-crown-5 (17.9 uL, 0.090 mmol) in DME (5.0 mL) and the reaction mixture was stirred at rt for 2 h. H₂O (2 mL) was added and the mixture was extracted with EtOAc (3x50 mL). The collected organics were washed with brine, dried over Na₂SO₄ and concentrated *in vacuo* to get a residue which was purified by flash chromatography (gradient elution 0-60% EtOAc in petroleum ether) to afford the title compound as a yellow oil (185 mg, 62%). ¹ H NMR (400 MHz, CDCl₃) δ 6.46 (s, 1H), 4.51 (s, 2H), 4.25 (d, *J* = 16.0 Hz, 1H), 3.98-3.80 (m, 1H), 3.66 (dd, *J₁* = 4.0 Hz, *J₂* = 16.0 Hz, 1H), 3.43 (s, 3H), 2.93-2.79 (m, 1H), 2.34-2.23 (m, 3H), 2.07-2.01 (m, 1H), 1.48 (s, 9H). LCMS (ES⁺) *m*/*z* 356 (M+Na)⁺, RT 1.46 min.

### Step 2: tert-butyl 7-(aminomethyl)-7-(5-(methoxymethyl)isoxazol-3-yl)-3-azabicyclo[4.1.0]heptane-3-carboxylate

A solution of tert-butyl 7-cyano-7-(5-(methoxymethyl)isoxazol-3-yl)-3-azabicyclo[4.1.0]heptane-3-carboxylate (185 mg, 0.550 mmol) in THF (6 mL) was treated with borane methylsulfanylmethane (2M in THF; 0.97 mL, 1.94 mmol) and the resulting mixture was stirred at 50°C for 2 h. After cooling, EtOH (4 mL) was added and the mixture stirred at reflux for 16 h. The volatiles were removed by reduced pressure and the title compound was used without further purification. LCMS (ES ⁺) *m*/*z* 338 (M+H)⁺, RT 0.97 min.

### Step 3: tert-butyl 7-((((benzyloxy)carbonyl)amino)methyl)-7-(5-(methoxymethyl)isoxazol-3-yl)-3-azabicyclo[4.1.0]heptane-3-carboxylate

A solution of tert-butyl 7-(aminomethyl)-7-(5-(methoxymethyl)isoxazol-3-yl)-3-azabicyclo[4.1.0]heptane-3-carboxylate (220 mg, 0.650 mmol) in DCM (6 mL) cooled at 0 °C was treated with TEA (0.27 mL, 1.96 mmol) and benzyl chloroformate (83.8 uL, 0.590 mmol) and the mixture was stirred at rt for 20 min. Brine was added and the mixture was extracted with EtOAc (2x20 mL). The collected organics were washed with H₂O, brine, dried over Na₂SO₄, and concentrated *in vacuo* to get a residue which was purified by flash chromatography (gradient elution 0-50% EtOAc in petroleum ether) to afford the title compound as a colorless oil (100 mg, 33%). LCMS (ES⁺) *m*/*z* 494 (M+Na)⁺, RT 1.80 min.

### Step 4: 7-((((benzyloxy)carbonyl)amino)methyl)-7-(5-(methoxymethyl)isoxazol-3-yl)-3-azabicyclo[4.1.0]heptan-3-ium chloride (Intermediate 23)

A solution of tert-butyl 7-((((benzyloxy)carbonyl)amino)methyl)-7-(5-(methoxymethyl)isoxazol-3-yl)-3-azabicyclo[4.1.0]heptane-3-carboxylate (100 mg, 0.210 mmol) in DCM (1 mL) was treated with HCl (4 M in 1,4-dioxane; 2.65 mL, 10.6 mmol) and the solution was stirred at rt for 1 h. The mixture was concentrated under reduced pressure to afford the title compound as a white solid which was used as a crude. LCMS (ES⁺) *m*/*z* 372 (M+H)⁺, RT 0.99 min.

**Intermediate 23** was used for the synthesis of **Example 47.**

### Intermediate 24: 7-((((benzyloxy)carbonyl)amino)methyl)-7-(5-cyclopropylisoxazol-3-yl)-3-azabicyclo[4.1.0]heptan-3-ium chloride

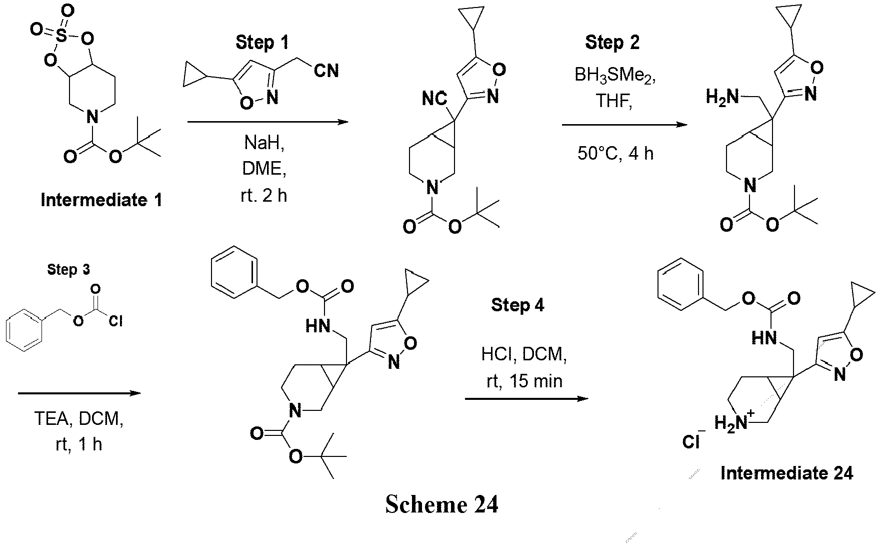

### Step 1: tert-butyl 7-cyano-7-(5-cyclopropylisoxazol-3-yl)-3-azabicyclo[4.1.0]heptane-3-carboxylate

A solution of sodium hydride (60% in mineral oil; 111.7 mg, 2.79 mmol) in DME (5.0 mL) was treated with a solution of *tert*-butyl tetrahydro-[1,3,2]dioxathiolo[4,5-c]pyridine-5(4H)-carboxylate 2,2-dioxide **(Intermediate 1;** 300 mg, 1.07 mmol), 2-(5-cyclopropylisoxazol-3-yl)acetonitrile (175.1 mg, 1.18 mmol) and 15-crown-5 (21.5 uL, 0.110 mmol) in DME (5.0 mL) and the reaction mixture was stirred at rt for 2 h. H₂O (2 mL) was added and the mixture was extracted with EtOAc (2x50 mL). The collected organics were washed with brine, dried over Na₂SO₄ and concentrated *in vacuo* to get a residue which was purified by flash chromatography (gradient elution 0-50% EtOAc in petroleum ether) to afford the title compound as a yellow oil (220 mg, 62%). LCMS (ES⁺) *m*/*z* 352 (M+Na)⁺, RT 1.88 min.

### Step 2: tert-butyl 7-(aminomethyl)-7-(5-cyclopropylisoxazol-3-yl)-3-azabicyclo[4.1.0]heptane-3-carboxylate

A solution of tert-butyl 7-cyano-7-(5-cyclopropylisoxazol-3-yl)-3-azabicyclo[4.1.0]heptane-3-carboxylate (220 mg, 0.670 mmol) in THF (6.5 mL) was treated with borane methylsulfanylmethane (2M in THF; 1.17 mL, 2.34 mmol) and the resulting mixture was stirred at 50 °C for 4 h. After cooling, EtOH (4 mL) was added and mixture stirred at reflux for 16 h. The volatiles were removed by reduced pressure and the title compound was used without further purification. LCMS (ES ⁺) *m*/*z* 334 (M+H)⁺, RT 1.09 min.

### Step 3: tert-butyl 7-((((benzyloxy)carbonyl)amino)methyl)-7-(5-cyclopropylisoxazol-3-yl)-3-azabicyclo[4.1.0]heptane-3-carboxylate

A solution of tert-butyl 7-(aminomethyl)-7-(5-cyclopropylisoxazol-3-yl)-3-azabicyclo[4.1.0]heptane-3-carboxylate (270 mg, 0.810 mmol) in DCM (7 mL) cooled at 0 °C was treated with TEA (0.34 mL, 2.43 mmol) and benzyl chloroformate (104.0 uL, 0.730 mmol) and the mixture was stirred at rt for 1 h. Brine was added and the mixture extracted with EtOAc (2x40 mL). The collected organics were washed with H₂O, brine, dried over Na₂SO₄, and concentrated *in vacuo* to get a residue which was purified by flash chromatography (gradient elution 0-40% EtOAc in petroleum ether) to afford the title compound as a colorless oil (83 mg, 22%). LCMS (ES⁺) *m*/*z* 490 (M+Na)⁺, RT 1.96 min.

### Step 4: 7-((((benzyloxy)carbonyl)amino)methyl)-7-(5-cyclopropylisoxazol-3-yl)-3-azabicyclo[4.1.0]heptan-3-ium chloride (Intermediate 24)

A solution of *tert*-butyl 7-((((benzyloxy)carbonyl)amino)methyl)-7-(5-cyclopropylisoxazol-3-yl)-3-azabicyclo[4.1.0]heptane-3-carboxylate (85 mg, 0.180 mmol) in DCM (1 mL) was treated with HCl (4M in 1,4-dioxane; 2.27 mL, 9.09 mmol) and the solution was stirred at rt for 15 min. The mixture was concentrated under reduced pressure to afford the title compound as a white solid which was used as a crude. LCMS (ES⁺) *m*/*z* 368 (M+H)⁺, RT 1.15 min.

**Intermediate 24** was used for the synthesis of **Example 48.**

### Intermediate 25: 7-((((benzyloxy)carbonyl)amino)methyl)-7-(3-fluoropyridin-2-yl)-3-azabicyclo[4.1.0]heptan-3-ium chloride

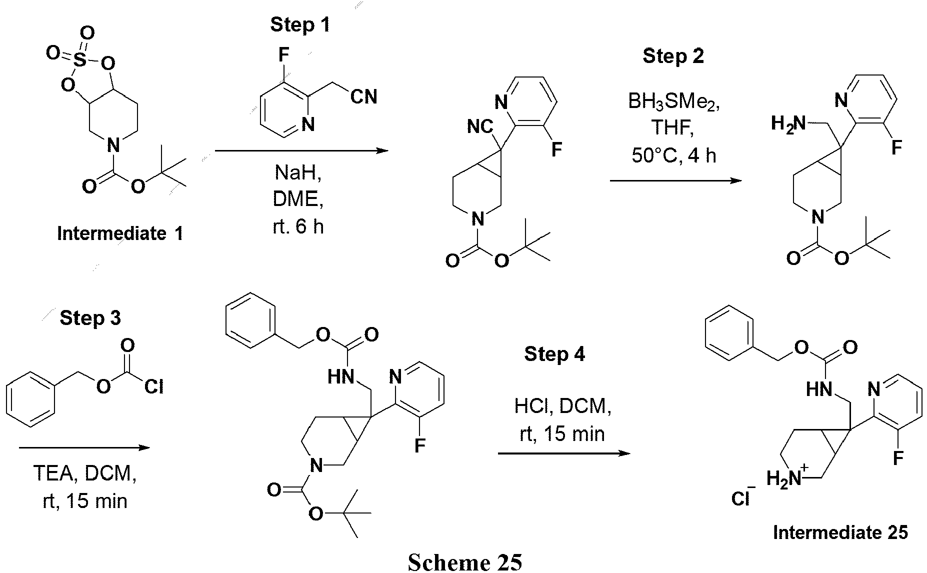

### Step 1: tert-butyl 7-cyano-7-(3-fluoropyridin-2-yl)-3-azabicyclo[4.1.0]heptane-3-carboxylate

A solution of sodium hydride (60% in mineral oil; 111.7 mg, 2.79 mmol) in DME (3.5 mL) was treated with a solution of tert-butyl tetrahydro-[1,3,2]dioxathiolo[4,5-c]pyridine-5(4H)-carboxylate 2,2-dioxide **(Intermediate 1;** 300 mg, 1.07 mmol), 2-(3-fluoropyridin-2-yl)acetonitrile (160.8 mg, 1.18 mmol) and 15-crown-5 (21.3 uL, 0.110 mmol) in DME (3.5 mL) and the reaction mixture was stirred at rt for 6 h. H₂O (4 mL) was added and the mixture was extracted with EtOAc (2x50 mL). The collected organics were washed with brine, dried over Na₂SO₄ and concentrated *in vacuo* to get a residue which was purified by flash chromatography (gradient elution 0-60% EtOAc in petroleum ether) to afford the title compound as a yellow oil (73 mg, 21%). LCMS (ES⁺) *m*/*z* 340 (M+Na)⁺, RT 1.70 min.

### Step 2: tert-butyl 7-(aminomethyl)-7-(3-fluoropyridin-2-yl)-3-azabicyclo[4.1.0]heptane-3-carboxylate

A solution of tert-butyl 7-cyano-7-(3-fluoropyridin-2-yl)-3-azabicyclo[4.1.0]heptane-3-carboxylate (73 mg, 0.230 mmol) in THF (3.0 mL) was treated with borane methylsulfanylmethane (2M in THF; 402.0 uL, 0.810 mmol) and the resulting mixture was stirred at 50 °C for 4 h. After cooling, EtOH (5 mL) was added and mixture stirred at reflux for 6 h. The volatiles were removed by reduced pressure and the title compound was used without further purification. LCMS (ES ⁺) *m*/*z* 322 (M+H)⁺, RT 1.15 min.

### Step 3: tert-butyl 7-((((benzyloxy)carbonyl)amino)methyl)-7-(3-fluoropyridin-2-yl)-3-azabicyclo[4.1.0]heptane-3-carboxylate

A solution of tert-butyl 7-(aminomethyl)-7-(3-fluoropyridin-2-yl)-3-azabicyclo[4.1.0]heptane-3-carboxylate (85 mg, 0.260 mmol) in DCM (3 mL) cooled at 0°C was treated with TEA (111 uL, 0.790 mmol) and benzyl chloroformate (34 uL, 0.240 mmol) and mixture was stirred at rt for 15 min. Brine was added and the mixture was extracted with EtOAc (2x20 mL). The collected organics were washed with H₂O, brine, dried over Na₂SO₄, and concentrated *in vacuo* to get a residue which was purified by flash chromatography (gradient elution 0-70% EtOAc in petroleum ether) to afford the title compound as a colorless oil (25 mg, 21%). LCMS (ES⁺) *m*/*z* 478 (M+Na)⁺, RT 1.70 min.

### Step 4: 7-((((benzyloxy)carbonyl)amino)methyl)-7-(3-fluoropyridin-2-yl)-3-azabicyclo[4.1.0]heptan-3-ium chloride (Intermediate 25)

A solution of tert-butyl 7-((((benzyloxy)carbonyl)amino)methyl)-7-(3-fluoropyridin-2-yl)-3-azabicyclo[4.1.0]heptane-3-carboxylate (23 mg, 0.050 mmol) in DCM (0.8 mL) was treated with HCl (4M in 1,4-dioxane; 0.5 mL, 2.02 mmol) and the solution stirred at rt for 15 min. The mixture was concentrated under reduced pressure to afford the title compound as a white solid which was used as a crude. LCMS (ES⁺) *m*/*z* 356 (M+H)⁺, RT 0.98 min.

**Intermediate 25** was used for the synthesis of **Example 49.**

### Intermediate 26: 7-((((benzyloxy)carbonyl)amino)methyl)-7-(5-fluoropyridin-2-yl)-3-azabicyclo[4.1.0]heptan-3-ium chloride

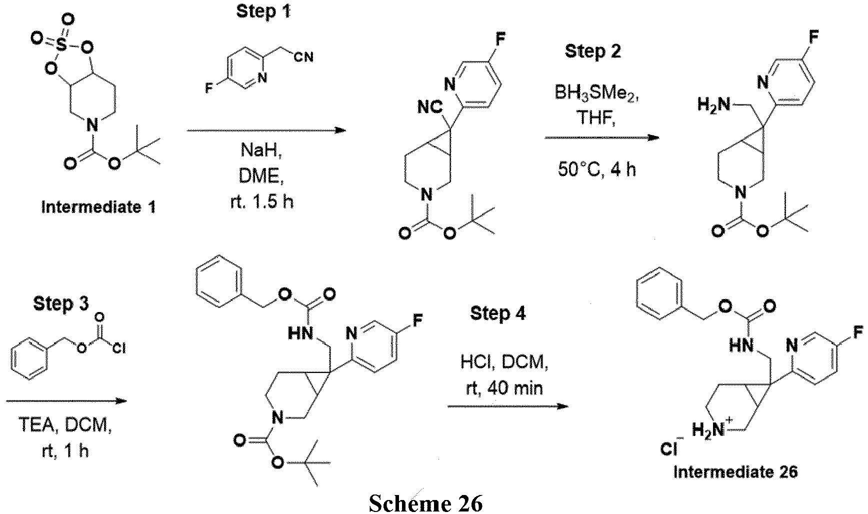

### Step 1: tert-butyl 7-cyano-7-(5-fluoropyridin-2-yl)-3-azabicyclo[4.1.0]heptane-3-carboxylate

A solution of sodium hydride (60% in mineral oil; 111.7 mg, 2.79 mmol) in DME (5 mL) was treated with a solution of tert-butyl tetrahydro-[1,3,2]dioxathiolo[4,5-c]pyridine-5(4H)-carboxylate 2,2-dioxide **(Intermediate 1;** 300 mg, 1.07 mmol), 2-(5-fluoropyridin-2-yl)acetonitrile (160.8 mg, 1.18 mmol) and 15-crown-5 (21.3 uL, 0.110 mmol) in DME (5 mL) and the reaction mixture was stirred at rt for 1.5 h. H₂O (2 mL) was added and the mixture was extracted with EtOAc (2x50 mL). The collected organics were washed with brine, dried over Na₂SO₄ and concentrated *in vacuo* to get a residue which was purified by flash chromatography (gradient elution 0-50% EtOAc in petroleum ether) to afford the title compound as a yellow oil (275 mg, 73%). LCMS (ES⁺) *m*/*z* 218 (M+H-Boc)⁺, RT 1.92 min.

### Step 2: tert-butyl 7-(aminomethyl)-7-(5-fluoropyridin-2-yl)-3-azabicyclo[4.1.0]heptane-3-carboxylate

A solution of tert-butyl 7-cyano-7-(5-fluoropyridin-2-yl)-3-azabicyclo[4.1.0]heptane-3-carboxylate (275 mg, 0.870 mmol) in THF (6.5 mL) was treated with borane methylsulfanylmethane (2M in THF; 1.52 mL, 3.03 mmol) and the resulting mixture was stirred at 50 °C for 4 h. After cooling, EtOH (4 mL) was added and mixture stirred at reflux for 16 h. The volatiles were removed by reduced pressure and the title compound was used without further purification. LCMS (ES ⁺) *m*/*z* 322 (M+H)⁺, RT 1.08 min.

### Step 3: tert-butyl 7-((((benzyloxy)carbonyl)amino)methyl)-7-(5-fluoropyridin-2-yl)-3-azabicyclo[4.1.0]heptane-3-carboxylate

A solution of tert-butyl 7-(aminomethyl)-7-(5-fluoropyridin-2-yl)-3-azabicyclo[4.1.0]heptane-3-carboxylate (275 mg, 0.390 mmol) in DCM (3 mL) cooled at 0 °C was treated with TEA (0.16 mL, 1.16 mmol) and benzyl chloroformate (49.0 uL, 0.350 mmol) and the mixture was stirred at rt for 1 h. Brine was added and mixture extracted with EtOAc (2x100 mL). The collected organics were washed with H₂O, brine, dried over Na₂SO₄, and concentrated *in vacuo* to get a residue which was purified by flash chromatography (gradient elution 0-65% EtOAc in petroleum ether) to afford the title compound as a yellow oil (52 mg, 29%). LCMS (ES⁺) *m*/*z* 478 (M+Na)⁺, RT 1.90 min.

### Step 4: 7-((((benzyloxy)carbonyl)amino)methyl)-7-(5-fluoropyridin-2-yl)-3-azabicyclo[4.1.0]heptan-3-ium chloride (Intermediate 26)

A solution of *tert*-butyl 7-((((benzyloxy)carbonyl)amino)methyl)-7-(5-fluoropyridin-2-yl)-3-azabicyclo[4.1.0]heptane-3-carboxylate (25 mg, 0.050 mmol) in DCM (10 mL) was treated with HCl (4M in 1,4-dioxane; 0.55 mL, 2.2 mmol) and the solution stirred at rt for 40 min. The mixture was concentrated under reduced pressure to afford the title compound as a white solid which was used as a crude. LCMS (ES⁺) *m*/*z* 356 (M+H)⁺, RT 1.05 min.

**Intermediate 26** was used for the synthesis of **Example 50.**

### Intermediate 27: 3,3-difluoro-1-methyl-4-(4,4,5,5-tetramethyl-1,3,2-dioxaborolan-2-yl)indolin-2-one

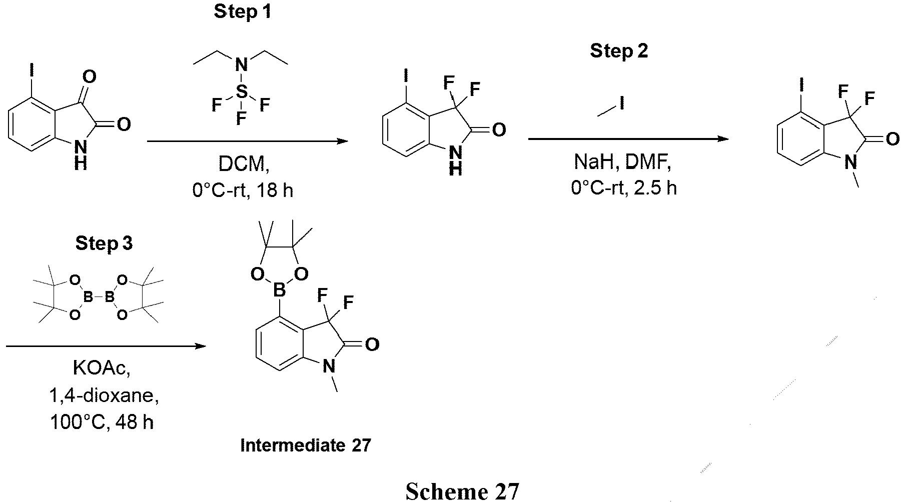

### Step 1: 3,3-difluoro-4-iodoindolin-2-one

A solution of 4-iodoindoline-2,3-dione (600 mg, 2.2 mmol) in DCM (8 mL) cooled at 0°C was treated with N,N-diethyl-1,1,1-trifluoro- • 4-sulfanamine (1.06 g, 6.59 mmol). The resulting mixture was stirred at rt for 18 h and then treated dropwise with NaHCO₃ sat. sol.in H₂O (1.5 g in 8 mL) under vigorous stirring. The layers were separated, and the aqueous phase was extracted with DCM (2x40 mL). The collected organics were washed with brine, dried over Na₂SO₄ and concentrated *in vacuo* to get a residue which was purified by flash chromatography (gradient elution 0-50% EtOAc in petroleum ether) to afford the title compound as a purple solid (330 mg, 51%). ¹H NMR (400 MHz, DMSO-*d₆*) δ 11.29 (bs, 1H), 7.53 (d, *J* = 8.0 Hz, 1H), 7.24 (t, *J* = 8.1 Hz, 1H), 6.98 (d, *J=* 8.0 Hz, 1H). LCMS (ES⁺) *m*/*z* 296 (M+H)⁺, RT 1.61 min

### Step 2: 3,3-difluoro-4-iodo-1-methylindolin-2-one

A solution of 3,3-difluoro-4-iodoindolin-2-one (100 mg, 0.340 mmol) in DMF (2 mL) cooled at 0°C was treated with sodium hydride (60% in mineral oil; 27 mg, 0.680 mmol). The resulting mixture was stirred at rt for 30 min then was treated with iodomethane (42 uL, 0.680 mmol) and stirred at rt for 2 h. H₂O (2 mL) was added dropwise and mixture extracted with EtOAc (2x20 mL). Organic layer was separated and washed with brine, dried over Na₂SO₄ and concentrated *in vacuo* to obtain a residue which was purified by flash chromatography (gradient elution 0-50% EtOAc in petroleum ether) to afford the title compound as a yellow solid (83 mg, 79%). ¹ H NMR (400 MHz, CDCl₃) δ 7.54 (d, *J* = 8.0 Hz, 1H), 7.18 (t, *J* = 8.1 Hz, 1H), 6.86 (d, *J* = 8.0 Hz, 1H), 3.21 (s, 3H). LCMS (ES⁺) *m*/*z* 310 (M+H)⁺, RT 1.75 min

### Step 3: 3,3-difluoro-1-methyl-4-(4,4,5,5-tetramethyl-1,3,2-dioxaborolan-2-yl)indolin-2-one (Intermediate 27)

A solution of [1,1'-Bis(diphenylphosphino)ferrocene]dichloropalladium(II) complex with dichloromethane (16.1 mg, 0.020 mmol), 3,3-difluoro-4-iodo-1-methylindolin-2-one (60 mg, 0.190 mmol), 4,4,4',4',5,5,5',5'-octamethyl-2,2'-bi(1,3,2-dioxaborolane) (108.5 mg, 0.430 mmol), and potassium acetate (57.2 mg, 0.580 mmol) in degassed 1,4-dioxane (1 mL) was stirred at 100°C for 48 h. After cooling, the mixture was filtered on a pad of solka floc and the filtrate was washed with EtOAc. The organic phase was concentrated *in vacuo* to obtain a residue which was purified by flash chromatography (gradient elution 0-50% EtOAc in petroleum ether) to afford the title compound as a yellow oil (45 mg, 75%). LCMS (ES⁺) *m*/*z* 310 (M+H)⁺, RT 2.00 min

**Intermediate 27** was used for the synthesis of **Example 51.**

### Intermediate 28: 7-((((Benzyloxy)carbonyl)amino)methyl)-7-phenyl-3-azabicyclo[4.1.0] heptan-3-ium chloride

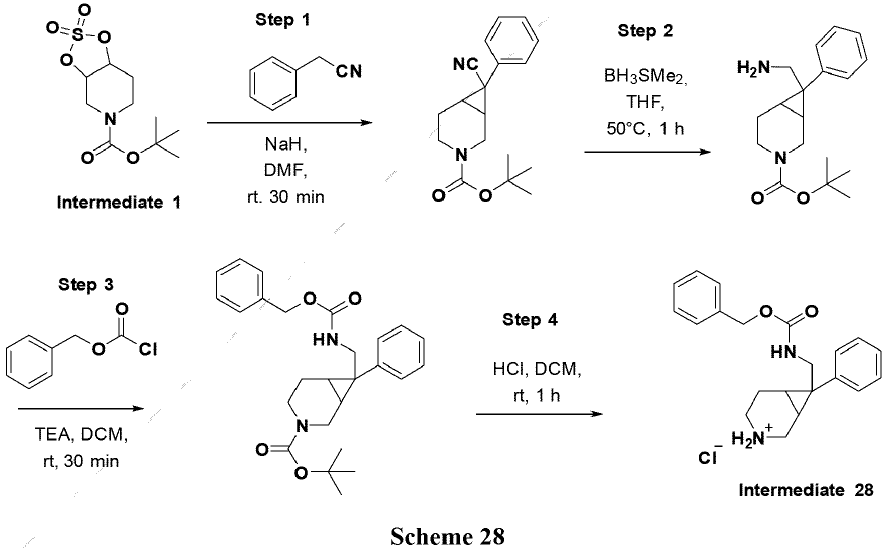

### Step 1: tert-butyl 7-cyano-7-phenyl-3-azabicyclo[4.1.0]heptane-3-carboxylate

*tert-butyl* tetrahydro-[1,3,2]dioxathiolo[4,5-c]pyridine-5(4H)-carboxylate 2,2-dioxide **(Intermediate 1;** 560 mg, 2.01 mmol) and 2-phenylacetonitrile (258 mg, 2.21 mmol) were dissolved in dry DMF (4.5 mL) and the resulting solution was added dropwise over a period of 30 min to a suspension of sodium hydride (60% in mineral oil; 200 mg, 5, 01 mmol) e) in dry DMF (2.8 mL) cooled to 0 °C under N₂ atmosphere. The reaction mixture was stirred at 0 °C for 10 min then at rt for 30 min. The reaction was quenched by addition of brine then diluted with EtOAc. The organic layer was washed with brine (x2) and H₂O (x2), dried over Na₂SO₄, filtered and concentrated *in vacuo.* The residue was purified by flash chromatography on silica gel (from 10% to 30% EtOAc in petroleum ether) to afford the title compound as a light-yellow oil (452 mg, 76%). ¹H NMR (CDCl₃) δ 7.42-7.33 (m, 2H), 7.32-7.24 (m, 3H), 4.15 (m, 1H), 4.04-3.64 (m, 2H), 3.16-2.73 (m, 1H), 2.25 (m, 1H), 2.09 (m, 1H), 2.00 (m, 1H), 1.92 (m, 1H), 1.51 (s, 9H); LCMS (ES⁺) Method 1: *m*/*z* 299 (M+H)⁺, RT 2.17 min.

### Step 2: tert-butyl 7-(aminomethyl)-7-phenyl-3-azabicyclo[4.1.0]heptane-3-carboxylate

*tert-butyl* 7-cyano-7-phenyl-3-azabicyclo[4.1.0]heptane-3-carboxylate (120 mg, 0.40 mmol) and CoCl₂ (208 mg, 1.6 mmol) were dissolved in MeOH (5 mL) in a pressure flask. The dark solution was cooled to 0 °C. NaBH₄ (456 mg, 12 mmol) was added portionwise. Then, the flask was sealed, and the reaction mixture was vigorously stirred for 12 h at rt. The solvent was removed *in vacuo* to obtain the crude compound, that was used in the next step without further purification. LCMS (ES⁺) Method 1: *m*/*z* 303 (M+H)⁺, RT 1.37 min.

### Step 3: tert-butyl 7-((((benzyloxy)carbonyl)amino)methyl)-7-phenyl-3-azabicyclo[4.1.0]heptane-3-carboxylate

To a solution of crude *tert-butyl* 7-(aminomethyl)-7-phenyl-3-azabicyclo[4.1.0]heptane-3-carboxylate (0.40 mmol) in DCM (5 mL), cooled to 0 °C, benzyl chloroformate (0.12 mL, 0.80 mmol) and TEA (0.09 mL, 0.60 mmol) were sequentially added. The reaction mixture was stirred at 0 °C for 30 min. H₂O was added and the phases were separated. Then the organic layer was washed with 5% citric acid solution (x2 mL), dried over Na₂SO₄, filtered and concentrated under reduced pressure. The resulting crude was purified by flash chromatography on silica gel (from 100% DCM to 10% EtOAc/DCM) affording the title compound as a colourless oil (100 mg, 57% over two steps). LCMS (ES⁺) Method 1: *m*/*z* 437 (M+H)⁺, RT 2.44 min.

### Step 4: 7-((((Benzyloxy)carbonyl)amino)methyl)-7-phenyl-3-azabicyclo[4.1.0]heptan-3-ium chloride (Intermediate 28)

*tert*-Butyl 7-((((benzyloxy)carbonyl)amino)methyl)-7-phenyl-3-azabicyclo[4.1.0]heptane-3-carboxylate (35 mg, 0.08 mmol) was dissolved in DCM (1 mL) and treated with HCl (4N in 1,4-dioxane, 1 mL, 4.0 mmol). The solution was stirred at rt for 1.5 h, then solvent was removed *in vacuo* to afford the title compound that was used in the next step without further purification (35 mg, 99%). LCMS (ES⁺) Method 1: *m*/*z* 337 (M+H)⁺, RT 1.42 min.

### Intermediate 29: Benzyl ((7-(pyridin-2-yl)-3-azabicyclo[4.1.0]heptan-7-yl)methyl)carbamate dihydrochloride

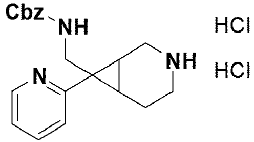

### Step 1: tert-butyl 7-cyano-7-(pyridin-2-yl)-3-azabicyclo[4.1.0]heptane-3-carboxylate

*tert-butyl* tetrahydro-[1,3,2]dioxathiolo[4,5-c]pyridine-5(4H)-carboxylate 2,2-dioxide **(Intermediate 1;** 520 mg, 1.86 mmol), and 2-(pyridin-2-yl)acetonitrile (242 mg, 2.05 mmol) were dissolved in dry DME (3.7 mL), and the resulting solution was added dropwise over a period of 30 min to a suspension of sodium hydride (60% in mineral oil; 186 mg, 4.6 mmol) in DME (6.7 mL) at rt under N₂ atmosphere. The reaction mixture was stirred at rt for 3 h, then quenched by addition of H₂O and extracted with EtOAc (x2). The combined organic layers were dried over Na₂SO₄, filtered and concentrated *in vacuo.* The residue was purified by reverse phase chromatography on a C18 cartridge (from 10% to 40% MeCN/H₂O + 0.1% TFA) to afford the title compound as a yellow oil (225 mg, 40%). ¹H NMR (CDCl₃) δ 8.45 (m, 1H), 7.80-7.64 (m, 2H), 7.16 (m, 1H), 4.22 (d, *J*=14.6 Hz, 1H), 4.09-3.78 (m, 1H), 3.72 (dd, *J*=14.6 and 5.3 Hz, 1H), 3.11-2.76 (m, 1H), 2.50-2.33 (m, 2H), 2.25 (m, 1H), 2.12-1.98 (m, 1H), 1.51 (s, 9H); LCMS (ES⁺) Method 1: *m*/*z* 300 (M+H)⁺, RT 1.93 min.

### Step 2: tert-butyl 7-(aminomethyl)-7-(pyridin-2-yl)-3-azabicyclo[4.1.0]heptane-3-carboxylate

*tert-*Butyl 7-cyano-7-(pyridin-2-yl)-3-azabicyclo[4.1.0]heptane-3-carboxylate (150 mg, 0.50 mmol) and CoCl₂ (357 mg, 1.5 mmol) were dissolved in MeOH (7 mL) in a pressure flask. The dark solution was cooled to 0 °C and NaBH₄ (568 mg, 15 mmol) was added portionwise. The flask was sealed and the reaction mixture was vigorously stirred for 12 h at rt. The solvent was removed *in vacuo* to obtain the crude compound that was used in the next step without further purification. LCMS (ES⁺) Method 1: *m*/*z* 304 (M+H)⁺, RT 1.02 min.

### Step 3: Tert-butyl 7-((((benzyloxy)carbonyl)amino)methyl)-7-(pyridin-2-yl)-3-azabicyclo[4.1.0]heptane-3-carboxylate

*tert*-Butyl 7-(aminomethyl)-7-(pyridin-2-yl)-3-azabicyclo[4.1.0]heptane-3-carboxylate from previous step (0.50 mmol) was reacted as described in the synthesis of **Intermediate 28,** Step 3. The residue was purified by preparative HPLC (from 5 to 45% MeCN/H₂O + 0.1% TFA) affording the title compound as a yellow solid (45 mg, 16% over two steps). LCMS (ES⁺) Method 1: *m*/*z* 438 (M+H)⁺, RT 1.52 min.

### Step 4: Benzyl ((7-(pyridin-2-yl)-3-azabicyclo[4.1.0]heptan-7-yl)methyl)carbamate dihydrochloride (Intermediate 29)

*tert*-Butyl 7-((((benzyloxy)carbonyl)amino)methyl)-7-(pyridin-2-yl)-3-azabicyclo[4.1.0]heptane-3-carboxylate (22 mg, 0.04 mmol) was dissolved in DCM (1 mL) and treated with HCl (4N in 1,4-dioxane, 1 mL, 4.0 mmol). The solution was stirred at rt for 1.5 h, then the solvent was removed *in vacuo* to afford the title compound (18 mg, 99%). LCMS (ES⁺) Method 1: *m*/*z* 338 (M+H)⁺, RT 0.85 min.

### Intermediate 30: 7-((((Benzyloxy)carbonyl)amino)methyl)-7-(pyrimidin-2-yl)-3-azabicyclo[4.1.0] heptan-3-ium chloride

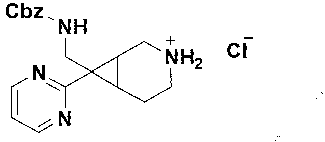

### Step 1: tert-butyl 7-cyano-7-(pyrimidin-2-yl)-3-azabicyclo[4.1.0]heptane-3-carboxylate

*tert-Butyl* tetrahydro-[1,3,2]dioxathiolo[4,5-c]pyridine-5(4H)-carboxylate 2,2-dioxide (**Intermediate 1;** 350 mg, 1.25 mmol), and 2-(pyrimidin-2-yl)acetonitrile (246 mg, 2.07 mmol, 1.65 eq) were dissolved in dry DME (0.5 M) and the resulting solution was added dropwise over a period of 30 min to a suspension of sodium hydride (60% in mineral oil; 2.6 eq) in DME (0.7 M) at rt under N₂ atmosphere. The reaction mixture was stirred at rt for 3 h, then quenched by addition of H₂O and extracted with EtOAc (x 2). Combined organic layers were dried over Na₂SO₄, filtered and concentrated *in vacuo.* The residue was purified by reverse phase chromatography on a C18 cartridge (from 10% to 40% MeCN/H₂O + 0.1% TFA) to afford the title compound as an orange foam (150 mg, 40%). ¹H NMR (CDCl₃) δ 8.67 (d, *J*=4.9 Hz, 2H), 7.18 (t, *J*=4.9 Hz, 1H), 4.18 (m, 1H), 3.99 (m, 1H), 3.75 (m, 1H), 3.29-2.76 (m, 1H), 2.58-2.19 (m, 3H), 2.09 (m, 1H), 1.51 (s, 9H); LCMS (ES⁺) Method 1: *m*/*z* 301 (M+H)⁺, RT 1.73 min.

### Step 2: tert-butyl 7-(aminomethyl)-7-(pyrimidin-2-yl)-3-azabicyclo[4.1.0]heptane-3-carboxylate

*tert-butyl* 7-cyano-7-(pyrimidin-2-yl)-3-azabicyclo[4.1.0]heptane-3-carboxylate (85 mg, 0.28 mmol) and CoCl₂ (3 eq) were dissolved in MeOH (0.07 M) in a pressure flask. The dark solution was cooled to 0 °C and NaBH₄ (30 eq) was added portionwise. The flask was sealed and the reaction mixture was vigorously stirred for 12 h at rt. The solvent was removed *in vacuo* to obtain the crude compound that was used in the next step without further purification. LCMS (ES⁺) Method 1: *m*/*z* 305 (M+H)⁺, RT 1.16 min.

### Step 3: tert-butyl 7-((((benzyloxy)carbonyl)amino)methyl)-7-(pyrimidin-2-yl)-3-azabicyclo[4.1.0]heptane-3-carboxylate

*tert*-Butyl 7-(aminomethyl)-7-(pyrimidin-2-yl)-3-azabicyclo[4.1.0]heptane-3-carboxylate (0.28 mmol) was reacted as described in the synthesis of **Intermediate 28,** Step 3. The residue was purified by flash chromatography on silica gel (from 10% to 50% EtOAc in petroleum ether) affording the title compound as a colourless oil (18 mg, 15% over two steps). ¹H NMR (CDCl₃) δ 8.59 (d, *J*=4.9 Hz, 2H), 7.47-7.29 (m, 5H), 7.07 (t, *J*=4.9 Hz, 1H), 6.29-5.75 (m, 1H), 5.11 (m, 2H), 3.89 (m, 4H), 3.65 (m, 1H), 2.88 (m, 1H), 2.21-2.01 (m, 2H), 1.90 (m, 1H), 1.63 (m, 1H), 1.51 (s, 9H); LCMS (ES⁺) Method 1: *m*/*z* 439 (M+H)⁺, RT 2.23 min.

### Step 4: 7-((((Benzyloxy)carbonyl)amino)methyl)-7-(pyrimidin-2-yl)-3-azabicyclo[4.1.0]heptan-3-ium (Intermediate 30)

*tert*-Butyl 7-((((benzyloxy)carbonyl)amino)methyl)-7-(pyrimidin-2-yl)-3-azabicyclo[4.1.0]heptane-3-carboxylate (18 mg, 0.04 mmol) was dissolved in DCM (1 mL) and treated with HCl (4N in 1,4-dioxane, 1 mL, 4.0 mmol). The solution was stirred at rt for 1.5 h, then solvent was removed *in vacuo* to afford the title compound to give the title compound (15 mg; 97%). LCMS (ES⁺) Method 1: *m*/*z* 339 (M+H)⁺, RT 1.24 min.

### Intermediate 31: 7-((((Benzyloxy)carbonyl)amino)methyl)-7-(thiophen-3-yl)-3-azabicyclo[4.1.0] heptan-3-ium chloride

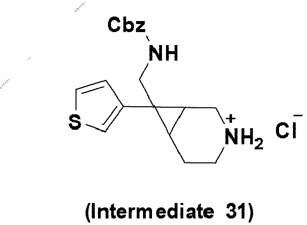

### Step 1: tert-butyl 7-cyano-7-(thiophen-3-yl)-3-azabicyclo[4.1.0]heptane-3-carboxylate

*tert-Butyl* tetrahydro-[1,3,2]dioxathiolo[4,5-c]pyridine-5(4H)-carboxylate 2,2-dioxide **(Intermediate 1;** 350 mg, 1.25 mmol) and 2-(thiophen-3-yl)acetonitrile (170 mg, 1.38 mmol) were dissolved in dry DMF (0.45 M) and the resulting solution was added dropwise over a period of 30 min to a suspension of sodium hydride (60% in mineral oil; 2.5 eq) in dry DMF (1.8 M) cooled to 0 °C under N₂ atmosphere. The reaction mixture was stirred at 0 °C for 10 min then at rt for 30 min. The reaction was quenched by addition of brine then diluted with EtOAc. The organic layer was washed with brine (x 2) and H₂O (x 2), dried over Na₂SO₄, filtered and concentrated *in vacuo.* The residue was purified by flash chromatography on silica gel (from 0% to 5% EtOAc in petroleum ether) to afford the title compound as a yellow oil (140 mg, 37%). ¹H NMR (CDCl₃) δ 7.33 (m, 1H), 7.17 (m, 1H), 6.90 (m, 1H), 4.14 (m, 1H), 4.04-3.61 (m, 2H), 3.13-2.71 (m, 1H), 2.22 (m, 1H), 2.11-1.75 (m, 3H), 1.50 (s, 9H); LCMS (ES⁺) Method 1: *m*/*z* 305 (M+H)⁺, RT 2.12 min.

### Step 2: Tert-butyl 7-(aminomethyl)-7-(thiophen-3-yl)-3-azabicyclo[4.1.0]heptane-3-carboxylate

*tert-Butyl* 7-cyano-7-(thiophen-3-yl)-3-azabicyclo[4.1.0]heptane-3-carboxylate (65 mg, 0.21 mmol) and CoCl₂ (3 eq) were dissolved in MeOH (0.07 M) in a pressure flask. The dark solution was cooled to 0 °C and NaBH₄ (30 eq) was added portionwise. The flask was sealed and the reaction mixture was vigorously stirred for 12 h at rt. The solvent was removed *in vacuo* to obtain crude compound that was used in the next step without further purification. LCMS (ES⁺) Method 1: *m*/*z* 309 (M+H)⁺, RT 1.32 min.

### Step 3: Tert-butyl 7-((((benzyloxy)carbonyl)amino)methyl)-7-(thiophen-3-yl)-3-azabicyclo[4.1.0]heptane-3-carboxylate

*tert-Butyl* 7-(aminomethyl)-7-(thiophen-3-yl)-3-azabicyclo[4.1.0]heptane-3-carboxylate (0.21 mmol) was reacted as described in the synthesis of **Intermediate 28,** Step 3. The residue was purified by preparative HPLC (from 30% to 75% MeCN/H₂O + 0.1% HCOOH) affording the title compound as a white solid (5 mg, 5% over two steps). LCMS (ES⁺) Method 1: *m*/*z* 443 (M+H)⁺, RT 2.41 min.

### Step 4: 7-((((Benzyloxy)carbonyl)amino)methyl)-7-(thiophen-3-yl)-3-azabicyclo[4.1.0]heptan-3-ium chloride (Intermediate 31)

*tert-Butyl* 7-((((benzyloxy)carbonyl)amino)methyl)-7-(thiophen-3-yl)-3-azabicyclo[4.1.0]heptane-3-carboxylate (5 mg, 0.01 mmol) was dissolved in DCM (1 mL) and treated with 0.5 mL of HCl (4N in 1,4-dioxane). LCMS (ES⁺) Method 1: *m*/*z* 343 (M+H)⁺, RT 1.36 min.

### Intermediate 32: 7-((((Benzyloxy)carbonyl)amino)methyl)-7-(4-methylthiazol-2-yl)-3-azabicyclo[4.1.0]heptan-3-ium chloride

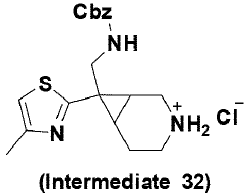

### Step 1: tert-butyl 7-cyano-7-(4-methylthiazol-2-yl)-3-azabicyclo[4.1.0]heptane-3-carboxylate

*Tert*-butyl tetrahydro-[1,3,2]dioxathiolo[4,5-c]pyridine-5(4H)-carboxylate 2,2-dioxide **(Intermediate 1;** 154 mg, 0.55 mmol), 15-crown-5 (11 µL, 0.055 mmol) and 2-(4-methylthiazol-2-yl)acetonitrile (84 mg, 0.61 mmol) were dissolved in dry DME (0.5 M) and the resulting solution was added dropwise over a period of 30 min to a suspension of sodium hydride (60% in mineral oil; 2.6 eq) in DME (0.7 M) at rt under N2 atmosphere. The reaction mixture was stirred at rt for 3 h, then quenched by addition of H₂O and extracted with EtOAc (x2). The combined organic layers were dried over Na₂SO₄, filtered and concentrated in vacuo. The residue was purified by flash chromatography on silica gel (from 0% to 20% EtOAc in petroleum ether) to afford the title compound as a red oil (100 mg, 57%). ¹H NMR (CDCl₃) δ 6.76 (br d, *J*=0.9 Hz, 1H), 4.25 (d, *J*=14.8 Hz, 1H), 4.10-3.75 (m, 1H), 3.69 (dd, *J*=14.80 and 5.0 Hz, 1H), 3.05-2.70 (m, 1H), 2.55 - 2.40 (m, 2H), 2.38 (d, *J*=0.9 Hz, 3H), 2.33-2.15 (m, 1H), 2.13-1.98 (m, 1H), 1.50 (s, 9H); LCMS (ES⁺) Method 1: *m*/*z* 320 (M+H)⁺, RT 2.00 min.

### Step 2: Tert-butyl 7-(aminomethyl)-7-(4-methylthiazol-2-yl)-3-azabicyclo[4.1.0]heptane-3-carboxylate

To a solution of *tert-butyl* 7-cyano-7-(4-methylthiazol-2-yl)-3-azabicyclo[4.1.0]heptane-3-carboxylate (120 mg, 0.38 mmol) in dry THF (0.16 M), with borane methylsulfanylmethane (2M in THF; 4 eq) was added and the reaction mixture was stirred at 50 °C for 1 h. Additional borane methylsulfanylmethane (2M in THF; 2 eq) was added and the reaction mixture was stirred at 50 °C for 2 h. The reaction was quenched by addition of EtOH (0.08 M) and the resulting solution was stirred at 70 °C for 3 h. The solvent was removed *in vacuo* to obtain the crude title compound, which was used in the next step without further purification. LCMS (ES⁺) Method 1: *m*/*z* 324 (M+H)⁺, RT 1.29 min.

### Step 3: Tert-butyl 7-((((benzyloxy)carbonyl)amino)methyl)-7-(4-methylthiazol-2-yl)-3-azabicyclo[4.1.0]heptane-3-carboxylate

*t*ert-Butyl 7-(aminomethyl)-7-(4-methylthiazol-2-yl)-3-azabicyclo[4.1.0]heptane-3-carboxylate (0.38 mmol) was prepared according to the procedure described for **Intermediate 28,** Step 3. The residue was purified by flash chromatography on silica gel (from 0% to 100 % EtOAc in DCM) affording the title compound as a light-yellow oil (29 mg, 17% over two steps). ¹H NMR (CDCl₃) δ 7.43-7.30 (m, 5H), 6.70 (s, 1H), 5.86-5.42 (m, 1H), 5.11 (m, 2H), 4.03 (d, *J*=14.8 Hz, 1H), 3.96-3.69 (m, 3H), 3.64-3.46 (m, 1H), 2.95-2.53 (m, 1H), 2.40 (s, 3H), 2.22-2.03 (m, 1H), 2.01-1.77 (m, 2H), 1.70 (m, 1H), 1.48 (s, 9H); LCMS (ES⁺) Method 1: *m*/*z* 458 (M+H)⁺, RT 2.10 min.

### Step 4: 7-((((Benzyloxy)carbonyl)amino)methyl)-7-(4-methylthiazol-2-yl)-3-azabicyclo[4.1.0]heptan-3-ium chloride (Intermediate 32)

*Tert-butyl* 7-((((benzyloxy)carbonyl)amino)methyl)-7-(4-methylthiazol-2-yl)-3-azabicyclo[4.1.0]heptane-3-carboxylate (29 mg, 0.06 mmol) was dissolved in DCM (1 mL) and treated with 1 mL of HCl (4N in 1,4-dioxane). The solution was stirred at rt for 1.5 h, then the solvent was removed *in vacuo* to afford the title compound (29 mg; 99%). LCMS (ES⁺) Method 1: *m*/*z* 358 (M+H)⁺, RT 1.26 min.

### Intermediate 33: 7-((((Benzyloxy)carbonyl)amino)methyl)-7-(1-methyl-1H-pyrazol-3-yl)-3-azabicyclo[4.1.0]heptan-3-ium chloride

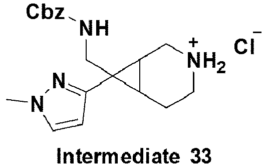

### Step 1: tert-butyl 7-cyano-7-(1-methyl-1H-pyrazol-3-yl)-3-azabicyclo[4.1.0]heptane-3-carboxylate

*tert-Butyl* tetrahydro-[1,3,2]dioxathiolo[4,5-c]pyridine-5(4H)-carboxylate 2,2-dioxide **(Intermediate 1;** 100 mg, 0.36 mmol) and 2-(1-methyl-1H-pyrazol-3-yl)acetonitrile (47 mg, 0.39 mmol) were dissolved in dry DMF (0.45 M) and the resulting solution was added dropwise over a period of 30 min to a suspension of sodium hydride (60% in mineral oil; 2.5 eq) in dry DMF (1.8 M) cooled to 0 °C under N₂ atmosphere. The reaction mixture was stirred at 0 °C for 10 min then at rt for 30 min. The reaction was quenched by addition of brine then diluted with EtOAc. The organic layer was washed with brine (x2) and H₂O (x2), dried over Na₂SO₄, filtered and concentrated *in vacuo.* The residue was purified by flash chromatography on silica gel (from 0% to 100% EtOAc in petroleum ether) to afford the title compound as yellow oil (24 mg, 22%). ¹H NMR (300 MHz, CDCl₃) δ 7.22-7.15 (m, 1H), 6.23 (d, *J*=2.2 Hz, 1H), 4.04 (br d, *J*=13.7 Hz, 1H), 3.93-3.51 (m, 5H), 2.97-2.70 (m, 1H), 2.19-1.81 (m, 4H), 1.40 (s, 9H); LCMS (ES⁺) Method 1: *m*/*z* 303 (M+H)⁺, RT 1.72 min.

### Step 2: Tert-butyl 7-(aminomethyl)-7-(1-methyl-1H-pyrazol-3-yl)-3-azabicyclo[4.1.0]heptane-3-carboxylate

To a solution of *tert*-butyl 7-cyano-7-(1-methyl-1H-pyrazol-3-yl)-3-azabicyclo[4.1.0]heptane-3-carboxylate (23 mg, 0.08 mmol) in dry THF (0.16 M), borane methylsulfanylmethane (2M in THF; 4 eq) was added and the reaction mixture was stirred at 50 °C for 1 h. Additional borane methylsulfanylmethane (2M in THF; 2 eq) was added and the reaction mixture was stirred at 50 °C for 2 h. The reaction was quenched by addition of EtOH (0.08 M) and the resulting solution was stirred at 70 °C for 3 h. The solvent was removed *in vacuo* to obtain the crude title compound, which was used in the next step without further purification. LCMS (ES⁺) Method 1: *m*/*z* 307 (M+H)⁺, RT 1.21 min.

### Step 3: Tert-butyl 7-((((benzyloxy)carbonyl)amino)methyl)-7-(1-Methyl-1H-pyrazol-3-yl)-3-azabicyclo[4.1.0]heptane-3-carboxylate

*Tert*-butyl 7-(aminomethyl)-7-(1-methyl-1H-pyrazol-3-yl)-3-azabicyclo[4.1,0]heptane-3-carboxylate (0.08 mmol) was reacted as described in the synthesis of **Intermediate 28,** Step 3. The residue was purified by flash chromatography on silica gel (from 0% to 100% EtOAc/DCM) affording the title compound as a pale yellow oil (17 mg, 49% over two steps). ¹H NMR (300 MHz, CDCl₃) δ 7.34-7.11 (m, 6H), 5.89 (br s, 1H), 5.50-5.25 (m, 1H), 5.14-4.90 (m, 2H), 3.93-3.39 (m, 8H), 2.80-2.52 (m, 1H), 2.06-1.91 (m, 1H), 1.78-1.54 (m, 3H), 1.39 (s, 9H); LCMS (ES⁺) Method 1: *m*/*z* 441 (M+H)⁺, RT 2.05 min.

### Step 4: 7-((((Benzyloxy)carbonyl)amino)methyl)-7-(1-methyl-1H-pyrazol-3-yl)-3-azabicyclo[4.1.0]heptan-3-ium chloride (Intermediate 33)

*tert*-Butyl 7-((((benzyloxy)carbonyl)amino)methyl)-7-(1-methyl-1H-pyrazol-3 -yl)-3 - azabicyclo[4.1.0]heptane-3-carboxylate (17 mg, 0.039 mmol) was dissolved in DCM (1 mL) and treated with 1 mL of HCl (4N in 1,4-dioxane). The solution was stirred at rt for 1.5 h, then the solvent was removed *in vacuo* to give the title compound (17 mg; yield). LCMS (ES⁺) Method 1: *m*/*z* 341 (M+H)⁺, RT 1.19 min.

### Intermediate 34: 7-((((Benzyloxy)carbonyl)amino)methyl)-7-(1-methyl-1H-pyrazol-5-yl)-3-azabicyclo[4.1.0]heptan-3-ium chloride

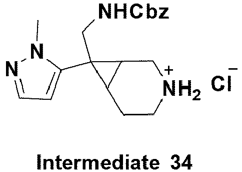

### Step 1: tert-butyl 7-cyano- 7-(1-methyl-1H-pyrazol-5-yl)-3-azabicyclo[4.1.0]heptane-3-carboxylate

*tert*-Butyl tetrahydro-[1,3,2]dioxathiolo[4,5-c]pyridine-5(4H)-carboxylate 2,2-dioxide **(Intermediate 1;** 100 mg, 0.36 mmol) and 2-(1-methyl-1H-pyrazol-5-yl)acetonitrile (50 mg, 0.39 mmol) were dissolved in dry DMF (0.45 M) and the resulting solution was added dropwise over a period of 30 min to a suspension of sodium hydride (60% in mineral oil; 2.5 eq) in dry DMF (1.8 M) cooled to 0 °C under N₂ atmosphere. The reaction mixture was stirred at 0 °C for 10 min then at rt for 30 min. The reaction was quenched by addition of brine then diluted with EtOAc. The organic layer was washed with brine (x2) and H₂O (x2), dried over Na₂SO₄, filtered and concentrated *in vacuo.* The residue was purified by flash chromatography on silica gel (from 0% to100% EtOAc in petroleum ether) to afford the title compound as a yellow oil (44 mg, 41%). ¹H NMR (300 MHz, CDCl₃) δ 7.31 (s, 1H), 6.01 (s, 1H), 4.20 (br s, 1H), 3.92-3.60 (m, 5H), 2.93-2.70 (m, 1H), 2.17 (br s, 1H), 2.07-1.72 (m, 3H), 1.40 (s, 9H); LCMS (ES⁺) Method 1: *m*/*z* 303 (M+H)⁺, RT 1.66 min.

### Step 2: Tert-butyl 7-(aminomethyl)-7-(1-methyl-1H-pyrazol-5-yl)-3-azabicyclo[4.1.0]heptane-3-carboxylate

To a solution of *tert*-butyl 7-cyano-7-(1-methyl-1H-pyrazol-5-yl)-3-azabicyclo[4.1.0]heptane-3-carboxylate (40 mg, 0.13 mmol) in dry THF (0.16 M), borane methylsulfanylmethane (2M in THF; 4 eq) was added and the reaction mixture was stirred at 50 °C for 1 h. Additional borane methylsulfanylmethane (2M in THF ; 2 eq) was added and the reaction mixture was stirred at 50 °C for 2 h. The reaction was quenched by addition of EtOH (0.08 M) and the resulting solution was stirred at 70 °C for 3 h. The solvent was removed *in vacuo* to obtain crude title compound, which was used in the next step without further purification. LCMS (ES⁺) Method 1 *m*/*z* 307 (M+H)⁺, RT 1.10 min.

### Step 3: Tert-butyl 7-((((benzyloxy)carbonyl)amino)methyl)-7-(1-Methyl-1H-pyrazol-5-yl)-3-azabicyclo[4.1.0]heptane-3-carboxylate

The compound was prepared according to the procedure described in Step 3 of the synthesis of **Intermediate 28,** starting from *tert*-butyl 7-(aminomethyl)-7-(1-methyl-1H-pyrazol-5-yl)-3-azabicyclo[4.1.0]heptane-3-carboxylate (0.13 mmol). The residue was purified by flash chromatography on silica gel (from 0% to 100% EtOAc in DCM) affording the title compound as a light-yellow oil (30 mg, 52% over two steps). ¹H NMR (300 MHz, CDCl₃) δ 7.34-7.17 (m, 6H), 5.97 (s, 1H), 4.97-4.76 (m, 3H), 4.21-3.20 (m, 9H), 2.57 (br s, 1H), 2.13-1.91 (m, 1H), 1.83-1.47 (m, 2H), 1.39 (s, 9H); Method 1: *m*/*z* 441 (M+H)⁺, RT 1.93 min.

### Step 4: 7-((((Benzyloxy)carbonyl)amino)methyl)-7-(1-methyl-1H-pyrazol-5-yl)-3-azabicyclo[4.1.0]heptan-3-ium chloride

*tert*-Butyl 7-((((benzyloxy)carbonyl)amino)methyl)-7-(1-methyl-1H-pyrazol-5 -yl)-3 - azabicyclo[4.1.0]heptane-3-carboxylate (30 mg, 0.07 mmol) was dissolved in DCM (1 mL) and treated with 1 mL of HCl (4N in 1,4-dioxane). The solution was stirred at rt for 1.5 h, then solvent was removed *in vacuo* to give the title compound (30 mg). LCMS (ES⁺) Method 1: *m*/*z* 341 (M+H)⁺, RT 1.07 min.

### Intermediate 35: 7-((((Benzyloxy)carbonyl)amino)methyl)-7-(isoxazol-3-yl)-3-azabicyclo[4.1.0] heptan-3-ium chloride

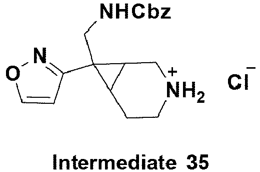

### Step 1: Tert-butyl 7-cyano-7-(isoxazol-3-yl)-3-azabicyclo[4.1.0]heptane-3-carboxylate

*Tert*-butyl tetrahydro-[1,3,2]dioxathiolo[4,5-c]pyridine-5(4H)-carboxylate 2,2-dioxide **(Intermediate 1;** 100 mg, 0.36 mmol), 15-crown-5 (7 µL, 0.055 mmol) and 2-(isoxazol-3-yl)acetonitrile (42 mg, 0.39 mmol) were dissolved in dry DME (0.5 M), and the resulting solution was added dropwise over a period of 30 min to a suspension of sodium hydride (60% in mineral oil; 2.6 eq) in DME (0.7 M) at rt under N₂ atmosphere. The reaction mixture was stirred at rt for 3 h, then quenched by addition of H₂O and extracted with EtOAc (x2). Combined organic layers were dried over Na₂SO₄, filtered and concentrated *in vacuo.* The residue was purified by flash chromatography on silica gel (from 0% to 100% EtOAc in petroleum ether) to afford the title compound as a yellow oil (48 mg, 46%). ¹H NMR (300 MHz, CDCl₃) δ 8.30 (d, *J*=1.6 Hz, 1H), 6.47 (d, *J*=1.6 Hz, 1H), 4.20 (br d, *J*=14.7 Hz, 1H), 3.97-3.72 (m, 1H), 3.58 (dd, *J*=14.7, 5.0 Hz, 1H), 2.92-2.62 (m, 1H), 2.30-2.09 (m, 3H), 2.05-1.89 (m, 1H), 1.40 (s, 9H); LCMS (ES⁺) Method 1: *m*/*z* 290 (M+H)⁺, RT 1.76 min.

### Step 2: Tert-butyl 7-(aminomethyl)-7-(isoxazol-3-yl)-3-azabicyclo[4.1.0]heptane-3-carboxylate

To a solution of *tert*-butyl 7-cyano-7-(isoxazol-3-yl)-3-azabicyclo[4.1.0]heptane-3-carboxylate (48 mg, 0.17 mmol) in dry THF (0.16 M), borane methylsulfanylmethane (2M in THF ; 4 eq) was added and the reaction mixture was stirred at 50 °C for 1 h. Additional borane methylsulfanylmethane (2M in THF; 2 eq) was added and the reaction mixture was stirred at 50 °C for 2 h. The reaction was quenched by addition of EtOH (0.08 M) and the resulting solution was stirred at 70 °C for 3 h. Solvent was removed *in vacuo* to obtain the crude title compound, which was used in the next step without further purification. LCMS (ES⁺) Method 1: *m*/*z* 294 (M+H)⁺, RT 1.13 min.

### Step 3: Tert-butyl 7-((((benzyloxy)carbonyl)amino)methyl)-7-(isoxazol-3-yl)-3-azabicyclo[4.1.0]heptane-3-carboxylate

*tert*-Butyl 7-(aminomethyl)-7-(isoxazol-3-yl)-3-azabicyclo[4.1.0]heptane-3-carboxylate (0.16 mmol) was prepared according to the procedure described for **Intermediate 28,** Step 3. The residue was purified by flash chromatography on silica gel (from 0% to 50% EtOAc in DCM) affording the title compound as a pale yellow oil (25 mg; 36% over two steps). Method 1: *m*/*z* 428 (M+H)⁺, RT 2.14 min.

### Step 4: 7-((((Benzyloxy)carbonyl)amino)methyl)-7-(isoxazol-3-yl)-3-azabicyclo[4.1.0]heptan-3-ium chloride (Intermediate 35)

*tert-butyl* 7-((((benzyloxy)carbonyl)amino)methyl)-7-(isoxazol-3-yl)-3-azabicyclo[4.1.0]heptane-3-carboxylate (25 mg , 0.06 mmol) was dissolved in DCM (1 mL) and treated with an excess of HCl 4N in 1,4-dioxane. The solution was stirred at rt for 1.5 h, then the solvent was removed *in vacuo* to give the title compound (25 mg; 99%). LCMS (ES⁺) Method 1: *m*/*z* 328 (M+H)⁺, RT 1.21 min.

### Intermediate 36: 7-((1,3-Dioxoisoindolin-2-yl)methyl)-7-(4-methylthiazol-2-yl)-3-azabicyclo[4.1.0]heptan-3-ium chloride

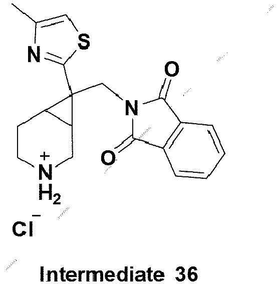

### Step 1: Tert-butyl 7-((1,3-dioxoisoindolin-2-yl)methyl)-7-(4-methylthiazol-2-yl)-3-azabicyclo[4.1. 0]heptane-3-carboxylate

To a solution of *tert*-butyl 7-(aminomethyl)-7-(4-methylthiazol-2-yl)-3-azabicyclo[4.1.0]heptane-3-carboxylate (prepared as described in the synthesis of **Intermediate 32,** Step 2; 185 mg, 0.57 mmol) and phthalic anhydride (102 mg, 0.69 mmol) in dry toluene (6.5 mL), TEA (0.12 mL, 0.86 mmol) was added and the solution was stirred at 100 °C for 8 h. The reaction mixture was cooled to rt, then diluted with EtOAc and washed with 5% citric acid solution (x2), NaHCO₃ sat. sol. (x2) and brine (x2). The organic layer was dried over Na₂SO₄, filtered and concentrated under reduced pressure. The resulting crude was purified by flash chromatography on silica gel (from 0% to 40% EtOAc/DCM) affording the title compound as an off-white foam (173 mg, 67%). ¹H NMR (300 MHz, CDCl₃) δ 7.80 (m, 2H), 7.69 (m, 2H), 6.58 (s, 1H), 4.52 (m, 1H), 4.20 (d, *J*=14.4 Hz, 1H), 4.11-3.80 (m, 2H), 3.52 (dd, *J*=14.4 and 6.1 Hz, 1H), 2.69 (m, 1H), 2.44-2.15 (m, 2H), 2.11 (s, 3H), 1.97 (m, 1H), 1.71 (m, 1H), 1.47 (s, 9H); LCMS (ES⁺) Method 1: *m*/*z* 454 (M+H)⁺, RT 2.03 min.

### Step 2: 7-((1,3-Dioxoisoindolin-2-yl)methyl)-7-(4-methylthiazol-2-yl)-3-azabicyclo[4.1.0]heptan-3-ium chloride (Intermediate 36)

*tert-*butyl 7-((1,3-dioxoisoindolin-2-yl)methyl)-7-(4-methylthiazol-2-yl)-3-azabicyclo[4.1.0]heptane-3-carboxylate (173 mg, 0.38 mmol) was dissolved in DCM (1 mL) and treated with an excess of HCl 4N in 1,4-dioxane. The solution was stirred at rt for 1.5 h, then solvent was removed *in vacuo* to afford the title compound (169 mg; 99%). LCMS (ES⁺) *m*/*z* 354 (M+H)⁺, RT 1.08 min.

### Intermediate 37: 7-((1,3-Dioxoisoindolin-2-yl)methyl)-7-(thiophen-3-yl)-3-azabicyclo[4.1.0] heptan-3-ium chloride

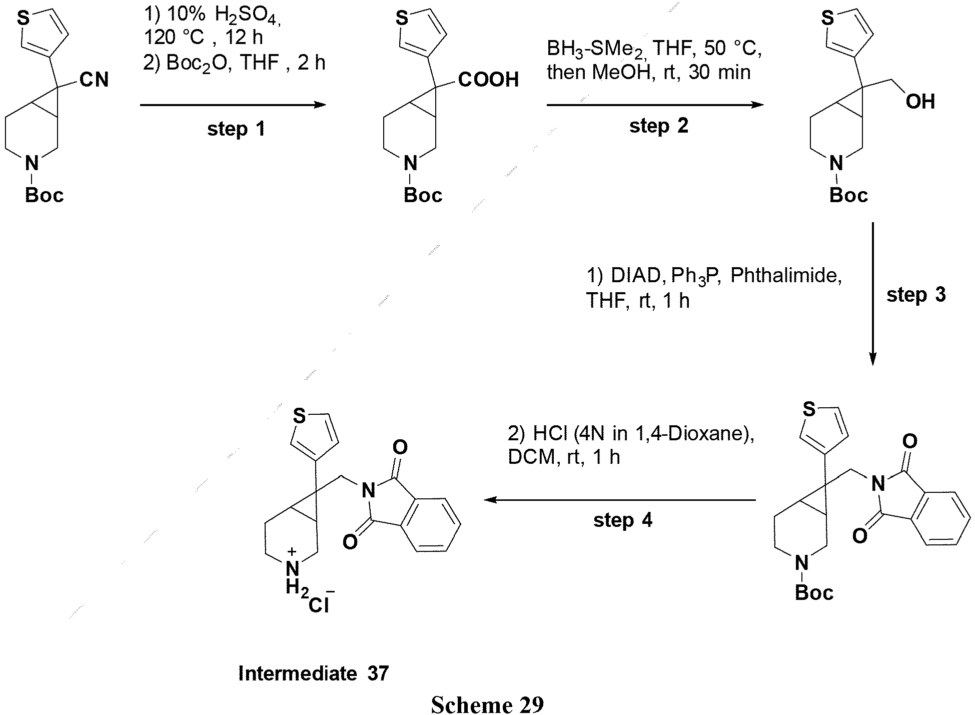

### Step 1: 3-(tert-butoxycarbonyl)-7-(thiophen-3-yl)-3-azabicyclo[4.1.0]heptane-7-carboxylic acid

*Tert*-butyl 7-cyano-7-(thiophen-3-yl)-3-azabicyclo[4.1.0]heptane-3-carboxylate (prepared as described in Step 1 for the synthesis of Intermediate 31 (140 mg, 0.46 mmol) was dissolved in 10% aq. H₂SO₄ solution (5 mL) and the reaction mixture was stirred for 15 h at 130 °C. The reaction was then cooled to 0 °C and treated with a solution of 10N NaOH until pH=10. The reaction mixture was diluted with THF (5 mL) di-*tert*-butyl dicarbonate (351 mg, 1.61 mmol) was added and was vigorously stirred at rt for 2.5 h, then diluted with DCM and the layers were separated. The aqueous phase was treated with 6N HCl solution until pH=2 and extracted with EtOAc (x2). Collected organic layers were dried over Na₂SO₄, filtered and evaporated to afford the title compound as a beige solid, which was used in the next step without further purification (139 mg, 93%). LCMS (ES⁺) Method 1: *m*/*z* 324 (M+H)⁺, RT 1.91 min.

### Step 2: Tert-butyl 7-(hydroxymethyl)-7-(thiophen-3-yl)-3-azabicyclo[4.1.0]heptane-3-carboxylate

To a solution of 3-(*tert*-butoxycarbonyl)-7-(thiophen-3-yl)-3-azabicyclo[4.1.0]heptane-7-carboxylic acid (100 mg, 0.31 mmol) in dry THF (2 mL), borane methylsulfanylmethane (2M in THF; 0.54 mL, 1.08 mmol) was added and the reaction mixture was stirred at rt for 12 h. The reaction was quenched by addition of MeOH (4 mL), stirred at rt for 30 min then solvent evaporated under reduced pressure. The resulting crude was purified by flash chromatography on silica gel (from 0% to 30% EtOAc in DCM) to afford the title compound as a colourless oil (78 mg, 82%). LCMS (ES⁺) Method 1: *m*/*z* 310 (M+H)⁺, RT 1.94 min.

### Step 3: Tert-butyl 7-((1,3-dioxoisoindolin-2-yl)methyl)-7-(thiophen-3-yl)-3-azabicyclo[4.1.0]heptane-3-carboxylate

To a solution of *tert*-butyl 7-(hydroxymethyl)-7-(thiophen-3-yl)-3-azabicyclo[4.1.0]heptane-3-carboxylate (110 mg, 0.36 mmol), phthalimide (63 mg, 0.43 mmol) and PPh₃ (158.5 mg, 0.60 mmol) in dry THF (3.4 mL), DIAD (0.12 mL, 0.60 mmol) was added dropwise at rt. The resulting yellow solution was stirred at rt for 1 h, then solvent was removed *in vacuo* and the residue was purified by flash chromatography on silica gel (from 10% to 20% EtOAc in petroleum ether) to afford the title compound as a white foam (145 mg, 93%). LCMS (ES⁺) Method 1: *m*/*z* 439 (M+H)⁺, RT 2.41 min.

### Step 4: 2-((7-(Thiophen-3-yl)-3-azabicyclo[4.1.0]heptan-7-yl)methyl)isoindoline-1,3-dione (Intermediate 37)

*tert*-Butyl 7-((1,3-dioxoisoindolin-2-yl)methyl)-7-(thiophen-3-yl)-3-azabicyclo[4.1.0]heptane-3-carboxylate (145 mg, 0.33 mmol) was dissolved in DCM (2 mL) and treated with HCl (4N in dioxane 2.07 mL, 8.27 mmol). The solution was stirred at rt for 1 h, then solvent was removed *in vacuo* to afford the crude title compound that was used in the next step without further purification (145 mg; 99%). LCMS (ES⁺) Method 1: *m*/*z* 339 (M+H)⁺, RT 1.28 min.

### Intermediate 38: 2-((3-Azabicyclo[4.1.0]heptan-7-yl)methyl)isoindoline-1,3-dione

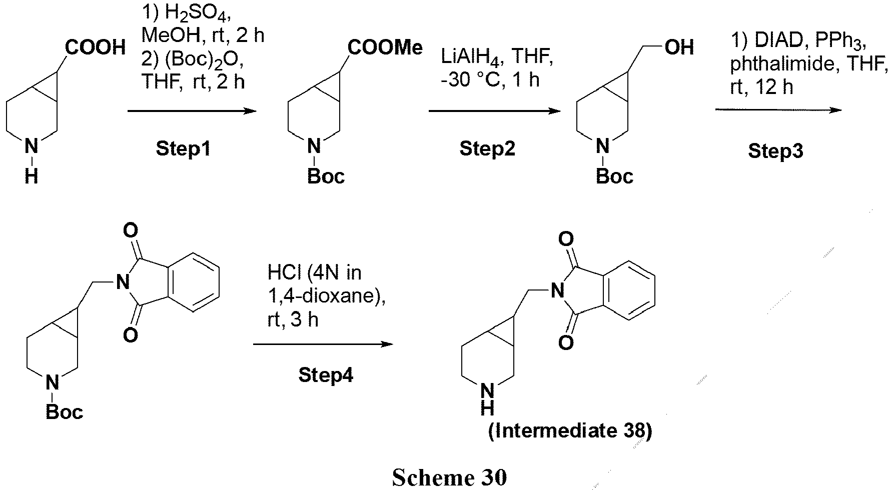

### Step 1: 3-(Tert-butyl) 7-methyl 3-azabicyclo[4.1.0]heptane-3,7-dicarboxylate

To a solution of 3-azabicyclo[4.1.0]heptane-7-carboxylic acid hydrochloride (100.mg, 0.56 mmol) in MeOH (1 mL) 1 drop of conc. H₂SO₄ was added. The resulting mixture was stirred at rt for 2 h and then treated with 2M NaOH until pH = 7.5. After dilution in THF (1 mL) di-*tert*-butyl dicarbonate (154 mg, 0.71 mmol) and DIPEA (0.19 mL, 1.06 mmol) were added. The resulting mixture was stirred for 2 h at rt; MeOH was evaporated and the aqueous residue was extracted with EtOAc (x2). The collected organic layers were washed with brine (x2), dried over Na₂SO₄ filtered and concentrated affording the title compound which was used in the next step without further purification (130 mg). LCMS (ES⁺) Method 1: *m*/*z* 256 (M+H)⁺, RT 1.96.min.

### Step 2: Tert-butyl 7-(hydroxymethyl)-3-azabicyclo[4.1.0]heptane-3-carboxylate

3-(*Tert*-butyl) 7-methyl 3-azabicyclo[4.1.0]heptane-3,7-dicarboxylate from previous step (130 mg, 0.51 mmol) was dissolved in dry THF (23 mL) under N₂ atmosphere, and the solution was cooled to -30 °C. LiAlH₄ (solution 2M in THF, 0.28 mL,0.56 mmol) was added dropwise, and the reaction was stirred between -30 °C and -20 °C for 1 h. The reaction was diluted with EtOAc (10 mL) and quenched with saturated solution of Rochelle salt and H₂O (10 mL). After about 30 min stirring at rt, the mixture was dried over Na₂SO₄, filtered and concentrated under reduced pressure. The resulting crude compound was used in the next step without any further purification (100 mg; yield). LCMS (ES⁺) Method 1: *m*/*z* 228 (M+H)⁺, RT 1.50 min.

### Step 3: Tert-butyl 7-((1,3-dioxoisoindolin-2-yl)methyl)-3-azabicyclo[4.1.0]heptane-3-carboxylate

*tert*-Butyl 7-(hydroxymethyl)-3-azabicyclo[4.1.0]heptane-3-carboxylate (100 mg, 0.44mmol), phthalimide (77 mg, 0.53 mmol), PPh₃ (196 mg, 0.75 mmol) were dissolved in dry THF (3.5 mL) then DIAD (0.15 mL,0.75 mmol) was added dropwise. The resulting yellow solution was stirred at rt for 2 h. Solvent was removed under reduced pressure and the resulting crude was purified by flash chromatography on silica gel (from 10% to 20% EtOAc in petroleum ether), affording the title compound (100 mg, 64% over 3 steps). ¹H NMR (300 MHz, CDCl₃) δ 7.83-7.74 (m, 2H), 7.70-7.61 (m, 2H), 3.66-3.38 (m, 4H), 3.24-3.12 (m, 1H), 2.97-2.84 (m, 1H), 1.91-1.76 (m, 1H), 1.65-1.56 (m, 1H), 1.38-1.30 (m, 9H), 1.09-0.92 (m, 3H); LCMS (ES⁺) Method 1: *m*/*z* 357 (M+H)⁺, RT 2.20 min.

### Step 4: 2-((3-Azabicyclo[4.1.0]heptan-7-yl)methyl)isoindoline-1,3-dione (Intermediates 38)

*Tert*-butyl 7-((1,3-dioxoisoindolin-2-yl)methyl)-3-azabicyclo[4.1.0]heptane-3-carboxylate (100 mg, 0.28 mmol) was treated with 1 mL of HCl (4N in 1,4-dioxane) and the resulting solution was stirred at rt for 3 h. After evaporation, the compound was used without further purification. LCMS (ES⁺) Method 1: *m*/*z* 257 (M+H)⁺, RT 1.02 min.

### Intermediates 39: Benzyl (((1S,6R,7S)-7-(4-methylthiazol-2-yl)-3-azabicyclo[4.1.0]heptan-7-yl)methyl)carbamate

The synthesis of **Intermediate 39** and of **Intermediate 40** were performed as described in the **Scheme 31** below:

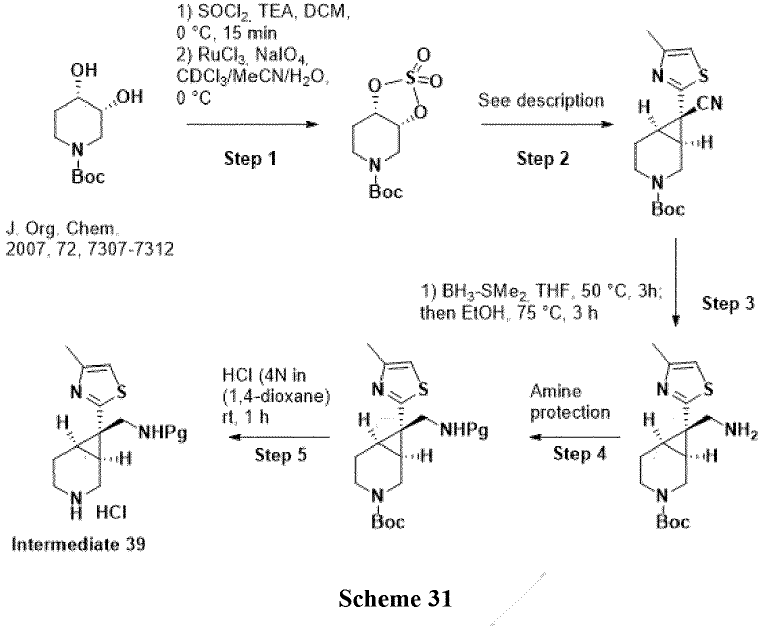

### Step 1: Tert-butyl (3aR,7aS)-tetrahydro-[1,3,2]dioxathiolo[4,5-c]pyridine-5(4H)-carboxylate 2,2-dioxide

Step 1 was carried out according to the procedure described in Step 2 of synthesis of **Intermediate 1,** starting from tert-butyl (3R,4S)-3,4-dihydroxypiperidine-1-carboxylate (prepared as described in J.Org. Chem 2007, 72, pp. 7307-7312; 170 mg, 0.78 mmol). LCMS (ES⁺) Method 1: *m*/*z* 280 (M+H)⁺, RT 1.80 min.

### Step 2: Tert-butyl (1S,6R,7S)-7-cyano-7-(4-methylthiazol-2-yl)-3-azabicyclo[4.1.0]heptane-3-carboxylate

*tert*-Butyl (3aR,7aS)-tetrahydro-[1,3,2]dioxathiolo[4,5-c]pyridine-5(4H)-carboxylate 2,2-dioxide (1000 mg, 3.58 mmol), 15-crown-5 (71 µL, 0.036 mmol) and 2-(4-methylthiazol-2-yl)acetonitrile (544 mg, 3.93 mmol) were dissolved in dry DME (0.5 M) and the resulting solution was added dropwise over a period of 30 min to a suspension of sodium hydride (60% in mineral oil; 2.6 eq) in DME (0.7 M) at rt under N₂ atmosphere. The reaction mixture was stirred at rt for 3h, then quenched by addition of H₂O and extracted with EtOAc (x2). Combined organic layers were dried over Na₂SO₄, filtered and concentrated *in vacuo.* The residue was purified by flash chromatography on silica gel (from 0% to 20% EtOAc in DCM) to give the title compound as a red oil (693 mg, 60%). ¹H NMR (300 MHz, CDCl₃) δ 6.76 (br d, *J*=0.9 Hz, 1H), 4.25 (d, *J*=4.8 Hz, 1H), 4.10-3.75 (m, 1H), 3.69 (dd, *J*=14.8 and 5.0 Hz, 1H), 3.05-2.70 (m, 1H), 2.55 - 2.40 (m, 2H), 2.38 (d, *J*=0.9 Hz, 3H), 2.33-2.15 (m, 1H), 2.13-1.98 (m, 1H), 1.50 (s, 9H); LCMS (ES⁺) Method 1: *m*/*z* 320 (M+H)⁺, RT 2.00 min.

### Step 3: Tert-butyl (1S,6R,7S)-7-(aminomethyl)-7-(4-methylthiazol-2-yl)-3-azabicyclo[4.1.0]heptane-3-carboxylate

To a solution of *tert*-butyl (1S,6R,7S)-7-cyano-7-(4-methylthiazol-2-yl)-3-azabicyclo[4.1.0]heptane-3-carboxylate (680 mg, 2.13 mmol) in dry THF (0.16 M), borane methylsulfanylmethane (2M in THF; 8.52 mmol) was added and the reaction mixture was stirred at 50 °C for 1 h. Additional borane methylsulfanylmethane (2M in THF; 4.26 mmol) was added and the reaction mixture was stirred at 50 °C for 2 h. The reaction was quenched by addition of EtOH (0.08 M) and the resulting solution was stirred at 70 °C for 3 h. Volatiles were removed *in vacuo* and the residue was purified by flash chromatography on silica gel (from 0% to 10% MeOH in DCM) to give the title compound as a pale yellow oil (280 mg, 41%). LCMS (ES⁺) Method 1: *m*/*z* 324 (M+H)⁺, RT 1.29 min.

### Step 4: Tert-butyl (1S,6R,7S)-7-((((benzyloxy)carbonyl)amino)methyl)-7-(4-methylthiazol-2-yl)-3-azabicyclo[4.1.0]heptane-3-carboxylate

The title compound was prepared according to the procedure described in Step 3 of the synthesis of **Intermediate 28** starting from *tert*-butyl (1S,6R,7S)-7-(aminomethyl)-7-(4-methylthiazol-2-yl)-3-azabicyclo[4.1.0]heptane-3-carboxylate (91 mg, 0.28 mmol). LCMS (ES⁺) Method 1: *m*/*z* 458 (M+H)⁺, RT 2.07 min.

### Step 5: Benzyl (((1S,6R,7S)-7-(4-methylthiazol-2-yl)-3-azabicyclo[4.1.0]heplan-7-yl)methyl)carbamate (Intermediate 39)

*tert*-butyl (1S,6R,7S)-7-((((benzyloxy)carbonyl)amino)methyl)-7-(4-methylthiazol-2-yl)-3-azabicyclo[4.1.0]heptane-3-carboxylate (35 mg, 0.076 mmol) was treated with 1 mL of HCl (4N in 1,4-dioxane) and the resulting solution was stirred at rt for 3 h. After evaporation, the compound was used without further purification. LCMS (ES⁺) Method 1: *m*/*z* 358 (M+H)⁺, RT 1.28 min.

### Intermediate 40: 2-(((1S,6R,7S)-7-(4-methylthiazol-2-yl)-3-azabicyclo[4.1.0]heptan-7-yl)methyl)isoindoline-1,3-dione

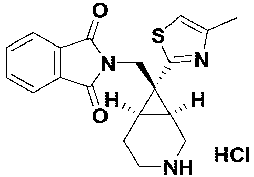

The compound was prepared following the procedure outlined in the **Scheme 31** for the synthesis of **Intermediate 39,** wherein Step 4 and Step 5 were carried out as indicated below.

### Step 4: Tert-butyl (1S,6R, 7S)-7-((1,3-dioxoisoindolin-2-yl)methyl)-7-(4-methylthiazol-2-yl)-3-azabicyclo[4.1.0]heptane-3-carboxylate

The title compound was prepared according to the procedure described in Step 1 of the synthesis of **Intermediate 36** starting from *tert*-butyl (1S,6R,7S)-7-(aminomethyl)-7-(4-methylthiazol-2-yl)-3-azabicyclo[4.1.0]heptane-3-carboxylate (198 mg, 0.61 mmol). LCMS (ES⁺) Method 1: *m*/*z* 454 (M+H)⁺, RT 2.03 min.

### Step 5: 2-(((1S,6R,7S)-7-(4-methylthiazol-2-yl)-3-azabicyclo[4.1.0]heplan-7-yl)methyl)isoindoline-1,3-dione (Intermediate 40)

*tert-Butyl* (1S,6R,7S)-7-((1,3-dioxoisoindolin-2-yl)methyl)-7-(4-methylthiazol-2-yl)-3-azabicyclo[4.1.0]heptane-3-carboxylate (194, 0.43 mmol) was treated with 1 mL of HCl (4N in 1,4-dioxane) and the resulting solution was stirred at rt for 3 h. After evaporation, the compound was used without further purification. LCMS (ES⁺) Method 1: *m*/*z* 354 (M+H)⁺, RT 1.09 min.

### Intermediate 41: 6-chloro-3-((2-(trifluoromethyl)pyridin-3-yl)thio)pyrazin-2-amine

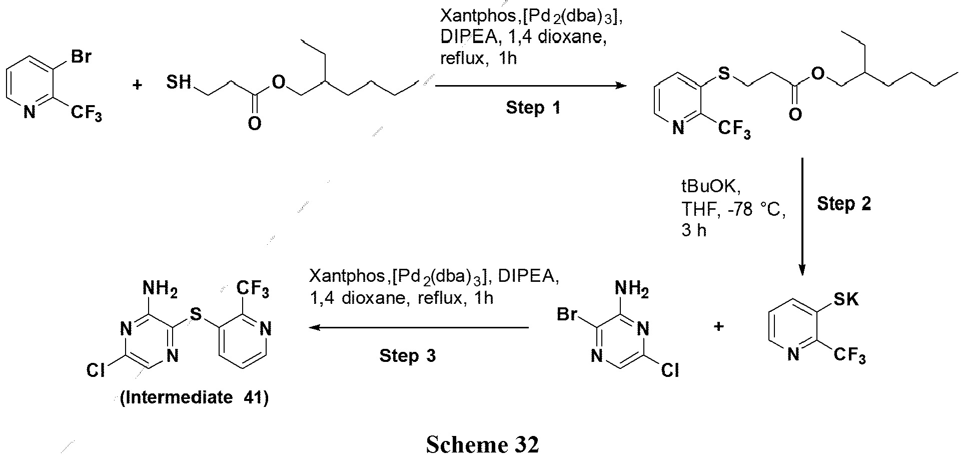

### Step1: 2-ethylhexyl 3-((2-(trifluoromethyl)pyridin-3-yl)thio)propanoate

To a mixture of 3-bromo-2-trifluoromethylpyridine (712 mg, 3.150 mmol), 2-ethylhexyl 3-sulfanylpropanoate (0.788 mL, 3.465 mmol), Pd₂(dba)₃ (289 mg, 0.316 mmol), Xantphos (183 mg, 0.316 mmol) in dry degassed 1,4 dioxane (7 mL) under N₂ atmosphere, DIPEA (1.7 mL, 9.76 mmol) was added and the resulting mixture was heated at 100 °C for 1 h. After solvent concentration the residue was purified by flash chromatography on silica gel (from 0% to 20% EtOAc in cyclohexane) to afford 2-ethylhexyl 3-((2-(trifluoromethyl)pyridin-3-yl)thio)propanoate as an orange solid (809 mg, 70%). ¹H NMR (300 MHz, DMSO-*d*₆) δ 8.52 (br d, *J*=4.2 Hz, 1H), 8.17 (br d, *J*=8.3 Hz, 1H), 7.72- 7.65 (m, 1H), 3.95 (d, *J*=5.5 Hz, 2H), 3.42-3.33 (m, 2H), 2.69 (t, *J*=6.5 Hz, 2H), 1.34-1.18 (m, 9H), 0.89-0.79 (m, 6H). LCMS (ES⁺) Method 3: *m*/*z* 364 (M+H)⁺, RT 2.08 min.

### Step 2: potassium 2-(trifluoromethyl)pyridine-3-thiolate

To a solution of 2-ethylhexyl 3-((2-(trifluoromethyl)pyridin-3-yl)thio)propanoate (500 mg, 1.38 mmol) in dry THF (4 mL) cooled to -78 °C, a suspension of *t*BuOK (463.1 mg, 4.13 mmol) in dry THF (20 mL) was added. After stirring at -78 °C for 20 min the reaction was quenched with aqueous K₂CO₃ (2M, 0.25 mL) and gradually warmed to rt (a brown solid precipitated). The solid was filtered off, washed with THF (x2) and dried under reduced pressure, to afford potassium 2-(trifluoromethyl)pyridine-3-thiolate as a brownish solid (300 mg, 98%). ¹H NMR (300 MHz, DMSO-*d*₆) δ 7.63 (d, *J*=4.0 Hz, 1H), 7.52 (d, *J*=8.3 Hz, 1H), 6.77 (dd, *J*=8.1, 4.2 Hz, 1H). LCMS (ES⁺) Method 1: *m*/*z* 180 (M+H)⁺, RT 1.69 min.

### Step 3: 6-chloro-3-((2-(trifluoromethyl)pyridin-3-yl)thio)pyrazin-2-amine

To a solution of 2-amino-3-bromo-6-chloropyrazine (464 mg, 2.2261 mmol) and potassium 2-(trifluoromethyl)pyridine-3-thiolate (483.6 mg, 2.23 mmol) in degassed dry 1,4-dioxane (10 mL) in a sealed tube, Pd₂(dba)₃ (204 mg, 0.22 mmol), Xantphos (129 mg, 0.22 mmol) were sequentially added followed by dry DIPEA (1 mL, 5.74 mmol) under N₂ atmosphere. The resulting mixture was refluxed for 1 h. The volatiles were evaporated under reduced pressure and the resulting residue purified by flash chromatography on silica gel (from 0% to 30% EtOAc in cyclohexane) to afford the title compound as yellow solid (390 mg, 57%). ¹H NMR (300 MHz, DMSO-*d*₆) δ 8.71 (dd, *J*=4.5, 1.0 Hz, 1H), 8.00-7.94 (m, 1H), 7.74-7.68 (m, 2H), 7.16 (s, 2H). LCMS (ES⁺) Method 1: *m*/*z* 307, 309 (M+H)⁺, RT 1.96 min.

### Intermediate 42: 3-((2-amino-3-chloropyridin-4-yl)thio)-6-chloropyrazin-2-amine

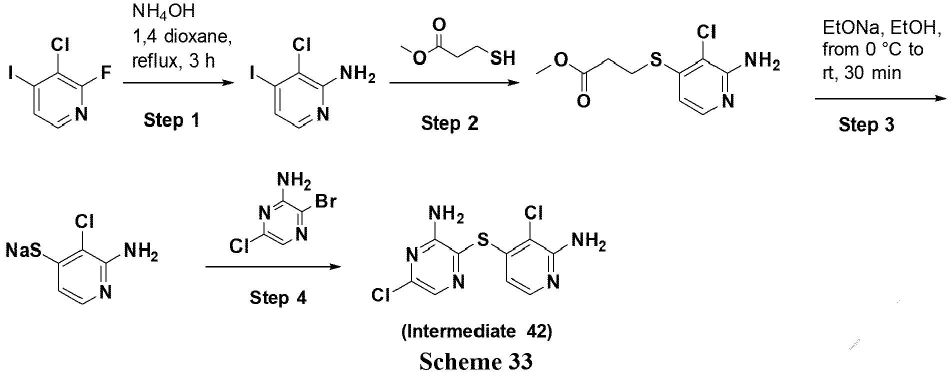

### Step 1: 3-chloro-4-iodopyridin-2-amine

A solution of 3-chloro-2-fluoro-4-iodopyridine (2.0 g, 7.769 mmol) in 1,4 dioxane (8 mL) and 30% NH₄OH (20 mL; 155.3 mmol) in H₂O was refluxed for 3 h. The mixture was diluted with EtOAc and H₂O, the phases were separated and the aqueous layer was extracted with EtOAc (3 x10 mL). The collected organic layers were washed with brine (x2), dried over Na₂SO₄, filtered and concentrated under reduced pressure to afford 3-chloro-4-iodopyridin-2-amine as a brownish solid (1.96 g, quantitative). ¹H NMR (300 MHz, DMSO-*d*₆) δ 7.59 (d, *J*=5.14 Hz, 1H), 7.11 (d, *J*=5.14 Hz, 1H), 6.56 (s, 2H). LCMS (ES⁺) Method 1: *m*/*z* 255 (M+H)⁺, RT 0.92 min.

### Step 2: methyl 3-((2-amino-3-chloropyridin-4-yl)thio)propanoate

To a solution of 3-chloro-4-iodopyridin-2-amine (1.96 g, 7.70 mmol) and methyl 3-mercaptopropionate (0.92 mL, 8.48 mmol) in degassed dry 1,4-dioxane (30 mL), Pd₂(dba)₃ (1 g, 1.15 mmol), [(t-Bu)₃PH]BF₄ (334 mg, 1.15 mmol) were sequentially added followed by degassed dry DIPEA (4.0 mL, 23.76 mmol) under N₂ atmosphere. The mixture was stirred at 90 °C for 2 h. The volatiles were removed under reduced pressure and the residue was purified by flash chromatography on silica (from 20% to 100% EtOAc in petroleum ether) to afford methyl 3-((2-amino-3-chloropyridin-4-yl)thio)propanoate as an orange solid (1.8 g, 93%). ¹H NMR (300 MHz, DMSO-*d*₆) δ 7.81 (d, *J*=5.32 Hz, 1H), 6.57 (d, *J*=5.4 Hz, 1H), 6.25 (s, 2H) 3.64 (s, 3H), 3.22 (t, *J*=7.0 Hz, 2H), 2.73 (t, *J*=7.0 Hz, 2H); LCMS (ES⁺) Method 1: *m*/*z* 247 (M+H)⁺, RT 0.98 min.

### Step 3: 2-amino-3-bromo-6-chloropyrazine

To a solution of methyl 3-((2-amino-3-chloropyridin-4-yl)thio)propanoate (1.8 g, 7.29 mmol) in EtOH (22 mL), EtONa (solution 20% wt in EtOH, 3.6 mL, 9.24 mmol) was added at 0 °C. The mixture was gradually warmed to rt. After 30 min EtOH was removed under reduced pressure and the residue diluted with DCM (50 mL). Product precipitation was observed and the solid was filtered and washed with DCM (x3) to afford the title compound as a red solid (1.3 g, 92%). ¹H NMR (300 MHz, DMSO-*d*₆) δ 7.03 (d, *J*=5.4 Hz, 1H), 6.46 (d, *J*=5.3 Hz, 1H), 5.01 (s, 2H). LCMS (ES⁺) Method 1: *m*/*z* 161,163 (M+H)⁺, RT 0.56 min.

### Step 4: 3-((2-amino-3-chloropyridin-4-yl)thio)-6-chloropyrazin-2-amine

To a solution of 2-amino-3-bromo-6-chloropyrazine (419 mg, 2.01 mmol) and sodium 2-amino-3-chloropyridine-4-thiolate (367 mg, 2.01 mmol) in degassed dry 1,4-dioxane (8 mL), Pd₂(dba)₃ (184 mg, 0.20 mmol) and [(t-Bu)₃PH]BF₄ (58.5 mg, 0.202 mmol) were sequentially added followed by dry DIPEA (0.87 mL, 5.02 mmol) under N₂ atmosphere. The resulting mixture was stirred at 105 °C for 1.5 h. Volatiles were removed under reduced pressure and the residue was purified by flash chromatography on C18 cartridge (from 5 to 45% MeCN, H₂O + 0.1% HCOOH) to afford the title compound as an orange solid (116 mg, 20%). ¹H NMR (300 MHz, DMSO-*d*₆) δ 7.89 (s, 1H), 7.70 (d, *J*=5.3 Hz, 1H), 7.17 (s, 2 H), 6.39 (s, 2 H), 5.97 (d, *J*=5.3 Hz, 1H). LCMS (ES⁺) Method 1: m/z 288 (M+H)⁺, RT 1.13 min

### Intermediate 43: 8-bromo-5-chloroimidazo[1,2-c]pyrimidine

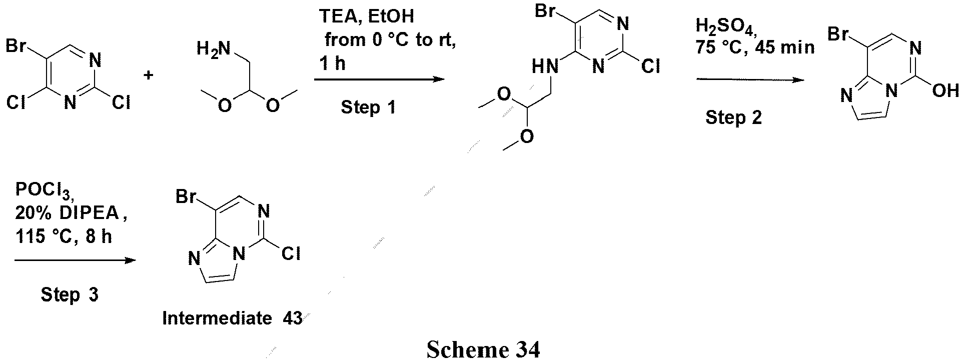

### Step 1: 5-bromo-2-chloro-N-(2,2-dimethoxyethyl)pyrimidin-4-amine

Aminoacetaldehyde dimethyl acetal (1.5 mL, 13.76 mmol) was added to a solution of 5-bromo-2,4-dichloro-pyrimidine (2.05 g, 8.99 mmol) and dry TEA (2.0 mL, 14.35 mmol) in EtOH (15 mL) at 0 °C. The mixture was gradually warmed to rt and stirred for 1 h. The mixture was concentrated, then diluted with EtOAc and washed with H₂O (x2). The organic layer was washed with brine (x2) dried over Na₂SO₄, filtered and concentrated under reduced pressure to afford the title compound as a white powder (2.63 g, 98%). ¹H NMR (300 MHz, DMSO-*d*₆) δ 8.28 (s, 1H), 7.65 (br t, *J*=5.7 Hz, 1H), 4.58 (t, *J*=5.4 Hz, 1H), 3.48 (t, *J*=5.7 Hz, 2H), 3.31-3.26 (m, 6H); LCMS (ES⁺) Method 1: *m*/*z* 296 (M+H)⁺, RT 1.62 min.

### Step 2: 8-bromoimidazo[1,2-c]pyrimidin-5-ol

A suspension of 5-bromo-2-chloro-N-(2,2-dimethoxyethyl)pyrimidin-4-amine (2.63 g, 8.86 mmol) conc. H₂SO₄ (8 mL) was stirred at 75 °C for 40 min. The mixture was cooled at 0 °C and 5N NaOH (50 mL) was added until pH =6 and a precipitate was observed. The solid was filtered and the filtrate was extracted with EtOAc (3x5 mL). The collected organic layers were washed with brine (x 2) dried over Na₂SO₄, filtered and concentrated under reduced pressure to afford a beige powder (1.52 g). ¹H NMR (300 MHz, DMSO-*d*₆) δ 11.90 (br s, 1H), 7.89 (d, *J*=1.5 Hz, 1H), 7.64 (d, *J*=4.6 Hz, 1H), 7.43 (d, *J*=1.6 Hz, 1H); LCMS (ES⁺) Method 1: *m*/*z* 214 (M+H)⁺, RT 0.64 min.

### Step 3: 8-bromo-5-chloroimidazo[1,2-c]pyrimidine

In a sealed microwave vial, dry DIPEA (1.6 mL) was added to a suspension of 8-bromoimidazo[1,2-c]pyrimidin-5-ol (380 mg, 1.78 mmol) in POCl₃ (8 mL) at 0 °C. The mixture was stirred at 115 °C for 9 h and then cooled and slowly added to a stirred mixture of cold H₂O and EtOAc. The organic layer was washed with H₂O (2x10 mL), dried over Na₂SO₄, filtered and concentrated under reduced pressure. The crude compound was purified by flash chromatography on C18 cartridge (from 0% to 35% MeCN/H₂O + 0.1% TFA) to afford the title compound as beige powder (170 mg, 41%). ¹H NMR (300 MHz, DMSO-*d*₆) δ 8.22-8.18 (m, 2H) 7.85 (d, *J*=1.5 Hz, 1H). LCMS (ES⁺) Method 1: *m*/*z* 232 (M+H)⁺, RT 1.21 min.

### Intermediate 44: 6-chloro-3-iodo-1-(tetrahydro-2H-pyran-2-yl)-1H-pyrazolo[3,4-b]pyrazine

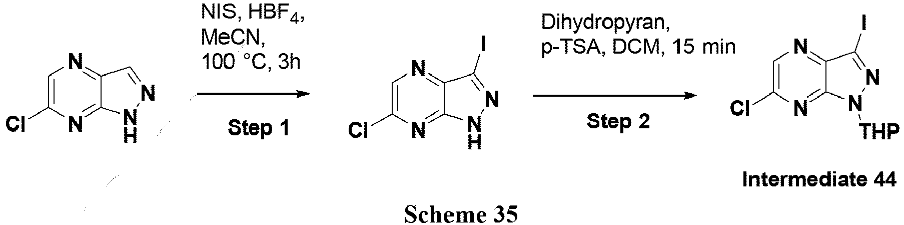

### Step 1: 6-chloro-3-iodo-1H-pyrazolo[3,4-b]pyrazine

6-chloro-1H-pyrazolo[3,4-b]pyrazine (850 mg, 5.5 mmol) was suspended in MeCN (7 mL) then N-iodosuccinimide (1.86 g, 8.25 mmol) and HBF₄ (48% in H₂O 1.72 mL, 13.14 mmol) were sequentially added. The resulting mixture was refluxed for 3 h, then cooled by ice bath and filtered. The yellow precipitate was washed with cold MeCN (x3) to obtain the title compound as a yellow solid (1.25 g, 81%). ¹H NMR (300 MHz, DMSO-*d*₆) δ 14.66 (br s, 1H), 8.70 (s, 1H); LCMS (ES⁺) Method 1: *m*/*z* 281 (M+H)⁺, RT 1.65 min.

### Step 2: 6-chloro-3-iodo-1-(tetrahydro-2H-pyran-2-yl)-1H-pyrazolo[3,4-b]pyrazine

6-chloro-3-iodo-1H-pyrazolo[3,4-b]pyrazine (754 mg, 2.69 mmol) was dissolved in DCM (13 mL) and treated with 3,4-dihydro-2H-pyran (0.76 mL, 8.33 mmol) and *p*-toluenesulfonic acid (155 mg, 0.90 mmol). The reaction was stirred at rt for 30 min, then was poured into NaHCO₃ sat. sol. (30 mL) and extracted with DCM (x3). The combined organic extracts were evaporated *in vacuo* and the residue was purified by flash chromatography on silica gel (from 0% to 20% EtOAc/petroleum ether) to afford the title compound as a light-yellow solid (600 mg, 61%). ¹H NMR (300 MHz, CDCl₃) δ 8.59 (s, 1H), 5.99 (dd, *J*=10.4 and 2.6 Hz, 1H), 4.14 (m, 1H), 3.82 (m, 1H), 2.67 (m, 1H), 2.19 (m, 1H), 2.00 (m, 1H), 1.92-1.74 (m, 2H), 1.67 (m, 1H); LCMS (ES⁺) Method 1: *m*/*z* 365 (M+H)⁺, RT 2.16 min.

### Intermediate 45: 4-Chloro-2-methyl-5-(4,4,5,5-tetramethyl-1,3,2-dioxaborolan-2-yl)-2H-indazole

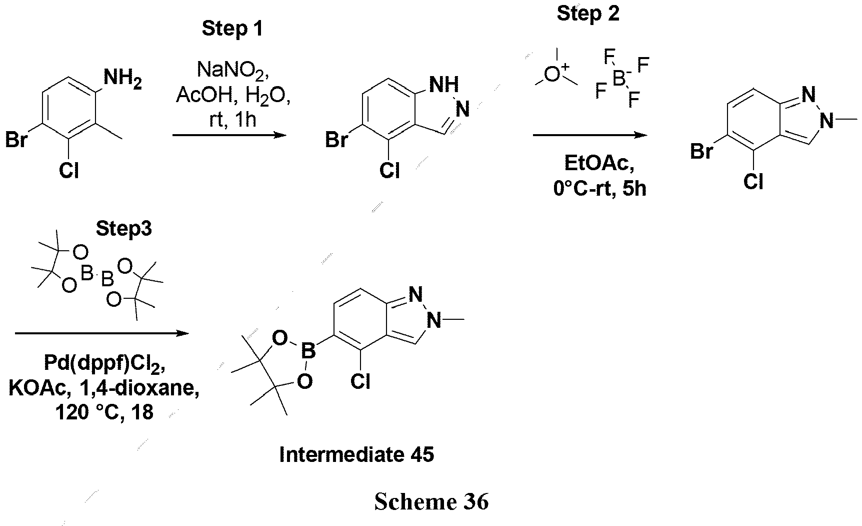

### Step 1: 5-Bromo-4-chloro-1H-indazole

A solution of 4-bromo-3-chloro-2-methylaniline (867 mg, 3.93 mmol) in AcOH (17 mL) was treated with NaNO2 (339.0 mg, 4.91 mmol) in H₂O (1.5 mL). The mixture was stirred at rt for 1 h. After the mixture was concentrated and the residue was suspended in H₂O, filtered then washed with H₂O and *n*-heptane. The organic phase was concentrated under reduced pressure to give a residue which was purified by flash chromatography (gradient elution 0-30% EtOAc in cyclohexane) to afford the title compound as a light orange solid (328 mg, 36%). LCMS (ES⁺) *m*/*z* 231 (M+H)⁺, RT 1.59 min.

### Step 2: 5-Bromo-4-chloro-2-methyl-2H-indazole

A solution of 5-bromo-4-chloro-1H-indazole (1.49 g, 6.45 mmol) in EtOAc (32 mL) at 0 °C was treated with trimethyloxonium tetrafluoroborate (1.43 g, 9.65 mmol). The resultant mixture was allowed to warm to rt and stirred for 5 h. After completion, the mixture was quenched with NaHCO₃ sat. sol. and extracted with EtOAc. The organic layer was washed with brine, dried over Na₂SO₄, filtered, and concentrated *in vacuo.* The crude product was purified by flash chromatography (gradient elution 0-30% EtOAc in *n*-heptane) to afford the title compound as an orange solid (900 mg, 57%). ¹H NMR (500 MHz, DMSO-d₆): δ 8.49 (s, 1H), 7.52 (d, *J* = 9.0 Hz, 1H), 7.44 (d, *J* = 9.0 Hz, 1H), 4.16 (s, 3H). LCMS (ES⁺) *m*/*z* 245 (M+H)⁺, RT 1.69 min.

### Step 3: 4-Chloro-2-methyl-5-(4,4,5,5-tetramethyl-1,3,2-dioxaborolan-2-yl)-2H-indazole

A suspension of 5-bromo-4-chloro-2-methyl-2H-indazole (548.0 mg, 2.23 mmol), bis(pinacolato)diboron (1.36 g, 5.36 mmol), [1,1'-Bis(diphenylphosphino)ferrocene]dichloropalladium(II) complex in DCM (163.3 mg, 0.223 mmol) and KOAc (657.2 mg, 6.70 mmol) in 1,4-dioxane (11 mL) was heated to 120 °C for 18 h. After completion, the mixture was cooled to rt, the solid precipitate was filtered off and washed with EtOAc. The filtrate was concentrated in vacuo and the residue was dissolved in toluene and n-heptane until precipitation occurred. The suspension was filtered through a short pad of Celite and the filtrate was concentrated in vacuo. The residue was triturated with n-heptane, the solid was filtered off and the filtrate was concentrated under reduced pressure to afford the title compound (1.15 g, 99%), as a light brown solid. ¹H NMR (300 MHz DMSO-d6) δ 8.52 (s, 1H), 7.50 (d, J =8.7 Hz, 1H), 7.42 (d, J =8.7 Hz, 1H), 4.18 (s, 3H), 1.32 (s, 12H). LCMS (ES+) m/z 293 (M+H)+, RT 1.89 min.

**Intermediate 45** was used for the synthesis of **Examples 15, 24, 46, 47, 48, 49** and **50.**

### Intermediate 46: 3,4-dichloro-2-methyl-5-(4,4,5,5-tetramethyl-1,3,2-dioxaborolan-2-yl)-2H-indazole

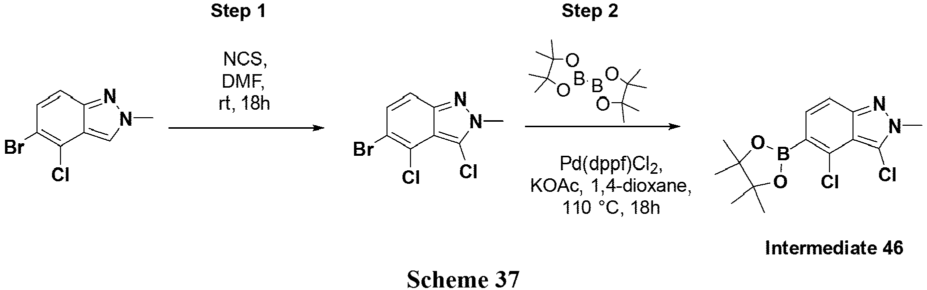

### Step 1: 5-Bromo-3,4-dichloro-2-methyl-2H-indazole

5-Bromo-4-chloro-2-methyl-2H-indazole (1.0 g, 4.1 mmol) in dry DMF (20 mL) was treated with NCS (575.7 mg, 4.3 mmol). The mixture was stirred at rt for 18 h. After completion, the mixture was diluted with H₂O and the precipitated solid was filtered off, washed with H₂O and dried. The crude product was triturated with a mixture of EtOAc and n-heptane, then washed with n-heptane and dried to afford the title compound (863 mg, 75%), as a yellow solid. LCMS (ES⁺) *m*/*z* 279 (M+H)⁺

### Step 2: 3,4-Dichloro-2-methyl-5-(4,4,5,5-tetramethyl-1,3,2-dioxaborolan-2-yl)-2H-indazole

A suspension of 5-bromo-3,4-dichloro-2-methyl-2H-indazole (647.0 mg, 2.31 mmol), bis(pinacolato)diboron (1.17 g, 4.62 mmol), [1,1'-bis(diphenylphosphino)ferrocene]dichloropalladium(II) complex in DCM (169.1 mg, 0.23 mmol) and KOAc (680.5 mg, 6.93 mmol) in 1,4-dioxane (11.5 mL) was heated at 110 °C for 18 h in a pressure vessel. After completion, the mixture was cooled to rt, the solid precipitate was filtered off and washed with EtOAc. The filtrate was concentrated *in vacuo* and n-heptane was added. The precipitate was filtered off and the filtrate was concentrated under reduced pressure. The crude product was triturated with MeCN and dried to afford the title compound (168 mg, 99%). ¹H NMR (500 MHz, DMSO-*d*₆) δ 7.50 (d, *J*=8.8 Hz, 1H), 7.40 (d, *J*=8.8 Hz, 1H), 4.10 (s, 3H), 1.29 (s, 12H). LCMS (ES⁺) *m*/*z* 327 (M+H)⁺

**Intermediate 46** was used for the synthesis of **Example 44**

### Intermediate 47: 6-chloro-3-iodo-5-methyl-1-(tetrahydro-2H-pyran-2-yl)-1,5-dihydro-4H-pyrazolo[3,4-d]pyrimidin-4-one

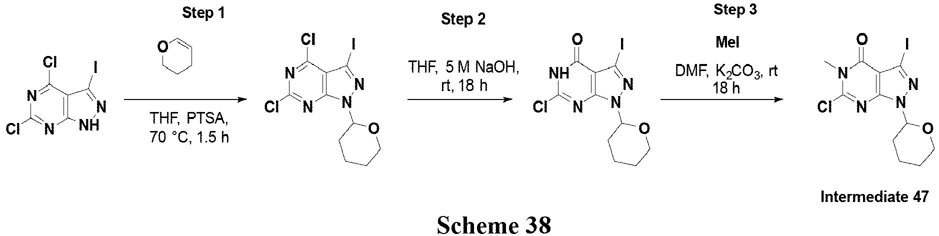

### Step 1: 4,6-Dichloro-3-iodo-1-(tetrahydro-2H-pyran-2-yl)-1H-pyrazolo[3,4-d]pyrimidine

A solution of 4,6-dichloro-3-iodo-1H-pyrazolo[3,4-d]pyrimidine (2.0 g, 6.35 mmol), 3,4-dihydro-2H-pyran (1.68 mL, 18.4 mmol) and p-toluenesulfonic acid monohydrate (241.6 mg, 1.27 mmol) in THF (29 mL) was heated at 70 °C for 1.5 h. After completion, the solvent was removed *in vacuo* and the crude product was purified by flash chromatography (gradient elution 0-5% MeOH in CHCl₃) to afford the title compound as white solid (2.23 g, 88%). LCMS (ES⁺) *m*/*z* 399 (M+H)⁺

### Step 2: 6-Chloro-3-iodo-1-(tetrahydro-2H-pyran-2-yl)-1,5-dihydro-4H-pyrazolo[3,4-d]pyrimidin-4-one

A solution of 4,6-dichloro-3-iodo-1-(tetrahydro-2H-pyran-2-yl)-1H-pyrazolo[3,4-d]pyrimidine (2.82 g, 7.0 mmol) in THF (28 mL) was treated with 5 M aq. NaOH solution (12.7 mL, 63.45 mmol). The mixture was stirred at rt for 5 h, then THF (14 mL) was added. The resultant mixture was stirred at rt for 18 h and then was acidified with 6 M aqueous HCl solution and extracted with EtOAc. The organic layer was washed with brine, dried over Na₂SO₄, filtered, and concentrated in *vacuo.* The crude product was triturated with *n*-heptane and dried to afford the title compound (1.8 g, 67%) as a white solid. ¹H NMR (300 MHz, DMSO-*d*₆) δ 13.40 (s, 1H), 5.66 (dd, *J* = 10.2, 2.2 Hz, 1H), 3.91 (d, *J* = 11.3 Hz, 1H), 3.71-3.59 (m, 1H), 2.35-2.17 (m, 1H), 2.04-1.90 (m, 1H), 1.89-1.78 (m, 1H), 1.77-1.62 (m, 1H), 1.60-1.48 (m, 2H).

### Step 3: 6-Chloro-3-iodo-5-methyl-1-(tetrahydro-2H-pyran-2-yl)-1,5-dihydro-4H-pyrazolo[3,4-d]pyrimidin-4-one

A solution of 6-chloro-3-iodo-1-(tetrahydro-2H-pyran-2-yl)-1,5-dihydro-4H-pyrazolo[3,4-d]pyrimidin-4-one (1.75 g, 4.6 mmol) in DMF (21 mL) was treated with K₂CO₃ (762.6 mg, 5.5 mmol) followed by slow addition of iodomethane (314.9 µL, 5.0 mmol). The mixture was stirred at rt for 18 h. After completion, the mixture was diluted with H₂O and the solid precipitate was collected by filtration. The crude product was triturated with EtOAc to afford the title compound (1.04 g, 57%) as a white solid. ¹H NMR (500 MHz, CDCl₃) δ 5.73 (dd, *J* =10.2, 1.5 Hz, 1H), 4.08 (d, *J* =11.5 Hz, 1H), 3.73 (t, *J* =11.3 Hz, 1H), 3.68 (s, 3H), 2.54-1.43 (m, 1H), 2.09 (d, *J* = 10.7 Hz, 1H), 1.86 (d, *J* = 13.3 Hz, 1H), 1.82-1.65 (m, 2H), 1.58 (d, *J* = 12.3 Hz, 1H). LCMS (ES⁺) *m*/*z* 311 (M+H-THP)⁺

Intermediate 47 was used for the synthesis of Example 62

### Intermediate 48: 3-Chloro-4-((6-chloro-5H-pyrrolo[2,3-b]pyrazin-2-yl)thio)pyridin-2-amine

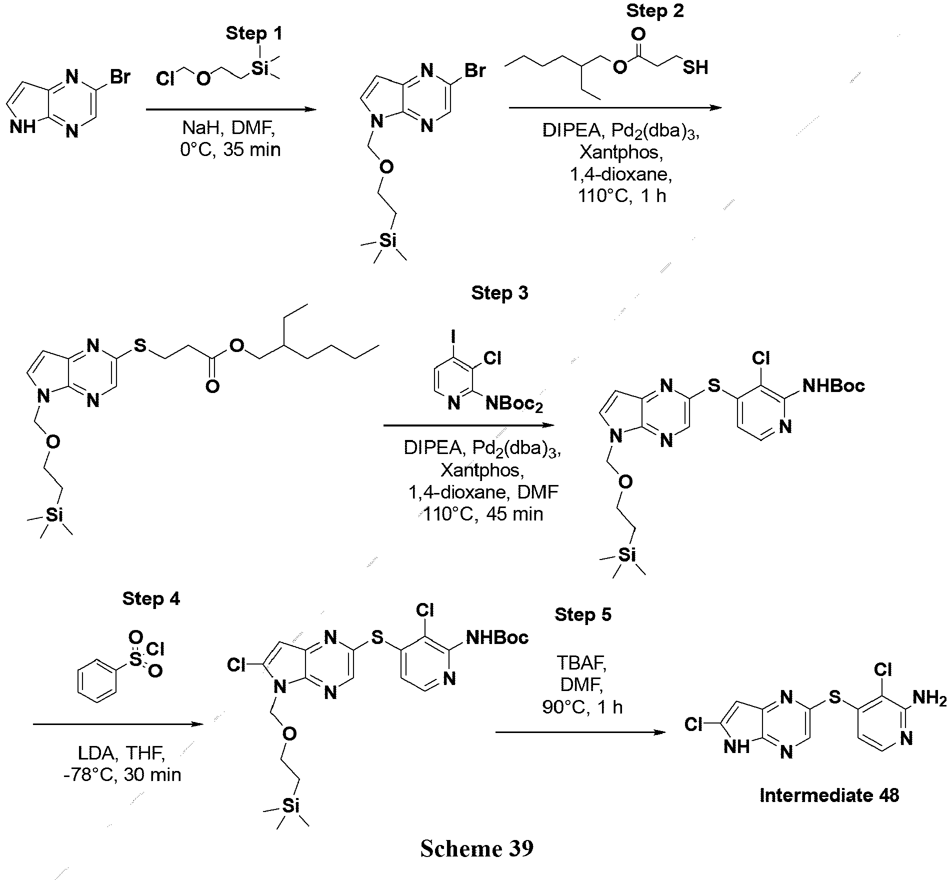

### Step 1: 2-bromo-5-((2-(trimethylsilyl)ethoxy)methyl)-5H-pyrrolo[2,3-b]pyrazine

2-Bromo-4H-pyrrolo[2,3-b]pyrazine (1 g, 5.05 mmol) was dissolved in DMF (34 mL) and the resulting solution was cooled to 0°C and sodium hydride (60% in mineral oil; 242 mg, 6.06 mmol) was added. The mixture was stirred at this temperature for 30 min then 2-(chloromethoxy)ethyl-trimethyl-silane (0.99 mL, 5.55 mmol) was added and the mixture stirred for 5 min. NaHCO₃ sat. sol. and EtOAc were added to the mixture. The organic phase was then isolated, washed with H₂O, dried over Na₂SO₄, filtered, and concentrated *in vacuo* to give a residue which was purified by flash chromatography (from 0-60% EtOAc in petroleum ether). The title compound was obtained as a pale yellow oil (1410 mg, 85%). ¹H NMR (400 MHz, DMSO-*d*₆) δ 8.45 (s, 1H), 8.14 (d, *J* = 4.0 Hz, 1H), 6.73 (d, *J* = 4.0 Hz, 1H), 5.63 (s, 2H), 3.51 (t, *J* = 8.0 Hz, 2H), 0.81 (t, *J* = 8.0 Hz, 2H), 0.11 (s, 9H). LCMS (ES⁺) *m*/*z* 328-330 (M+H)⁺, RT 2.36 min.

### Step 2: 2-Ethylhexyl 3-((5-((2-(trimethylsilyl)ethoxy)methyl)-5H-pyrrolo[2,3-b]pyrazin-2-yl)thio)propanoate

2-Ethylhexyl 3-sulfanylpropanoate (1032 mg, 4.72 mmol), DIPEA (1665 mg, 12.89 mmol), Pd₂(dba)₃ (393 mg, 0.430 mmol) and Xantphos (249 mg, 0.430 mmol) were dissolved in 1,4-dioxane (11 mL) and 2-bromo-5-((2-(trimethylsilyl)ethoxy)methyl)-5H-pyrrolo[2,3-b]pyrazine (1410 mg, 4.3 mmol) was added. The mixture was stirred at 110°C for 1 h then the solvent was concentrated *in vacuo* and the residue purified by flash chromatography (gradient elution 0-100% EtOAc in petroleum ether). The title compound was obtained as a yellow oil (2 g, 66% pure, 66%). LCMS (ES⁺) *m*/*z* 466 (M+H)⁺, RT 2.55 min.

### Step 3: tert-butyl (3-chloro-4-((5-((2-(trimethylsilyl)ethoxy)methyl)-5H-pyrrolo[2,3-b]pyrazin-2-yl)thio)pyridin-2-yl)carbamate

A suspension of 2-ethylhexyl 3-((5-((2-(trimethylsilyl)ethoxy)methyl)-5H-pyrrolo[2,3-b]pyrazin-2-yl)thio)propanoate (1.790 g, 3.84 mmol), tert-butyl (tert-butoxycarbonyl)(3-chloro-4-iodopyridin-2- yl)carbamate (1.850 g, 4.07 mmol), Pd₂(dba)₃ (281.57 mg, 0.310 mmol) and Xantphos (222.39 mg, 0.380 mmol) in a mixture of 1,4-dioxane (11.7 mL) and DMF (3.7 mL) was degassed with N₂ then treated with potassium 2-methylpropan-2-olate (8.46 mL, 8.46 mmol) and DIPEA (1.33 mL, 7.69 mmol). The reaction mixture was stirred for 45 min at 110°C. After cooling the solvent was concentrated under reduced pressure then DCM was added, and the precipitated powder was filtered. The organic solvent was concentrated *in vacuo* and the crude product was purified by flash chromatography (gradient elution 0-100% EtOAc in petroleum ether) to afford the title compound as brown solid (1.35 g, 69%). LCMS (ES⁺) *m*/*z* 506 (M-H)⁻, RT 2.47 min.

### Step 4: tert-butyl (3-chloro-4-((6-chloro-5-((2-(trimethylsilyl)ethoxy)methyl)-5H-pyrrolo[2,3-b]pyrazin-2-yl)thio)pyridin-2-yl)carbamate

A solution of tert-butyl (3-chloro-4-((5-((2-(trimethylsilyl)ethoxy)methyl)-5H-pyrrolo[2,3-b]pyrazin-2-yl)thio)pyridin-2-yl)carbamate (600 mg, 1.18 mmol) in THF (7.4 mL) cooled to -78 °C was treated with (diisopropylamino)lithium (1.18 mL, 1.18 mmol) and the mixture was stirred at this temperature for 30 min then benzenesulfonyl chloride (0.18 mL, 1.42 mmol) was added and the reaction mixture stirred for 5 min. NaHCO₃ sat. sol. and EtOAc were added then the organic phase was separated and washed with Na₂S₂O₃ sat sol, dried over Na₂SO₄, filtered and concentrated *in vacuo.* The crude product was purified by flash chromatography (gradient elution 0-100% EtOAc in petroleum ether) to afford the title compound as a yellow solid (300 mg, 47%). LCMS (ES⁺) *m*/*z* 540 (M-H)⁻, RT 2.6 min.

### Step 5: 3-chloro-4-((6-chloro-5H-pyrrolo[2,3-b]pyrazin-2-yl)thio)pyridin-2-amine

A solution of tert-butyl (3-chloro-4-((6-chloro-5-((2-(trimethylsilyl)ethoxy)methyl)-5H-pyrrolo[2,3-b]pyrazin-2-yl)thio)pyridin-2-yl)carbamate (50 mg, 0.090 mmol) in DMF (0.74 mL) was treated with TBAF (0.28 mL, 0.280 mmol) and the mixture was stirred for 1 h at 90°C. After cooling the solvent was removed *in vacuo* and the crude product was purified by flash chromatography (gradient elution 0-100% EtOAc in petroleum ether) to afford the title compound as yellow solid (16.7 mg, 58%). LCMS (ES⁺) *m*/*z* 311 (M+H)⁺, RT 0.77 min.

**Intermediate 48** was used for the synthesis of **Example 59**

### Intermediate 49: 2-chloro-6-((2-(trifluoromethyl)pyridin-3-yl)thio)-1H-imidazo[4,5-b]pyrazine

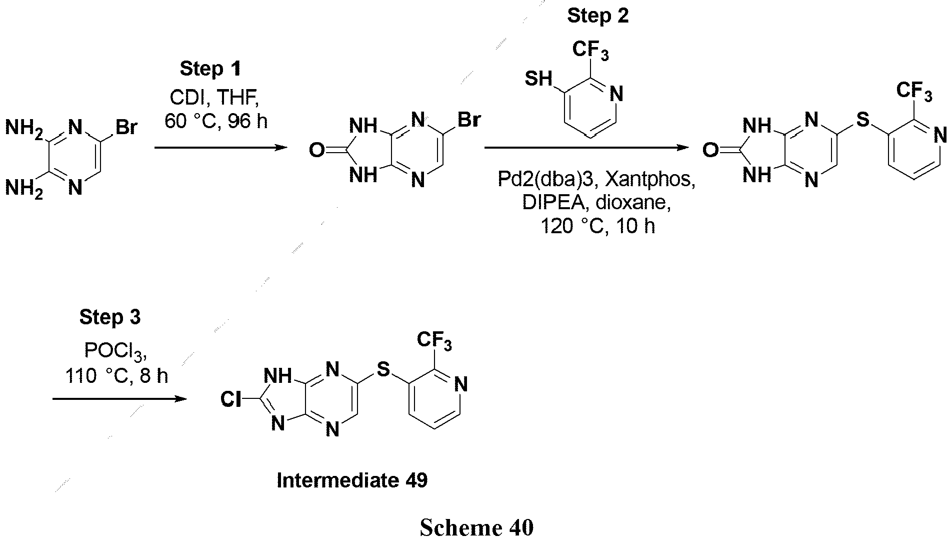

### Step 1: 5-Bromo-1,3-dihydro-2H-imidazo[4,5-b]pyrazin-2-one

A solution of 5-bromo-2,3-pyrazindiamine (4.1 g, 21.7 mmol) and CDI (7.0 g, 43.4 mmol) in THF (120 mL) was heated to 60 °C for 72 h. Then, a second portion of CDI (7.0 g, 43.4 mmol) was added and heating to 60 °C was continued for 24 h. After cooling, the mixture was concentrated *in vacuo* and the residue was purified by reverse phase chromatography (gradient elution 0-50% MeCN + 0.1% TFA/H₂O + 0.1% TFA). Evaporation of the fractions containing the desired product furnished a compound which was triturated with MeCN and filtered off to give the title compound as a beige solid (3.04 g, 65%). ¹H NMR (300 MHz, DMSO-*d*₆) δ 11.98 (br s, 1H), 11.91 (br s, 1H), 7.98 (s, 1H). LCMS (ES⁺) *m*/*z* 215, 217 (M+H)⁺, RT 0.76 min.

### Step 2: 5-[(2-(Trifluoromethyl)pyridin-3-yl)thio]-1,3-dihydro-2H-imidazo[4,5-b]pyrazin-2-one

A solution of 5-bromanyl-1,3-dihydroimidazo[4,5-b]pyrazin-2-one (1.0 g, 4.65 mmol) in 1,4-dioxane (47 mL) was degassed with N₂ for 5 min, then Pd₂(dba)₃ (0.21 g, 0.23 mmol), Xantphos (0.27 g, 0.47 mmol), 2-(trifluoromethyl)pyridine-3-thiol (1.19 g, 5.12 mmol) and DIPEA (1.62 mL, 9.3 mmol) were added sequentially. The reaction mixture was degassed with N₂ for further 5 min and heated to 100 °C for 2 h. After cooling, the mixture was concentrated *in vacuo* and the residue was treated with MeCN affording a precipitate which was filtered off to give the title compound as a beige solid (1.33 g, 91%). ¹H NMR (300 MHz, DMSO-*d*₆) δ 11.96 (br s, 2H), 8.56 (d, *J* = 3.7 Hz, 1H), 8.10 (s, 1H), 7.75 (d, *J* = 8.3 Hz, 1H), 7.58 (dd, *J* = 8.2 and 4.5 Hz, 1H). LCMS (ES⁺) *m*/*z* 314 (M+H)⁺, RT 1.24 min.

### Step 3: 2-Chloro-6-[(2-(trifluoromethyl)pyridin-3-yl)thio]-1H-imidazo[4,5-b]pyrazine

A solution of 5-[2-(trifluoromethyl)pyridin-3-yl]sulfanyl-1,3-dihydroimidazo[4,5-b]pyrazin-2-one (1.3 g, 4.15 mmol) in POCl₃ (20 mL, 0.21 mol) was heated to 120 °C in a sealed vial for 8 h. After cooling, the mixture was concentrated *in vacuo* to give a residue that was purified by reverse phase chromatography (gradient elution 0-100% MeCN + 0.1% TFA/H₂O+ 0.1% TFA) to furnish the title compound as a pale yellow solid (TFA salt; 0.26 g, 19%). ¹H NMR (300 MHz, DMSO-*d₆*) δ 8.71 (d, *J* =3.9 Hz, 1H), 8.55 (s, 1H), 8.05 (d, *J* =7.9 Hz, 1H), 7.70 (dd, *J* =8.1 and 4.6 Hz, 1H). LCMS (ES⁺) *m*/*z* 332 (M+H)⁺, RT 1.4 min.

**Intermediate 49** was used for the synthesis of **Example 63, Example 64, Example 65, Example 66, Example 67 and Example 68**

### Intermediate 50: 3-chloro-4-((2-(methylsulfonyl)-1H-imidazo[4,5-b]pyrazin-6-yl)thio)pyridin-2-amine

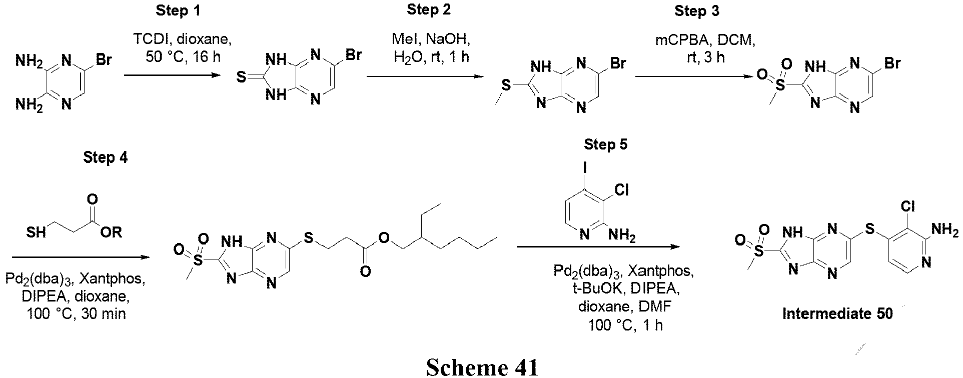

### Step 1: 5-Bromo-1,3-dihydro-2H-imidazo[4,5-b]pyrazine-2-thione

A solution of 5-bromopyrazine-2,3-diamine (5.0 g, 26.5 mmol) in 1,4-dioxane (55 mL) was treated with TDCI (6.6 g, 37.0 mmol). The mixture was heated at 50 °C for 16 h. After cooling, the reaction mixture was concentrated *in vacuo* and purified on silica gel (eluting with 0-50% EtOAc/petroleum ether) to give the title compound as a yellow solid (5.4 g, 79%). ¹H NMR (400 MHz, DMSO-d₆) δ 13.62 (br s, 2H), 8.22 (s, 1H). LCMS (ES⁺) *m*/*z* 231, 233 (M+H)⁺, RT 0.87 min.

### Step 2: 6-Bromo-2-(methylthio)-1H-imidazo[4,5-b]pyrazine

A solution of 5-bromo-1,3-dihydro-2H-imidazo[4,5-b]pyrazine-2-thione (3.0 g, 13.0 mmol) in H₂O (85 mL) at rt was treated with NaOH (799 mg, 19.5 mmol) and stirred at this temperature until complete dissolution of the starting material. Then, iodomethane (1.2 mL, 19.5 mmol) was added and the reaction mixture was stirred at rt for 1 h. A solution of NaOH 2N was added until clearness and the aqueous solution was washed with CHCl₃, concentrated and acidified to pH 6.5 with aqueous HCl 6N. The resulting precipitate was filtered off and washed with H₂O to give the title compound as a pale yellow solid (1.8 g, 56%). ¹H NMR (DMSO-d₆) δ 7.76 (s, 1H), 2.55 (s, 3H). LCMS (ES⁺) *m*/*z* 245, 247 (M+H)⁺, RT 1.03 min.

### Step 3: 6-Bromo-2-(methylsulfonyl)-1H-imidazo[4,5-b]pyrazine

*m*-CPBA (4.2 g, 18.4 mmol) was added portionwise to a solution of 6-bromo-2-(methylthio)-1*H-*imidazo[4,5-*b*]pyrazine (1.8 g, 7.3 mmol) in DCM (70 mL) at 0 °C, then the resulting reaction mixture was stirred at rt for 3 h. After addition of aqueous HCl 6N (10 mL) the mixture was extracted with a solution of CHCl₃/EtOH 95:5 (3x150 mL). The organic phase was washed with H₂O and brine. The dried organics were concentrated *in vacuo* and the residue was purified on reverse phase silica gel (eluting with 0-50% MeCN/H₂O + 0.1 % TFA) to give the title compound as a white solid (1.5 g, 75%). ¹H NMR (400 MHz, DMSO-*d*₆) δ 8.79 (s, 1H), 3.56 (s, 3H). LCMS (ES⁺) *m*/*z* 277, 279 (M+H)⁺, RT 0.86 min.

### Step 4: 2-ethylhexyl 3-((2-(methylsulfonyl)-1H-imidazo[4,5-b]pyrazin-6-yl)thio)propanoate

A pressure tube was charged with 6-bromo-2-(methylsulfonyl)-1H-imidazo[4,5-b]pyrazine (800 mg, 2.9 mmol), 2-ethylhexyl 3-mercaptopropanoate (722 uL, 3.2 mmol), DIPEA (1.0 mL, 5.8 mmol), Xantphos (83 mg, 0.14 mmol) and Pd₂(dba)₃ (66 mg, 0.07 mmol) in 1,4-dioxane (15 mL). The mixture was degassed with nitrogen for 1 min, capped and heated at 100 °C for 30 min. After cooling, the mixture was concentrated *in vacuo* and purified on silica gel (eluting with 10-100% EtOAc + 10% MeOH in petroleum ether) to give the title compound as a pale yellow solid (993 mg, 75%). LCMS (ES⁺) *m*/*z* 415 (M+H)⁺, RT 2.18 min.

### Step 5: 3-chloro-4-((2-(methylsulfonyl)-1H-imidazo[4,5-b]pyrazin-6-yl)thio)pyridin-2-amine

A solution of 2-ethylhexyl 3-((2-(methylsulfonyl)-1H-imidazo[4,5-b]pyrazin-6-yl)thio)propanoate (100 mg, 0.24 mmol), 3-chloro-4-iodopyridin-2-amine (73.7 mg, 0.29 mmol), Xantphos (6.9 mg, 0.012 mmol), Pd₂(dba)₃ (5.5 mg, 0.006 mmol) were dissolved in 1,4-dioxane (1.4 mL) and DMF (1.0 mL) under N₂ flux, then potassium 2-methylpropan-2-olate (40.6 mg, 0.36 mmol) and DIPEA (0.08 mL, 0.48 mmol) were added and stirred at 100 °C for 1 h. After cooling, the mixture was concentrated *in vacuo* and purified on silica gel (eluting with 5-100% EtOAc + 20% MeOH in EtOAc) to give the title compound as a yellow solid (75 mg, 87%). LCMS (ES⁺) *m*/*z* 356 (M+H)⁺, RT 0.73 min.

**Intermediate 50** was used for **Example 69** and **Example 70**

### Intermediate 51: benzyl (((1S,6R,7R)-7-(2-fluorophenyl)-3-azabicyclo[4.1.0]heptan-7-yl)methyl)carbamate hydrochloride

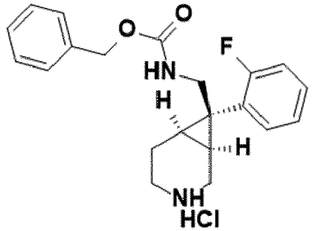

**Intermediate 51** was synthesized using the procedure reported for **Intermediate 39 (Scheme 31)** with the corresponding starting materials (the step 4 was performed using benzyl(2,5-dioxopyrrolidin-1-yl)carbonate in THF/H₂O instead of benzyl chloroformate in DCM while the step 5 was performed using DCM as co-solvent) and it was obtained as a white solid. LCMS (ES⁺) Method 2: *m*/*z* 355 (M+H)⁺, RT 1.20 min.

**Intermediate 51** was used for the synthesis of **Example 105**

### Intermediate 52: Benzyl (((1S,6R,7R)-7-(2,6-difluorophenyl)-3-azabicyclo[4.1.0]heptan-7-yl)methyl)carbamate hydrochloride

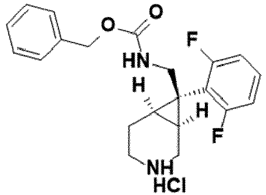

**Intermediate 52** was synthesized using the procedure reported for **Intermediate 39 (Scheme 31)** with the corresponding starting materials (the step 4 was performed using benzyl(2,5-dioxopyrrolidin-1-yl)carbonate in THF/H₂O instead of benzyl chloroformate in DCM while the step 5 was performed using DCM as co-solvent) and it was obtained as a white solid. LCMS (ES⁺) Method 2: *m*/*z* 373 (M+H)⁺, RT 1.27 min.

**Intermediate 52** was used for the synthesis of **Example 106**

### Intermediate 53: 2,4-dimethyl-5-(4,4,5,5-tetramethyl-1,3,2-dioxaborolan-2-yl)-2H-indazole

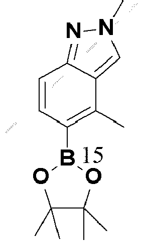

**Intermediate 53** was synthesized using the procedure reported for **Intermediate 45 (Scheme 36)** starting from step 2 using 5-bromo-4-methyl-1H-indazole (the step 3 was performed at 90°C) and it was obtained as a white solid. LCMS (ES⁺) Method 2: *m*/*z* 273 (M+H)⁺, RT 1.85 min.

**Intermediate 53** was used for the synthesis of **Example 106**

### Synthesis of Examples

### Example 1: [3-[7-(aminomethyl)-7-(thiophen-3-yl)-3-azabicyclo[4.1.0]heptan-3-yl]-6-(2,3-dichlorophenyl)-5-methylpyrazin-2-yl] methanol

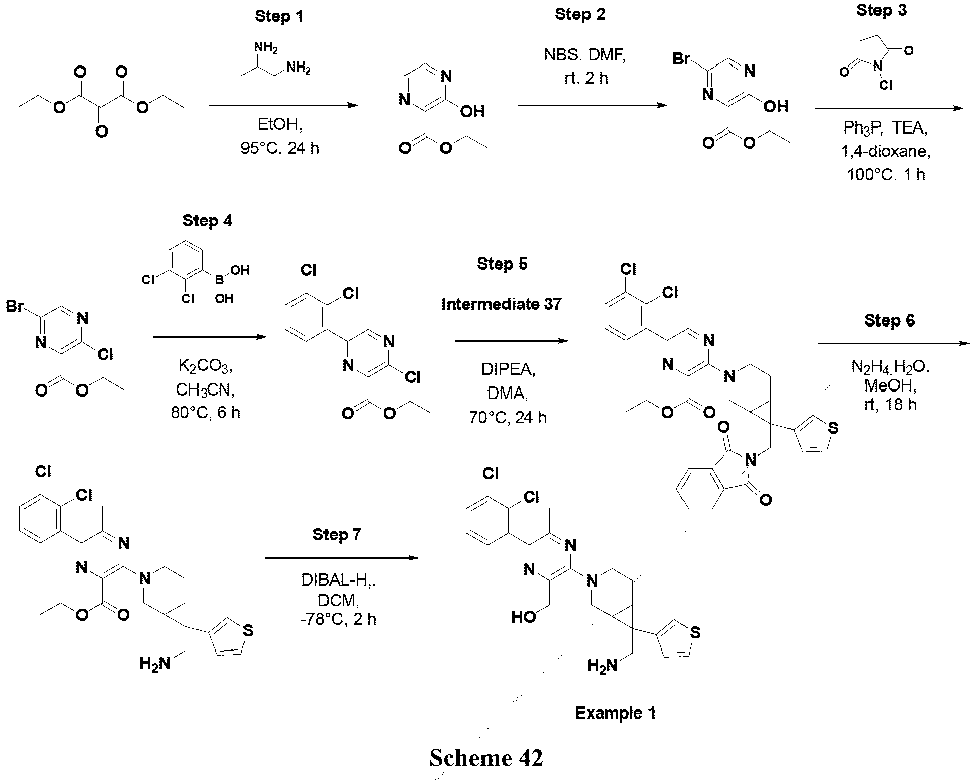

### Step 1: ethyl 3-hydroxy-5-methylpyrazine-2-carboxylate

A 50 ml flask was charged with ethanol (19.0 mL) and 1,2-diaminopropane (0.99 mL, 11.48 mmol) and the resulting clear, colourless solution was cooled to 0 °C. Diethyl 2-oxopropanedioate (1.75 mL, 11.48 mmol) was added to the solution and the reaction mixture was allowed to warm to room temperature and stirred at this temperature for 2 h and at 95 °C for 24 h. After cooling to rt the reaction mixture was concentrated under reduced pressure to give a residue which was purified by flash chromatography (gradient elution 40-100% EtOAc in petroleum ether) to afford the title compound as a yellow solid (360 mg, 17%). ¹H NMR (400 MHz, DMSO-*d*₆) δ 12.83 (br s, 1H), 7.33 (br s, 1H), 4.26 (q, *J* = 8.0 Hz, 2H), 2.24 (s, 3H), 1.27 (t, *J* = 8.0 Hz, 3H). LCMS (ES⁺) *m*/*z* 183 (M+H)⁺, RT 0.70 min.

### Step 2: ethyl 6-bromo-3-hydroxy-5-methylpyrazine-2-carboxylate

A 25 ml flask was charged with ethyl 3-hydroxy-5-methylpyrazine-2-carboxylate (300 mg, 1.65 mmol) in DMF (6.6 mL) and the resulting solution was cooled to 0°C. 1-Bromopyrrolidine-2,5-dione (307.7 mg, 1.73 mmol) was added and the reaction mixture warmed to rt and stirred for 2 h. The mixture was then diluted with H₂O and EtOAc. The organic phase was washed with brine, dried over Na₂SO₄, and concentrated *in vacuo* to afford the title compound as a crude (430 mg, 100%). LCMS (ES⁺) *m*/*z* 261-263 (M+H)⁺, RT 1.22 min.

### Step 3: ethyl 6-bromo-3-chloro-5-methylpyrazine-2-carboxylate

A 50 ml flask was charged with triphenylphosphine (1.3 g, 4.94 mmol) in 1,4-dioxane (15.0 mL) 1-chloropyrrolidine-2,5-dione (0.67 g, 5.02 mmol) was added and the resulting solution was stirred at rt for 30 min. Ethyl 6-bromo-3-hydroxy-5-methylpyrazine-2-carboxylate (430 mg, 1.65 mmol) was added and the resulting mixture was heated at 100°C for 1 h. After cooling the mixture, TEA (4 mL, 1.65 mmol) was added. The volatiles were concentrated *in vacuo* and the residue was purified by flash chromatography (gradient elution 35% EtOAc in petroleum ether) to afford the title compound as a yellow oil (310 mg, 67%). ¹H NMR (400 MHz, CDCl₃) δ 4.49 (q, *J* = 8.0 Hz, 2H), 2.74 (s, 3H), 1.44 (t, *J* = 8.0 Hz, 3H). LCMS (ES⁺) *m*/*z* 279-281 (M+H)⁺, RT 1.81 min.

### Step 4: ethyl 3-chloro-6-(2,3-dichlorophenyl)-5-methylpyrazine-2-carboxylate

A degassed mixture of ethyl 6-bromo-3-chloro-5-methylpyrazine-2-carboxylate (500 mg, 1.79 mmol), (2,3-dichlorophenyl)boronic acid (410 mg, 2.15 mmol) and dipotassium carbonate (980 mg, 7.09 mmol) in MeCN (17.9 mL) was treated with [1,1'-Bis(diphenylphosphino)ferrocene]dichloropalladium(II) complex in DCM (290 mg, 0.390 mmol). The reaction mixture was stirred at 80 °C for 6 h. After cooling, the reaction mixture was filtered on a pad of solka flok, the filtrate was concentrated *in vacuo* and purified by flash chromatography (gradient elution 0-30% EtOAc in petroleum ether) to afford the title compound (510 mg, 82%). ¹H NMR (400 MHz, CDCl₃) δ 7.60 (d, *J* = 8.0 Hz, 1H), 7.36 (t, *J* = 8.0 Hz, 1H), 7.28 (d, *J* = 8.0 Hz, 1H), 4.49 (q, *J* = 8.0 Hz, 2H), 2.48 (s, 3H), 1.44 (t, *J* = 8.0 Hz, 3H). LCMS (ES⁺) *m*/*z* 345-347 (M+H)⁺, RT 2.26 min.

### Step 5: ethyl 6-(2,3-dichlorophenyl)-3-(7-((1,3-dioxoisoindolin-2-yl)methyl)-7-(thiophen-3-yl)-3-azabicyclo[4.1.0]heptan-3-yl)-5-methylpyrazine-2-carboxylate

A mixture of ethyl 3-chloro-6-(2,3-dichlorophenyl)-5-methylpyrazine-2-carboxylate (18 mg, 0.050 mmol), **Intermediate 37** (19.5 mg, 0.050 mmol) and DIPEA (0.04 mL, 0.210 mmol) in DMA (0.46 mL) was stirred at 70 °C for 24 h. After cooling, the volatiles were concentrated *in vacuo* and the residue (33 mg, 98%) was used without further purification. LCMS (ES⁺) *m*/*z* 647-649 (M+H)⁺, RT 2.69 min.

### Step 6: ethyl 3-(7-(aminomethyl)-7-(thiophen-3-yl)-3-azabicyclo[4.1.0]heptan-3-yl)-6-(2,3-dichlorophenyl)-5-methylpyrazine-2-carboxylate

Ethyl 6-(2,3-dichlorophenyl)-3-(7-((1,3-dioxoisoindolin-2-yl)methyl)-7-(thiophen-3-yl)-3-azabicyclo[4.1.0]heptan-3-yl)-5-methylpyrazine-2-carboxylate (33 mg, 0.050 mmol) in MeOH (0.48 mL) was treated with hydrazine hydrate (15.7 uL, 0.200 mmol). The resulting mixture was stirred at rt for 18 h then concentrated under reduced pressure to afford the title compound as a yellow powder (26 mg, 59%) and used without further purification. LCMS (ES⁺) *m*/*z* 517-519 (M+H)⁺, RT 1.59 min

### Step 7: (3-(7-(aminomethyl)-7-(thiophen-3-yl)-3-azabicyclo[4.1.0]heptan-3-yl)-6-(2,3-dichlorophenyl)-5-methylpyrazin-2-yl) methanol

A solution of ethyl 3-(7-(aminomethyl)-7-(thiophen-3-yl)-3-azabicyclo[4.1.0]heptan-3-yl)-6-(2,3-dichlorophenyl)-5-methylpyrazine-2-carboxylate (26 mg, 0.030 mmol) in DCM (0.30 mL) was treated with DIBAL-H (1M in THF; 0.12 mL, 0.120 mmol) at -78°C. The mixture was stirred at this temperature for 30 min then additional DIBAL-H (1M in THF; 0.12 mL, 0.120 mmol) was added and the mixture was stirred for further 1 h and 30 min at -78°C. DCM (0.6 mL) was added to the mixture followed by H₂O (40 uL) and 2N NaOH (10 uL). The resulting slurry was stirred 30 min then the mixture was filtered and washed with (DCM : MeOH / 1: 5 mL). The filtrate was concentrated *in vacuo* and the residue was purified by preparative HPLC (from 20% to 100% MeCN/H₂O + 0.1% TFA) to obtain the title compound as a yellow solid (TFA salt; 0.9 mg, 6%). ¹H NMR (400 MHz, DMSO-*d*₆) δ 7.68 (d, *J* = 8.0 Hz, 2H), 7.49-7.47 (m, 1H), 7.42 (t, *J* = 8.0 Hz, 1H), 7.35-7.31 (m, 3H), 7.08 (d, *J* = 8.0 Hz, 1H), 6.45 (s, 1H), 5.31 (br s, 1H), 4.50-4.44 (m, 2H), 3.83 (d, *J* = 12.0 Hz, 1H), 3.69 (d, *J* = 8.0 Hz, 1H), 3.52-3.50 (m, 2H), 2.65-2.59 (m, 1H), 2.21-2.12 (m, 2H), 2.15 (s, 3H), 1.95-1.89 (m, 1H), 1.75-1.68 (m, 1H), 1.53-1.49 (m, 1H). LCMS (ES⁺) *m*/*z* 475 (M+H)⁺, RT 1.54 min

### Example 2: [3-[3-(2,3-dichlorophenyl)-1H-pyrazolo[3,4-b]pyrazin-6-yl]-7-(pyridin-3-yl)-3-azabicyclo[4.1.0] heptan-7-yl]methanamine

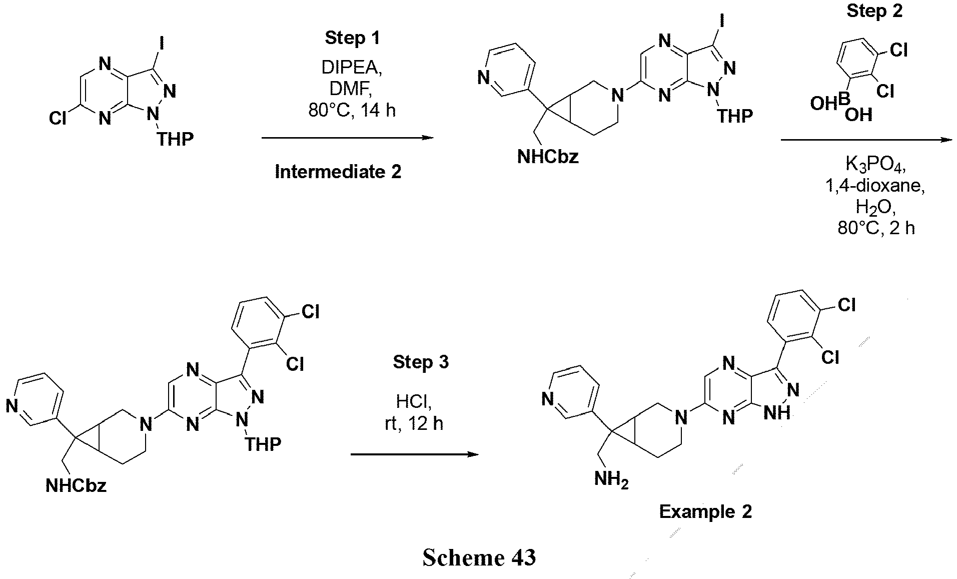

### Step 1: benzyl ((3-(3-iodo-1-(tetrahydro-2H-pyran-2-yl)-1H-pyrazolo[3,4-b]pyrazin-6-yl)-7-(pyridin-3-yl)-3-azabicyclo[4.1.0]heptan-7-yl)methyl)carbamate

A solution of **Intermediate 44** (9 mg, 0.02 mmol) and **Intermediate 2** (10 mg, 0.020 mmol) and DIPEA (26.4 uL, 0.150 mmol) in DMF (0.6 mL) was heated at 80 °C for 14 h. After cooling, EtOAc (20 mL) was added and the mixture washed with citric acid (5%), H₂O, brine, dried over Na₂SO₄ and concentrated *in vacuo* to afford the title compound as a yellow solid (14 mg, 85%) which was used as crude in the next step. LCMS (ES⁺) *m*/*z* 666 (M+H)⁺, RT 1.59 min.

### Step 2: benzyl ((3-(3-(2,3-dichlorophenyl)-1-(tetrahydro-2H-pyran-2-yl)-1H-pyrazolo[3,4-b]pyrazin-6-yl)-7-(pyridin-3-yl)-3-azabicyclo[4.1.0]heptan-7-yl)methyl)carbamate

A solution of benzyl ((3-(3-iodo-1-(tetrahydro-2H-pyran-2-yl)-1H-pyrazolo[3,4-b]pyrazin-6-yl)-7-(pyridin-3-yl)-3-azabicyclo[4.1.0]heptan-7-yl)methyl)carbamate (15 mg, 0.020 mmol), (2,3-dichlorophenyl)boronic acid (8.6 mg, 0.050 mmol), [1,1'-Bis(diphenylphosphino)ferrocene]dichloropalladium(II) complex with dichloromethane (3.73 mg, 0.005 mmol) and tripotassium phosphate (28.71 mg, 0.140 mmol) in a mixture of degassed 1,4-dioxane (0.7 mL) and H₂O (70 uL) was heated in a pressure tube at 80 °C for 2 h. After cooling, the mixture was diluted with EtOAc (20 mL), washed with brine (2x2 mL), dried over Na₂SO₄ and concentrated *in vacuo* to afford a residue which was purified by flash chromatography (gradient elution 0-100% EtOAc in petroleum ether) to obtain the title compound as a yellow solid (9 mg, 58%). LCMS (ES⁺) *m*/*z* 684 (M+H)⁺, RT 1.94 min.

### Step 3: (3-(3-(2,3-dichlorophenyl)-1H-pyrazolo[3,4-b]pyrazin-6-yl)-7-(pyridin-3-yl)-3-azabicyclo[4.1.0]heptan-7-yl)methanamine

Benzyl ((3-(3-(2,3-dichlorophenyl)-1-(tetrahydro-2H-pyran-2-yl)-1H-pyrazolo[3,4-b]pyrazin-6-yl)-7-(pyridin-3-yl)-3-azabicyclo[4.1.0]heptan-7-yl)methyl)carbamate (9 mg, 0.010 mmol) was treated with conc. HCl (37%; 0.5 mL, 0.010 mmol) and the mixture was stirred at rt for 12 h. After volatiles removal *in vacuo* the residue was purified by preparative HPLC (from 10% to 100% MeCN/H₂O + 0.1% TFA) to obtain the title compound as a white solid (TFA salt; 0.4 mg, 7%). LCMS (ES⁺) *m*/*z* 466 (M+H)⁺, RT 0.99 min.

The following examples were synthesized using the above procedure **(Scheme 43)** with the corresponding starting materials: **Example 3, Example 4** and **Example 5** (using **Intermediate 3); Example 6** (using **Intermediate 4); Example 7** (using **Intermediate 5); Example 8, Example 9** (using **Intermediate 6); Example 10, Example 11** and **Example 12** (using **Intermediate 7;** for **Example 12** the step 3 was performed using HCl (37%) in 1,4-dioxane); **Example 13** (using **Intermediate 9); Example 14** and **Example 15** (using **Intermediate 10** and 4N HCl in 1,4-dioxane in step 3 instead of 37% HCl, and for **Example 15** the **Intermediate 45** was used in step 2); **Example 16** (using **Intermediate 3** and **Intermediate 11**); **Example 17** (using **Intermediate 6** and **Intermediate 11**); **Example 18, Example 19, Example 20, Example 21, Example 22, Example 23, Example 24** (using **Intermediate 12** and for **Example 24** the **Intermediate 45** was used in step 2); **Example 25 Example 26, Example 27, Example 28, Example 29, Example 30, Example 31** (using **Intermediate 6**); **Example 32** (using **Intermediate 6** and HBr in acetic acid was used in step 3 instead of 37% HCl); **Example 33** (using **Intermediate 6** and **Intermediate 15**); **Example 34** (using **Intermediate 6** and **Intermediate 16**); **Example 35** (using **Intermediate 6** and HBr in acetic acid was used in step 3 instead of 37% HCl); **Example 36** (using **Intermediate 6** and **Intermediate 17**); **Example 37, Example 38, Example 39, Example 40** (using **Intermediate 18**); **Example 41** (using **Intermediate 18** and **Intermediate 19**); **Example 42** (using **Intermediate 18** and **Intermediate 20**); **Example 43** (using **Intermediate 18** and **Intermediate 21**); **Example 44** (using **Intermediate 18** and **Intermediate 46**); **Example 45** (using **Intermediate 18** and **Intermediate 22**); **Example 46** (using **Intermediate 18** and **Intermediate 45**); **Example 47** (using **Intermediate 23** and **Intermediate 45**); **Example 48** (using **Intermediate 24** and **Intermediate 45**); **Example 49** (using **Intermediate 25** and **Intermediate 45**); **Example 50** (using **Intermediate 26** and **Intermediate 45**); **Example 51** (using **Intermediate 18** and **Intermediate 27**); **Example 52** (using **Intermediate 10**).

| **Example #** | **¹H NMR (400 MHz, DMSO-d₆**) **δ** |
|---|---|
| 3 | δ 13.47 (br s, 1H), 8.43 (s, 1H), 7.78-7.69 (m, 5H), 7.53-7.49 (m, 2H), 7.40-7.35 (m, 1H), 7.23-7.19 (m, 2H), 4.44 (d, J = 12.0 Hz, 1H), 4.35-4.32 (m, 1H), 3.18-3.07 (m, 2H), 2.97-2.89 (m, 1H), 2.36-2.33 (m, 1H), 1.82-1.76 (m, 2H), 1.57-1.54 (m, 1H), 1.19-1.16 (m, 1 H) |
| 4 | 13.51 (br s, 1H), 8.89 (d, J = 4.0 Hz, 1H), 8.53 (s, 1H), 7.96 (d, J = 4.0 Hz, 1H), 7.85 (d, J = 4.0 Hz, 1H), 7.66-7.32 (m, 7H), 7.22 (d, J = 4.0 Hz, 2H), 4.47 (d, J = 16.0 Hz, 1H), 4.39-4.37 (m, 1H), 3.79-3.75 (m, 1H), 3.14-2.95 (m, 4H), 1.82-1.79 (m, 2H), 1.59-1.56 (m, 1H) |
| 5 | 13.55 (br s, 1H), 9.30 (d, J = 8.0 Hz, 1H), 9.01 (s, 1H), 8.49 (s, 1H), 8.42 (d, J = 8.0 Hz, 1H), 8.13 (d, J = 8.0 Hz, 1H), 7.96 (t, J = 8.0 Hz, 1H), 7.67-7.64 (m, 4H), 7.48-7.45 (m, 1H), 7.37-7.35 (m, 1H), 7.23-7.19 (m, 2H), 4.47 (d, J = 16.0 Hz, 1H), 4.37-4.34 (m, 1H), 3.79-3.73 (m, 1H), 3.18-2.95 (m, 4H), 1.84-1.80 (m, 2H), 1.57-1.54 (m, 1H) |
| 6 | 13.47 (br s, 1H), 8.40 (s, 1H), 7.93 (br s, 3H), 7.89 (d, J = 8.0 Hz, 1H), 7.68 (d, J = 8.0 Hz, 1H), 7.50 (t, J = 8.0 Hz, 1H), 6.28 (s, 1H), 4.33 (d, J = 12.0 Hz, 2H), 3.71-3.69 (m, 1H), 3.26-3.22 (m, 2H), 2.97-2.94 (m, 1H), 2.32-2.30 (m, 1H), 2.21 (s, 3H), 2.08-1.71 (m, 3H) |
| 7 | 8.33 (s, 1H), 7.69 (d, J = 8.0 Hz, 1H), 7.62 (d, J = 8.0 Hz, 1H), 7.54 (br s, 3H), 7.43 (t, J = 8.0 Hz, 1H), 7.40-7.32 (m, 1H), 7.25 (t, J = 8.0 Hz, 2H), 7.06 (t, J = 8.0 Hz, 1H), 4.35 (d, J = 16.0 Hz, 1H), 4.26 (dd, J1= 4.0 Hz, J2= 12 Hz, 1H), 3.64-3.61 (m, 1H), 3.22-3.18 (m, 1H), 3.10-3.06 (m, 1H), 2.84-2.81 (m, 1H), 2.27-2.22 (m, 1H), 1.80-1.48 (m, 3H) |
| 8 | 13.47 (br s, 1H), 8.40 (s, 1H), 7.87 (br s, 3H), 7.76 (d, J = 8.0 Hz, 1H), 7.69 (d, J = 8.0 Hz, 1H), 7.51 (t, J = 8.0 Hz, 1H), 6.15 (s, 1H), 4.33-4.29 (m, 2H), 3.75-3.70 (m, 1H), 3.27-3.18 (m, 2H), 2.99-2.92 (m, 1H), 2.39 (s, 3H), 2.31-2.28 (m, 1H), 1.98-1.92 (m, 1H), 1.78-1.72 (m, 2H) |
| 9 | 13.56 (br s, 1H), 9.28 (d, J = 8.0 Hz, 1H), 9.00 (s, 1H), 8.46 (s, 1H), 8.42 (d, J = 8.0 Hz, 1H), 8.13 (d, J = 8.0 Hz, 1H), 7.95 (t, J = 8.0 Hz, 1H), 7.88 (br s, 3H), 7.66-7.63 (m, 1H), 6.15 (s, 1H), 4.34 (d, J = 16.0 Hz, 2H), 3.77-3.72 (m, 2H), 3.25-3.20 (m, 1H), 2.99-2.95 (m, 1H), 2.39 (s, 3H), 2.31-2.28 (m, 1H), 1.99-1.95 (m, 1H), 1.77-1.73 (m, 2H) |
| 10 | 8.39 (s, 1H), 7.74 (d, J = 8.0 Hz, 1H), 7.65 (d, J = 8.0 Hz, 1H), 7.60 (br s, 3H), 7.53-7.47 (m, 3H), 7.18 (t, J = 8.0 Hz, 2H), 4.42 (d, J = 12.0 Hz, 1H), 4.31 (br, J= 16 Hz, 1H), 3.72-3.67 (m, 1H), 3.25-3.20 (m, 1H), 3.15-3.10 (m, 1H), 2.92-2.85 (m, 1H), 1.80-1.75 (m, 2H), 1.52-1.50 (m, 1H) |
| 11 | 13.51 (s, 1H), 9.17 (s, 1H), 8.97 (d, J = 16.0 Hz, 2H), 8.76 (d, J = 8.0 Hz, 1H), 8.56 (s, 1H), 8.23 (d, J = 8.0 Hz, 1H), 7.59 (br s, 3H), 7.52 (t, J = 8.0 Hz, 2H), 7.21 (t, J = 8.0 Hz, 2H), 4.45 (d, J = 16.0 Hz, 1H), 4.35 (br d, J= 16 Hz, 1H), 3.79-3.76 (m, 1H), 3.27-3.21 (m, 2H), 3.15-3.10 (m, 1H), 2.96-2.90 (m, 1H), 1.84-1.75 (m, 2H), 1.57-1.51 (m, 1H) |
| 12 | 9.04 (s, 1H), 8.67 (d, J = 8.0 Hz, 1H), 8.65 (br s, 1H), 8.45 (s, 1H), 8.18 (d, J = 8.0 Hz, 1H), 7.86 (t, J = 8.0 Hz, 1H), 7.76 (br s, 1H), 7.64 (br s, 3H), 7.53 (t, J = 8.0 Hz, 2H), 7.21 (t, J = 8.0 Hz, 2H), 4.48 (d, J = 16.0 Hz, 1H), 4.36 (br d, J= 12 Hz, 1H), 3.28-3.24 (m, 2H), 3.17-3.15 (m, 1H), 2.97-2.88 (m, 1H), 2.35-2.31 (m, 1H), 1.84-1.75 (m, 2H), 1.54-1.51 (m, 1H) |
| 13 | 8.40 (s, 1H),7.93 (s, 1H), 7.83 (br s, 3H), 7.76 (d, J = 8.0 Hz, 1H), 7.69 (d, J = 8.0 Hz, 1H), 7.51(t, J = 8.0 Hz, 1H), 4.31 (br d, J = 12.0 Hz, 2H), 4.03 (s, 3H), 3.77-3.72 (m, 2H), 3.25-3.20 (m, 1H), 2.98-2.81 (m, 1H), 2.33-2.30 (m, 1H), 1.89-1.62 (m, 3H) |
| 14 | 13.47 (br s, 1H), 8.67 (s, 1H), 8.42 (s, 1H), 7.82 (br s, 3H), 7.75 (d, J = 8.0 Hz, 1H), 7.66 (d, J = 8.0 Hz, 1H), 7.50 (t, J = 8.0 Hz, 1H), 4.41 (d, J = 12.0 Hz, 1H), 4.34 (d, J = 12.0 Hz, 1H), 3.31-3.14 (m, 2H), 2.95-2.92 (m, 1H), 2.35-2.32 (m, 1H), 2.08 (s, 3H), 2.01-1.98 (m, 1H), 1.76-1.71 (m, 2H) |
| 15 | 13.36 (br s, 1H), 8.68 (s, 1H), 8.56 (s, 1H), 8.42 (s, 1H), 7.79 (br s, 3H), 7.69-7.63 (m, 2H), 4.43 (d, J = 12.0 Hz, 1H), 4.34 (d, J = 12.0 Hz, 1H), 4.23 (s, 3H), 3.76-3.72 (m 1H), 3.24-3.12 (m, 2H), 2.95-2.92 (m, 1H), 2.35-2.32 (m, 1H), 2.08 (s, 3H), 2.01-1.98 (m, 1H), 1.76-1.71 (m, 2H) |
| 16 | 13.54 (br s, 1H), 8.45 (s, 1H), 8.08 (d, J = 4.0 Hz, 1H), 7.67 (br s, 3H), 7.47-7.21 (m, 4H), 7.02 (d, J = 4.0 Hz, 1H), 6.85 (br s, 1H), 4.44 (d, J = 16.0 Hz, 1H), 4.33 (d, J = 12.0 Hz, 1H), 3.76-3.72 (m 1H), 3.18-3.10 (m, 2H), 2.95-2.93 (m, 1H), 2.93 (s, 3H), 2.35-2.32 (m, 1H), 1.92-1.77 (m, 2H), 1.57-1.51 (m, 1H) |
| 17 | 8.48 (s, 1H), 8.08 (d, J = 8.0 Hz, 1H), 7.88 (br s, 3H), 7.55 (d, J = 8.0 Hz, 1H), 6.12 (s, 1H), 4.31-4.26 (m, 2H), 3.79-3.75 (m, 1H), 3.40-3.37 (m, 1H), 3.24-3.19 (m, 1H), 3.06 (s, 3H), 3.02-2.97 (m, 1H), 2.35-2.32 (m, 2H), 1.97-1.94 (m, 2H), 1.78-1.71 (m, 3H) |
| 18 | 3.89 (s, 1H), 8.87 (d, J = 4.0 Hz, 1H), 8.71 (d, J = 4.0 Hz, 1H), 8.56 (s, 1H), 8.24 (d, J = 4.0 Hz, 1H), 7.86 (br s, 3H), 7.65 (d, J = 4.0 Hz, 1H), 6.15 (s, 1H), 4.35 (d, J = 12.0 Hz, 2H), 3.82-3.78 (m, 1H), 3.40-3.20 (m, 2H), 3.04-3.02 (m, 1H), 2.39 (s, 3H), 2.35-2.32 (m, 1H), 1.97-1.94 (m, 1H), 1.78-1.71 (m, 2H) |
| 19 | 13.49 (s, 1H), 8.91 (d, J = 8.0 Hz, 1H), 8.69 (s, 1H), 8.49 (s, 1H), 7.86 (br s, 3H), 7.72-7.66 (m, 2H), 6.15 (s, 1H), 4.73 (s, 2H), 4.33 (d, J = 16.0 Hz, 2H), 3.79-3.75 (m, 1H), 3.30-3.20 (m, 2H), 3.04-2.95 (m, 1H), 2.39 (s, 3H), 2.35-2.32 (m, 1H), 1.97-1.94 (m, 1H), 1.78-1.71 (m, 2H) |
| 20 | 13.34 (s, 1H), 9.45 (s, 1H), 9.12 (s, 1H), 8.50 (s, 1H), 8.39 (d, J = 8.0 Hz, 1H), 8.28 (d, J = 8.0 Hz, 1H), 7.87 (br s, 3H), 6.15 (s, 1H), 4.35 (d, J = 12.0 Hz, 2H), 3.78-3.73 (m, 1H), 3.25-3.21 (m, 2H), 3.04-2.95 (m, 1H), 2.39 (s, 3H), 2.35-2.32 (m, 1H), 1.98-1.95 (m, 1H), 1.78-1.72 (m, 2H) |
| 21 | 13.48 (s, 1H), 9.23 (d, J = 8.0 Hz, 1H), 9.00 (d, J = 4.0 Hz, 1H), 8.45 (s, 1H), 8.26 (d, J = 8.0 Hz, 1H), 7.88 (br s, 3H), 7.79 (d, J = 8.0 Hz, 1H), 7.63-7.59 (m, 1H), 6.16 (s, 1H), 4.35 (d, J = 16.0 Hz, 2H), 3.79-3.73 (m, 1H), 3.45-3.41 (m, 1H), 3.25-3.21 (m, 1H), 3.04-2.95 (m, 1H), 2.81 (s, 3H), 2.40 (s, 3H), 2.35-2.32 (m, 1H), 2.00-1.94 (m, 1H), 1.78-1.72 (m, 2H) |
| 22 | 13.42 (br s, 1H), 9.33 (br d, J = 8.0 Hz, 1H), 8.94 (s, 1H), 8.44 (s, 1H), 8.37 (d, J = 8.0 Hz, 1H), 7.88 (br s, 2H), 7.68 (d, J = 8.0 Hz, 1H), 7.42 (d, J = 8.0 Hz, 1H), 6.16 (s, 1H), 4.35 (d, J = 12.0 Hz, 2H), 4.07 (s, 3H), 3.78-3.73 (m, 1H), 3.27-3.21 (m, 2H), 3.02-2.96 (m, 1H), 2.40 (s, 3H), 2.35-2.32 (m, 1H), 1.98-1.94 (m, 1H), 1.79-1.75 (m, 2H) |
| 23 | 13.60 (br s, 1H), 9.53 (s, 1H), 8.87 (br s, 1H), 8.71 (br d, J = 4.0 Hz, 1H), 8.61 (d, J = 8.0 Hz, 1H), 8.48 (s, 1H), 8.30 (d, J = 8.0 Hz, 1H), 7.94 (d, J = 8.0 Hz, 1H), 7.88 (br s, 3H), 6.16 (s, 1H), 4.35 (d, J = 12.0 Hz, 2H), 3.80-3.75 (m, 1H), 3.30-3.21 (m, 2H), 3.03-2.97 (m, 1H), 2.40 (s, 3H), 2.35-2.32 (m, 1H), 2.00-1.94 (m, 1H), 1.79-1.75 (m, 2H) |
| 24 | 13.40 (br s, 1H), 8.56 (s, 1H), 8.40 (s, 1H), 7.89 (br s, 3H), 7.70-7.63 (m, 2H), 6.15 (s, 1H), 4.32 (d, J = 16.0 Hz, 2H), 4.24 (s, 3H), 3.75-3.70 (m, 1H), 3.42-3.40 (m, 1H), 3.26-3.21 (m, 1H), 3.03-2.92 (m, 1H), 2.39 (s, 3H), 2.35-2.32 (m, 1H), 1.98-1.94 (m, 1H), 1.79-1.72 (m, 2H) |
| 26 | 13.37 (s, 1H), 8.81 (s, 1H), 8.47 (s, 1H), 8.46 (d, J = 8.0 Hz, 1H), 7.90 (br s, 3H), 7.63 (d, J = 8.0 Hz, 1H), 7.38 (t, J = 8.0 Hz, 1H), 6.15 (s, 1H), 4.34 (d, J = 16.0 Hz, 2H), 4.24 (s, 3H), 3.75-3.70 (m, 1H), 3.42-3.40 (m, 1H), 3.26-3.21 (m, 1H), 3.03-2.92 (m, 1H), 2.39 (s, 3H), 2.35-2.32 (m, 1H), 1.98-1.94 (m, 1H), 1.79-1.72 (m, 2H) |
| 27 | 13.84 (s, 1H), 9.11-9.07 (m, 2H), 8.52 (s, 1H), 8.43 (br d, J = 4.0 Hz, 1H), 8.15 (d, J = 8.0 Hz, 1H), 7.90-7.87 (br m, 4H), 7.73 (t, J = 8.0 Hz, 1H), 6.16 (s, 1H), 4.35 (d, J = 12.0 Hz, 2H), 3.80-3.75 (m, 1H), 3.45-3.40 (m, 1H), 3.25-3.21 (m, 1H), 3.04-2.98 (m, 1H), 2.40 (s, 3H), 2.35-2.32 (m, 1H), 2.00-1.96 (m, 1H), 1.79-1.74 (m, 2H) |
| 28 | 8.40 (s, 1H), 8.25 (br s, 2H), 7.87 (br s, 3H), 7.14 (br t, J = 8.0 Hz, 1H), 6.14 (s, 1H), 4.32 (d, J = 16.0 Hz, 2H), 3.90 (s, 3H), 3.74-3.72 (m, 1H), 3.40-3.19 (m, 2H), 2.97-2.94 (m, 1H), 2.38 (s, 3H), 2.35-2.32 (m, 1H), 1.97-1.93 (m, 1H), 1.76-1.73 (m, 2H) |
| 29 | 8.45 (s, 1H), 7.87 (br s, 3H), 7.75 (d, J = 4.0 Hz, 1H), 7.37 (br s, 1H), 6.14 (s, 1H), 4.64 (br s, J = 8.0 Hz, 1H), 4.32 (d, J = 16.0 Hz, 2H), 3.81-3.72 (m, 3H), 3.56-3.52 (m, 2H), 3.40-3.19 (m, 2H), 3.00-2.94 (m, 1H), 2.39 (s, 3H), 2.35-2.32 (m, 1H), 2.16-2.14 (m, 2H), 2.00-1.89 (m, 2H), 1.80-1.73 (m, 2H), 1.58-1.52 (m, 1H) |
| 30 | 13.55 (s, 1H), 9.31 (d, J = 8.0 Hz, 1H), 9.04 (d, J = 4.0 Hz, 1H), 8.46 (s, 1H), 8.38 (br t, J = 8.0 Hz, 1H), 7.89 (br s, 3H), 7.77 (t, J = 8.0 Hz, 1H), 7.73-7.69 (m, 1H), 6.16 (s, 1H), 4.34 (d, J = 16.0 Hz, 2H), 3.79-3.73 (m, 1H), 3.40-3.19 (m, 2H), 3.03-2.96 (m, 1H), 2.40 (s, 3H), 2.35-2.32 (m, 1H), 1.99-1.95 (m, 1H), 1.79-1.75 (m, 2H) |
| 31 | 13.78 (s, 1H), 8.52 (d, J = 4.0 Hz, 1H), 8.47 (s, 1H), 8.00 (d, J = 4.0 Hz, 1H), 7.86 (br s, 3H), 6.14 (s, 1H), 4.31 (d, J = 16.0 Hz, 2H), 3.78-3.73 (m, 1H), 3.40-3.19 (m, 2H), 3.01-2.95 (m, 1H), 2.39 (s, 3H), 2.35-2.32 (m, 1H), 1.97-1.94 (m, 1H), 1.77-1.72 (m, 2H) |
| 32 | 13.81 (s, 1H), 9.52 (d, J = 8.0 Hz, 1H), 9.16 (d, J = 4.0 Hz, 1H), 8.62 (d, J = 8.0 Hz, 1H), 8.55 (d, J = 8.0 Hz, 1H), 8.51 (s, 1H), 7.88 (br s, 3H), 7.83-7.79 (m, 1H), 6.16 (s, 1H), 4.35 (d, J = 12.0 Hz, 2H), 3.82-3.77 (m, 1H), 3.40-3.19 (m, 2H), 3.04-2.98 (m, 1H), 2.40 (s, 3H), 2.35-2.31 (m, 1H), 1.99-1.94 (m, 1H), 1.79-1.74 (m, 2H) |
| 33 | 13.60 (s, 1H), 9.38 (d, J = 4.0 Hz, 1H), 9.09 (d, J = 4.0 Hz, 1H), 8.47 (s, 1H), 8.38 (d, J = 4.0 Hz, 1H), 8.15 (d, J = 4.0 Hz, 1H), 7.87 (br s, 3H), 7.74-7.71 (m, 1H), 6.15 (s, 1H), 4.34 (d, J = 16.0 Hz, 2H), 3.79-3.73 (m, 1H), 3.40-3.20 (m, 2H), 3.04-2.98 (m, 1H), 2.39 (s, 3H), 2.35-2.31 (m, 1H), 1.99-1.95 (m, 1H), 1.79-1.74 (m, 2H) |
| 35 | 9.15 (d, J = 4.0 Hz, 1H), 9.00 (br s, 1H), 8.54 (d, J = 8.0 Hz, 1H), 8.40 (br s, 1H), 8.05 (br s, 1H), 7.88 (br m, 4H), 6.16 (s, 1H), 4.35 (br d, J = 16.0 Hz, 2H), 3.76-3.72 (m, 1H), 3.40-3.20 (m, 2H), 3.02-2.96 (m, 1H), 2.40 (s, 3H), 2.35-2.31 (m, 1H), 1.98-1.95 (m, 1H), 1.79-1.74 (m, 2H) |
| 36 | 8.49 (s, 1H), 8.09 (d, J = 8.0 Hz, 1H), 7.89 (br s, 3H), 7.65 (d, J = 8.0 Hz, 1H), 6.11 (s, 1H), 4.30 (d, J = 16.0 Hz, 2H), 3.79-3.74 (m, 1H), 3.40-3.35 (m, 1H), 3.25-3.19 (m, 1H), 3.01-2.97 (m, 1H), 2.37 (s, 3H), 2.33-2.28 (m, 1H), 1.98-1.93 (m, 1H), 1.77-1.72 (m, 2H) |
| 37 | 13.35 (s, 1H), 8.45 (s, 1H), 8.33 (d, J = 8.0 Hz, 1H), 8.20 (s, 1H), 7.87 (br s, 3H), 7.56 (d, J = 8.0 Hz, 1H), 6.14 (s, 1H), 4.32 (d, J = 16.0 Hz, 2H), 3.79-3.73 (m, 1H), 3.43-3.38 (m, 1H), 3.20-3.15 (m, 1H), 3.00-2.99 (m, 1H), 2.38 (s, 3H), 2.33-2.30 (m, 1H), 1.98-1.94 (m, 1H), 1.76-1.72 (m, 2H) |
| 38 | 13.87 (s, 1H), 9.53 (d, J = 8.0 Hz, 1H), 9.25 (d, J = 8.0 Hz, 1H), 9.16 (s, 1H), 8.54 (s, 1H), 8.52 (d, J = 8.0 Hz, 1H), 7.87 (br s, 3H), 7.54 (d, J = 8.0 Hz, 1H), 6.16 (s, 1H), 4.35 (d, J = 12.0 Hz, 2H), 3.82-3.79 (m, 1H), 3.43-3.15 (m, 2H), 3.00-2.97 (m, 1H), 2.40 (s, 3H), 2.33-2.30 (m, 1H), 1.98-1.94 (m, 1H), 1.79-1.74 (m, 2H) |
| 39 | 13.83 (s, 1H), 9.07 (s, 1H), 8.93 (d, J = 8.0 Hz, 1H), 8.54 (s, 2H), 8.21-8.19 (m, 1H), 7.87 (br s, 3H), 7.78-7.75 (m, 1H), 6.16 (s, 1H), 4.36 (d, J = 16.0 Hz, 2H), 3.82-3.79 (m, 1H), 3.43-3.22 (m, 2H), 3.01-2.98 (m, 1H), 2.40 (s, 3H), 2.33-2.30 (m, 1H), 1.98-1.94 (m, 1H), 1.79-1.74 (m, 2H) |
| 40 | 8.38 (s, 1H), 7.87 (br s, 3H), 7.39 (br s, 1H), 6.93 (s, 2H), 6.14 (s, 1H), 4.34-4.25 (m, 6H), 3.72-3.67 (m, 1H), 3.45-3.40 (m, 1H), 3.29-3.25 (m, 1H), 2.95-2.92 (m, 1H), 2.38 (s, 3H), 2.31-2.28 (m, 1H), 1.98-1.94 (m, 1H), 1.76-1.71 (m, 2H) |
| 41 | 13.46 (s, 1H), 9.06 (s, 1H), 8.32 (s, 1H), 8.02 (d, J = 8.0 Hz, 1H), 7.88-7.80 (m, 4H), 7.64 (s, 1H), 7.40-7.35 (m, 1H), 6.14 (s, 1H), 4.35-4.26 (m, 2H), 3.82-3.79 (m, 1H), 3.43-3.22 (m, 2H), 2.95-2.94 (m, 1H), 2.40 (s, 3H), 2.33-2.30 (m, 1H), 1.98-1.94 (m, 1H), 1.76-1.72 (m, 2H) |
| 42 | 13.85 (s, 1H), 9.31 (s, 1H), 9.10 (d, J = 8.0 Hz, 1H), 8.58 (s, 1H), 8.54 (s, 1H), 8.15 (d, J = 8.0 Hz, 1H), 7.88-7.86 (m, 4H), 6.16 (s, 1H), 4.36 (d, J = 12.0 Hz, 2H), 3.82-3.79 (m, 1H), 3.43-3.22 (m, 2H), 3.02-2.99 (m, 1H), 2.40 (s, 3H), 2.33-2.30 (m, 1H), 1.98-1.94 (m, 1H), 1.78-1.74 (m, 2H) |
| 43 | 13.74 (s, 1H), 9.41 (d, J = 8.0 Hz, 1H), 9.12 (s, 1H), 8.53-8.50 (m, 2H), 8.37 (d, J = 8.0 Hz, 1H), 7.89 (br s, 3H), 7.78-7.75 (m, 1H), 6.16 (s, 1H), 4.36 (d, J = 12.0 Hz, 2H), 3.82-3.79 (m, 1H), 3.45-3.22 (m, 2H), 3.02-2.99 (m, 1H), 2.40 (s, 3H), 2.35-2.31 (m, 1H), 1.98-1.95 (m, 1H), 1.78-1.74 (m, 2H) |
| 44 | 13.43 (s, 1H), 8.39 (s, 1H), 7.89 (br s, 3H), 7.70 (d, J = 8 Hz, 1H), 7.61 (d, J = 8 Hz, 1H), 6.14 (s, 1H), 4.31 (d, J = 16.0 Hz, 2H), 4.18 (s, 3H), 3.76-3.70 (m, 1H), 3.45-3.22 (m, 2H), 2.98-2.96 (m, 1H), 2.38 (s, 3H), 2.30-2.26 (m, 1H), 1.97-1.94 (m, 1H), 1.76-1.72 (m, 2H) |
| 45 | 13.83 (s, 1H), 9.16 (t, J = 8 Hz, 1H), 9.09 (d, J = 4 Hz, 1H), 8.52 (s, 1H), 8.39 (d, J = 4 Hz, 1H), 7.88-7.84 (br m, 4H), 7.64 (t, J = 8 Hz, 1H), 6.15 (s, 1H), 4.35 (d, J = 12.0 Hz, 2H), 3.80-3.75 (m, 1H), 3.45-3.22 (m, 2H), 3.02-2.98 (m, 1H), 2.39 (s, 3H), 2.30-2.26 (m, 1H), 1.98-1.94 (m, 1H), 1.78-1.74 (m, 2H) |
| 46 | 8.52 (s, 1H), 8.38 (s, 1H), 7.87 (br s, 3H), 7.64 (t, J = 8 Hz, 2H), 6.11 (s, 1H), 4.33 (d, J = 12.0 Hz, 2H), 4.22 (s, 3H), 3.73-3.70 (m, 1H), 3.44-3.40 (m, 1H), 3.26-3.21 (m, 1H), 2.99-2.96 (m, 1H), 2.37 (s, 3H), 2.33-2.27 (m, 1H), 1.96-1.92 (m, 1H), 1.76-1.71 (m, 2H) |
| 47 | 13.37 (s, 1H), 8.56 (s, 1H), 8.40 (s, 1H), 7.89 (br s, 3H), 7.69-7.62 (m, 2H), 6.44 (s, 1H), 4.52 (s, 2H), 4.33 (d, J = 12.0 Hz, 2H), 4.23 (s, 3H), 3.77-3.73 (m, 1H), 3.45-3.42 (m, 1H), 3.33 (s, 3H), 3.31-3.22 (m, 1H), 2.98-2.95 (m, 1H), 2.35-2.32 (m, 1H), 2.01-1.95 (m, 1H), 1.81-1.75 (m, 2H) |
| 48 | 13.37 (s, 1H), 8.55 (s, 1H), 8.39 (s, 1H), 7.86 (br s, 3H), 7.69-7.62 (m, 2H), 6.11 (s, 1H), 4.32 (d, J = 12.0 Hz, 2H), 4.23 (s, 3H), 3.75-3.72 (m, 1H), 3.45-3.42 (m, 1H), 3.27-3.22 (m, 1H), 2.98-2.95 (m, 1H), 2.35-2.32 (m, 1H), 2.10-2.00 (m, 1H), 1.95-1.89 (m, 1H), 1.76-1.71 (m, 2H), 1.10-1.06 (m, 2H), 0.85-0.82 (m, 2H) |
| 49 | 8.54 (s, 1H), 8.43 (s, 1H), 8.41 (s, 1H), 7.82-7.75 (m, 4H), 7.68-7.64 (m, 2H), 7.49-7.46 (m, 1H), 4.49 (d, J = 12.0 Hz, 1H), 4.39 (d, J = 12.0 Hz, 1H), 4.23 (s, 3H), 3.76-3.71 (m, 1H), 3.34-3.21 (m, 2H), 2.95-2.93 (m, 1H), 2.35-2.31 (m, 1H), 2.10-2.06 (m, 1H), 1.85-1.80 (m, 2H) |
| 50 | 13.37 (s, 1H), 8.56 (s, 1H), 8.50 (s, 1H), 8.41 (s, 1H), 7.79-7.77 (m, 4H), 7.68-7.64 (m, 2H), 7.34-7.31 (m, 1H), 4.37-4.34 (m, 2H), 4.24 (s, 3H), 3.79-3.72 (m, 1H), 3.56-3.49 (m, 2H), 3.01-2.95 (m, 1H), 2.38-2.32 (m, 1H), 2.08-2.03 (m, 1H), 1.85-1.76 (m, 2H) |
| 51 | 13.60 (s, 1H), 8.47 (s, 1H), 8.42 (d, J = 8 Hz, 1H), 7.91 (br s, 3H), 7.76 (t, J = 8 Hz, 1H), 7.25 (d, J = 8 Hz, 1H), 6.15 (s, 1H), 4.33 (d, J =16 Hz, 2H), 3.78-3.74 (m, 1H), 3.56-3.30 (m, 2H), 3.22 (s, 3H), 3.01-2.95 (m, 1H), 2.38 (s, 3H), 2.35-2.32 (m, 1H), 1.99-1.94 (m, 1H), 1.77-1.72 (m, 2H) |
| 52 | 13.33 (s, 1H), 8.77 (s, 1H), 8.68 (s, 1H), 8.62 (d, J = 8 Hz, 1H), 8.53 (s, 1H), 7.86-7.80 (m, 4H), 4.44 (d, J=16 Hz, 1H), 4.41-4.36 (m, 1H), 3.81-3.78 (m, 1H), 3.30-3.19 (m, 2H), 2.98-2.95 (m, 1H), 2.64 (s, 3H), 2.37-2.32 (m, 1H), 2.09 (s, 3H), 2.05-2.02 (m, 1H), 1.76-1.72 (m, 2H) |

### Example 53: 4-((6-(7-(aminomethyl)-7-(5-methylisoxazol-3-yl)-3-azabicyclo[4.1.0]heptan-3-yl)-1H-pyrazolo[3,4-b]pyrazin-3-yl)thio)-3-chloropyridin-2-amine

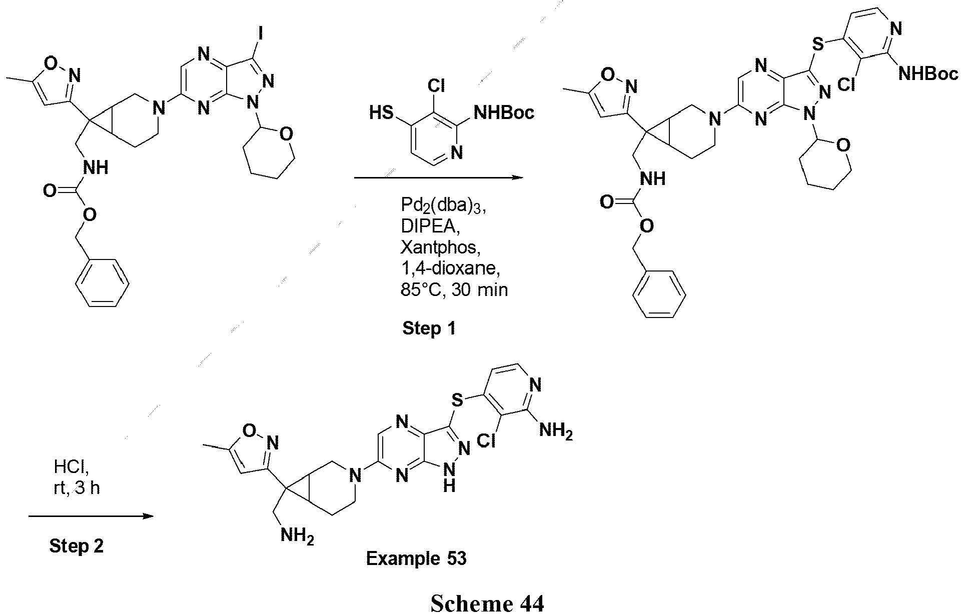

### Step 1: benzyl ((3-(3-((2-((tert-butoxycarbonyl)amino)-3-chloropyridin-4-yl)thio)-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate

A suspension of benzyl ((3-(3-iodo-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate (prepared with the procedure reported in **Scheme 43** step 1; 28.4 mg, 0.040 mmol, using **Intermediate 6** (12.2 mg, 0.050 mmol), Pd₂(dba)₃ (3.9 mg, 0.004 mmol), Xantphos (2.5 mg, 0.004 mmol) and DIPEA (11.0 mg, 0.085 mmol) in 1,4-dioxane (0.6 mL) was heated at 85°C for 1 h. After cooling, the solvent was concentrated *in vacuo* and EtOAc and H₂O were added. The organic phase was washed with brine, dried over Na₂SO₄ and concentrated *in vacuo* to get the title compound as a brown oil (34 mg, 100%) which was used as crude in the next step. LCMS (ES⁺) *m*/*z* 803 (M+H)⁺, RT 2.05 min.

### Step 2: 4-((6-(7-(aminomethyl)-7-(5-methylisoxazol-3-yl)-3-azabicyclo[4.1.0]heptan-3-yl)-1H-pyrazolo[3,4-b]pyrazin-3-yl)thio)-3-chloropyridin-2-amine

Benzyl ((3 -(3 -((2-((tert-butoxycarbonyl)amino)-3 -chloropyridin-4-yl)thio)-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate (34 mg, 0.040 mmol) was treated with conc. HCl (37%; 0.5 mL, 0.040 mmol) and the mixture was stirred at rt for 3 h. After solvent removal *in vacuo* the residue was purified by preparative HPLC (from 10% to 100% MeCN/H₂O + 0.1% TFA) to obtain the title compound as a white solid (TFA salt; 1.0 mg, 5%). ¹H NMR (400 MHz, DMSO-*d*₆) δ 13.90 (s, 1H), 8.41 (s, 1H), 7.86 (br s, 3H), 7.58 (d, *J=* 4.0 Hz, 1H), 6.51 (br s, 2H), 6.14 (s, 1H), 5.76 (s, 2H), 4.33-4.26 (m, 2H), 3.76-3.72 (m, 1H), 3.29-3.21 (m, 2H), 2.98-2.89 (m, 1H), 2.39 (s, 3H), 2.34-2.29 (m, 1H), 1.99-1.92 (m, 1H), 1.77-1.74 (m, 2H). LCMS (ES⁺) *m*/*z* 484 (M+H)⁺, RT 0.78 min

**Example 54** was synthesized using **Intermediate 14** (instead of tert-butyl (3-chloro-4-mercaptopyridin-2-yl)carbamate) and benzyl ((3-(3-iodo-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate with the same procedure reported in **Scheme 43. ¹H** NMR (400 MHz, DMSO-*d*₆) δ 7.79 (d, *J* = 8.0 Hz, 1H), 7.72 (d, *J* = 8.0 Hz, 1H), 7.65 (br s, 3H), 7.59-7.53 (m, 1H), 7.48-7.43 (m, 1H), 7.35-7.33 (m, 2H), 7.15-7.12 (m, 1H), 5.50-5.47 (m, 1H), 4.61 (d, *J* = 8.0 Hz, 1H), 4.01-3.95 (m, 2H), 3.66-3.57 (m, 2H), 2.70-2.68 (m, 1H), 2.47-2.45 (m, 2H), 2.33-2.28 (m, 1H), 1.82-1.80 (m, 2H), 1.77-1.74 (m, 1H).

### Example 55: (6-(7-(aminomethyl)-7-(3-fluorophenyl)-3-azabicyclo[4.1.0]heptan-3-yl)-3-(2,3-dichlorophenyl)-1H-pyrazolo[3,4-b]pyrazin-5-yl)methanol and Example 56: (6-(7-(aminomethyl)-7-(3-fluorophenyl)-3-azabicyclo[4.1.0]heptan-3-yl)-3-phenyl-1H-pyrazolo[3,4-b]pyrazin-5-yl)methanol

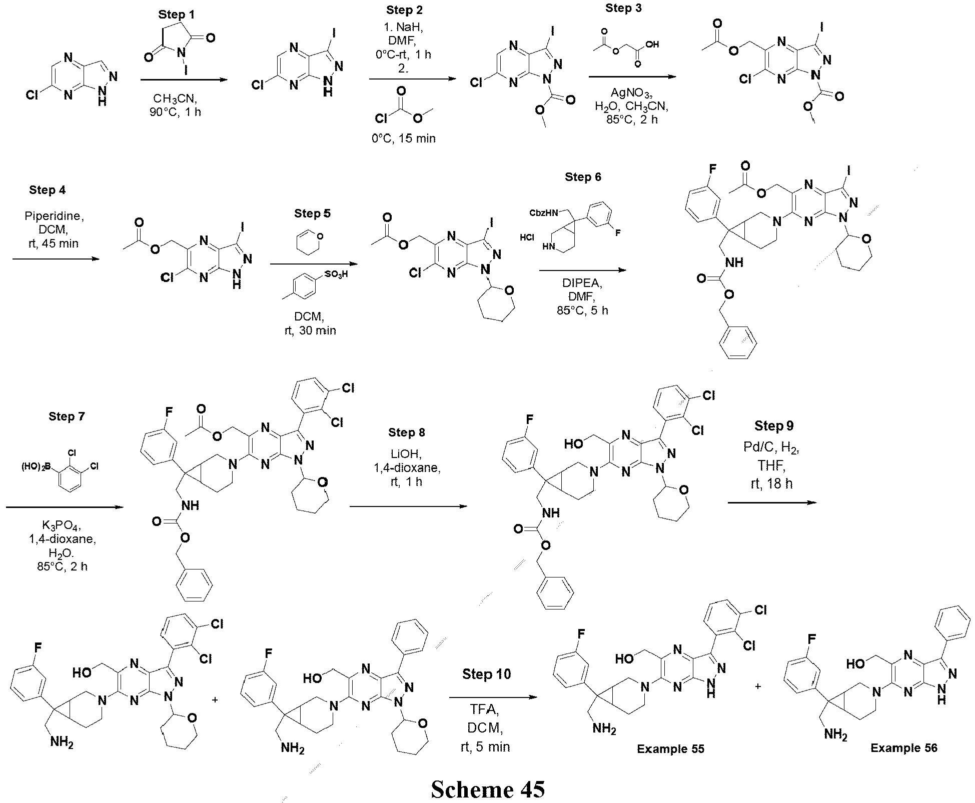

### Step 1: 6-chloro-3-iodo-1Hpyrazolo[3,4-b]pyrazine

A solution of 6-chloro-1H-pyrazolo[3,4-b]pyrazine (1.5 g, 9.71 mmol) in MeCN (32.0 mL) was treated with 1-iodopyrrolidine-2,5-dione (4.37 g, 19.41 mmol) and trifluoroborane hydrofluoride (3.8 mL, 29.12 mmol) and the resulting brown solution was stirred at 90°C for 1 h. After cooling to rt the mixture was placed in an ice bath for 30 min. The formed precipitate was collected by filtration and washed with cold MeCN to afford the title compound as a yellow solid (3.0 g, 97%). ¹H NMR (400 MHz, DMSO-*d*₆) δ 8.71 (s, 1H).

### Step 2: methyl 6-chloro-3-iodo-1H-pyrazolo[3,4-b]pyrazine-1-carboxylate

A solution of sodium hydride (60% dispersion in mineral oil; 941 mg, 23.53 mmol) in DMF (21 mL) was cooled to 0°C and treated with 6-chloro-3-iodo-1H-pyrazolo[3,4-b]pyrazine (3.0 g, 10.7 mmol). After stirring at rt for 1 h, the mixture was cooled to 0 °C and treated with methyl chloroformate (3.03 g, 32.09 mmol) and stirred at rt for 15 min. The solution was then poured in H₂O and the precipitate was collected by filtration to afford the title compound as a brown solid (2.49 g, 69%). ¹H NMR (400 MHz, DMSO-*d*₆) δ 8.93 (s, 1H), 4.07 (s, 3H). LCMS (ES⁺) *m*/*z* 338 (M+H)⁺, RT 1.40 min

### Step 3: methyl 5-(acetoxymethyl)-6-chloro-3-iodo-1H-pyrazolo[3,4-b]pyrazine-1-carboxylate

A solution of methyl 6-chloro-3-iodo-1H-pyrazolo[3,4-b]pyrazine-1-carboxylate (500 mg, 1.48 mmol), 2-acetyloxyethanoic acid (1.07 mL, 11.82 mmol) and silver nitrate (50.2 mg, 0.300 mmol) in MeCN (15 mL) and H₂O (9 mL) at 85°C was treated with ammonium persulfate (2.7 g, 11.82 mmol) and heated at 85°C for 2 h. After cooling to rt the mixture was poured into EtOAc and brine. The organic layer was separated and purified by flash chromatography (gradient elution 0-60% EtOAc in petroleum ether) to afford the title compound as a yellow solid (330 mg, 54%). LCMS (ES⁺) *m*/*z* 411 (M+H)⁺, RT 1.50 min

### Step 4: (6-chloro-3-iodo-1H-pyrazolo[3,4-b]pyrazin-5-yl)methyl acetate

A solution of methyl 5-(acetoxymethyl)-6-chloro-3-iodo-1H-pyrazolo[3,4-b]pyrazine-1-carboxylate (310 mg, 0.760 mmol) in DCM (5.6 mL) was treated with piperidine (0.09 mL, 0.910 mmol) and stirred at rt for 45 min. The solvent was concentrated *in vacuo* and the residue purified by flash chromatography (gradient elution 0-50% EtOAc in petroleum ether) to afford the title compound as a yellow solid (130 mg, 49%). LCMS (ES⁺) *m*/*z* 353 (M+H)⁺, RT 1.40 min

### Step 5: (6-chloro-3-iodo-1-(tetrahydro-2H-pyran-2-yl)-1H-pyrazolo[3,4-b]pyrazin-5-yl)methyl acetate

A solution of (6-chloro-3-iodo-1H-pyrazolo[3,4-b]pyrazin-5-yl)methyl acetate (130 mg, 0.370 mmol) in DCM (1.8 mL) was treated with 3,4-dihydro-2H-pyran (0.1 mL, 1.11 mmol), 4-methylbenzenesulfonic acid (19.0 mg, 0.110 mmol) and stirred at rt for 30 min. The solvent was concentrated *in vacuo* and the residue was purified by flash chromatography (gradient elution 0-50% EtOAc in petroleum ether) to afford the title compound as a pale yellow solid (52 mg, 32%). ¹H NMR (400 MHz, DMSO-*d*₆) δ 5.92 (dd, *J₁*= 4.2 Hz, *J₂*= 8.0 Hz, 1H), 5.42 (q, *J=* 12.0 Hz, 2H), 3.95-3.93 (m, 1H), 3.76-3.70 (m, 1H), 2.45-2.40 (m, 1H), 2.15 (s, 3H), 2.05-1.94 (m, 2H), 1.80-1.74 (m, 1H), 1.61-1.44 (m, 2H). LCMS (ES⁺) *m*/*z* 437 (M+H)⁺, RT 1.98 min

### Step 6: (6-(7-((((benzyloxy)carbonyl)amino)methyl)-7-(3-fluorophenyl)-3-azabicyclo[4.1.0]heptan-3-yl)-3-iodo-1-(tetrahydro-2H-pyran-2-yl)-1H-pyrazolo[3,4-b]pyrazin-5-yl)methyl acetate

A solution of (6-chloro-3-iodo-1-(tetrahydro-2H-pyran-2-yl)-1H-pyrazolo[3,4-b]pyrazin-5-yl)methyl acetate (29 mg, 0.070 mmol), benzyl ((7-(3-fluorophenyl)-3-azabicyclo[4.1.0]heptan-7-yl)methyl)carbamate hydrochloride (25.9 mg, 0.070 mmol) and DIPEA (71 uL, 0.400 mmol) in DMF (1.33 mL) was stirred at 85°C for 5 h. After cooling EtOAc (20 mL) was added and the mixture washed with 5% citric acid (1x3 mL), H₂O (1x3 mL), brine (1x3 mL), dried over Na₂SO₄ and concentrated *in vacuo* to afford the title compound as a brown oil (50 mg, 99%) which was used as crude in the next step. LCMS (ES⁺) *m*/*z* 755 (M+H)⁺, RT 2.51-2.53 min

### Step 7: (6-(7-((((benzyloxy)carbonyl)amino)methyl)-7-(3-fluorophenyl)-3-azabicyclo[4.1.0]heptan-3-yl)-3-(2,3-dichlorophenyl)-1-(tetrahydro-2H-pyran-2-yl)-1H-pyrazolo[3,4-b]pyrazin-5-yl)methyl acetate

A solution of (6-(7-((((benzyloxy)carbonyl)amino)methyl)-7-(3-fluorophenyl)-3-azabicyclo[4.1.0]heptan-3-yl)-3-iodo-1-(tetrahydro-2H-pyran-2-yl)-1H-pyrazolo[3,4-b]pyrazin-5-yl)methyl acetate (50 mg, 0.070 mmol), (2,3-dichlorophenyl)boronic acid (25.3 mg, 0.130 mmol), 1,1-bis(diphenylphosphino)ferrocenedichloropalladium (II) complex in DCM (10.9 mg, 0.010 mmol) and tripotassium phosphate (84.4 mg, 0.400 mmol) in a degassed mixture of (1,4-dioxane: H₂O = 0.8 mL: 0.08 mL) was heated in a pressure tube at 85°C for 2 h. After cooling, the mixture was diluted with EtOAc (25 mL), washed with brine (2x5 mL), dried over Na₂SO₄ and concentrated under reduced pressure to give a residue which was purified by flash chromatography (gradient elution 0-100% EtOAc in petroleum ether) to afford the title compound as a yellow solid (43 mg, 84%). LCMS (ES⁺) *m*/*z* 773 (M+H)⁺, RT 2.33-2.35 min

### Step 8: benzyl ((3-(3-(2, 3-dichlorophenyl)-5-(hydroxymethyl)-1-(tetrahydro-2H-pyran-2-yl)-1H-pyrazolo[3,4-b]pyrazin-6-yl)-7-(3-fluorophenyl)-3-azabicyclo[4.1.0]heptan-7-yl)methyl)carbamate

A solution of (6-(7-((((benzyloxy)carbonyl)amino)methyl)-7-(3-fluorophenyl)-3-azabicyclo[4.1.0]heptan-3-yl)-3-(2,3-dichlorophenyl)-1-(tetrahydro-2H-pyran-2-yl)-1H-pyrazolo[3,4-b]pyrazin-5-yl)methyl acetate (43 mg, 0.060 mmol) in 1,4-dioxane (0.32 mL) was treated with a solution of LiOH (9.3 mg, 0.220 mmol) in H₂O (0.32 mL). The mixture was stirred at rt for 1 h. EtOAc and brine were added to the mixture and then the organic phase was isolated, dried over Na₂SO₄ and concentrated *in vacuo* to afford the title compound as a yellow oil (40 mg, 98%) which was used as crude. LCMS (ES⁺) *m*/*z* 731 (M+H)⁺, RT 2.27-2.29 min

### Step 9: (6-(7-(aminomethyl)-7-(3-fluorophenyl)-3-azabicyclo[4.1.0]heptan-3-yl)-3-(2,3-dichlorophenyl)-1-(tetrahydro-2H-pyran-2-yl)-1H-pyrazolo[3,4-b]pyrazin-5yl)methanol and (6-(7-(aminomethyl)-7-(3-fluorophenyl)-3-azabicyclo[4.1.0]heptan-3-yl)-3-phenyl-1-(tetrahydro-2H-pyran-2-yl)-1H-pyrazolo[3,4-b]pyrazin-5-yl)methanol

A solution of benzyl ((3-(3-(2,3-dichlorophenyl)-5-(hydroxymethyl)-1-(tetrahydro-2H-pyran-2-yl)-1H-pyrazolo[3,4-b]pyrazin-6-yl)-7-(3-fluorophenyl)-3-azabicyclo[4.1.0]heptan-7-yl)methyl)carbamate (40 mg, 0.050 mmol) in THF (0.5 mL) was treated with Pd/C (10 mg) and the mixture was stirred under H₂ atmosphere for 18 h. After fluxing N₂, the catalyst was filtered on a pad of solka floc and washed with MeOH. After removal of solvent the title compounds were obtained in a mixture 1:1 and used as crude. LCMS (ES⁺) *m*/*z* 529 (M+H)⁺, RT 1.40-1.43 min, *m*/*z* 597 (M+H)⁺ RT 1.45-1.49 min

### Step 10: (6-(7-(aminomethyl)-7-(3-fluorophenyl)-3-azabicyclo[4.1.0]heptan-3-yl)-3-(2,3-dichlorophenyl)-1H-pyrazolo[3,4-b]pyrazin-5-yl)methanol and (6-(7-(aminomethyl)-7-(3-fluorophenyl)-3-azabicyclo[4.1.0]heptan-3-yl)-3-phenyl-1H-pyrazolo[3,4-b]pyrazin-5-yl)methanol

A solution of a mixture of (6-(7-(aminomethyl)-7-(3-fluorophenyl)-3-azabicyclo[4.1.0]heptan-3-yl)-3-(2,3-dichlorophenyl)-1-(tetrahydro-2H-pyran-2-yl)-1H-pyrazolo[3,4-b]pyrazin-5-yl)methanol and (6-(7-(aminomethyl)-7-(3-fluorophenyl)-3-azabicyclo[4.1.0]heptan-3-yl)-3-phenyl-1-(tetrahydro-2H-pyran-2-yl)-1H-pyrazolo[3,4-b]pyrazin-5-yl)methanol (34 mg, 0.06 mmol) in DCM (0.70 mL) was treated with TFA (0.27 mL) and the mixture was stirred at rt for 5 min. The solvent was concentrated under reduced pressure and the residue was purified by preparative HPLC (from 15% to 100% MeCN/H₂O + 0.1% TFA) to obtain the title compounds as yellow solids. **Example 55:** (TFA salt; 0.7 mg, 2%); ¹H NMR (400 MHz, DMSO-*d*₆) δ 7.79 (d, *J* = 8.0 Hz, 1H), 7.72 (d, *J=* 8.0 Hz, 1H), 7.65 (br s, 3H), 7.59-7.53 (m, 1H), 7.48-7.43 (m, 1H), 7.35-7.33 (m, 2H), 7.15-7.12 (m, 1H), 5.50-5.47 (m, 1H), 4.61 (d, *J* = 8.0 Hz, 1H), 4.01-3.95 (m, 2H), 3.66-3.57 (m, 2H), 2.70-2.68 (m, 1H), 2.47-2.45 (m, 2H), 2.33-2.28 (m, 1H),1.82-1.80 (m, 2H), 1.77-1.74 (m, 1H). LCMS (ES⁺) *m*/*z* 513 (M+H)⁺, RT 1.30 min. **Example 56:** (TFA salt; 0.88 mg, 3%); ¹H NMR (400 MHz, DMSO-*d*₆) δ 8.44 (d, *J* = 8.0 Hz, 2H), 7.69-7.65 (br s, 3H), 7.53-7.51 (m, 2H), 7.44-7.42 (m, 2H), 7.32-7.30 (m, 2H), 7.15-7.12 (m, 1H), 5.56-5.54 (m, 1H), 4.70 (d, *J* = 4.0 Hz, 2H), 4.60 (m, 1H), 4.01-3.89 (m, 2H), 3.64-3.60 (m, 2H), 2.70-2.67 (m, 1H), 2.33-2.30 (m, 1H), 2.10-1.99 (m, 1H), 1.82-1.80 (m, 1H), 1.65-1.63 (m, 1H). LCMS (ES⁺) *m*/*z* 445 (M+H)⁺, RT 1.19 min

### Example 57: (3-((2-amino-3-chloropyridin-4-yl)thio)-6-(7-(aminomethyl)-7-(5-methylisoxazol-3-yl)-3-azabicyclo[4.1.0]heptan-3-yl)-1H-pyrazolo[3,4-b]pyrazin-5-yl)methanol

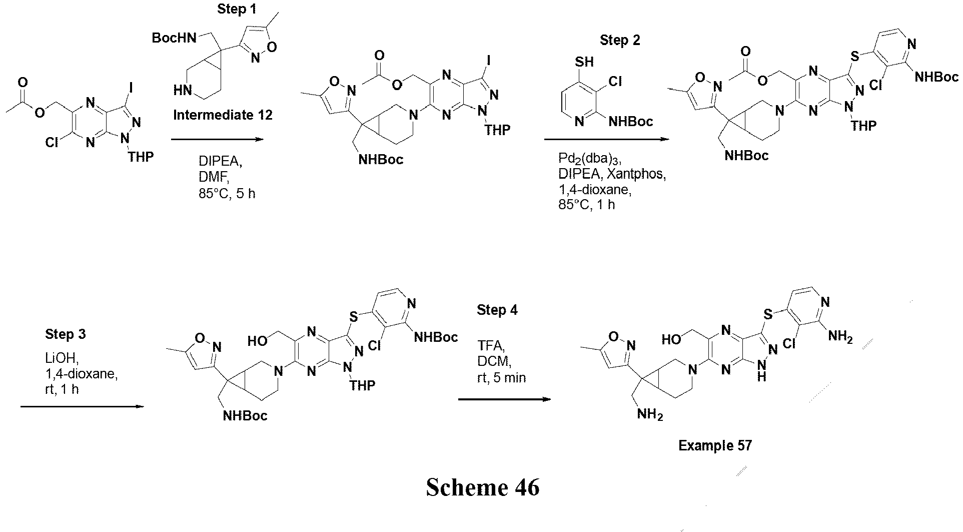

### Step 1: (6-(7-(((tert-butoxycarbonyl)amino)methyl)-7-(5-methylisoxazol-3-yl)-3-azabicyclo[4.1.0]heptan-3-yl)-3-iodo-1-(tetrahydro-2H-pyran-2-yl)-1H-pyrazolo[3,4-b]pyrazin-5-yl)methyl acetate

A solution of (6-chloro-3-iodo-1-(tetrahydro-2H-pyran-2-yl)-1H-pyrazolo[3,4-b]pyrazin-5-yl)methyl acetate (prepared as reported in steps 1-5 **Scheme 45**; 160 mg, 0.370 mmol), **Intermediate 12** (112.6 mg, 0.370 mmol) and DIPEA (391 uL, 2.2 mmol) in DMF (6.0 mL) was stirred at 85°C for 5 h. After cooling, EtOAc (20 mL) was added and mixture washed with 5% citric acid (1x3 mL), H₂O (1x3 mL), brine (1x3 mL), dried over Na₂SO₄ and concentrated *in vacuo* to get the title compound as a brown solid (250 mg, 96%) which was used as crude. LCMS (ES⁺) *m*/*z* 708 (M+H)⁺, RT 2.35 min

### Step 2: (3-((2-((tert-butoxycarbonyl)amino)-3-chloropyridin-4-yl)thio)-6-(7-(((tert-butoxycarbonyl)amino)methyl)-7-(5-methylisoxazol-3-yl)-3-azabicyclo[4.1.0]heptan-3-yl)-1-(tetrahydro-2H-pyran- 2-yl)-1H-pyrazolo[3,4-b]pyrazin-5-yl)methyl acetate

A solution of (6-(7-(((tert-butoxycarbonyl)amino)methyl)-7-(5-methylisoxazol-3-yl)-3-azabicyclo[4.1.0]heptan-3-yl)-3-iodo-1-(tetrahydro-2H-pyran-2-yl)-1H-pyrazolo[3,4-b]pyrazin-5-yl)methyl acetate (30 mg, 0.040 mmol), tert-butyl N-(3-chloranyl-4-sulfanyl-pyridin-2-yl)carbamate (12.2 mg, 0.050 mmol), Pd₂(dba)₃ (3.88 mg, 0.004 mmol), Xantphos (2.45 mg, 0.004 mmol) and DIPEA (10.9 mg, 0.080 mmol) in 1,4-dioxane (0.6 mL) was heated in a pressure tube at 85°C for 1 h. After cooling mixture was concentrated under reduced pressure to afford the title compound as a brown oil (35 mg, 98%) which was used as crude in the next step. LCMS (ES⁺) *m*/*z* 840-842 (M+H)⁺, RT 2.13 min

### Step 3: tert-butyl ((3-(3-((2-((tert-butoxycarbonyl)amino)-3-chloropyridin-4-yl)thio)-5-(hydroxymethyl)-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate

A solution of (3-((2-((tert-butoxycarbonyl)amino)-3-chloropyridin-4-yl)thio)-6-(7-(((tert-butoxycarbonyl)amino)methyl)-7-(5-methylisoxazol-3-yl)-3-azabicyclo[4.1.0]heptan-3-yl)-1-(tetrahydro-2H-pyran-2-yl)-1H-pyrazolo[3,4-b]pyrazin-5-yl)methyl acetate (35 mg, 0.040 mmol) in 1,4-dioxane (0.26 mL) was treated with a solution of LiOH (7.0 mg, 0.170 mmol) in H₂O (0.26 mL). The mixture was stirred at rt for 1 h. EtOAc and brine were added to the mixture and then the organic phase was isolated, dried over Na₂SO₄ and concentrated *in vacuo* to afford the title compound as a brown oil (33 mg, 99%) which was used as crude in the next step. LCMS (ES⁺) *m*/*z* 799 (M+H)⁺, RT 2.04 min

### Step 4: (3-((2-amino-3-chloropyridin-4-yl)thio)-6-(7-(aminomethyl)-7-(5-methylisoxazol-3-yl)-3-azabicyclo[4.1.0]heptan-3-yl)-1H-pyrazolo[3,4-b]pyrazin-5-yl)methanol

A solution of tert-butyl ((3-(3-((2-((tert-butoxycarbonyl)amino)-3-chloropyridin-4-yl)thio)-5-(hydroxymethyl)-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate (34 mg, 0.040 mmol) in DCM (0.70 mL) was treated with TFA (0.27 mL) and the mixture was stirred at rt for 5 min. The solvent was concentrated under reduced pressure and the residue was purified by preparative HPLC (from 10% to 100% MeCN/H₂O + 0.1% TFA) to obtain the title compound as a white solid (TFA salt; 1.2 mg, 5%). ¹H NMR (400 MHz, DMSO-*d*₆) δ 7.88 (br s, 3H), 7.59 (d, *J* = 4.0 Hz, 1H), 6.56 (br s, 2H), 6.14 (s, 1H), 5.72 (d, *J* = 8.0 Hz, 1H), 4.58 (s, 2H), 3.96-3.85 (m, 2H), 3.71-3.63 (m, 3H), 2.76-2.73 (m, 2H), 2.39 (s, 3H), 2.29-2.25 (m, 1H), 2.10-2.05 (m, 1H), 1.95-1.89 (m, 1H), 1.82-1.77 (m, 1H). LCMS (ES⁺) *m*/*z* 514 (M+H)⁺, RT 0.83 min.

### Example 58: (3-(2-amino-3-chloropyridin-4-yl)-6-(7-(aminomethyl)-7-(5-methylisoxazol-3-yl)-3-azabicyclo[4.1.0]heptan-3-yl)-1H-pyrazolo[3,4-b]pyrazin-5-yl)methanol

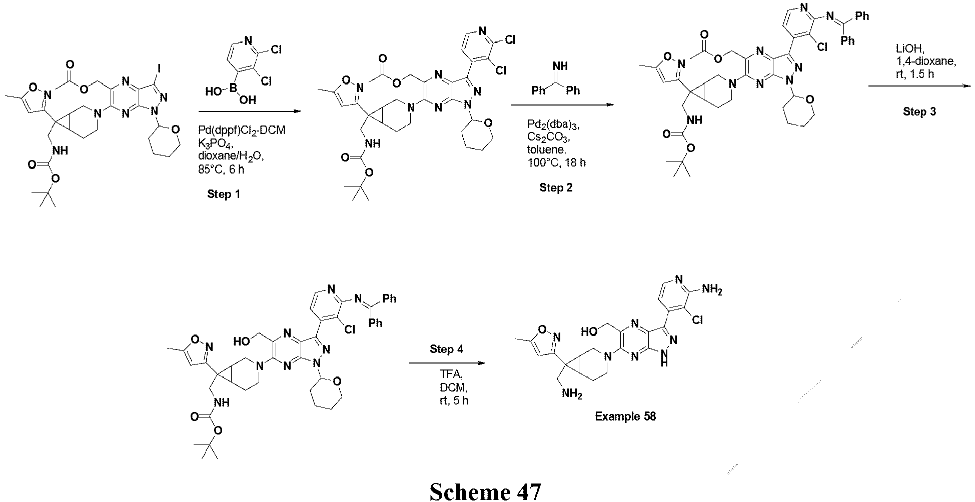

### Step 1: (6-(7-(((tert-butoxycarbonyl)amino)methyl)-7-(5-methylisoxazol-3-yl)-3-azabicyclo[4.1.0]heptan-3-yl)-3-(2,3-dichloropyridin-4-yl)-1-(tetrahydro-2H-pyran-2-yl)-1H-pyrazolo[3,4-b]pyrazin-5-yl)methyl acetate

(6-(7-(((tert-butoxycarbonyl)amino)methyl)-7-(5-methylisoxazol-3-yl)-3-azabicyclo[4.1.0]heptan-3-yl)-3-iodo-1-(tetrahydro-2H-pyran-2-yl)-1H-pyrazolo[3,4-b]pyrazin-5-yl)methyl acetate (prepared as described in **Scheme 46** step 1; 50 mg, 0.07 mmol), (2,3-dichloropyridin-4-yl)boronic acid (16 mg, 0.08 mmol), [1,1'-bis(diphenylphosphino)ferrocene]dichloropalladium(II) complex in DCM (11 mg, 0.01 mmol), K₃PO₄ (90 mg, 0.42 mmol) were dissolved in a mixture of dioxane (2.2 mL) and H₂O (0.4 mL). The mixture was degassed under N₂ and then heated at 85°C for 6 h. After cooling down, EtOAc and H₂O were added to the mixture and the organic phase was separated, washed with brine, dried over Na₂SO₄ and concentrated *in vacuo.* The residue was purified by flash chromatography (gradient elution 0-100% EtOAc in petroleum ether) to afford the title compound as an orange oil (45 mg, 87%). LCMS (ES⁺) *m*/*z* 727/729 (M+H)⁺, RT 2.55 min.

### Step 2: (6-(7-(((tert-butoxycarbonyl)amino)methyl)-7-(5-methylisoxazol-3-yl)-3-azabicyclo[4.1.0]heptan-3-yl)-3-(3-chloro-2-((diphenylmethylene)amino)pyridin-4-yl)-1-(tetrahydro-2H-pyran-2-yl)-1H-pyrazolo[3,4-b]pyrazin-5-yl)methyl acetate

A suspension of (6-(7-(((tert-butoxycarbonyl)amino)methyl)-7-(5-methylisoxazol-3-yl)-3-azabicyclo[4.1.0]heptan-3-yl)-3-(2,3-dichloropyridin-4-yl)-1-(tetrahydro-2H-pyran-2-yl)-1H-pyrazolo[3,4-b]pyrazin-5-yl)methyl acetate (45 mg, 0.060 mmol), [1-(2-diphenylphosphanyl-1-naphthyl)-2-naphthyl]-diphenyl-phosphane (7.7 mg, 0.012 mmol), diphenylmethanimine (16.8 mg, 0.093 mmol), Pd₂(dba)₃ (5.66 mg, 0.010 mmol), Cs₂CO₃ (60.3 mg, 0.190 mmol) in toluene (1.5 mL) was stirred at 100°C for 18 h. After cooling down the mixture, DCM and H₂O were added and the organic phase was separated, washed with brine, dried over Na₂SO₄ and concentrated *in vacuo* to afford the title compound as a brown oil (53 mg) which was used as crude. LCMS (ES⁺) *m*/*z* 872, 873 (M+H)⁺, RT 2.41 min.

### Step 3: tert-butyl ((3-(3-(3-chloro-2-((diphenylmethylene)amino)pyridin-4-yl)-5-(hydroxymethyl)-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate

A solution of (6-(7-(((tert-butoxycarbonyl)amino)methyl)-7-(5-methylisoxazol-3-yl)-3-azabicyclo[4.1.0]heptan-3-yl)-3-(3-chloro-2-((diphenylmethylene)amino)pyridin-4-yl)-1-(tetrahydro-2H-pyran-2-yl)-1H-pyrazolo[3,4-b]pyrazin-5-yl)methyl acetate (53 mg, 0.06 mmol) in 1,4-dioxane (0.76 mL) was treated with a solution of LiOH (10 mg, 0.24 mmol) in H₂O (0.76 mL). The mixture was stirred at rt for 1.5 h. EtOAc and brine were added to the mixture and then the organic phase was isolated, dried over Na₂SO₄ and concentrated *in vacuo* to afford the title compound as a brown oil (50 mg) which was used as crude in the next step. LCMS (ES⁺) *m*/*z* 831 (M+H)⁺, RT 2.32 min.

### Step 4: (3-(2-amino-3-chloropyridin-4-yl)-6-(7-(aminomethyl)-7-(5-methylisoxazol-3-yl)-3-azabicyclo[4.1.0]heptan-3-yl)-1H-pyrazolo[3,4-b]pyrazin-5-yl)methanol

A solution of tert-butyl ((3-(3-(3-chloro-2-((diphenylmethylene)amino)pyridin-4-yl)-5-(hydroxymethyl)-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate (50 mg, 0.060 mmol) in DCM (1.0 mL) was treated with TFA (0.39 mL) and the mixture was stirred at rt for 5 h. The solvent was concentrated under reduced pressure and the residue was purified by flash chromatography (gradient elution 0-60% EtOAc in petroleum ether) to afford a residue which was dissolved in HCl in MeOH (1.25 M; 1.0 mL) and the mixture was stirred at rt for 18 h. After removal of the solvent under reduced pressure the residue was purified by preparative HPLC (from 10% to 100% MeCN/H₂O + 0.1% TFA) to obtain the title compound as a yellow solid (TFA salt; 0.61 mg, 2%). ¹H NMR (400 MHz, DMSO-*d*₆) δ 8.02 (d, *J* = 8.0 Hz, 1H), 7.89-7.87 (br s, 3H), 7.07 (d, *J* = 8.0 Hz, 1H), 6.54 (br s, 2H), 6.14 (s, 1H), 4.62 (s, 2H), 3.94-3.88 (m, 2H), 3.70-3.66 (m, 3H), 2.75-2.71 (m, 2H), 2.40 (s, 3H), 2.33-2.26 (m, 1H), 2.10-2.05 (m, 1H), 1.95-1.89 (m, 1H), 1.82-1.77 (m, 1H). LCMS (ES⁺) *m*/*z* 482 (M+H)⁺, RT 0.72 min.

### Example 59: 4-((6-(7-(aminomethyl)-7-(2-fluorophenyl)-3-azabicyclo[4.1.0]heptan-3-yl)-5H-pyrrolo[2,3-b]pyrazin-2-yl)thio)-3-chloropyridin-2-amine

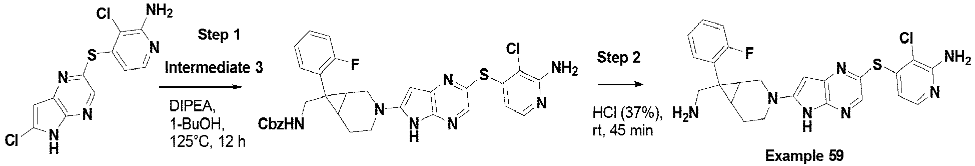

### Step 1: benzyl ((3-(2-((2-amino-3-chloropyridin-4-yl)thio)-5H-pyrrolo[2,3-b]pyrazin-6-yl)-7-(2-fluorophenyl)-3-azabicyclo[4.1.0]heptan-7-yl)methyl)carbamate

A solution of **Intermediate 48** (11.5 mg, 0.040 mmol), **Intermediate 3** (15.8 mg, 0.040 mmol) and DIPEA (0.03 mL, 0.150 mmol) in 1-Butanol (0.25 mL) was stirred at 125 °C for 12 h. After cooling the solvent was concentrated under reduced pressure, the residue was dissolved in EtOAc (1 mL), the organic phase was washed with H₂O, brine, dried over Na₂SO₄ and concentrated *in vacuo* to afford the title compound which was used as crude. LCMS (ES⁺) *m*/*z* 630 (M+H)⁺, RT 1.57 min.

### Step 2: 4-((6-(7-(αminomethyl)-7-(2-fluorophenyl)-3-azabicyclo[4.1.0]heptan-3-yl)-5H-pyrrolo[2,3-b]pyrazin-2-yl)thio)-3-chloropyridin-2-amine

Benzyl ((3-(2-((2-amino-3-chloropyridin-4-yl)thio)-5H-pyrrolo[2,3-b]pyrazin-6-yl)-7-(2-fluorophenyl)-3-azabicyclo[4.1.0]heptan-7-yl)methyl)carbamate (35 mg, 0.060 mmol) was treated with conc. HCl (37%; 0.8 mL) and the mixture was stirred at rt for 45 min. After removal of the solvent *in vacuo* the residue was purified by preparative HPLC (from 10% to 100% MeCN/H₂O + 0.1% TFA) to obtain the title compound as a yellow solid (TFA salt; 0.5 mg, 2%). LCMS (ES⁺) *m*/*z* 496 (M+H)⁺, RT 0.92 min.

### Example 60: (3-(3-(3,4-dihydro-1,5-naphthyridin-1(2H)-yl)-1H-pyrazolo[3,4-b]pyrazin-6-yl)-7-(5-methylisoxazol-3-yl)-3-azabicyclo[4.1.0]heptan-7-yl)methanamine

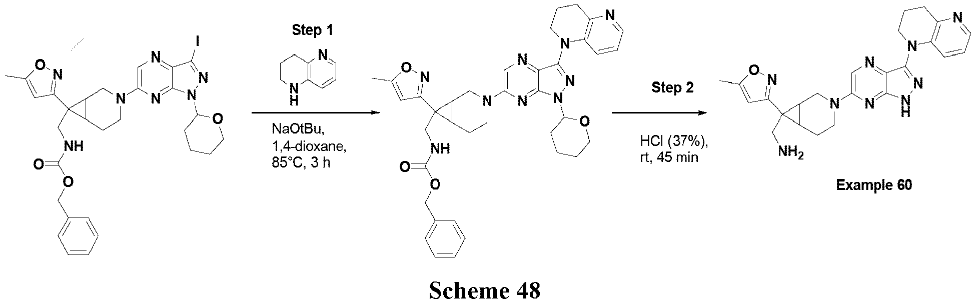

### Step 1: benzyl ((3-(3-(3,4-dihydro-1,5-naphthyridin-1(2H)-yl)-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate

A solution of benzyl ((3-(3-iodo-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate (prepared with the procedure reported in **Scheme 43**, step 1; 28.4 mg, 0.040 mmol) using **Intermediate 6** (23 mg, 0.030 mmol), 1,2,3,4-tetrahydro-1,5-naphthyridine (4.61 mg, 0.030 mmol), sodium 2-methylpropan-2-olate (9.9 mg, 0.100 mmol), dicyclohexyl(2',6'-diisopropoxybiphenyl-2-yl)phosphine (1.6 mg, 0003 mmol) and [2'-(methylamino)-2-biphenylyl]palladium methanesulfonate dicyclohexyl(2',6'-diisopropoxy-2-biphenylyl)phosphine (2.9 mg, 0.003 mmol) in degassed 1,4-dioxane (1 mL) was heated in a sealed vial at 85°C for 3 h. After cooling, the mixture was diluted with EtOAc (35 mL), washed with brine (2x5 mL), dried over Na₂SO₄ and concentrated *in vacuo* to afford the title compound as a yellow solid (21 mg, 90%) which was used as crude. LCMS (ES⁺) *m*/*z* 677 (M+H)⁺, RT 1.60 min.

### Step 2: (3-(3-(3,4-dihydro-1,5-naphthyridin-1(2H)-yl)-1H-pyrazolo[3,4-b]pyrazin-6yl)-7-(5-methylisoxazol-3-yl)-3-azabicyclo[4.1.0]heptan-7-yl)methanamine

Benzyl ((3-(3-(3,4-dihydro-1,5-naphthyridin-1(2H)-yl)-1-(tetrahydro-2H-pyran-2-yl)-1H-pyrazolo[3,4-b]pyrazin-6-yl)-7-(5-methylisoxazol-3-yl)-3-azabicyclo[4.1.0]heptan-7-yl)methyl)carbamate (20 mg, 0.030 mmol) was treated with conc. HCl (37%; 0.6 mL) and the mixture was stirred at rt for 16 h. After removal of the solvent *in vacuo* the residue was purified by preparative HPLC (from 10% to 100% MeCN/H₂O + 0.1% TFA) to obtain the title compound as a yellow solid (TFA salt; 1.0 mg, 7%). LCMS (ES⁺) *m*/*z* 458 (M+H)⁺, RT 0.74 min

### Example 61: (3-(7-(aminomethyl)-3-(3-(4-chloro-2-methyl-2H-indazol-5-yl)-1H-pyrazolo[3,4-b]pyrazin-6-yl)-3-azabicyclo[4.1.0]heptan-7-yl)isoxazol-5-yl)methanol

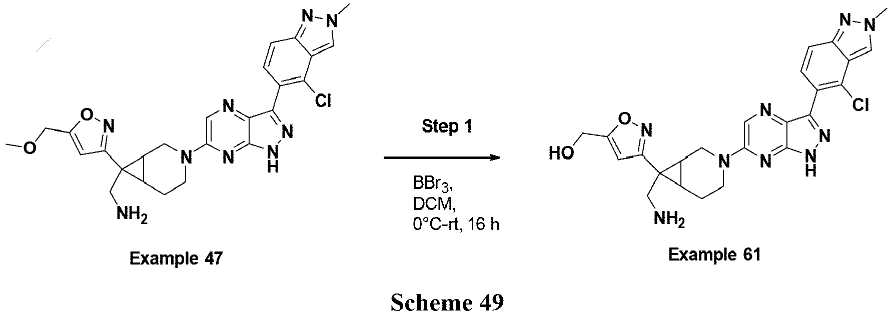

### Step 1: (3-(7-(aminomethyl)-3-(3-(4-chloro-2-methyl-2H-indazol-5-yl)-1H-pyrazolo[3,4-b]pyrazin-6-yl)-3-azabicyclo[4.1.0]heptan-7-yl)isoxazol-5-yl)methanol

A solution of (3-(3-(4-chloro-2-methyl-2H-indazol-5-yl)-1H-pyrazolo[3,4-b]pyrazin-6-yl)-7-(5-(methoxymethyl)isoxazol-3-yl)-3-azabicyclo[4.1.0]heptan-7-yl)methanamine (**Example 47**; 13 mg, 0.030 mmol) in DCM (1 mL) cooled at 0°C was treated dropwise with boron tribromide (1M in hexanes, 75 uL, 0.08 mmol). The mixture was left warming to rt and stirred at this temperature for 16 h. MeOH (1 mL) was added to the mixture and solvent removal *in vacuo* the residue was purified by preparative HPLC (from 10% to 100% MeCN/H₂O + 0.1% TFA) to afford the title compound as a white solid (TFA salt; 0.4 mg, 3%). LCMS (ES⁺) *m*/*z* 506 (M+H)⁺, RT 0.85 min.

### Example 62: 6-(7-(aminomethyl)-7-(5-methylisoxazol-3-yl)-3-azabicyclo[4.1.0]heptan-3-yl)-3-(4-chloro-2-methyl-2H-indazol-5-yl)-5-methyl-1,5-dihydro-4H-pyrazolo[3,4-d]pyrimidin-4-one

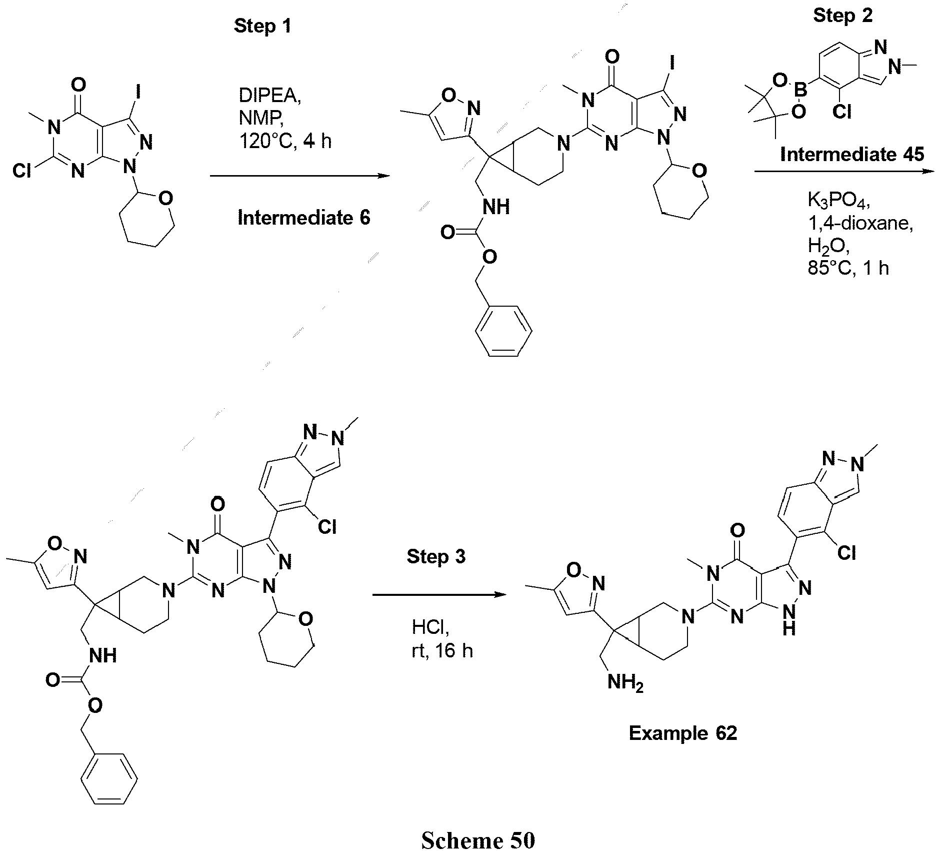

### Step 1: benzyl ((3-(3-iodo-5-methyl-4-oxo-1-(tetrahydro-2H-pyran-2-yl)-4,5-dihydro-1H-pyrazolo[3,4-d]pyrimidin-6-yl)-7-(5-methylisoxazol-3-yl)-3-azabicyclo[4.1.0]heptan-7-yl)methyl)carbamate

A solution of **Intermediate 47** (100 mg, 0.250 mmol), **Intermediate 6** (95.8 mg, 0.250 mmol), DIPEA (0.22 mL, 1.27 mmol) in NMP (2 mL) was stirred at 120°C for 4 h. After cooling EtOAc (40 mL) was added and the mixture washed with H₂O, brine, dried over Na₂SO₄ and concentrated *in vacuo* to get a residue which was purified by flash chromatography (gradient elution 0-100% EtOAc in petroleum ether) to afford the title compound as a colourless oil (>100% for residual NMP). LCMS (ES⁺) *m*/*z* 700 (M+H)⁺, RT 1.93 min.

### Step 2: benzyl ((3-(3-(4-chloro-2-methyl-2H-indazol-5-yl)-5-methyl-4-oxo-1-(tetrahydro-2H-pyran-2-yl)-4,5-dihydro-1H-pyrazolo[3,4-d]pyrimidin-6-yl)-7-(5-methylisoxazol-3-yl)-3-azabicyclo[4.1.0]heptan-7-yl)methyl)carbamate

A solution of benzyl ((3-(3-iodo-5-methyl-4-oxo-1-(tetrahydro-2H-pyran-2-yl)-4,5-dihydro-1H-pyrazolo[3,4-d]pyrimidin-6-yl)-7-(5-methylisoxazol-3-yl)-3-azabicyclo[4.1,0]heptan-7-yl)methyl)carbamate (77 mg, 0.060 mmol), **Intermediate 45** (20.9 mg, 0.070 mmol), [1,1'-bis(diphenylphosphino)ferrocene]dichloropalladium(II) complex in DCM (9.1 mg, 0.010 mmol) and tripotassium phosphate (70.1 mg, 0.330 mmol) in a mixture of degassed 1,4-dioxane (1 mL) and H₂O (0.1 mL) was heated at 85 °C for 1 h. After cooling, the mixture was diluted with EtOAc (35 mL), washed with brine, dried over Na₂SO₄ and concentrated *in vacuo* to afford a residue which was purified by flash chromatography (gradient elution 0-100% EtOAc in petroleum ether) to afford the title compound as a yellow oil (25 mg, 62%). LCMS (ES⁺) *m*/*z* 738 (M+H)⁺, RT 1.92 min.

### Step 3: 6-(7-(aminomethyl)-7-(5-methylisoxazol-3-yl)-3-azabicyclo[4.1.0]heptan-3-yl)-3-(4-chloro-2-methyl-2H-indazol-5-yl)-5-methyl-1,5-dihydro-4H-pyrazolo[3,4-d]pyrimidin-4-one

Benzyl ((3-(3-(4-chloro-2-methyl-2H-indazol-5-yl)-5-methyl-4-oxo-1-(tetrahydro-2H-pyran-2-yl)-4,5-dihydro-1H-pyrazolo[3,4-d]pyrimidin-6-yl)-7-(5-methylisoxazol-3-yl)-3-azabicyclo[4.1.0]heptan-7-yl)methyl)carbamate (22 mg, 0.030 mmol) was treated with conc. HCl (37%; 0.6 mL, 0.030 mmol) and the mixture was stirred at rt for 16 h. After volatiles removal *in vacuo* the residue was purified by preparative HPLC (from 10% to 100% MeCN/H₂O + 0.1% TFA) to obtain the title compound as a white solid (TFA salt; 5 mg, 32%). ¹H NMR (400 MHz, DMSO-*d*₆) δ 13.55 (br s, 1H), 8.53 (s, 1H), 7.96 (br s, 3H), 7.61 (d, *J* = 8.0 Hz, 1H), 7.31 (d, *J* = 8.0 Hz, 1H), 6.12 (s, 1H), 4.23 (s, 3H), 3.71-3.65 (m, 5H), 3.34 (s, 3H), 2.67-2.58 (m, 1H), 2.39 (s, 3H), 2.33-2.26 (m, 1H), 1.99-1.95 (m, 1H), 1.89-1.87 (m, 1H), 1.77-1.74 (m, 1H). LCMS (ES⁺) *m*/*z* 520 (M+H)⁺, RT 0.87 min.

### Example 63: (7-Phenyl-3-(5-((2-(trifluoromethyl)pyridin-3-yl)thio)-1H-imidazo[4,5-b]pyrazin-2-yl)-3-azabicyclo[4.1.0]heptan-7-yl)methanamine

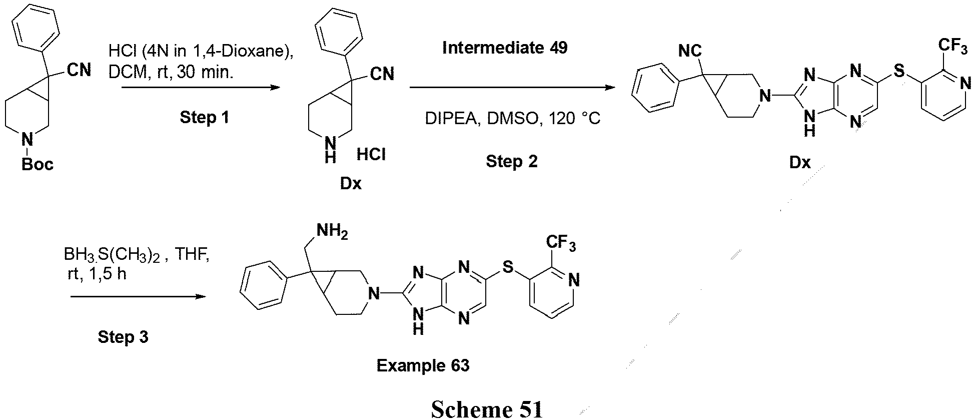

### Step 1: 7-Phenyl-3-azabicyclo[4.1.0]heptane-7-carbonitrile

*tert-*butyl 7-cyano-7-phenyl-3-azabicyclo[4.1.0]heptane-3-carboxylate (prepared as described in **Scheme 28**, step 1; 50 mg, 0.17 mmol) was dissolved in DCM (1 mL) and treated with 1 mL of 4N HCl in 1,4-dioxane. The solution was stirred at rt for 1.5 h, then solvent was removed *in vacuo* to to give the title compound (44 mg; 99%). LCMS (ES⁺) Method 1: *m*/*z* 199 (M+H)⁺, RT 1.04 min.

### Step 2: 7-Phenyl-3-(5-((2-(trifluoromethyl)pyridin-3-yl)thio)-1H-imidazo[4,5-b]pyrazin-2-yl)-3-azabicyclo[4.1.0]heptane-7-carbonitrile

To a solution of **Intermediate 49** (56 mg, 0.17 mmol) and 7-phenyl-3-azabicyclo[4.1.0]heptane-7-carbonitrile (39 mg, 0.17 mmol) in DMSO (1.3 mL), dry DIPEA (0.12 mL, 0.67 mmol) was added. The reaction mixture was stirred at 120 °C for 2 h. The reaction was diluted with EtOAc (x2) and washed with H₂O (x2), 5% citric acid solution (x2), NaHCO₃ sat. sol. (x2). Organic layer was dried over Na₂SO₄ filtered and concentrated *in vacuo.* The resulting crude was purified by flash chromatography on silica gel (from 80% to 100% EtOAc/petroleum ether) to obtain the title compound as light-yellow solid (60 mg, 72%). LCMS (ES⁺) Method 1: *m*/*z* 494 (M+H)⁺, RT 1.94 min.

### Step 3: (7-Phenyl-3-(5-((2-(trijluoromethyl)pyridin-3-yl)thio)-1H-imidazo[4,5-b]pyrazin-2-yl)-3-azabicyclo[4.7.0]heptan-7-yl)methanamine

7-Phenyl-3-(5-((2-(trifluoromethyl)pyridin-3-yl)thio)-1H-imidazo[4,5-b]pyrazin-2-yl)-3-azabicyclo[4.1.0]heptane-7-carbonitrile (46 mg, 0.09 mmol) was dissolved in dry THF (0.5 mL) and borane methylsulfanylmethane (2M in THF; 0.16 mL, 0.33 mmol) was added at rt under N₂ atmosphere. The reaction mixture was stirred at RT for 1 h and 40 min then more borane methylsulfanylmethane (2M in THF; 0.2 mL, 0.40 mmol) was added and the resulting solution was stirred at rt for 12 h. The reaction was quenched by addition of MeOH (2 mL) and concentrated *in vacuo.* The residue was dissolved in THF (0.5 mL), 6N HCl aqueous solution (2 mL) was added and the mixture was stirred at rt for 3.5 h. Volatiles were removed *in vacuo* and the residue was purified by preparative HPLC (from 10% to 60% MeCN/H₂O + 0.1% TFA) to obtain the title compound as a light-yellow powder (TFA salt; 9.5 mg, 16%). ¹H NMR (DMSO-*d₆*+TFA) δ 8.61 (br d, *J*=4.6 Hz, 1H), 8.24 (s, 1H), 7.84 (d, *J*=8.1 Hz, 1H), 7.75-7.57 (m, 4H), 7.45 (m, 2H), 7.37 (m, 2H), 7.29 (m, 1H), 4.22 (br d, *J*=14.4 Hz, 1H), 4.02-3.84 (m, 2H), 3.23 (m, 1H), 3.18-2.99 (m, 2H), 2.34 (m, 1H), 1.87-1.63 (m, 2H), 1.52 (m, 1H); LCMS (ES⁺) Method 2: *m*/*z* 498 (M+H)⁺, RT 2.38 min.

### Example 64: (7-(Thiophen-3-yl)-3-(5-((2-(trifluoromethyl)pyridin-3-yl)thio)-1H-imidazo [4,5-b]pyrazin-2-yl)-3-azabicyclo [4.1.0] heptan-7-yl)methanamine

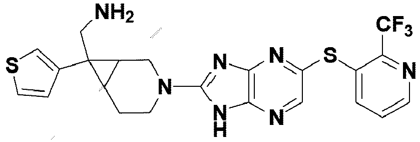

### Step 1: Benzyl ((7-(thiophen-3-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate

To a solution of **Intermediate 49** (4.5 mg, 0.01 mmol) and **Intermediate 31** (4.3 mg, 0.01mmol) in DMSO (0.2 mL), dry DIPEA (0.01mL, 0.06 mmol) was added. The reaction mixture was stirred at 120 °C for 3 h. The reaction was directly purified by preparative HPLC (from 20% to 75% MeCN/H₂O + 0.1% HCOOH) to obtain the title compound as a beige solid (5 mg, 69%). LCMS (ES⁺) Method 1: *m*/*z* 638 (M+H)⁺, RT 2.13 min.

### Step 2: (7-(Thiophen-3-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine

Benzyl ((7-(thiophen-3-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate (5 mg, 0.01 mmol) was dissolved in conc. HCl (37%; 0.5 mL) and the resulting yellow solution was stirred at rt for 12 h.

The reaction was directly purified by preparative HPLC (5% to 45% MeCN/H₂O + 0.1% TFA) to obtain the title compound as an off-white solid (TFA salt; 4 mg, 81%). ¹H NMR (DMSO-*d₆*+TFA) δ 8.61 (br d, *J*=4.6 Hz, 1H), 8.25 (s, 1H), 7.84 (d, *J*=8.2 Hz, 1H), 7.75 (br s, 3H), 7.62 (m, 1H), 7.54 (m, 1H), 7.41 (br s, 1H), 7.13 (d, *J*=4.8 Hz, 1H), 4.11 (d, *J*=14.2 Hz, 1H), 3.99-3.84 (m, 2H), 3.32 (m, 1H), 3.20-2.99 (m, 2H), 2.31 (m, 1H), 1.86 (m, 1H), 1.68 (m, 1H), 1.54 (m, 1H); LCMS (ES⁺) Method 2: *m*/*z* 504 (M+H)⁺, RT 2.34 min.

### Example 65: (7-(Pyridin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine

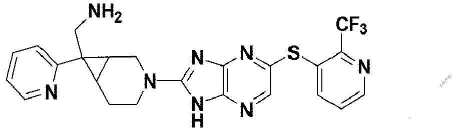

### Step 1: Benzyl ((7-(pyridin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate

Benzyl ((7-(pyridin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate was prepared according to the procedure described in step 1 of the synthesis of **Example 64** starting from **Intermediate 29** (16.4 mg, 0.04 mmol). The reaction was purified by preparative HPLC (from 5% to 50% MeCN/H₂O + 0.1% TFA) to obtain the title compound as a beige solid (16 mg, 63%). ¹H NMR (DMSO-*d₆*+TFA) δ 8.83 (br d, *J*=5.6 Hz, 1H), 8.60 (m, 1H), 8.43 (dt, *J*=7.9 and 1.5 Hz, 1H), 8.27 (s, 1H), 8.01-7.80 (m, 3H), 7.60 (m, 1H), 7.42 (m, 1H), 7.37-7.24 (m, 3H), 7.15 (m, 2H), 4.80 (s, 2H), 4.30 (d, *J*=4.3 Hz, 1H), 4.05-3.83 (m, 2H), 3.60 (m, 1H), 3.36 (m, 1H), 3.15 (m, 1H), 2.28 (m, 1H), 2.07-1.90 (m, 2H), 1.82 (m, 1H); LCMS (ES⁺) Method 1: *m*/*z* 633 (M+H)⁺, RT 1.58 min.

### Step 2: (7-(Pyridin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine

(7-(Pyridin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine was prepared according to the procedure described in step 2 of the synthesis of **Example 64** starting from benzyl ((7-(pyridin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate (15 mg, 0.02 mmol). The reaction was purified by preparative HPLC (from 5% to 45% MeCN/H₂O + 0.1% TFA) to obtain the title compound as a light-yellow solid (TFA salt; 10 mg, 69%). ¹H NMR (DMSO-*d₆*+TFA) δ 8.74 (br d, *J*=5.3 Hz, 1H), 8.60 (m, 1H), 8.26-8.15 (m, 2H), 7.96-7.76 (m, 4H), 7.70-7.56 (m, 3H), 4.29 (d, *J*=14.7 Hz, 1H), 4.00 (m, 1H), 3.88 (m, 1H), 3.45 (m, 1H), 3.28-3.04 (m, 2H), 2.33 (m, 1H), 2.12 (m, 1H), 1.92-1.68 (m, 2H); LCMS (ES⁺) Method 2. *m*/*z* 499 (M+H)⁺, RT 2.05 min.

### Example 66: (7-(Pyrimidin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine

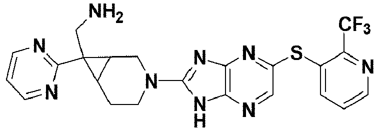

### Step 1: Benzyl ((7-(pyrimidin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate

Benzyl ((7-(pyrimidin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate was prepared according to the procedure described in step 1 of the synthesis of **Example 64** starting from **Intermediate 30** (15 mg, 0.04 mmol). The reaction was purified by preparative HPLC (from 20% to 65% MeCN/H₂O + 0.1% TFA) to obtain the title compound as a yellow solid (22 mg, 87%). ¹H NMR (DMSO-*d₆*+TFA) δ 8.70 (d, *J*=4.9 Hz, 2H), 8.61 (m, 1H), 8.33 (s, 1H), 7.90 (d, *J*=8.2 Hz, 1H), 7.61 (m, 1H), 7.38-7.21 (m, 6H), 7.05 (m, 1H), 4.84 (m, 2H), 4.15-3.89 (m, 3H), 3.70 (m, 2H), 3.23 (m, 1H), 2.28 (m, 1H), 2.14 (m, 1H), 2.00 (m, 1H), 1.92-1.74 (m, 1H); LCMS (ES⁺) Method 1 *m*/*z* 634 (M+H)⁺, RT 2.00 min.

### Step 2: (7-(Pyrimidin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine

(7-(Pyrimidin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine was prepared according to the procedure described in step 2 of the synthesis **Example 64** starting from benzyl ((7-(pyrimidin-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate (21 mg, 0.03 mmol). The reaction was purified by reverse phase chromatography on C18 cartridge (from 5% to 40% MeCN/H₂O + 0.1% TFA) to obtain the title compound as a light-yellow solid (TFA salt; 15 mg, 74%). ¹H NMR (DMSO-*d₆*+TFA) δ 8.76 (d, *J*=4.9 Hz, 2H), 8.60 (m, 1H), 8.22 (s, 1H), 7.90 (br s, 3H), 7.81 (m, 1H), 7.62 (m, 1H), 7.39 (t, *J*=4.9 Hz, 1H), 4.22 (d, *J*=4.5 Hz, 1H), 4.04 (m, 1H), 3.91 (m, 1H), 3.55 (m, 1H), 3.31-3.08 (m, 2H), 2.40-2.20 (m, 1H), 2.04 (m, 1H), 1.78 (m, 1H); LCMS (ES⁺) Method 2: *m*/*z* 500 (M+H)⁺, RT 2.43 min.

### Example 67: (7-(4-Methylthiazol-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine

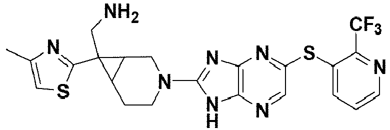

### Step 1: Benzyl ((7-(4-methylthiazol-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate

Benzyl ((7-(4-methylthiazol-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate was prepared according to the procedure described in step 1 of the synthesis of **Example 64** starting from **Intermediate 32** (18 mg, 0.05 mmol). The reaction was purified by preparative HPLC (from 20% to 65% MeCN /H₂O + 0.1% TFA) to obtain the title compound as a beige solid (19 mg, 63%). ¹H NMR (DMSO-*d₆*+TFA) δ 8.61 (br d, *J*=4.6 Hz, 1H), 8.30 (s, 1H), 7.88 (d, *J*=8.1 Hz, 1H), 7.61 (m, 1H), 7.45 (m, 1H), 7.38-7.22 (m, 5H), 7.13 (s, 1H), 4.92 (s, 2H), 4.10 (d, *J*=14.3 Hz, 1H), 4.02-3.82 (m, 2H), 3.68 (m, 1H), 3.45 (m, 1H), 3.16 (m, 1H), 2.30 (m, 4H), 2.07 (m, 1H), 1.94 (m, 1H), 1.81 (m, 1H); LCMS (ES⁺) Method 1: *m*/*z* 653 (M+H)⁺, RT 1.98 min.

### Step 2: (7-(4-Methylthiazol-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine

(7-(4-Methylthiazol-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methanamine was prepared according to the procedure described in step 2 of the synthesis of **Example 64** starting from benzyl ((7-(4-methylthiazol-2-yl)-3-(5-((2-(trifluoromethyl)pyridin-3-yl)thio)-1H-imidazo[4,5-b]pyrazin-2-yl)-3-azabicyclo[4.1.0]heptan-7-yl)methyl)carbamate (17 mg, 0.03 mmol). The reaction was purified by preparative HPLC (from 5% to 50% MeCN/H₂O + 0.1% TFA) to obtain the title compound as a light-yellow solid (TFA salt; 11 mg, 68%). ¹H NMR (DMSO-*d₆*+TFA) δ 8.61 (m, 1H), 8.23 (s, 1H), 7.95 (br s, 3H), 7.83 (m, 1H), 7.62 (m, 1H), 7.18 (br d, *J*=1.0 Hz, 1H), 4.18 (d, *J*=14.6 Hz, 1H), 4.05-3.83 (m, 2H), 3.49 (m, 1H), 3.32-3.03 (m, 2H), 2.36 (m, 4H), 2.02 (m, 1H), 1.88-1.67 (m, 2H); LCMS (ES⁺) Method 2: *m*/*z* 519 (M+H)⁺, RT 2.33 min.

### Example 68: (3-(5-((2-(Trifluoromethyl)pyridin-3-yl)thio)-1H-imidazo[4,5-b]pyrazin-2-yl)-3-azabicyclo[4.1.0]heptan-7-yl)methanamine

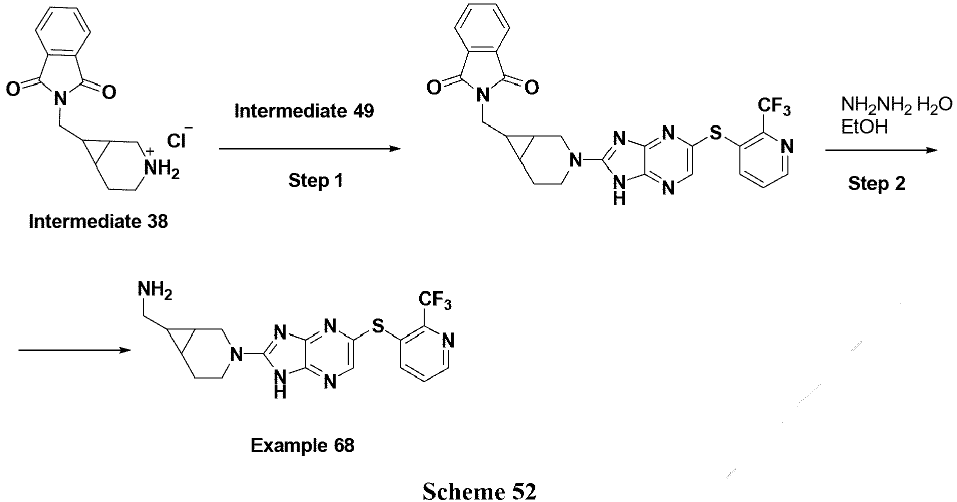

### Step 1: 2-((3-(5-((2-(Trifluoromethyl)pyridin-3-yl)thio)-1H-imidazo[4,5-b]pyrazin-2-yl)-3-azabicyclo[4.1.0]heptan-7-yl)methyl)isoindoline-1,3-dione

2-((3-(5-((2-(Trifluoromethyl)pyridin-3-yl)thio)-1H-imidazo[4,5-b]pyrazin-2-yl)-3-azabicyclo[4.1.0]heptan-7-yl)methyl)isoindoline-1,3-dione was prepared according to the procedure in step 1 of the synthesis of **Example 64** starting from **Intermediate 38** (21 mg, 0.07 mmol). The reaction was purified by preparative HPLC (from 0% to 45% MeCN/H₂O + 0.1% TFA) to afford the title compound as a colorless oil (27 mg, 81%). LCMS (ES⁺) Method 1: *m*/*z* 552 (M+H)⁺, RT 1.90 min.

### Step 2: (3-(5-((2-(Trifluoromethyl)pyridin-3-yl)thio)-1H-imidazo[4,5-b]pyrazin-2-yl)-3-azabicyclo[4.1.0]heptan-7-yl)methanamine

To a solution 2-((3-(5-((2-(trifluoromethyl)pyridin-3-yl)thio)-1H-imidazo[4,5-b]pyrazin-2-yl)-3-azabicyclo[4.1.0]heptan-7-yl)methyl)isoindoline-1,3-dione (27 mg, 0.05 mmol) in EtOH (2 mL) hydrazine monohydrate (27 uL, 0.54 mmol) was added. The resulting mixture was stirred at rt for 3 h. Volatiles were removed under reduced pressure and the crude was purified by preparative HPLC (from 5% to 45% MeCN/H₂O + 0.1% TFA) to afford pure desired compound (TFA salt; 20 mg, 96%). ¹H NMR (300 MHz, DMSO-*d*₆+TFA) δ 8.61 - 8.65 (m, 1H), 8.30 (s, 1H), 7.89 (d, *J*=8.0 Hz, 1H), 7.74 (br s, 3H), 7.63 (dd, *J*=8.1, 4.5 Hz, 1H), 4.03-3.93 (m, 1H), 3.90-3.81 (m, 1H), 3.57-3.34 (m, 2H), 2.80-2.71 (m, 2 H), 2.20-2.08 (m, 1H), 1.85-1.73 (m, 1H), 1.31-1.15 (m, 2H), 1.04-0.89 (m, 1H); LCMS (ES⁺) Method 2: *m*/*z* 422 (M+H)⁺, RT 2.06 min.

### Example 69: 4-((2-(7-(aminomethyl)-7-phenyl-3-azabicyclo[4.1.0]heptan-3-yl)-1H-imidazo[4,5-b]pyrazin-5-yl)thio)-3-chloropyridin-2-amine

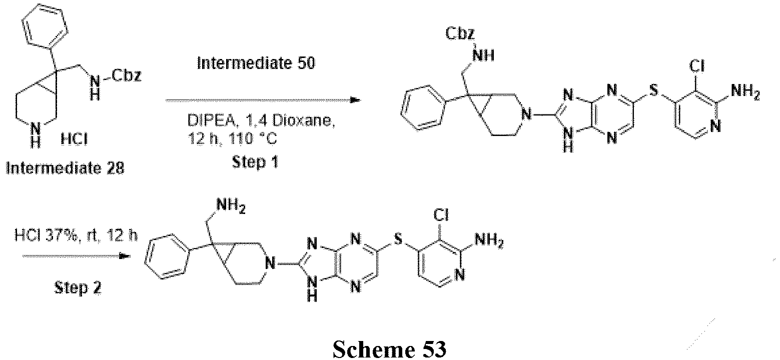

### Step 1: benzyl ((3-(5-((2-amino-3-chloropyridin-4-yl)thio)-1H-imidazo[4,5-b]pyrazin-2-yl)-7-phenyl-3-azabicyclo[4.1.0]heptan-7-yl)methyl)carbamate

A mixture of **Intermediate 28** (20 mg, 0.06 mmol), **Intermediate 50** (20 mg, 0.06 mmol) and DIPEA (50 uL, 0.3 mmol) in 1,4-dioxane was stirred in a sealed tube at 120 °C for 24 h. The reaction was purified by preparative HPLC (from 10% to 65% MeCN/H₂O + 0.1% TFA) affording the desired compound as a colorless oil. LCMS (ES⁺) Method 1: *m*/*z* 613, 615 (M+H)⁺, RT 1.66 min.

### Step 2: 4-((2-(7-(aminomethyl)-7-phenyl-3-azabicyclo[4.1.0]heptan-3-yl)-1H-imidazo[4,5-b]pyrazin-5-yl)thio)-3-chloropyridin-2-amine

The compound was prepared according to the procedure described in step 2 of the synthesis of **Example 64** starting from benzyl ((7-phenyl-3-azabicyclo[4.1.0]heptan-7-yl)methyl)carbamate. The reaction was purified by preparative HPLC (from 0% to 45% MeCN/H₂O + 0.1% TFA) to obtain the title compound as a light-yellow solid (TFA salt; 2 mg, 7% over two steps). ¹H NMR (300 MHz, DMSO-*d*₆+TFA) δ 8.32 (s, 1H), 7.76-7.66 (m, 4H), 7.49-7.44 (m, 2H), 7.37 (t, *J*=7.4 Hz, 2H), 7.32-7.25 (m, 1H), 6.31-6.18 (m, 1H), 4.39-4.21 (m, 1H), 4.06-3.89 (m, 2H), 3.31-3.04 (m, 3H), 2.43-2.32 (m, 1H), 1.92-1.68 (m, 2H), 1.6-1.48 (m, 1H); LCMS (ES⁺) Method 2: *m*/*z* 479, 481 (M+H)⁺, RT 1.74 min.

### Example 70: 4-((2-(7-(aminomethyl)-7-(4-methylthiazol-2-yl)-3-azabicyclo[4.1.0]heptan-3-yl)-1H-imidazo[4,5-b]pyrazin-5-yl)thio)-3-chloropyridin-2-amine

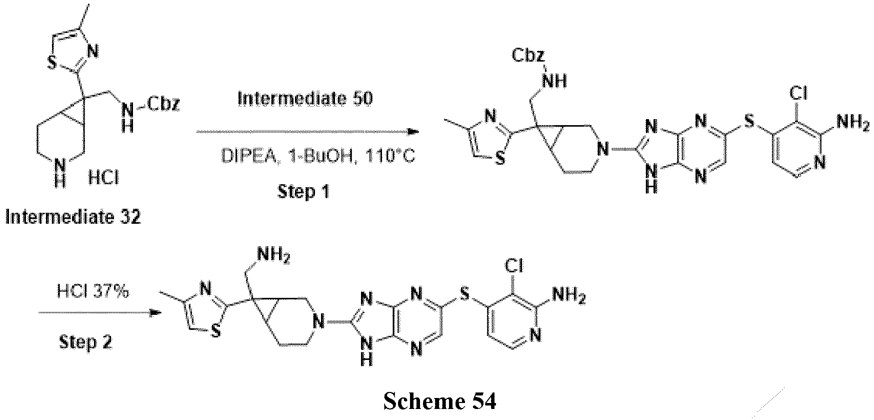

### Step 1: benzyl ((3-(5-((2-amino-3-chloropyridin-4-yl)thio)-1H-imidazo[4,5-b]pyrazin-2-yl)-7-(4-methylthiazol-2-yl)-3-azabicyclo[4.1.0]heptan-7-yl)methyl)carbamate

To a solution of **Intermediate 50** (21 mg, 0.06 mmol) and **Intermediate 32** (25 mg, 0.06 mmol) in 1-butanol (0.7 mL), dry DIPEA (0.05 mL, 0.29 mmol) was added. The reaction mixture was stirred at 110 °C for 23 h, then concentrated *in vacuo.* The resulting crude was purified by reverse phase chromatography on C18 cartridge (from 5% to 45% MeCN/H₂O + 0.1% TFA) to obtain the title compound as a yellow solid (13 mg, 35%). LCMS (ES⁺) Method 1: *m*/*z* 634 (M+H)⁺, RT 1.48 min.

### Step 2: 4-((2-(7-(aminomethyl)-7-(4-methylthiazol-2-yl)-3-azabicyclo[4.1.0]heptan-3-yl)-1H-imidazo[4,5-b]pyrazin-5-yl)thio)-3-chloropyridin-2-amine

The compound was prepared according to the procedure described in step 2 of the synthesis of **Example 64** starting from benzyl ((3-(5-((2-amino-3-chloropyridin-4-yl)thio)-1H-imidazo[4,5-b]pyrazin-2-yl)-7-(4-methylthiazol-2-yl)-3-azabicyclo[4.1.0]heptan-7-yl)methyl)carbamate (13 mg, 0.02 mmol). The reaction was purified by reverse phase chromatography on C18 cartridge (from 0% to 30% MeCN/H₂O + 0.1% TFA) to obtain the title compound as an off-white solid (TFA salt; 6 mg, 49%). ¹H NMR (DMSO-*d₆*+TFA) δ 8.30 (s, 1H), 8.00 (br s, 3H) 7.73 (d, *J*=6.9 Hz, 1H), 7.19 (br d, *J*=1.0 Hz, 1H), 6.23 (d, *J*=6.9 Hz, 1H), 4.28 (d, *J*=14.6 Hz, 1H), 4.05 (m, 1H), 3.94 (m, 1H), 3.50 (m, 1H), 3.33-3.07 (m, 2H), 2.37 (m, 4H), 2.03 (m, 1H), 1.89-1.72 (m, 2H); LCMS (ES⁺) Method 2: *m*/*z* 500 (M+H)⁺, RT 1.66 min.

### Example 71: 6-(7-(Aminomethyl)-7-phenyl-3-azabicyclo[4.1.0]heptan-3-yl)-3-((2-(trifluoromethyl)pyridin-3-yl)thio)pyrazin-2-amine

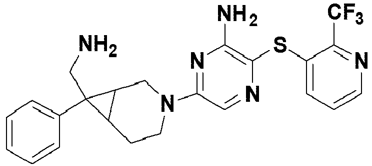

### Step 1: Benzyl ((3-(6-amino-5-((2-(trifluoromethyl)pyridin-3-yl)thio)pyrazin-2-yl)-7-phenyl-3-azabicyclo[4.1.0]heptan-7-yl)methyl)carbamate

Benzyl ((3-(6-amino-5-((2-(trifluoromethyl)pyridin-3-yl)thio)pyrazin-2-yl)-7-phenyl-3-azabicyclo[4.1.0]heptan-7-yl)methyl)carbamate was prepared by reacting **Intermediate 28** (32 mg, 0.09 mmol) and **Intermediate 41** (20 mg, 0.06 mmol) under the same conditions described in the synthesis of **Example 64**, step 1. The reaction was purified by preparative HPLC (from 15% to 60% MeCN/H₂O + 0.1% HCOOH) to obtain the title compound as a brown solid (20 mg, 50%). ¹H NMR (300 MHz, DMSO-*d*₆) δ 8.45 (d, *J*=4.3 Hz, 1H), 7.62-7.56 (m, 1H), 7.55-7.47 (m, 1H), 7.41-7.01 (m, 13H), 6.20 (br s, 1H), 4.87 (s, 2H), 4.31-4.24 (m, 2H), 3.70-3.62 (m, 1H), 3.46-3.28 (m, 2H), 2.83-2.74 (m, 1H), 2.25-2.10 (m, 1H), 1.87-1.70 (m, 1H), 1.69- 1.58 (m, 1H), 1.43-1.33 (m, 1H); LCMS (ES⁺) Method 1: m/z 607 (M+H)⁺, RT 2.47 min.

### Step 2: 6-(7-(Aminomethyl)-7-phenyl-3-azabicyclo[4.1.0]heptan-3-yl)-3-((2-(trifluoromethyl)pyridin-3-yl)thio)pyrazin-2-amine

6-(7-(Aminomethyl)-7-phenyl-3-azabicyclo[4.1.0]heptan-3-yl)-3-((2-(trifluoromethyl)pyridin-3-yl)thio)pyrazin-2-amine was prepared from benzyl ((3-(6-amino-5-((2-(trifluoromethyl)pyridin-3-yl)thio)pyrazin-2-yl)-7-phenyl-3-azabicyclo[4.1.0]heptan-7-yl)methyl)carbamate (20 mg, 0.03 mmol) according to the procedure described in the synthesis of **Example 64**, step 2. The reaction was purified by preparative HPLC (from 5% to 45% MeCN/H₂O + 0.1% TFA) to obtain the title compound as a brown solid (TFA salt; 7.36 mg, 47%). ¹H NMR (300 MHz, DMSO-*d*₆ + TFA) δ 8.47 (br d, *J*=4.4 Hz, 1H) 7.70-7.59 (m, 4H), 7.57-7.42 (m, 3H), 7.41-7.25 (m, 4H), 4.34-4.19 (m, 2H), 3.63 (br dd, *J*=14.2, 6.3 Hz, 1H), 3.31-3.20 (m, 1H), 3.17-3.06 (m, 1H), 2.66-2.84 (m, 1H), 2.36-2.18 (m, 1H), 1.85-1.61 (m, 2H), 1.52-1.42 (m, 1H); LCMS (ES⁺) Method 2: m/z 473 (M+H)⁺, RT 2.60 min.

### Example 72: 6-(7-(Aminomethyl)-7-(pyridin-2-yl)-3-azabicyclo[4.1.0]heptan-3-yl)-3-((2-(trifluoromethyl)pyridin-3-yl)thio)pyrazin-2-amine

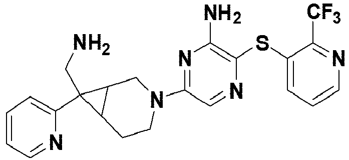

### Step 1: Benzyl ((3-(6-amino-5-((2-(trifluoromethyl)pyridin-3-yl)thio)pyrazin-2-yl)-7-(pyridin-2-yl)-3-azabicyclo[4.1.0]heptan-7-yl)methyl)carbamate

Benzyl ((3-(6-amino-5-((2-(trifluoromethyl)pyridin-3-yl)thio)pyrazin-2-yl)-7-(pyridin-2-yl)-3-azabicyclo[4.1.0]heptan-7-yl)methyl)carbamate was prepared by reacting **Intermediate 29** (10 mg, 0.024 mmol) and **Intermediate 41** (10 mg, 0.03 mmol) under the same conditions described in the synthesis of **Example 64**, step 1. The reaction was purified by preparative HPLC (from 15% to 60% MeCN/H₂O + 0.1% TFA) to obtain the title compound as a brown solid (6 mg, 41%). Method 1: *m*/*z* 608 (M+H)⁺, RT 1.66 min.

### Step 2: 6-(7-(Aminomethyl)-7-(pyridin-2-yl)-3-azabicyclo[4.1.0]heptan-3yl)-3-((2-(trifluoromethyl)pyridin-3-yl)thio)pyrazin-2-amine

6-(7-(Aminomethyl)-7-(pyridin-2-yl)-3-azabicyclo[4.1.0]heptan-3-yl)-3-((2-(trifluoromethyl)pyridin-3-yl)thio)pyrazin-2-amine was prepared from benzyl ((3-(6-amino-5-((2-(trifluoromethyl)pyridin-3-yl)thio)pyrazin-2-yl)-7-(pyridin-2-yl)-3-azabicyclo [4.1.0]heptan-7-yl)methyl)carbamate (6 mg, 0.01 mmol) according to the procedure described in the synthesis of **Example 64**, step 2. The reaction was purified by preparative HPLC (from 5% to 45% MeCN/H₂O + 0.1% TFA) to afford the title compound as a brown solid (TFA salt; 4 mg, 69%). ¹H NMR (DMSO-*d₆*+TFA) δ 8.74 (br d, *J*=5.1 Hz, 1H), 8.48 (br d, *J*=4.7 Hz, 1H), 8.18 (m, 1H), 7.88 (br s, 3H), 7.70-7.59 (m, 3H), 7.55 (m, 1H), 7.33 (d, *J*=8.2 Hz, 1H), 4.37-4.20 (m, 2H), 3.61 (m, 1H), 3.47 (m, 1H), 3.26 (m, 1H), 2.81 (m, 1H), 2.29 (m, 1H), 2.09 (m, 1H), 1.86-1.63 (m, 2H); LCMS (ES⁺) Method 2 *m*/*z* 474 (M+H)⁺, RT 2.12 min.

### Example 73: 6-(7-(Aminomethyl)-7-(4-methylthiazol-2-yl)-3-azabicyclo[4.1.0]heptan-3-yl)-3-((2-(trifluoromethyl)pyridin-3-yl)thio)pyrazin-2-amine

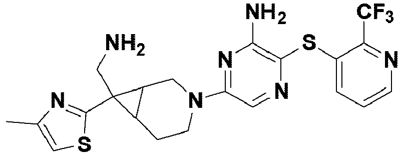

### Step 1: Benzyl ((3-(6-amino-5-((2-(trifluoromethyl)pyridin-3-yl)thio)pyrazin-2-yl)-7-(4-methylthiazol-2-yl)-3-azabicyclo[4.1.0]heptan-7-yl)methyl)carbamate

Benzyl ((3-(6-amino-5-((2-(trifluoromethyl)pyridin-3-yl)thio)pyrazin-2-yl)-7-(4-methylthiazol-2-yl)-3-azabicyclo[4.1.0]heptan-7-yl)methyl)carbamate was prepared by reacting **Intermediate 32** (13 mg, 0.03 mmol) and **Intermediate 41** (12 mg, 0.04 mmol) under the same conditions described in the synthesis of **Example 64**, step 1. The reaction was purified by preparative HPLC (from 20% to 70% MeCN/H₂O + 0.1% TFA) to obtain the title compound as a brown solid (8 mg, 39%). LCMS (ES⁺) Method 1: *m*/*z* 628 (M+H)⁺, RT 2.20 min.

### Step 2: 6-(7-(Aminomethyl)-7-(4-methylthiazol-2-yl)-3-azabicyclo[4.1.0]heptan-3-yl)-3-((2-(trifluoromethyl)pyridin-3-yl)thio)pyrazin-2-amine

6-(7-(Aminomethyl)-7-(4-methylthiazol-2-yl)-3-azabicyclo[4.1.0]heptan-3-yl)-3-((2-(trifluoromethyl)pyridin-3-yl)thio)pyrazin-2-amine was prepared from benzyl ((3-(6-amino-5-((2-(trifluoromethyl)pyridin-3-yl)thio)pyrazin-2-yl)-7-(4-methylthiazol-2-yl)-3-azabicyclo[4.1.0]heptan-7-yl)methyl)carbamate (8 mg, 0.013 mmol) according to the procedure described in the synthesis of **Example 64**, step 2. The reaction was purified by preparative HPLC (from 5% to 55% MeCN/H₂O + 0.1% TFA) to afford the title compound as an off-white solid (TFA salt; 4 mg, 62%). ¹H NMR (DMSO-*d₆*+TFA) δ 8.48 (m, 1H), 7.95 (br s, 3H), 7.61 (s, 1H), 7.54 (m, 1H), 7.33 (d, *J*=8.2 Hz, 1H), 7.17 (br d, *J*=1.0 Hz, 1H), 4.34-4.16 (m, 2H), 3.64 (m, 1H), 3.49 (m, 1H), 3.27 (m, 1H), 2.80 (m, 1H), 2.36 (s, 3H), 2.28 (m, 1H), 2.00 (m, 1H), 1.82-1.61 (m, 2H); LCMS (ES⁺) *m*/*z* 494 (M+H)⁺, RT 2.53 min.

### Example 74: 3-((2-Amino-3-chloropyridin-4-yl)thio)-6-(7-(aminomethyl)-7-(4-methylthiazol-2-yl)-3-azabicyclo[4.1.0]heptan-3-yl)pyrazin-2-amine

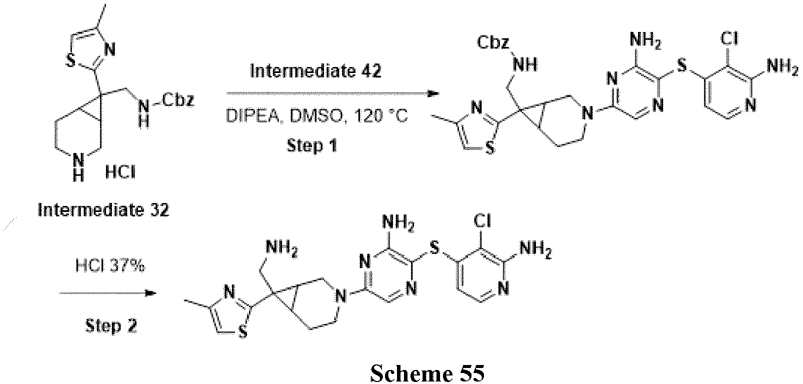

### Step 1: benzyl ((3-(6-amino-5-((2-amino-3-chloropyridin-4-yl)thio)pyrazin-2-yl)-7-(4-methylthiazol-2-yl)-3-azabicyclo[4.1.0]heptan-7-yl)methyl)carbamate

benzyl ((3-(6-amino-5-((2-amino-3-chloropyridin-4-yl)thio)pyrazin-2-yl)-7-(4-methylthiazol-2-yl)-3-azabicyclo[4.1.0]heptan-7-yl)methyl)carbamate was prepared by reacting **Intermediate 32** (13 mg, 0.03 mmol) and **Intermediate 42** (12 mg, 0.43 mmol), heating under MW irradiation. The reaction was purified by preparative HPLC (from 10% to 65% MeCN/H₂O + 0.1% TFA) to obtain the title compound as a brown solid (5 mg, 25%). LCMS (ES⁺) Method 1: *m*/*z* 609 (M+H)⁺, RT 1.61 min.

### Step 2: 3-((2-Amino-3-chloropyridin-4-yl)thio)-6-(7-(aminomethyl)-7-(4-methylthiazol-2-yl)-3-azabicyclo[4.1.0]heptan-3-yl)pyrazin-2-amine

The compound was prepared from benzyl ((3-(6-amino-5-((2-amino-3-chloropyridin-4-yl)thio)pyrazin-2-yl)-7-(4-methylthiazol-2-yl)-3-azabicyclo[4.1.0]heptan-7-yl)methyl)carbamate (5 mg, 0.008 mmol) according to the procedure described in the synthesis of **Example 64**, step 2. The reaction was purified by preparative HPLC (from 0% to 35% MeCN/H₂O + 0.1% TFA) to afford the title compound as an off-white solid (TFA salt; 2 mg, 49%). ¹H NMR (DMSO-*d₆*+TFA) δ 8.30 (s, 1H) 7.96 (br s, 3H), 7.75 (d, *J*=6.9 Hz, 1H), 7.64 (s, 1H), 7.17 (br d, *J*=1.0 Hz, 1H), 6.09 (d, *J*=6.9 Hz, 1H), 4.33-4.15 (m, 2H), 3.68 (m, 1H), 3.51 (m, 1H), 3.23 (m, 1H), 2.83 (m, 1H), 2.36 (s, 3H), 2.29 (m, 1H), 2.00 (m, 1H), 1.81-1.65 (m, 2H); LCMS (ES⁺) *m*/*z* Method 2: 475 (M+H)⁺, RT 1.78 min.

### Example 75: 6-(7-(Aminomethyl)-3-azabicyclo[4.1.0]heptan-3-yl)-3-((2-(trifluoromethyl)pyridin-3-yl)thio)pyrazin-2-amine

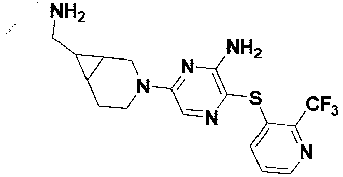

### Step 1: 2-((3-(6Amino-5-((2-(trifluoromethyl)pyridin-3-yl)thio)pyrazin-2-yl)-3-azabicyclo[4.1.0]heptan-7-yl)methyl)isoindoline-1,3-dione

2-((3-(6-Amino-5-((2-(trifluoromethyl)pyridin-3-yl)thio)pyrazin-2-yl)-3-azabicyclo[4.1.0]heptan-7-yl)methyl)isoindoline-1,3-dione was prepared by reacting **Intermediate 38** (20 mg, 0.07 mmol) and **Intermediate 41** (20 mg, 0.08 mmol) under the same conditions described in the synthesis of **Example 64**, step 1. The reaction was purified by preparative HPLC (from 20% to 75% MeCN/H₂O + 0.1% TFA) to afford the title compound as yellow solid (20 mg, 55%). LCMS (ES⁺) Method 1: m/z 527 (M+H)⁺, RT 2.28 min.

### Step 2: 6-(7-(Aminomethyl)-3-azabicyclo[4.1.0]heptan-3-yl)-3-((2-(trifluoromethyl)pyridin-3-yl)thio)pyrazin-2-amine

To a solution of 2-((3-(6-amino-5-((2-(trifluoromethyl)pyridin-3-yl)thio)pyrazin-2-yl)-3-azabicyclo[4.1.0]heptan-7-yl)methyl)isoindoline-1,3-dione (20 mg, 0.04 mmol) in EtOH (2 mL) hydrazine monohydrate (27 uL, 0.54 mmol) was added and the resulting mixture was stirred at rt for 3 h. The reaction was purified by preparative HPLC (from 5% to 20% MeCN/H₂O + 0.1% TFA) to obtain the title compound as an off-white solid (TFA salt; 12 mg, 81%). ¹H NMR (300 MHz, DMSO-*d*₆ + TFA) δ 8.47-8.43 (m, 1H), 7.79-7.63 (m, 3H), 7.56-7.49 (m, 2H), 7.32 (d, *J*=7.1 Hz, 1H), 4.02 (d, *J*=13.3 Hz, 1H), 3.76-3.66 (m, 1H), 3.62-3.50 (m, 1H), 3.29-3.16 (m, 1H), 2.83-2.65 (m, 2H), 2.09-1.96 (m, 1H), 1.84-1.70 (m, 1H), 1.20-1.11 (m, 2H), 0.91-0.79 (m, 1H); LCMS (ES⁺) Method 2: m/z 397 (M+H)⁺, RT 2.20 min.

### Example 76: 4-((5-(7-(aminomethyl)-7-(4-methylthiazol-2-yl)-3-azabicyclo[4.1.0]heptan-3-yl)imidazo[1,2-c]pyrimidin-8-yl)thio)-3-chloropyridin-2-amine

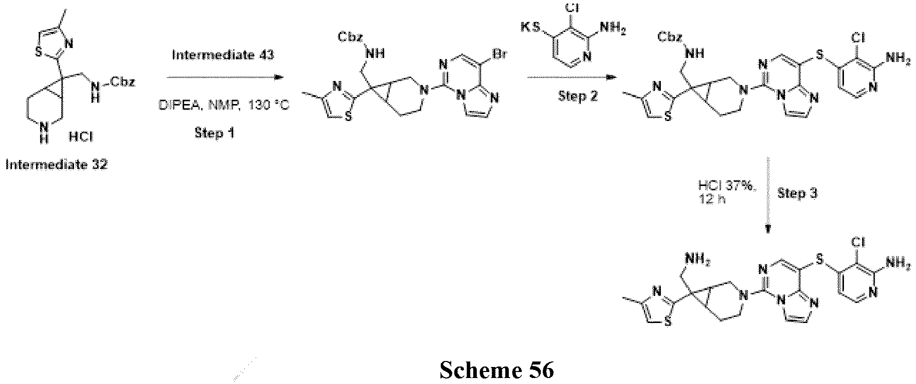

### Step 1: benzyl ((3-(8-bromoimidazo[1,2-c]pyrimidin-5-yl)-7-(4-methylthiazol-2-yl)-3-azabicyclo[4.7.0]heptan-7-yl)methyl)carbamate

To a solution of **Intermediate 43** (15 mg, 0.04 mmol) and **Intermediate 32** (10 mg, 0.04 mmol) in dry NMP (0.5 mL), dry DIPEA (0.03 mL, 0.15 mmol) was added. The reaction mixture was heated in a sealed vial at 130 °C for 2 h, then directly purified by preparative HPLC (eluting from 10% to 65% MeCN/H₂O + 0.1% TFA). The fractions were collected, 2N NaOH aqueous solution was added until pH=8, then aqueous phase was extracted with EtOAc (x2). The organic layer was dried over Na₂SO₄ filtered and concentrated *in vacuo* to obtain the title compound (19 mg, 90%). LCMS (ES⁺) Method 1: *m*/*z* 553, 555 (M+H)⁺, RT 1.72 min.

### Step 2: benzyl ((3-(8-((2-amino-3-chloropyridin-4-yl)thio)imidazo[1,2-c]pyrimidin-5-yl)-7-(4-methylthiazol-2-yl)-3-azabicyclo[4.1.0]heptan-7yl)methyl)carbamate

To a mixture of benzyl ((3-(8-bromoimidazo[1,2-c]pyrimidin-5-yl)-7-(4-methylthiazol-2-yl)-3-azabicyclo[4.1.0]heptan-7-yl)methyl)carbamate (19 mg, 0.03 mmol), potassium 2-amino-3-chloropyridine-4-thiolate (prepared with the same procedure used for **Intermediate 41**, **Scheme 32** step 2; 14 mg, 0.07 mmol), Xantphos (6 mg, 0.01 mmol) and Pd₂(dba)₃ (9.4 mg, 0.01 mmol) in degassed dry 1,4-dioxane (0.5 mL), dry DIPEA (0.03 mL, 0.17 mmol) was added and the reaction mixture was stirred at 130 °C for 3.5 h. The reaction was diluted with EtOAc and H₂O, then the organic layer was washed with 5% citric acid solution, dried over Na₂SO₄ filtered and concentrated *in vacuo.* The resulting crude was purified by preparative HPLC (from 5% to 55% MeCN/H₂O + 0.1% TFA) to obtain the title compound as a white solid (TFA salt; 14 mg, 64%). LCMS (ES⁺) Method 1: *m*/*z* 633 (M+H)⁺, RT 1.54 min.

### Step 3: 4-((5-(7-(aminomethyl)-7-(4-methylthiazol-2-yl)-3-azabicyclo[4.1.0]heptan-3-yl)imidazo[1,2-c]pyrimidin-8-yl)thio)-3-chloropyridin-2-amine

4-((5-(7-(aminomethyl)-7-(4-methylthiazol-2-yl)-3-azabicyclo[4.1.0]heptan-3-yl)imidazo[1,2-c]pyrimidin-8-yl)thio)-3-chloropyridin-2-amine was prepared from benzyl ((3-(8-((2-amino-3-chloropyridin-4-yl)thio)imidazo[1,2-c]pyrimidin-5-yl)-7-(4-methylthiazol-2-yl)-3-azabicyclo[4.1.0]heptan-7-yl)methyl)carbamate (14 mg, 0.02 mmol) according to the procedure described in the synthesis of **Example 64**, step 2. The reaction was purified by preparative HPLC (from 0% to 35% MeCN/H₂O + 0.1% TFA) to obtain the title compound as a white solid (TFA salt; 2 mg, 15%). ¹H NMR (DMSO-*d₆*+TFA) δ 8.42 (s, 1H), 8.13-7.98 (m, 5H), 7.73 (d, *J*=6.8 Hz, 1H), 7.20 (br d, *J*=1.0 Hz, 1H), 6.37 (d, *J*=6.8 Hz, 1H), 4.13-3.98 (m, 2H), 3.92 (m, 1H), 3.64 (m, 2H), 3.08 (m, 1H), 2.43 (m, 1H), 2.37 (s, 3H), 2.19 (m, 1H), 2.05 (m, 1H), 1.92 (m, 1H); LCMS (ES⁺) Method 2: *m*/*z* 499, 501 (M+H)⁺, RT 1.69 min.

### Example 77: (3-(3-(2,3-Dichlorophenyl)-1H-pyrazolo[3,4-b]pyrazin-6-yl)-7-(4-methylthiazol-2-yl)-3-azabicyclo[4.1.0]heptan-7-yl)methanamine

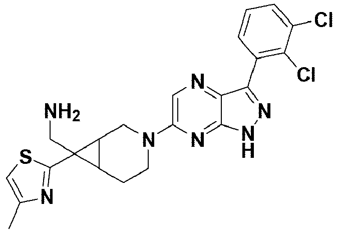

### Step 1: Benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

A mixture of **Intermediate 44** (70 mg, 0.19 mmol), **Intermediate 32** (72 mg, 0.18 mmol) and dry DIPEA (0.19 mL, 1.09 mmol) in dry DMF (1.7 mL) was heated to 80 °C in a sealed vial for 16 h. The reaction was diluted with EtOAc and washed with 5% citric acid solution (x2), H₂O (x2) and brine (x2). The organic layer was dried over Na₂SO₄ filtered and evaporated to obtain a crude compound which was used in the next step without further purification (124 mg). LCMS (ES⁺) Method 1: *m*/*z* 686 (M+H)⁺, RT 2.32 min.

### Step 2: Benzyl ((3-(3-(2,3-dichlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

A mixture of benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (50 mg, 0.07 mmol), K₃PO₄ (94 mg, 0.44 mmol), 2,3-dichlorophenylboronic acid (28 mg, 0.15 mmol) and Pd(dppf)Cl₂ complex in DCM (12 mg, 0.010 mmol) in degassed 1,4-dioxane/H₂O (9:1; 2.5 mL) was stirred for 1 h at 80 °C. The black suspension was diluted with EtOAc and washed with brine (x3). Organic layer was dried over Na₂SO₄ filtered and concentrated *in vacuo.* The resulting crude was purified by flash chromatography on silica gel (from 30% to 70% EtOAc in petroleum ether) to afford the title compound as a yellow oil (44 mg, 86% over two steps). LCMS (ES⁺) Method 1: *m*/*z* 704, 706 (M+H)⁺, RT 2.62 min.

### Step 3: (3-(3-(2,3-Dichlorophenyl)-1H-pyrazolo[3,4-b]pyrazin-6-yl)-7-(4-methylthiazol-2-yl)-3-azabicyclo[4.1.0]heptan-7-yl)methanamine

Benzyl ((3-(3-(2,3-dichlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (44 mg, 0.06 mmol) was dissolved in conc. HCl (37%; 2 mL) and the resulting yellow solution was stirred at rt for 12 h. The reaction was directly purified by preparative HPLC (from 5% to 55% MeCN/H₂O + 0.1% TFA) to obtain the title compound as a light-yellow solid (TFA salt; 16 mg, 43%). ¹H NMR (DMSO-*d₆*+TFA) δ 8.40 (s, 1H), 7.92 (br s, 3H), 7.72 (m, 2H), 7.49 (t, *J*=7.9 Hz, 1H), 7.17 (br d, *J*=1.0 Hz, 1H), 4.43-4.26 (m, 2H), 3.73 (m, 1H), 3.52 (m, 1H), 3.31 (m, 1H), 2.96 (m, 1H), 2.36 (m, 4H), 2.03 (m, 1H), 1.92-1.74 (m, 2H); LCMS (ES⁺) Method 2: *m*/*z* 486,488 (M+H)⁺, RT 2.67 min.

### Example 78: ((1S,6R,7S)-3-(3-(2,3-dichlorophenyl)-1H-pyrazolo[3,4-b]pyrazin-6-yl)-7-(4-methylthiazol-2-yl)-3-azabicyclo[4.1.0]heptan-7-yl)methanamine

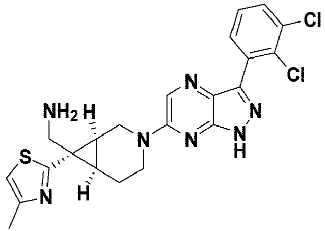

### Step 1: Benzyl (((1S,6R,7S)-3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

The compound was prepared according to the procedure described in the **Example 77** using **Intermediate 39** (21 mg, 0.05 mmol) instead of **Intermediate 32.** LCMS (ES⁺) Method 1: m/z 686 (M+H)⁺, RT 2.32 min.

### Step 2: Benzyl (((1S,6R,7S)-3-(3-(2, 3-dichlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

Benzyl (((1S,6R,7S)-3-(3-(2,3-dichlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate was prepared from benzyl (((1S,6R,7S)-3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (15 mg, 0.022 mmol) using the same procedure described in step 2 of the synthesis of **Example 77.** LCMS (ES⁺) Method 1: *m*/*z* 704, 706 (M+H)⁺, RT 2.62 min.

### Step 3: ((1S,6R,7S)-3-(3-(2,3-dichlorophenyl)-1H-pyrazolo[3,4-b]pyrazin-6-yl)-7-(4-methylthiazol-2-yl)-3-azabicyclo[4.1.0]heptan-7-yl)methanamine

((1S,6R,7S)-3-(3-(2,3-Dichlorophenyl)-1H-pyrazolo[3,4-b]pyrazin-6-yl)-7-(4-methylthiazol-2-yl)-3-azabicyclo[4.1.0]heptan-7-yl)methanamine was prepared from benzyl (((1S,6R,7S)-3-(3-(2,3-dichlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (15 mg, 0.02 mmol) using the same procedure described in step 3 of the synthesis of **Example 77.** The reaction was directly purified by preparative HPLC (from 5% to 55% MeCN/H₂O + 0.1% TFA) to obtain the title compound as a light-yellow solid (TFA salt; 16 mg, 43%). ¹H NMR (DMSO-*d₆*+TFA) δ 8.40 (s, 1H), 7.92 (br s, 3H), 7.72 (m, 2H), 7.49 (t, *J*=7.9 Hz, 1H), 7.17 (br d, *J*=1.0 Hz, 1H), 4.43-4.26 (m, 2H), 3.73 (m, 1H), 3.52 (m, 1H), 3.31 (m, 1H), 2.96 (m, 1H), 2.36 (m, 4H), 2.03 (m, 1H), 1.92-1.74 (m, 2H); LCMS (ES⁺) Method 2: *m*/*z* 486-488 (M+H)⁺, RT 2.67 min.

### Example 79: ((1S,6R,7S)-3-(3-(4-chloro-2-methyl-2H-indazol-5-yl)-1H-pyrazolo[3,4-b]pyrazin-6-yl)-7-(4-methylthiazol-2-yl)-3-azabicyclo[4.1.0]heptan-7-yl)methanamine

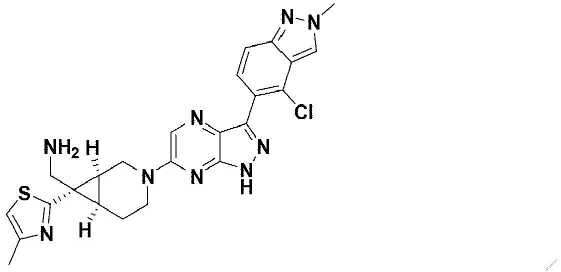

### Step 1: benzyl (((1S,6R,7S)-3-(3-(2,4-dimethyl-2H-indazol-5-yl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

Benzyl (((1S,6R,7S)-3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of the synthesis of **Example 77;** 20 mg, 0.03 mmol) and **Intermediate 45** (17 mg, 0. 06 mmol) were reacted using the same procedure described in step 2 of the synthesis of **Example 77.** The resulting crude was purified by flash chromatography on silica gel (from 0% to 100% EtOAc in petroleum ether) to afford the title compound as a yellow oil (6.6 mg, 31%). LCMS (ES⁺) Method 1: m/z 724, 726 (M+H)⁺, RT 2.30 min.

### Step 2: ((1S,6R,7S)-3-(3-(4-chloro-2-methyl-2H-indazol-5-yl)-1H-pyrazolo[3,4-b]pyrazin-6-yl)-7-(4-methylthiazol-2-yl)-3-azabicyclo[4.1.0]heptan-7-yl)methanamine

((1S,6R,7S)-3-(3-(4-chloro-2-methyl-2H-indazol-5-yl)-1H-pyrazolo[3,4-b]pyrazin-6-yl)-7-(4-methylthiazol-2-yl)-3-azabicyclo[4.1.0]heptan-7-yl)methanamine was prepared from benzyl (((1S,6R,7S)-3-(3-(4-chloro-2-methyl-2H-indazol-5-yl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (6.5 mg, 0.009 mmol) using the same procedure described in step 3 of the synthesis of **Example 77.** The reaction was directly purified by preparative HPLC (from 0% to 45% MeCN/H₂O + 0.1% TFA) to obtain the title compound as a light-yellow solid (TFA salt; 3 mg, 48%). ¹H NMR (300 MHz, DMSO-*d*₆+ TFA) δ 8.52 (s, 1H) 8.40 (s, 1H), 7.98-7.86 (m, 3H), 7.70-7.61 (m, 2H), 7.18-7.12 (m, 1H), 4.46-4.29 (m, 2H), 4.23 (s, 3H), 3.77-3.68 (m, 1H), 3.56-3.47 (m, 1H), 3.38-3.28 (m, 1H), 3.02- 2.89 (m, 1H), 2.35 (s, 4H), 2.08-1.97 (m, 1H), 1.90-1.78 (m, 2H). LCMS (ES⁺) Method 2: m/z 506, 508 (M+H)⁺, RT 2.24 min.

### Example 80: (3-(3-(2,3-Dichlorophenyl)-1H-pyrazolo[3,4-b]pyrazin-6-yl)-7-phenyl-3-azabicyclo[4.1.0]heptan-7-yl)methanamine

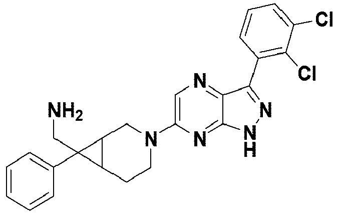

### Step 1: Benzyl ((3-(3-iodo-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate was prepared by reacting **Intermediate 28** (35 mg, 0.094 mmol) and **Intermediate 44** (70 mg, 0.19 mmol) according to the procedure described in step 1 of the synthesis of **Example 77.** The crude compound was used in the next step without further purification (60 mg). LCMS (ES⁺) Method 1: *m*/*z* 665 (M+H)⁺, RT 2.61 min.

### Step 2: Benzyl ((3-(3-(2,3-dichlorophenyl)-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate

Benzyl ((3-(3-(2,3-dichlorophenyl)-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate was prepared according to the procedure described in step 2 of the synthesis of **Example 77,** starting from benzyl ((3-(3-iodo-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate (25 mg, 0.04 mmol). The residue was purified by flash chromatography on silica gel (from 0% to 30% EtOAc in petroleum ether) to give the title compound as an off-white solid (16 mg, 62% over two steps). LCMS (ES⁺) Method 1: *m*/*z* 684, 686 (M+H)⁺, RT 2.39 min.

### Step 3: (3-(3-(2,3-Dichlorophenyl)-1H-pyrazolo[3,4-b]pyrazin-6-yl)-7-phenyl-3-azabicyclo [4.1.0]heptan-7-yl)methanamine

(3-(3-(2,3-Dichlorophenyl)-1H-pyrazolo[3,4-b]pyrazin-6-yl)-7-phenyl-3-azabicyclo[4.1.0] heptan-7-yl)methanamine was prepared according to the procedure described in step 3 of the synthesis of **Example 77** starting from benzyl ((3-(3-(2,3-dichlorophenyl)-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate (16 mg, 0.023 mmol). The reaction was directly purified by preparative HPLC (from 10% to 60% MeCN/H₂O + 0.1% TFA) to obtain the title compound as a light-yellow solid (TFA salt; 4 mg, 37%). ¹H NMR (DMSO-*d₆*+TFA) δ 8.41 (s, 1H), 7.72 (m, 2H), 7.63 (br s, 3H), 7.54-7.44 (m, 3H), 7.38 (m, 2H), 7.29 (m, 1H), 4.50-4.24 (m, 2H), 3.72 (m, 1H), 3.26 (m, 1H), 3.15 (m, 1H), 2.92 (m, 1H), 2.33 (m, 1H), 1.89-1.67 (m, 2H), 1.53 (m, 1H); LCMS (ES⁺) Method 2: *m*/*z* 465, 467 (M+H)⁺, RT 2.75 min.

### Example 81: (3-(3-(4-Chloro-2-methyl-2H-indazol-5-yl)-1H-pyrazolo[3,4-b]pyrazin-6-yl)-7-phenyl-3-azabicyclo[4.1.0]heptan-7-yl)methanamine

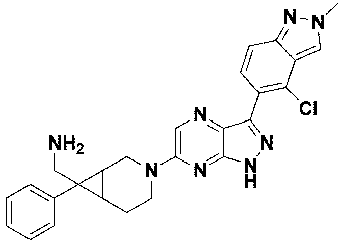

### Step 1: Benzyl ((3-(3-(4-chloro-2-methyl-2H-indazol-5-yl)-1-(tetrahydro-2H-pyran-2-yl)-1H-pyrazolo[3,4-b]pyrazin-6-yl)-7-phenyl-3-azabicyclo[4.1.0]heplan-7-yl)methyl)carbamate

Benzyl ((3-(3-(4-chloro-2-methyl-2H-indazol-5-yl)-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate was prepared from benzyl ((3-(3-iodo-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate (15 mg, 0.023 mmol) and **Intermediate 45** (13 mg, 0.04 mmol) according to the procedure described in step 2 of the synthesis of **Example 77.** The residue was purified by flash chromatography on silica gel (from 0% to 100% EtOAc in petroleum ether) to give the title compound as an off-white solid (5 mg, 31% over two steps). LCMS (ES⁺) Method 1: m/z 703, 705 (M+H)⁺, RT 2.58 min.

### Step 2: (3-(3-(4-Chloro-2-methyl-2H-indazol-5-yl)-1H-pyrazolo[3,4-b]pyrazin-6-yl)-7-phenyl-3-azabicyclo[4.1.0]heptan-7-yl)methanamine

(3-(3-(4-Chloro-2-methyl-2H-indazol-5-yl)-1H-pyrazolo[3,4-b]pyrazin-6-yl)-7-phenyl-3-azabicyclo[4.1.0]heptan-7-yl)methanamine was prepared from benzyl ((3-(3-(4-chloro-2-methyl-2H-indazol-5-yl)-1-(tetrahydro-2H-pyran-2-yl)-1H-pyrazolo[3,4-b]pyrazin-6-yl)-7-phenyl-3-azabicyclo[4.1.0]heptan-7-yl)methyl)carbamate (4.5 mg, 0.006 mmol) according to the procedure described in step 3 of the synthesis of **Example 77.** The reaction was directly purified by preparative HPLC (from 5% to 50% MeCN/H₂O + 0.1% TFA) to obtain the title compound as a light-yellow solid (TFA salt; 2 mg, 62%). ¹H NMR (300 MHz, DMSO-*d*₆+TFA) δ 8.52 (s, 1H), 8.40 (s, 1H), 7.66 (m, 5H), 7.50-7.45 (m, 2H), 7.36 (t, *J*=7.7 Hz, 2H), 7.31-7.23 (m, 1H), 4.45 (d, *J*=13.7 Hz, 1 H), 4.38-4.30 (m, 1H), 4.23 (s, 3H), 3.71 (dd, *J*=14.3, 6.4 Hz, 1H), 3.35-3.12 (m, 2H), 2.98-2.85 (m, 1H), 2.40-2.31 (m, 1H), 1.86-1.75 (m, 2H), 1.56-1.50 (m, 1H); LCMS (ES⁺) Method 2: m/z 485, 487 (M+H)⁺, RT 2.32 min.

### Example 82: (3-(3-(2,3-Dichlorophenyl)-1H-pyrazolo[3,4-b]pyrazin-6-yl)-7-(pyridin-2-yl)-3-azabicyclo [4.1.0]heptan-7-yl)methanamine

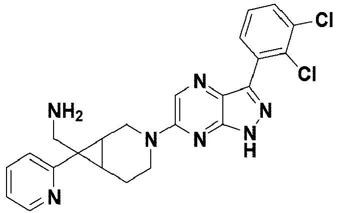

### Step 1: Benzyl ((3-(3-iodo-1-(tetrahydro-2H-pyran-2-yl)-1H-pyrazolo[3,4-b]pyrazin-6-yl)-7-(pyridin-2-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(pyridin-2-yl)-3-azabicyclo[4.1.0]heptan-7-yl)methyl)carbamate was prepared by reacting **Intermediate 29** (18 mg, 0.044 mmol) and **Intermediate 44** (66 mg, 0.09 mmol) according to the procedure described in step 1 of the synthesis of **Example 77.** The crude title compound was used in the next step without further purification (29 mg). LCMS (ES⁺) Method 1: *m*/*z* 666 (M+H)⁺, RT 1.71 min.

### Step 2: Benzyl ((3-(3-(2,3-dichlorophenyl)-1-(tetrahydro-2H-pyran-2-yl)-1H-pyrazolo[3,4-b]pyrazin-6-yl)-7-(pyridin-2-yl)-3-azabicyclo[4.1.0]heptan-7-yl)methyl)carbamate

Benzyl ((3-(3-(2,3-dichlorophenyl)-1-(tetrahydro-2H-pyran-2-yl)-1H-pyrazolo[3,4-b]pyrazin-6-yl)-7-(pyridin-2-yl)-3-azabicyclo[4.1.0]heptan-7-yl)methyl)carbamate was prepared according to the procedure described in step 2 of the synthesis of **Example 77,** starting from benzyl ((3-(3-iodo-1-(tetrahydro-2H-pyran-2-yl)-1H-pyrazolo[3,4-b]pyrazin-6-yl)-7-(pyridin-2-yl)-3-azabicyclo[4.1.0]heptan-7-yl)methyl)carbamate (29 mg, 0.044 mmol). The residue was purified by flash chromatography on silica gel (from 30% to 80% EtOAc in petroleum ether) to give the title compound as an off-white foam (15 mg, 50% over two steps). Method 1 *m*/*z* 684, 686 (M+H)⁺, RT 2.01 min.

### Step 3: (3-(3-(2, 3-Dichlorophenyl)-1H-pyrazolo[3,4-b]pyrazin-6-yl)-7-(pyridin-2-yl)-3-azabicyclo[4.1.0]heptan-7-yl)methanamine

The compound was prepared from benzyl ((3-(3-(2,3-dichlorophenyl)-1-(tetrahydro-2H-pyran-2-yl)-1H-pyrazolo[3,4-b]pyrazin-6-yl)-7-(pyridin-2-yl)-3-azabicyclo[4.1.0]heptan-7-yl)methyl)carbamate (15 mg, 0.02 mmol) according to the procedure described in step 3 of the synthesis of **Example 77.** The reaction was directly purified by preparative HPLC (from 0% to 40% MeCN/H₂O + 0.1% TFA) to obtain the title compound as a light-yellow solid (TFA salt; 8 mg, 63%). ¹H NMR (DMSO-*d₆*+TFA) δ 8.76 (dd, *J*=5.6 and 1.5 Hz, 1H), 8.41 (s, 1H), 8.22 (dt, *J*=7.8 and 1.5 Hz, 1H), 7.84 (br s, 3H), 7.78-7.62 (m, 4H), 7.50 (t, *J*=7.9 Hz, 1H), 4.53-4.30 (m, 2H), 3.70 (m, 1H), 3.47 (m, 1H), 3.29 (m, 1H), 2.96 (m, 1H), 2.34 (m, 1H), 2.12 (m, 1H), 1.94-1.75 (m, 2H); LCMS (ES⁺) Method 2: *m*/*z* 466-468 (M+H)⁺, RT 2.29 min.

### Example 83: (3-(3-(2,3-Dichlorophenyl)-1H-pyrazolo[3,4-b]pyrazin-6-yl)-7-(pyrimidin-2-yl)-3-azabicyclo [4.1.0]heptan-7-yl)methanamine

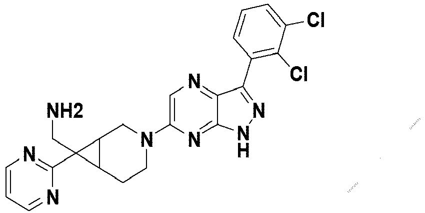

### Step 1: Benzyl ((3-(3-iodo-1-(tetrahydro-2H-pyran-2-yl)-1H-pyrazolo[3,4-b]pyrazin-6-yl)-7-(pyrimidin-2-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(pyrimidin-2-yl)-3-azabicyclo[4.1.0]heptan-7-yl)methyl)carbamate was prepared by reacting **Intermediate 30** (8.5 mg, 0.023 mmol) and **Intermediate 44** (34 mg, 0,05 mmol) according to the procedure described in step 1 of the synthesis of **Example 77.** The crude title compound was used in the next step without further purification (15 mg). LCMS (ES⁺) Method 1: *m*/*z* 667 (M+H)⁺, RT 2.43 min.

### Step 2: Benzyl ((3-(3-(2,3-dichlorophenyl)-1-(tetrahydro-2H-pyran-2-yl)-1H-pyrazolo[3,4-b]pyrazin-6-yl)-7-(pyrimidin-2-yl)-3-azabicyclo[4.1.0]heptan-7-yl)methyl)carbamate

Benzyl ((3-(3-(2,3-dichlorophenyl)-1-(tetrahydro-2H-pyran-2-yl)-1H-pyrazolo[3,4-b]pyrazin-6-yl)-7-(pyrimidin-2-yl)-3-azabicyclo[4.1.0]heptan-7-yl)methyl)carbamate was prepared according to the procedure described in step 2 of the synthesis of **Example 77,** starting from benzyl ((3-(3-iodo-1-(tetrahydro-2H-pyran-2-yl)-1H-pyrazolo[3,4-b]pyrazin-6-yl)-7-(pyrimidin-2-yl)-3-azabicyclo[4.1.0]heptan-7-yl)methyl)carbamate (15 mg, 0.023 mmol). The crude title compound was used in the next step without further purification (15 mg). LCMS (ES⁺) Method 1: *m*/*z* 685, 687 (M+H)⁺, RT 2.74 min.

### Step 3: (3-(3-(2,3-Dichlorophenyl)-1H-pyrazolo[3,4-b]pyrazin-6-yl)-7-(pyrimidin-2-yl)-3-azabicyclo[4.1.0]heptan-7-yl)methanamine

Crude benzyl ((3-(3-(2,3-dichlorophenyl)-1-(tetrahydro-2H-pyran-2-yl)-1H-pyrazolo[3,4-b]pyrazin-6-yl)-7-(pyrimidin-2-yl)-3-azabicyclo[4.1.0]heptan-7-yl)methyl)carbamate (15 mg, 0.02 theoretical mmol) was treated with HCl (4N in dioxane; 0.5 mL, 2.0 mmol) and the resulting solution was stirred at rt for 30 min. The reaction mixture was directly purified by preparative HPLC (from 30% to 90% MeCN/H₂O + 0.1% TFA) to obtain the product (without THP protecting group) as a yellow solid (8 mg). The compound was then dissolved in conc. HCl (37%; 0.5 mL) and stirred at rt for 18 h. The reaction was directly purified by preparative HPLC (from 10 to 55% MeCN/H₂O + 0.1% TFA) to afford the title compound as an off-white powder (TFA salt; 0.7 mg, 5% over two steps). LCMS (ES⁺) Method 2: *m*/*z* 467, 469 (M+H)⁺, RT 2.50 min.

### Example 84: (3-(3-(2-Chlorophenyl)-1H-pyrazolo[3,4-b]pyrazin-6-yl)-7-(4-methylthiazol-2-yl)-3-azabicyclo [4.1.0] heptan-7-yl)methanamine

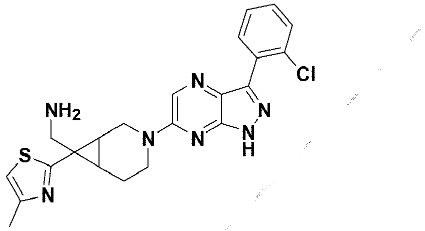

Benzyl ((3-(3-(2-chlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate was prepared according to the procedure described in Step 2 of the synthesis of **Example 77,** reacting benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of **Example 77;** 12 mg, 0.018 mmol) with 2-chlorophenylboronic acid (5.5 mg, 0.035 mmol). The residue was purified by flash chromatography on silica gel (from 30% to 70% EtOAc in petroleum ether) to give the title compound as a yellow oil (10 mg, 85% over two steps). LCMS (ES⁺) Method 1: *m*/*z* 670 (M+H)⁺, RT 2.48 min.

### Step 3: (3-(3-(2-Chlorophenyl)-1H-pyrazolo[3,4-b]pyrazin-6-yl)-7-(4-methylthiazol-2-yl)-3-azabicyclo[4.1.0]heptan-7-yl)methanamine

(3-(3-(2-Chlorophenyl)-1H-pyrazolo[3,4-b]pyrazin-6-yl)-7-(4-methylthiazol-2-yl)-3-azabicyclo[4.1.0]heptan-7-yl)methanamine was prepared from benzyl ((3-(3-(2-chlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (10 mg, 0.015 mmol) according to the procedure described in step 3 of the synthesis of **Example 77.** The reaction was directly purified by preparative HPLC (from 5% to 45% MeCN/H₂O + 0.1% TFA) to obtain the title compound as a light-yellow solid (TFA salt; 4 mg, 47%). ¹H NMR (DMSO-*d₆*+TFA) δ 8.39 (s, 1H), 7.92 (br s, 3H), 7.79-7.71 (m, 1H), 7.64-7.56 (m, 1H), 7.53-7.42 (m, 2H), 7.17 (br d, *J*=1.0 Hz, 1H), 4.45-4.26 (m, 2H), 3.74 (m, 1H), 3.52 (m, 1H), 3.32 (m, 1H), 2.96 (m, 1H), 2.42-2.29 (m, 4H), 2.03 (m, 1H), 1.93-1.71 (m, 2H); LCMS (ES⁺) Method 2: *m*/*z* 452-454 (M+H)⁺, RT 2.42 min.

### Example 85: (3-(3-(3-Chlorophenyl)-1H-pyrazolo[3,4-b|pyrazin-6-yl)-7-(4-methylthiazol-2-yl)-3-azabicyclo[4.1.0]heptan-7-yl)methanamine

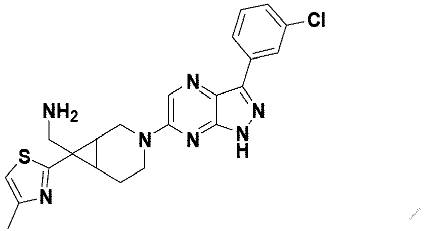

### Step 1: Benzyl ((3-(3-(3-chlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of the synthesis of **Example 77;** 15.5 mg, 0.023 mmol) and 3-chlorophenylboronic acid (7 mg, 0.045 mmol) were reacted according to the procedure described in step 2 of the synthesis of **Example 77.** The residue was purified by flash chromatography on silica gel (from 10% to 70% EtOAc/petroleum ether) to give the title compound as a yellow oil (11 mg, 73% over two steps). LCMS (ES⁺) Method 1: *m*/*z* 670, 672 (M+H)⁺, RT 2.76 min.

### Step 2: (3-(3-(3-Chlorophenyl)-1H-pyrazolo[3,4-b]pyrazin-6-yl)-7-(4-methylthiazol-2-yl)-3-azabicyclo[4.1.0]heptan-7-yl)methanamine

(3-(3-(3-Chlorophenyl)-1H-pyrazolo[3,4-b]pyrazin-6-yl)-7-(4-methylthiazol-2-yl)-3-azabicyclo[4.1.0]heptan-7-yl)methanamine was prepared from benzyl ((3-(3-(3-chlorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (11 mg, 0.016 mmol) according to the procedure described in step 3 of the synthesis of **Example 77.** The reaction was purified by preparative HPLC (from 15% to 60% MeCN/H₂O + 0.1% TFA) to obtain the title compound as a light-yellow solid (TFA salt; 4 mg, 44%). ¹H NMR (DMSO-*d₆*+TFA) δ 8.48 (s, 1H), 8.41 (m, 1H), 8.29 (m, 1H), 7.92 (br s, 3H), 7.53 (t, *J*=7.9 Hz, 1H), 7.44 (m, 1H), 7.17 (br d, *J*=1.0 Hz, 1H), 4.45-4.26 (m, 2H), 3.77 (m, 1H), 3.50 (m, 1H), 3.29 (m, 1H), 2.98 (m, 1H), 2.42-2.30 (m, 4H), 2.03 (m, 1H), 1.90-1.74 (m, 2H); LCMS (ES⁺) Method 2: *m*/*z* 452, 454 (M+H)⁺, RT 2.85 min.

### Example 86: (3-(3-(2,3-Difluorophenyl)-1H-pyrazolo[3,4-b]pyrazin-6-yl)-7-(4-methylthiazol-2-yl)-3-azabicyclo[4.1.0]heptan-7-yl)methanamine

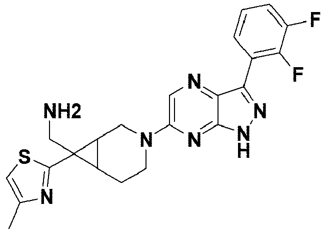

### Step 1: Benzyl ((3-(3-(2,3-difluorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of the synthesis of Example 77; 12 mg, 0.018 mmol) and 2,3-difluorophenylboronic acid (5.5 mg, 0.035 mmol) were reacted according to the procedure described in step 2 of the synthesis of **Example 77.** The residue was purified by flash chromatography on silica gel (from 30% to 70% EtOAc in petroleum ether) to give the title compound as a yellow oil (10 mg, 85% over two steps). Method 1: *m*/*z* 672 (M+H)⁺, RT 2.52 min.

### Step 2: (3-(3-(2,3-Difluorophenyl)-1H-pyrazolo[3,4-b]pyrazin-6-yl)-7-(4-methylthiazol-2-yl)-3-azabicyclo[4.1.0]heptan-7-yl)methanamine

The compound was prepared from benzyl ((3-(3-(2,3-difluorophenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (10 mg, 0.015 mmol) according to the procedure described in step 3 of the synthesis of **Example 77.** The reaction was directly purified by preparative HPLC (from 5% to 45% MeCN/H₂O + 0.1% TFA) to obtain the title compound as a light-yellow solid (TFA salt; 4 mg, 47%). ¹H NMR (DMSO-*d₆*+TFA) δ 8.45 (s, 1H), 8.01 (m, 1H), 7.92 (br s, 3H), 7.49 (m, 1H), 7.36 (m, 1H), 7.18 (br d, *J*=1.0 Hz, 1H), 4.46-4.26 (m, 2H), 3.76 (m, 1H), 3.51 (m, 1H), 3.30 (m, 1H), 2.98 (m, 1H), 2.42-2.30 (m, 4H), 2.03 (m, 1H), 1.90-1.74 (m, 2H); LCMS (ES⁺) Method 2: *m*/*z* 454 (M+H)⁺, RT 2.49 min.

### Example 87: (3-(3-(2-Chloro-3-methoxyphenyl)-1H-pyrazolo[3,4-b]pyrazin-6-yl)-7-(4-methylthiazol-2-yl)-3-azabicyclo [4.1.0] heptan-7-yl)methanamine

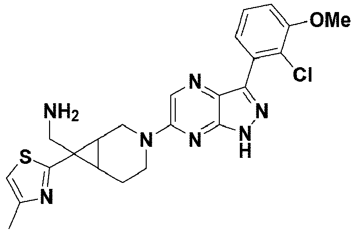

### Step 1: Benzyl ((3-(3-(2-chloro-3-methoxyphenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of the synthesis of **Example 77;** 15.4 mg, 0.022 mmol) and 2-chloro-3-methoxyphenylboronic acid (8.4 mg, 0.045 mmol) were reacted according to the procedure described in step 2 of the synthesis of **Example 77.** The residue was purified by flash chromatography on silica gel (from 30 to 70% EtOAc in petroleum ether) to give the title compound as a yellow oil (13 mg, 83% over two steps). LCMS (ES⁺) Method 1: *m*/*z* 700, 702 (M+H)⁺, RT 2.42 min.

### Step 2: (3-(3-(2-Chloro-3-methoxyphenyl)-1H-pyrazolo[3,4-b]pyrazin-6-yl)-7-(4-methylthiazol-2-yl)-3-azabicyclo[4.1.0]heptan-7-yl)methanamine

(3-(3-(2-Chloro-3-methoxyphenyl)-1H-pyrazolo[3,4-b]pyrazin-6-yl)-7-(4-methylthiazol-2-yl)-3-azabicyclo[4.1.0]heptan-7-yl)methanamine was prepared from benzyl ((3-(3-(2-chloro-3-methoxyphenyl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (13 mg, 0.02 mmol) according to the procedure described in step 3 of the synthesis of **Example 77.** The reaction was directly purified by preparative HPLC (from 5% to 55% MeCN/H₂O + 0.1% TFA) to obtain the title compound as a light-yellow solid (TFA salt; 5 mg, 44%). ¹H NMR (DMSO-*d₆*+TFA) δ 8.38 (s, 1H), 7.92 (br s, 3H), 7.42 (t, *J*=7.9 Hz, 1H), 7.29-7.21 (m, 2H), 7.17 (br d, *J*=1.0 Hz, 1H), 4.43-4.26 (m, 2H), 3.92 (s, 3H), 3.72 (m, 1H), 3.52 (m, 1H), 3.31 (m, 1H), 2.95 (m, 1H), 2.42-2.32 (m, 4H), 2.02 (m, 1H), 1.90-1.74 (m, 2H); LCMS (ES⁺) *m*/*z* 482, 484 (M+H)⁺, RT 2.40 min.

### Example 88: (7-(4-Methylthiazol-2-yl)-3-(3-(quinolin-5-yl)-1H-pyrazolo[3,4-b]pyrazin-6-yl)-3-azabicyclo [4.1.0] heptan-7-yl)methanamine

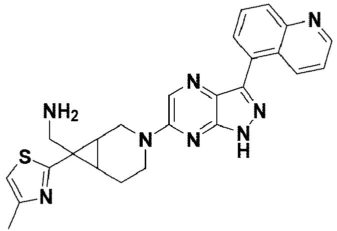

### Step 1: benzyl ((7-(4-methylthiazol-2-yl)-3-(3-(quinolin-5-yl)-1-(tetrahydro-2H-pyran-2-yl)-1H-pyrazolo[3,4-b]pyrazin-6-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of the synthesis of **Example 77;** 15 mg, 0.022 mmol) and quinoline-5-boronic acid (7.6 mg, 0.044 mmol) were reacted according to the procedure described in step 2 of the synthesis of **Example 77.** The residue was purified by flash chromatography on silica gel (from 50% to 100% EtOAc in petroleum ether) to give the title compound as an orange oil (10 mg, 67% over two steps). LCMS (ES⁺) *m*/*z* 687 (M+H)⁺, RT 1.86 min.

### Step 2: (7-(4-Methylthiazol-2-yl)-3-(3-(quinolin-5-yl)-1H-pyrazolo[3,4-b]pyrazin-6-yl)-3-azabicyclo[4.1.0]heptan-7-yl)methanamine

(7-(4-Methylthiazol-2-yl)-3-(3-(quinolin-5-yl)-1H-pyrazolo[3,4-b]pyrazin-6-yl)-3-azabicyclo[4.1.0]heptan-7-yl)methanamine was prepared from benzyl ((7-(4-methylthiazol-2-yl)-3-(3-(quinolin-5-yl)-1-(tetrahydro-2H-pyran-2-yl)-1H-pyrazolo[3,4-b]pyrazin-6-yl)-3-azabicyclo[4.1.0]heptan-7-yl)methyl)carbamate (10 mg, 0.015 mmol) according to the procedure described in step 3 of the synthesis of **Example 77.** The reaction was purified by preparative HPLC (from 0% to 30% MeCN/H₂O + 0.1% TFA) to obtain the title compound as a yellow solid (TFA salt; 5 mg, 57%). ¹H NMR (DMSO-*d₆*+TFA) δ 10.10 (d, *J=* 8.7 Hz, 1H), 9.41 (dd, *J=* 5.3 and 1.4 Hz, 1H), 8.79 (dd, *J=* 6.4 and 2.1 Hz, 1H), 8.51 (s, 1H), 8.38-8.26 (m, 2H), 8.19 (dd, *J=* 8.7 and 5.3 Hz, 1H), 7.95 (br s, 3H), 7.18 (br d, *J*=1.0 Hz, 1H), 4.44-4.28 (m, 2H), 3.82 (m, 1H), 3.54 (m, 1H), 3.31 (m, 1H), 3.03 (m, 1H), 2.44-2.31 (m, 4H), 2.05 (m, 1H), 1.92-1.76 (m, 2H); LCMS (ES⁺) Method 2: *m*/*z* 469 (M+H)⁺, RT 1.78 min.

### Example 89: (7-(4-methylthiazol-2-yl)-3-(3-(naphthalen-1-yl)-1H-pyrazolo[3,4-b]pyrazin-6-yl)-3-azabicyclo[4.1.0]heptan-7-yl)methanamine

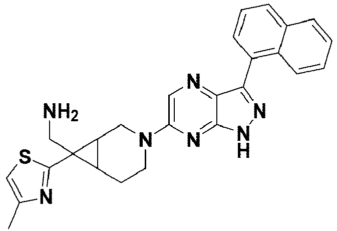

### Step 1: Benzyl ((7-(4-methylthiazol-2-yl)-3-(3-(naphthalen-1-yl)-1-(tetrahydro-2H-pyran-2-yl)-1H-pyrazolo[3,4-b]pyrazin-6-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of the synthesis of **Example 77;** 12 mg, 0.018 mmol) and naphthalen-1-ylboronic acid (6 mg, 0.035 mmol) were reacted according to the procedure described in step 2 of the synthesis of **Example 77.** The residue was purified by flash chromatography on silica gel (from 30% to 70% EtOAc in petroleum ether) to give the title compound as a yellow oil (11 mg, 92% over two steps). LCMS (ES⁺) Method 1: *m*/*z* 686 (M+H)⁺, RT 2.62 min.

### Step 2: (7-(4-methylthiazol-2-yl)-3-(3-(naphthalen-1-yl)-1H-pyrazolo[3,4-b]pyrazin-6-yl)-3-azabicyclo[4.1.0]heptan-7-yl)methanamine

(7-(4-methylthiazol-2-yl)-3-(3-(naphthalen-1-yl)-1H-pyrazolo[3,4-b]pyrazin-6-yl)-3-azabicyclo[4.1.0]heptan-7-yl)methanamine was prepared from benzyl ((7-(4-methylthiazol-2-yl)-3-(3-(naphthalen-1-yl)-1-(tetrahydro-2H-pyran-2-yl)-1H-pyrazolo[3,4-b]pyrazin-6-yl)-3-azabicyclo[4.1.0]heptan-7-yl)methyl)carbamate (11 mg, 0.016 mmol) according to the procedure described in step 3 of the synthesis of **Example 77.** The reaction was purified by preparative HPLC (from 10% to 60% MeCN/H₂O + 0.1% TFA) to obtain the title compound as a yellow solid (TFA salt; 5 mg, 51%). ¹H NMR (DMSO-*d₆*+TFA) δ 8.69 (m, 1H), 8.43 (s, 1H), 8.16 (dd, *J*=7.2 and 1.1 Hz, 1H), 8.06-7.98 (m, 2H), 7.94 (br s, 3H), 7.65 (m, 1H), 7.61-7.50 (m, 2H), 7.18 (br d, *J*=1.0 Hz, 1H), 4.48-4.28 (m, 2H), 3.77 (dd, *J*=14.6 and 6.2 Hz, 1H), 3.53 (m, 1H), 3.33 (m, 1H), 2.99 (m, 1H), 2.43-2.29 (m, 4H), 2.04 (dt, *J*=9.8 and 3.7 Hz, 1H), 1.94-1.74 (m, 2H); LCMS (ES⁺) Method 2: *m*/*z* 468 (M+H)⁺, RT 2.71 min.

### Example 90: (7-(4-Methylthiazol-2-yl)-3-(3-(2-(trifluoromethyl)pyridin-3-yl)-1H-pyrazolo[3,4-b]pyrazin-6-yl)-3-azabicyclo[4.1.0]heptan-7-yl)methanamine

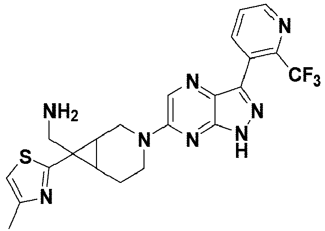

### Step 1: Benzyl ((7-(4-methylthiazol-2-yl)-3-(1-(tetrahydro-2H-pyran-2-yl)-3-(2-(trifluoromethyl)pyridin-3-yl)-1H-pyrazolo[3,4-b]pyrazin-6-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of the synthesis of **Example 77;** 18 mg, 0.026 mmol) and (2-(trifluoromethyl)pyridin-3-yl)boronic acid (10 mg, 0.05 mmol) were reacted according to the procedure described in step 2 of the synthesis of **Example 77.** The crude compound (18 mg) was used in the next step without purification. LCMS (ES⁺) Method 1: *m*/*z* 705 (M+H)⁺, RT 2.37 min.

### Step 2: (7-(4-Methylthiazol-2-yl)-3-(3-(2-(trijluoromethyl)pyridin-3-yl)-1H-pyrazolo[3,4-b]pyrazin-6-yl)-3-azabicyclo[4.1.0]heptan-7-yl)methanamine

The compound was prepared from benzyl ((7-(4-methylthiazol-2-yl)-3-(1-(tetrahydro-2H-pyran-2-yl)-3-(2-(trifluoromethyl)pyridin-3-yl)-1H-pyrazolo[3,4-b]pyrazin-6-yl)-3-azabicyclo[4.1.0]heptan-7-yl)methyl)carbamate (20 mg, 0.03 mmol) according to the procedure described in step 3 of the synthesis of **Example 77.** The reaction was directly purified by preparative HPLC (from 5% to 45% MeCN/H₂O + 0.1% TFA) to obtain the title compound as a yellow solid (TFA salt; 3 mg, 18%). ¹H NMR (DMSO-*d*₆) δ 13.56 (s, 1H), 8.86 (br d, *J*=4.7 Hz, 1H), 8.41 (s, 1H), 8.27 (br d, *J*=8.1 Hz, 1H), 8.00-7.83 (m, 4H), 7.19 (br d, *J*=1.0 Hz, 1H), 4.41-4.27 (m, 2H), 3.76 (m, 1H), 3.51 (m, 1H), 3.30 (m, 1H), 2.98 (m, 1H), 2.42-2.30 (m, 4H), 2.03 (m, 1H), 1.91-1.74 (m, 2H); LCMS (ES⁺) Method 2: *m*/*z* 487 (M+H)⁺, RT 2.23 min.

### Example 91: (3-(3-(Benzo[d][1,3]dioxol-4-yl)-1H-pyrazolo[3,4-b]pyrazin-6-yl)-7-(4-methylthiazol-2-yl)-3-azabicyclo[4.1.0]heptan-7-yl)methanamine

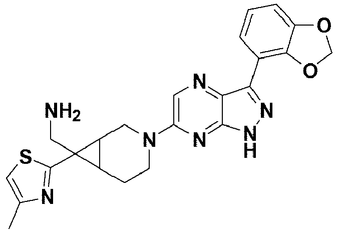

### Step 1: 2-((3-(3-Iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)isoindoline-1,3-dione

**Intermediate 36** (107 mg, 0.273 mmol) was reacted with **Intermediate 44** (104 mg, 0.28 mmol) according to the procedure described in step 1 of the synthesis of **Example 77.** The crude title compound was used in the next step without further purification (215 mg). LCMS (ES⁺) Method 1: *m*/*z* 682 (M+H)⁺, RT 2.28 min.

### Step 2: tert-butyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

2-((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)isoindoline-1,3-dione (186 mg, 0.27 mmol) was suspended in EtOH (8 mL), hydrazine monohydrate (0.41 mL, 8.19 mmol) was added and the reaction mixture was stirred at rt for 3 h. The volatiles were removed under reduced pressure to obtain crude primary amine (231 mg). The residue was taken up in DCM (5 mL) then a solution of di-*tert*-butyl dicarbonate (119 mg, 0.55mmol) in DCM (1 mL) and DIPEA (0.19 mL, 1.09 mmol) were added, and the reaction mixture was stirred at rt for 1.5 h. The reaction was diluted with DCM and washed with 5% citric acid solution (x2). The aqueous phase was extracted again with DCM (x2) then the collected organic layers were dried over Na₂SO₄, filtered and concentrated under reduced pressure. The resulting crude was purified by flash chromatography on silica gel (from 30% to 50% EtOAc in petroleum ether) to afford the title compound as a yellow foam (161 mg, 90% over four steps). ¹H NMR (300 MHz, CDCl₃) δ 8.18 (d, *J*=2.9 Hz, 1H), 6.78 (s, 1H), 5.80 (dd, *J*=10.4 and 2.2 Hz, 1H), 5.43 (br s, 1H), 4.38-4.07 (m, 3H), 3.98 (m, 1H), 3.89-3.63 (m, 3H), 3.15 (m, 1H), 2.68 (m, 1H), 2.47 (s, 3H), 2.39 (m, 1H), 2.17 (m, 1H), 2.10-1.87 (m, 4H), 1.79 (m, 2H), 1.58 (m, 1H), 1.41 (s, 9H); LCMS (ES⁺) Method 1: *m*/*z* 652 (M+H)⁺, RT 2.25 min.

### Step 3: tert-butyl ((3-(3-(benzo[d][1,3]dioxol-4-yl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

*tert*-Butyl((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (10 mg, 0.015 mmol) and benzo[d][1,3]dioxol-4-ylboronic acid (5 mg, 0.031 mmol) were reacted according to the procedure described in step 2 of the synthesis of **Example 77.** The residue was purified by flash chromatography on silica gel (from 30% to 70% EtOAc in petroleum ether) to give the title compound as a yellow oil (6 mg, 61%). LCMS (ES⁺) Method 1: *m*/*z* 646 (M+H)⁺, RT 2.36 min.

### Step 4: 3-(3-(Benzo[d][1,3]dioxol-4-yl)-1H-pyrazolo[3,4-b]pyrazin-6-yl)-7-(4-methylthiazol-2-yl)-3-azabicyclo[4.1.0]heptan-7-yl)methanamine

*tert-butyl* ((3-(3-(benzo[d][1,3]dioxol-4-yl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (6 mg, 0.009 mmol) was dissolved in DCM (0.5 mL) and treated with HCl (4N in dioxane; 0.2 mL, 0.8 mmol). The solution was stirred at rt for 1 h, then solvent was removed *in vacuo.* The resulting crude was purified by preparative HPLC (from 5% to 50% MeCN/H₂O + 0.1% TFA) to afford the title compound as an off-white solid (TFA salt; 4 mg, 77%). ¹H NMR (DMSO-*d₆*+TFA) δ 8.43 (s, 1H), 8.04-7.81 (m, 4H), 7.17 (br d, *J*=1.0 Hz, 1H), 7.02-6.91 (m, 2H), 6.10 (s, 2H), 4.48-4.26 (m, 2H), 3.75 (dd, *J*=14.6 and 6.3 Hz, 1H), 3.51 (m, 1H), 3.30 (m, 1H), 3.06-2.87 (m, 1H), 2.43-2.30 (m, 4H), 2.03 (m, 1H), 1.91-1.73 (m, 2H); LCMS (ES⁺) Method 2: *m*/*z* 462 (M+H)⁺, RT 2.40 min.

### Example 92: (3-(3-(8-Fluoroquinolin-5-yl)-1H-pyrazolo[3,4-b]pyrazin-6-yl)-7-(4-methylthiazol-2-yl)-3-azabicyclo [4.1.0] heptan-7-yl)methanamine

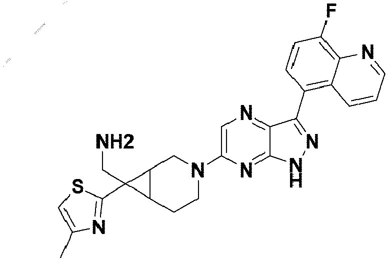

### Step 1: Tert-butyl ((3-(3-(8-fluoroquinolin-5-yl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

*tert-Butyl* ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 2 of the synthesis of **Example 91;** 16 mg, 0.025 mmol) and 8-fluoro-5-(4,4,5,5-tetramethyl-1,3,2-dioxaborolan-2-yl)quinoline (13.4 mg, 0.049 mmol) were reacted according to the procedure described in step 2 of the synthesis of **Example 77.** The residue was purified by flash chromatography on silica gel (from 50% to 100% EtOAc in petroleum ether) to give the title compound as a yellow oil (16 mg, 97%). LCMS (ES⁺) Method 1: *m*/*z* 671 (M+H)⁺, RT 2.22 min.

### Step 2: (3-(3-(8-Fluoroquinolin-5-yl)-1H-pyrazolo[3,4-b]pyrazin-6-yl)-7-(4-methylthiazol-2-yl)-3-azabicyclo[4.1.0]heptan-7-yl)methanamine

(3-(3-(8-Fluoroquinolin-5-yl)-1H-pyrazolo[3,4-b]pyrazin-6-yl)-7-(4-methylthiazol-2-yl)-3-azabicyclo[4.1.0]heptan-7-yl)methanamine was prepared from *tert*-butyl ((3-(3-(8-fluoroquinolin-5-yl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (16 mg, 0.024 mmol) according to the procedure described in step 4 of the synthesis of **Example 91.** The residue was directly purified by preparative HPLC (from 5% to 45% MeCN/H₂O + 0.1% TFA) to obtain the title compound as a yellow solid (TFA salt; 13 mg, 92%). ¹H NMR (DMSO-*d₆*+TFA) δ 9.43 (m, 1H), 9.07 (dd, *J*=4.2 and 1.4 Hz, 1H), 8.50-8.40 (m, 2H), 7.93 (br s, 3H), 7.87-7.73 (m, 2H), 7.18 (br d, *J*=1.0 Hz, 1H), 4.47-4.28 (m, 2H), 3.79 (dd, *J*=14.6 and 6.3 Hz, 1H), 3.53 (m, 1H), 3.31 (m, 1H), 3.00 (m, 1H), 2.43-2.30 (m, 4H), 2.04 (m, 1H), 1.93-1.73 (m, 2H); LCMS (ES⁺) Method 2: *m*/*z* 487 (M+H)⁺, RT 2.14 min.

### Example 93: 4-((6-(7-(Aminomethyl)-7-(4-methylthiazol-2-yl)-3-azabicyclo[4.1.0]heptan-3-yl)-1H-pyrazolo[3,4-b]pyrazin-3-yl)thio)-3-chloropyridin-2-amine

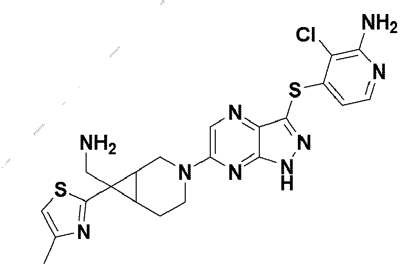

A mixture of benzyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 1 of **Example 77;** 17 mg, 0.03 mmol) and potassium 2-amino-3-chloropyridine-4-thiolate (prepared by using the procedure described for **Intermediate 42,** step 3 using potassium tert-butoxide, 16 mg, 0.078 mmol) in 1,4-dioxane (1 mL), DIPEA (18 uL, 0.100 mmol), Xantphos (1.5 mg, 0.003 mmol) and Pd₂(dba)₃ (4.76mg, 0.010mmol) were sequentially added. The reaction mixture was stirred for 3 h at 110 °C. The reaction was diluted with EtOAc (1 mL), washed with H₂O (3x 1mL) and the collected organic layers were concentrated to afford crude benzyl ((3-(3-((2-amino-3-chloropyridin-4-yl)thio)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate, which was directly treated with conc. HCl (37%; 780 uL). The residue was purified by preparative HPLC (from 5% to 45% MeCN/H₂O + 0.1% TFA) to obtain the title compound as a yellow solid (TFA salt; 2 mg, 14% over two steps). LCMS (ES⁺) Method 2: *m*/*z* 500, 502 (M+H)⁺, RT 1.82 min.

### Example 94: (3-(3-(2,3-Dichlorophenyl)-1H-pyrazolo[3,4-b]pyrazin-6-yl)-7-(thiophen-3-yl)-3-azabicyclo [4.1.0]heptan-7-yl)methanamine

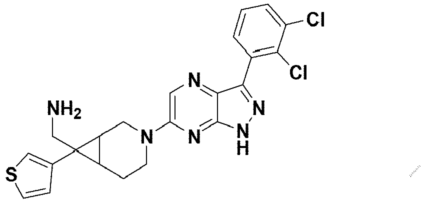

### Step 1: 2-((3-(3-Iodo-1-(tetrahydro-2H-pyran-2-yl)-1H-pyrazolo[3,4-b]pyrazin-6-yl)-7-(thiophen-3-yl)-3-azabicyclo[4.1.0]heptan-7-yl)methyl)isoindoline-1,3-dione

A mixture of **Intermediate 44** (52 mg, 0.14 mmol), **Intermediate 37** (51 mg, 0.14 mmol) in dry DMF (0.8 mL) was treated with DIPEA (0.12 mL, 0.68 mmol) and heated under MW irradiation to 85 °C for 4 h. The reaction was diluted with EtOAc and the organic phase was washed with H₂O, dried over Na₂SO₄, filtered and concentrated. The residue was purified by flash chromatography on silica gel (from 0% to 30% EtOAc in petroleum ether) to afford the title compound as an off-white solid (69 mg, 76%). LCMS (ES⁺) Method 1: *m*/*z* 667 (M+H)⁺, RT 2.62 min.

### Step 2: 2-((3-(3-(2,3-Dichlorophenyl)-1-(tetrahydro-2H-pyran-2-yl)-1H-pyrazolo[3,4-b]pyrazin-6-yl)-7-(thiophen-3-yl)-3-azabicyclo[4.1.0]heptan-7-yl)methyl)isoindoline-1,3-dione

2-((3-(3-iodo-1-(tetrahydro-2H-pyran-2-yl)-1H-pyrazolo[3,4-b]pyrazin-6-yl)-7-(thiophen-3-yl)-3-azabicyclo[4.1.0]heptan-7-yl)methyl)isoindoline-1,3-dione (56 mg, 0.09 mmol) and 2,3-dichlorophenylboronic acid (16 mg, 0.08 mmol) were reacted according to the procedure described in step 2 of the synthesis of **Example 77.** The mixture was diluted with toluene (2 mL) and washed with H₂O (3x2 mL). The collected organic layers were concentrated under reduced pressure, affording the desired compound was used in the next step without further purification (56 mg). LCMS (ES⁺) Method 1: *m*/*z* 685, 687 (M+H)⁺, RT 2.52 min.

### Step 3: (3-(3-(2,3-Dichlorophenyl)-1H-pyrazolo[3,4-b]pyrazin-6-yl)-7-(thiophen-3-yl)-3-azabicyclo[4.1.0]heptan-7-yl)methanamine

2-((3-(3-(2,3-Dichlorophenyl)-1-(tetrahydro-2H-pyran-2-yl)-1H-pyrazolo[3,4-b]pyrazin-6-yl)-7-(thiophen-3-yl)-3-azabicyclo[4.1.0]heptan-7-yl)methyl)isoindoline-1,3-dione (56 mg, 0.08 mmol) was dissolved in MeOH (2 mL) and treated with HCl (4N in dioxane 1 mL, 4.0 mmol). The reaction was stirred at rt for 1 h then the volatiles were removed *in vacuo.* The residue was dissolved in EtOAc and NaHCO₃ sol. sat., then the organic layer was dried over Na₂SO₄, filtered and evaporated *in vacuo.* The crude was dissolved in MeOH (2 mL), treated with hydrazine monohydrate (20 uL, 0.245 mmol) and stirred at rt for 2 h. The volatiles were removed under reduced pressure and the residue was dissolved in EtOAc and H₂O and the aqueous layer was extracted with EtOAc (x3). The combined organic extracts were concentrated and purified by reverse phase chromatography on C18 cartridge (from 10% to 50% MeCN/H₂O + 0.1% TFA) to obtain the title compound (TFA salt; 8.4 mg, 18%). ¹H NMR (DMSO-*d₆*+TFA) δ 8.39 (s, 1H), 7.81-7.61 (m, 5H), 7.59-7.45 (m, 2H), 7.41 (m, 1H), 7.16 (m, 1H), 4.31 (m, 2H), 3.71 (m, 1H), 3.33 (m, 1H), 3.17 (m, 1H), 2.93 (m, 1H), 2.29 (m, 1H), 1.85 (m, 1H), 1.73 (m, 1H), 1.57 (m, 1H); LCMS (ES⁺) Method 2: *m*/*z* 471 (M+H)⁺, RT 2.70 min.

### Example 95: (7-(Thiophen-3-yl)-3-(3-(2-(trifluoromethyl)pyridin-4-yl)-1H-pyrazolo[3,4-b]pyrazin-6-yl)-3-azabicyclo[4.1.0]heptan-7-yl)methanamine

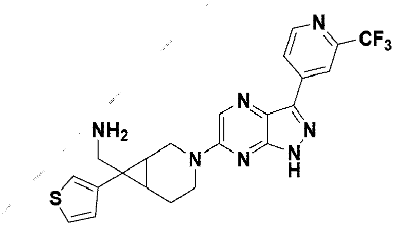

### Step 1: 2-((3-(1-(Tetrahydro-2H-pyran-2-yl)-3-(2-(trifluoromethyl)pyridin-4-yl)-1H-pyrazolo[3,4-b]pyrazin-6-yl)-7-(thiophen-3-yl)-3-azabicyclo[4.1.0]heptan-7-yl)methyl)isoindoline-1,3-dione

2-((3-(3-iodo-1-(tetrahydro-2H-pyran-2-yl)-1H-pyrazolo[3,4-b]pyrazin-6-yl)-7-(thiophen-3-yl)-3-azabicyclo[4.1.0]heptan-7-yl)methyl)isoindoline-1,3-dione (prepared as described in step 1 of the synthesis of **Example 94;** 25 mg, 0.038 mmol) and (2-(trifluoromethyl)pyridin-4-yl)boronic acid (14 mg, 0.07 mmol) were reacted according to the procedure described in step 2 of the synthesis of **Example 77.** The residue was purified by flash chromatography on silica gel (from 0% to 50% EtOAc in petroleum ether) to give the title compound as off white solid (12 mg, 47%). LCMS (ES⁺) Method 1: *m*/*z* 686 (M+H)⁺, RT 2.44 min.

### Step 2: (7-(Thiophen-3-yl)-3-(3-(2-(trijluoromethyl)pyridin-4-yl)-1H-pyrazolo[3,4-b]pyrazin-6-yl)-3-azabicyclo[4.1.0]heptan-7-yl)methanamine

(7-(Thiophen-3-yl)-3-(3-(2-(trifluoromethyl)pyridin-4-yl)-1H-pyrazolo[3,4-b]pyrazin-6-yl)-3-azabicyclo[4.1.0]heptan-7-yl)methanamine was prepared from 2-((3-(1-(tetrahydro-2H-pyran-2-yl)-3-(2-(trifluoromethyl)pyridin-4-yl)-1H-pyrazolo[3,4-b]pyrazin-6-yl)-7-(thiophen-3-yl)-3-azabicyclo[4.1.0]heptan-7-yl)methyl)isoindoline-1,3-dione (12 mg, 0.017 mmol) according to the procedure described in step 3 of the synthesis of **Example 94.** The reaction was directly purified by preparative HPLC (from 10% to 60% MeCN/H₂O + 0.1% TFA) to obtain the title compound (TFA salt; 9.6 mg, 96%). ¹H NMR (DMSO-*d₆*+TFA) δ 8.88 (d, *J*=5.1 Hz, 1H), 8.73 (br s, 1H), 8.56 (s, 1H), 8.52 (m, 1H), 7.71 (br s, 3H), 7.55 (dd, *J*=4.9 and 2.9 Hz, 1H), 7.41 (m, 1H), 7.15 (dd, *J*=5.1 and 1.3 Hz, 1H), 4.33 (m, 2H), 3.77 (m, 1H), 3.32 (m, 1H), 3.14 (m, 1H), 2.97 (m, 1H), 2.31 (m, 1H), 1.86 (m, 1H), 1.72 (m, 1H), 1.57 (m, 1H); LCMS (ES⁺) Method 2: *m*/*z* 472 (M+H)⁺, RT 2.69 min.

### Example 96: (7-(Thiophen-3-yl)-3-(3-(2-(trifluoromethyl)pyridin-3-yl)-1H-pyrazolo[3,4-b]pyrazin-6-yl)-3-azabicyclo[4.1.0]heptan-7-yl)methanamine

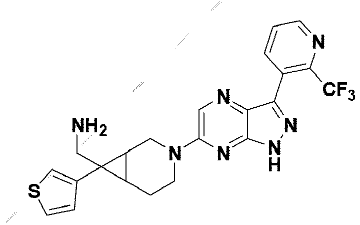

### Step 1: 2-((3-(1-(Tetrahydro-2H-pyran-2-yl)-3-(2-(trifluoromethyl)pyridin-3-yl)-1H-pyrazolo[3,4-b]pyrazin-6-yl)-7-(thiophen-3-yl)-3-azabicyclo[4.1.0]heptan-7-yl)methyl)isoindoline-1,3-dione

2-((3-(3-iodo-1-(tetrahydro-2H-pyran-2-yl)-1H-pyrazolo[3,4-b]pyrazin-6-yl)-7-(thiophen-3-yl)-3-azabicyclo[4.1.0]heptan-7-yl)methyl)isoindoline-1,3-dione (prepared as described in step 1 of the synthesis of **Example 94;** 25 mg, 0.038 mmol) and (2-(trifluoromethyl)pyridin-3-yl)boronic acid (14 mg, 0.07 mmol) were reacted according to the procedure described in step 2 of the synthesis of **Example 77.** The mixture was diluted with toluene (2 mL) and washed with H₂O (3x2 mL). The collected organic layers were concentrated under reduce pressure, affording the desired compound used in the next step without further purification (25 mg). LCMS (ES⁺) Method 1: *m*/*z* 686 (M+H)⁺, RT 2.64 min.

### Step 2: (7-(Thiophen-3-yl)-3-(3-(2-(trifluoromethyl)pyridin-3-yl)-1H-pyrazolo[3,4-b]pyrazin-6-yl)-3-azabicyclo[4.1.0]heptan-7-yl)methanamine

(7-(Thiophen-3-yl)-3-(3-(2-(trifluoromethyl)pyridin-3-yl)-1H-pyrazolo[3,4-b]pyrazin-6-yl)-3-azabicyclo[4.1.0]heptan-7-yl)methanamine was prepared from 2-((3-(1-(tetrahydro-2H-pyran-2-yl)-3-(2-(trifluoromethyl)pyridin-3-yl)-1H-pyrazolo[3,4-b]pyrazin-6-yl)-7-(thiophen-3-yl)-3-azabicyclo[4.1.0]heptan-7-yl)methyl)isoindoline-1,3-dione (12 mg, 0.017 mmol) according to the procedure described in step 3 of the synthesis of **Example 94.** The reaction was directly purified by preparative HPLC (from 5% to 50% MeCN/H₂O + 0.1% TFA) to obtain the title compound (TFA salt; 5.3 mg, 53%). ¹H NMR (DMSO-*d₆*+TFA) δ 8.85 (br d, *J*=4.9 Hz, 1H), 8.40 (s, 1H), 8.27 (br d, *J*=8.0, 1H), 7.87 (dd, *J*=7.9 and 4.7 Hz, 1H), 7.71 (br s, 3H), 7.56 (dd, *J*=5.0 and 2.9 Hz, 1H), 7.41 (m, 1H), 7.16 (dd, *J*=5.0 and 1.2 Hz, 1H), 4.31 (m, 2H), 3.72 (m, 1H), 3.34 (m, 1H), 3.17 (m, 1H), 2.94 (m, 1H), 2.31 (m, 1H), 1.85 (m, 1H), 1.73 (m, 1H), 1.57 (m, 1H); LCMS (ES⁺) Method 2: *m*/*z* 472 (M+H)⁺, RT 2.26 min.

### Example 97: (3-(3-(2,3-Dichlorophenyl)-1H-pyrazolo[3,4-b]pyrazin-6-yl)-7-(1-methyl-1H-pyrazol-5-yl)-3-azabicyclo [4.1.0] heptan-7-yl)methanamine

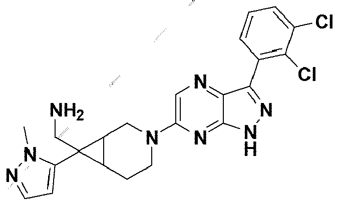

### Step 1: Benzyl ((3-(3-iodo-1-(tetrahydro-2H-pyran-2-yl)-1H-pyrazolo[3,4-b]pyrazin-6-yl)-7-(1-methyl-1H-pyrazol-5-yl)-3-azabicyclo[4.1.0]heptan-7-yl)methyl)carbamate

**Intermediate 34** (24 mg, 0.06 mmol) was reacted with **Intermediate 44** (24 mg, 0.06 mmol) according to the procedure described in step 1 of the synthesis of **Example 77.** The crude title compound was used in the next step without further purification (45 mg). LCMS (ES⁺) Method 1: m/z 669 (M+H)⁺, RT 2.14 min.

### Step 2: Benzyl ((3-(3-(2,3-dichlorophenyl)-1-(tetrahydro-2H-pyran-2-yl)-1H-pyrazolo[3,4-b]pyrazin-6-yl)-7-(1-methyl-1H-pyrazol-5-yl)-3-azabicyclo[4.1.0]heptan-7-yl)methyl)carbamate

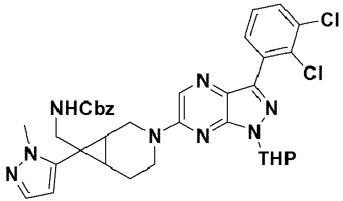

Benzyl ((3-(3-iodo-1-(tetrahydro-2H-pyran-2-yl)-1H-pyrazolo[3,4-b]pyrazin-6-yl)-7-(1-methyl-1H-pyrazol-5-yl)-3-azabicyclo[4.1.0]heptan-7-yl)methyl)carbamate (20 mg, 0.03 mmol) and (2,3-dichlorophenyl)boronic acid (11 mg, 0.06 mmol) were reacted according to the procedure described in step 2 of the synthesis of **Example 77.** The residue was purified by flash chromatography on silica gel (from 0 to 100% EtOAc in petroleum ether) to give the title compound as a yellow oil (12 mg, 58% over two steps). LCMS (ES⁺) Method 1: m/z 687, 689 (M+H)⁺, RT 2.47 min.

### Step 3: (3-(3-(2,3-Dichlorophenyl)-1H-pyrazolo[3,4-b]pyrazin-6-yl)-7-(1-methyl-1H-pyrazol-5-yl)-3-azabicyclo[4.1.0]heptan-7-yl)methanamine

(3-(3-(2,3-Dichlorophenyl)-1H-pyrazolo[3,4-b]pyrazin-6-yl)-7-(1-methyl-1H-pyrazol-5-yl)-3-azabicyclo[4.1.0]heptan-7-yl)methanamine was prepared from benzyl ((3-(3-(2,3-dichlorophenyl)-1-(tetrahydro-2H-pyran-2-yl)-1H-pyrazolo [3,4-b]pyrazin-6-yl)-7-(1-methyl-1H-pyrazol-5-yl)-3-azabicyclo[4.1.0]heptan-7-yl)methyl)carbamate (20 mg, 0.03 mmol) and treated according to the procedure described in step 3 of the synthesis of **Example 77.** The reaction was directly purified by preparative HPLC (from 5% to 45% MeCN/H₂O + 0.1% TFA) to obtain the title compound (TFA salt; 2 mg, 14%). ¹H NMR (300 MHz, DMSO-*d*₆) δ 8.42 (s, 1H), 7.79-7.65 (m, 5H), 7.54-7.38 (m, 2H), 6.26 (d, *J*=1.8 Hz, 1H), 4.53-4.45 (m, 1H), 4.39-4.31 (m, 1H), 3.89 (s, 3H), 3.75-3.66 (m, 1H), 3.21-3.04 (m, 2H), 2.96-2.85 (m, 1H), 2.39-2.31 (m, 1H), 1.91-1.76 (m, 2H), 1.69-1.59 (m, 1H); LCMS (ES⁺) Method 2: m/z 469, 471 (M+H)⁺, RT 2.36 min.

### Example 98: (3-(3-(2,3-dichlorophenyl)-1H-pyrazolo[3,4-b]pyrazin-6-yl)-7-(1-methyl-1H-pyrazol-3-yl)-3-azabicyclo [4.1.0] heptan-7-yl)methanamine

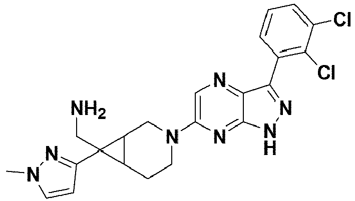

### Step 1: Benzyl ((3-(3-iodo-1-(tetrahydro-2H-pyran-2-yl)-1H-pyrazolo[3,4-b]pyrazin-6-yl)-7-(1-methyl-1H-pyrazol-3-yl)-3-azabicyclo[4.1.0]heptan-7-yl)methyl)carbamate

**Intermediate 33** (17 mg, 0.04 mmol) was reacted with **Intermediate 44** (17 mg, 0.04 mmol) according to the procedure described in step 1 of the synthesis of **Example 77.** The crude title compound was used in the next step without further purification (30 mg). LCMS (ES⁺) Method 1: m/z 669 (M+H)⁺, RT 2.27 min.

### Step 2: Benzyl ((3-(3-(2,3-dichlorophenyl)-1-(tetrahydro-2H-pyran-2-yl)-1H-pyrazolo[3,4-b]pyrazin-6-yl)-7-(1-methyl-1H-pyrazol-3-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(1-methyl-1H-pyrazol-3-yl)-3-azabicyc1o[4.1.0]heptan-7-yl)methyl)carbamate (30 mg, 0.04 mmol) and (2,3-dichlorophenyl)boronic acid (17 mg, 0.09 mmol) were reacted according to the procedure described in step 2 of the synthesis of **Example 77.** The residue was purified by flash chromatography on silica gel (from 0% to 100% EtOAc in petroleum ether) to give the title compound as a yellow oil (6 mg, 19%, over two steps). LCMS (ES⁺) Method 1: m/z 687, 689 (M+H)⁺, RT 2.58 min.

### Step 3: (3-(3-(2,3-dichlorophenyl)-1H-pyrazolo[3,4-b]pyrazin-6-yl)-7-(1-methyl-1H-pyrazol-3-yl)-3-azabicyclo[4.1.0]heptan-7-yl)methanamine

(3-(3-(2,3-dichlorophenyl)-1H-pyrazolo[3,4-b]pyrazin-6-yl)-7-(1-methyl-1H-pyrazol-3-yl)-3-azabicyclo[4.1.0]heptan-7-yl)methanamine was prepared from benzyl ((3-(3-(2,3-dichlorophenyl)-1-(tetrahydro-2H-pyran-2-yl)-1H-pyrazolo[3,4-b]pyrazin-6-yl)-7-(1-methyl-1H-pyrazol-3-yl)-3-azabicyclo[4.1.0]heptan-7-yl)methyl)carbamate (5.5 mg, 0.008 mmol) according to the procedure described in step 3 of the synthesis of **Example 77.** The reaction was directly purified by preparative HPLC (from 5% to 50% MeCN/H₂O + 0.1% TFA) to obtain the title compound (TFA salt; 2 mg, 53%). ¹H NMR (300 MHz, DMSO-*d*₆ +TFA) δ 8.37 (s, 1H), 7.82 - 7.66 (m, 5H), 7.61 (d, *J*=2.2 Hz, 1H), 7.50 (t, *J*=8.2 Hz, 1H), 6.01 (d, *J*=2.3 Hz, 1H), 4.29 (br d, *J*=13.9 Hz, 2H), 3.80 (s, 3H), 3.72 (dd, *J*=14.4, 6.4 Hz, 1H), 3.42-3.31 (m, 1H), 3.27-3.15 (m, 1H), 3.01-2.88 (m, 1H), 1.90-1.68 (m, 3H), 1.66-1.55 (m, 1H); LCMS (ES⁺) Method 2: m/z 469, 471 (M+H)⁺, RT 2.52 min.

### Example 99: (3-(3-(2,3-Dichlorophenyl)-1H-pyrazolo[3,4-b]pyrazin-6-yl)-7-(isoxazol-3-yl)-3-azabicyclo[4.1.0]heptan-7-yl)methanamine

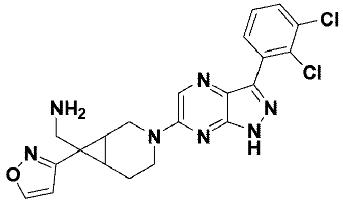

### Step 1: Benzyl ((3-(3-iodo-1-(tetrahydro-2H-pyran-2-yl)-1H-pyrazolo[3,4-b]pyrazin-6-yl)-7-(isoxazol-3-yl)-3-azabicyclo[4.1.0]heptan-7-yl)methyl)carbamate

**Intermediate 35** (22 mg, 0.06 mmol) was reacted with **Intermediate 44** (23 mg, 0.06 mmol) according to the procedure described in step 1 of the synthesis of **Example 77.** The crude title compound was used in the next step without further purification (38 mg). LCMS (ES⁺) Method 1: m/z 656 (M+H)⁺, RT 2.32 min.

### Step 2: Benzyl ((3-(3-(2,3-dichlorophenyl)-1-(tetrahydro-2H-pyran-2-yl)-1H-pyrazolo[3,4-b]pyrazin-6-yl)-7-(isoxazol-3-yl)-3-azabicyclo[4.1.0]heptan-7-yl)methyl)carbamate

Benzyl ((3-(3-iodo-1-(tetrahydro-2H-pyran-2-yl)-1H-pyrazolo[3,4-b]pyrazin-6-yl)-7-(isoxazol-3-yl)-3-azabicyclo[4.1.0]heptan-7-yl)methyl)carbamate (38 mg, 0.06 mmol) and (2,3-dichlorophenyl)boronic acid (22 mg, 0.12 mmol) were reacted according to the procedure described in step 2 of the synthesis of **Example 77.** The residue was purified by flash chromatography on silica gel (from 0 to 100% EtOAc in petroleum ether) to give the title compound as a yellow oil (38 mg, 97%, over two steps). LCMS (ES⁺) Method 1: m/z 674, 676 (M+H)⁺, RT 2.62 min.

### Step 3: (3-(3-(2, 3-Dichlorophenyl)-1H-pyrazolo[3,4-b]pyrazin-6-yl)-7-(isoxazol-3-yl)-3-azabicyclo[4.1.0]heptan-7-yl)methanamine

(3-(3-(2,3-Dichlorophenyl)-1H-pyrazolo[3,4-b]pyrazin-6-yl)-7-(isoxazol-3-yl)-3-azabicyclo[4.1.0]heptan-7-yl)methanamine was prepared from benzyl ((3-(3-(2,3-dichlorophenyl)-1-(tetrahydro-2H-pyran-2-yl)-1H-pyrazolo[3,4-b]pyrazin-6-yl)-7-(isoxazol-3-yl)-3-azabicyclo[4.1.0]heptan-7-yl)methyl)carbamate (39 mg, 0.06 mmol) according to the procedure described in Step 4 of the synthesis of **Example 91.** The reaction was directly purified by preparative HPLC (from 5% to 50% MeCN/H₂O + 0.1% TFA) to obtain the title compound (TFA salt; 6 mg, 22%). ¹H NMR (300 MHz, DMSO-*d*₆ +TFA) δ 8.83 (d, *J*=1.7 Hz, 1H), 8.39 (s, 1H), 7.91 (br s, 3H), 7.75-7.64 (m, 2H), 7.46 (t, *J*=7.9 Hz, 1H), 6.47 (d, *J*=1.7 Hz, 1H), 4.43-4.28 (m, 2H), 3.71 (dd, *J*=14.6, 6.24 Hz, 1H), 3.54-3.41 (m, 1H), 3.32-3.21 (m, 1H), 3.04-2.87 (m, 1H), 2.37-2.30 (m, 1H), 2.07-1.95 (m, 1H), 1.86-1.72 (m, 2H). LCMS (ES⁺) Method 2: m/z 456, 458 (M+H)⁺, RT 2.43 min.

### Example 100: (3-(3-(2,3-Dichlorophenyl)-1H-pyrazolo[3,4-b]pyrazin-6-yl)-3-azabicyclo [4.1.0] heptan-7-yl)methanamine

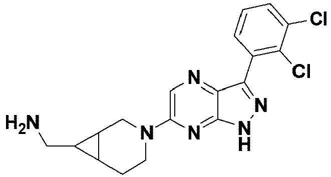

### Step 1: 2-((3-(3-iodo-1-(tetrahydro-2H-pyran-2-yl)-1H-pyrazolo[3,4-b]pyrazin-6-yl)-3-azabicyclo[4.1.0]heptan-7-yl)methyl)isoindoline-1,3-dione

**Intermediate 38** (11 mg, 0.04 mmol) was reacted with **Intermediate 44** (25 mg, 0.05 mmol) according to the procedure described in step 1 of the synthesis of **Example 77.** The crude title compound was used in the next step without further purification (22 mg). LCMS (ES⁺) Method 1: *m*/*z* 584, 586 (M+H)⁺, RT 2.43 min.

### Step 2: 2-((3-(3-(2,3-dichlorophenyl)-1-(tetrahydro-2H-pyran-2-yl)-1H-pyrazolo[3,4-b]pyrazin-6-yl)-3-azabicyclo[4.1.0]heptan-7-yl)methyl)isoindoline-1,3-dione

2-((3-(3-iodo-1-(tetrahydro-2H-pyran-2-yl)-1H-pyrazolo[3,4-b]pyrazin-6-yl)-3-azabicyclo[4.1.0]heptan-7-yl)methyl)isoindoline-1,3-dione (22 mg, 0.039 mmol) and 2,3-dichlorophenylboronic acid (7.4 mg, 0.039 mmol) were reacted according to the procedure described in step 2 of the synthesis of **Example 77.** The residue was purified by flash chromatography on silica gel (from 20% to 100% EtOAc in petroleum ether) to give the title compound (10 mg, 42 %, over two steps). LCMS (ES⁺) Method 1: *m*/*z* 603, 605 (M+H)⁺, RT 2.73 min.

### Step 3: (3-(3-(2,3-Dichlorophenyl)-1H-pyrazolo[3,4-b]pyrazin-6-yl)-3-azabicyclo[4.1.0]heptαn-7-yl)methanamine

(3-(3-(2,3-Dichlorophenyl)-1H-pyrazolo[3,4-b]pyrazin-6-yl)-3-azabicyclo[4.1.0]heptan-7-yl)methanamine was prepared from 2-((3-(3-(2,3-dichlorophenyl)-1-(tetrahydro-2H-pyran-2-yl)-1H-pyrazolo[3,4-b]pyrazin-6-yl)-3-azabicyclo[4.1.0]heptan-7-yl)methyl)isoindoline-1,3-dione (10 mg, 0.017 mmol) according to the procedure described in step 3 of the synthesis of **Example 94.** The reaction was directly purified by preparative HPLC (from 5% to 50% MeCN/H₂O + 0.1% TFA) to afford the title compound (TFA salt; 6.2 mg, 98%). ¹H NMR (300 MHz, DMSO-*d*₆) δ 8.31 (s, 1H), 7.59-7.79 (m, 5H), 7.49 (t, *J*=8.1 Hz, 1H), 4.22 (d, *J*=13.3 Hz, 1H), 3.85-3.68 (m, 2H), 3.38-3.27 (m, 1H), 2.81- 2.66 (m, 2H), 2.14-2.03 (m, 1H), 1.91-1.78 (m, 1H), 1.27-1.12 (m, 2H), 0.89-0.76 (m, 1H). LCMS (ES⁺) Method 2: m/z 389, 391 (M+H)⁺, RT 2.37 min.

### Example 101: (6-(7-(Aminomethyl)-7-(4-methylthiazol-2-yl)-3-azabicyclo[4.1.0]heptan-3-yl)-3-(quinolin-5-yl)-1H-pyrazolo[3,4-b]pyrazin-5-yl)methanol

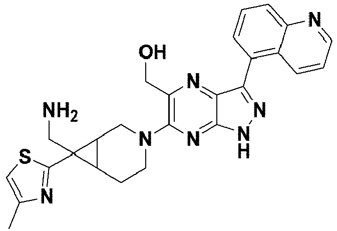

### Step 1: 6-(7-((1,3-Dioxoisoindolin-2-yl)methyl)-7-(4-methylthiazol-2-yl)-3-azabicyclo[4.1.0]heptan-3-yl)-3-iodo-1-(tetrahydro-2H-pyran-2-yl)-1H-pyrazolo[3,4-b]pyrazin-5-yl)methyl acetate

**Intermediate 36** (34 mg, 0.087 mmol) and 6-chloro-3-iodo-1-(tetrahydro-2H-pyran-2-yl)-1H-pyrazolo[3,4-b]pyrazin-5-yl)methyl acetate (prepared as reported in steps 1-5 of **Scheme 45;** 40 mg, 0.092 mmol) were reacted according to the procedure described in step 1 of the synthesis of **Example 77.** The crude title compound was used in the next step without further purification (78 mg). LCMS (ES⁺) Method 1: *m*/*z* 754 (M+H)⁺, RT 2.30 min.

### Step 2: (6-(7-((1,3-Dioxoisoindolin-2-yl)methyl)-7-(4-methylthiazol-2-yl)-3-azabicyclo[4.1.0]heptan-3-yl)-3-(quinolin-5-yl)-1-(tetrahydro-2H-pyran-2-yl)-1H-pyrazolo[3,4-b]pyrazin-5-yl)methyl acetate

(6-(7-((1,3-Dioxoisoindolin-2-yl)methyl)-7-(4-methylthiazol-2-yl)-3-azabicyclo[4.1.0]heptan-3-yl)-3-iodo-1-(tetrahydro-2H-pyran-2-yl)-1H-pyrazolo[3,4-b]pyrazin-5-yl)methyl acetate (43 mg, 0.057 mmol) and quinoline-5-boronic acid (20 mg, 0.114 mmol) were reacted according to the procedure described in step 2 of the synthesis of **Example 77.** The residue was purified by flash chromatography on silica gel (from 50% to 100% EtOAc in petroleum ether) to give the title compound as a yellow oil (31 mg, 72% over two steps). LCMS (ES⁺) Method 1: *m*/*z* 755 (M+H)⁺, RT 1.84 min.

### Step 3:(6-(7-(Aminomethyl)-7-(4-methylthiazol-2-yl)-3-azabicyclo[4.1.0]heptan-3-yl)-3-(quinolin-5-yl)-1H-pyrazolo[3,4-0]pyrazin-5-yl)methanol

(6-(7-((1,3-Dioxoisoindolin-2-yl)methyl)-7-(4-methylthiazol-2-yl)-3-azabicyclo[4.1.0]heptan-3-yl)-3-(quinolin-5-yl)-1-(tetrahydro-2H-pyran-2-yl)-1H-pyrazolo[3,4-b]pyrazin-5-yl)methyl acetate (31 mg, 0.04 mmol) was suspended in EtOH (1 mL) and hydrazine monohydrate (0.06 mL, 1.23 mmol) was added. The reaction mixture was stirred at rt for 5 h, then was filtered and solid washed with DCM (x2). The mother liquors were concentrated under reduced pressure and the residue was taken up in DCM and filtered again. Volatiles were removed *in vacuo* to obtain a yellow foam (27mg). The product was dissolved again in DCM (0.7 mL) and HCl (4N in 1,4-dioxane; 0.25 mL, 1 mmol) was added. The reaction mixture was stirred at rt for 1 h, then solvent was removed under reduced pressure. The residue was direcly purified by preparative HPLC (from 0% to 35% MeCN/H₂O + 0.1% TFA) to obtain the title compound (TFA salt; 13 mg, 52%) as a yellow solid. ¹H NMR (DMSO-*d*₆+TFA) δ 10.09 (d, *J*=8.7 Hz, 1H), 9.41 (dd, *J*=5.2 and 1.4 Hz, 1H), 8.92 (dd, *J*=6.8 and 1.6 Hz, 1H), 8.40-8.24 (m, 2H), 8.18 (dd, *J*=8.7 and 5.2 Hz, 1H), 7.98 (br s, 3H), 7.18 (br d, *J*=1.0 Hz, 1H), 4.70 (s, 2H), 4.01-3.85 (m, 2H), 3.84-3.65 (m, 3H), 2.77 (m, 1H), 2.44-2.25 (m, 4H), 2.15 (m, 1H), 2.01 (m, 1H), 1.95-1.82 (m, 1H); LCMS (ES⁺) Method 2: *m*/*z* 499 (M+H)⁺, RT 1.77 min.

### Example 102: (6-(7-(Aminomethyl)-7-(4-methylthiazol-2-yl)-3-azabicyclo[4.1.0]heptan-3-yl)-3-(2,3-dichlorophenyl)-1H-pyrazolo[3,4-b]pyrazin-5-yl)methanol

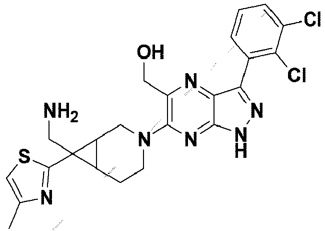

### Step 1: (3-(2, 3-dichlorophenyl)-6-(7-((1, 3-dioxoisoindolin-2-yl)methyl)-7-(4-methylthiazol-2-yl)-3-azabicyclo[4.1.0]heptan-3-yl)-1-(tetrahydro-2H-pyran-2-yl)-1H-pyrazolo[3,4-b]pyrazin-5-yl)methyl acetate

(6-(7-((1,3-Dioxoisoindolin-2-yl)methyl)-7-(4-methylthiazol-2-yl)-3-azabicyclo[4.1.0]heptan-3-yl)-3-iodo-1-(tetrahydro-2H-pyran-2-yl)-1H-pyrazolo[3,4-b]pyrazin-5-yl)methyl acetate (36 mg, 0.048 mmol) and 2,3-dichlorophenylboronic acid (18 mg, 0.096 mmol) were reacted according to the procedure described in step 2 of the synthesis of **Example 77.** The residue was purified by flash chromatography on silica gel (from 30% to 70% EtOAc in petroleum ether) to give the title compound as a yellow oil (25 mg, 68% over two steps). LCMS (ES⁺) Method 1: *m*/*z* 772, 774 (M+H)⁺, RT 2.58 min.

### Step 2: 6-(7-(Aminomethyl)-7-(4-methylthiazol-2-yl)-3-azabicyclo[4.1.0]heptan-3-yl)-3-(2,3-dichlorophenyl)-1H-pyrazolo[3,4-b]pyrazin-5-yl)methanol

6-(7-(Aminomethyl)-7-(4-methylthiazol-2-yl)-3-azabicyclo[4.1.0]heptan-3-yl)-3-(2,3-dichlorophenyl)-1H-pyrazolo[3,4-b]pyrazin-5-yl)methanol was prepared (3-(2,3-dichlorophenyl)-6-(7-((1,3-dioxoisoindolin-2-yl)methyl)-7-(4-methylthiazol-2-yl)-3-azabicyclo[4.1.0]heptan-3-yl)-1-(tetrahydro-2H-pyran-2-yl)-1H-pyrazolo[3,4-b]pyrazin-5-yl)methyl acetate (25 mg, 0.032 mmol) according to the procedure described in step 3 of the synthesis of **Example 101.** The residue was directly purified by preparative HPLC (from 5% to 55% MeCN/H₂O + 0.1% TFA) to obtain the title compound as a yellow solid (TFA salt; 5 mg, 25%). ¹H NMR (DMSO-*d*₆+TFA) δ 7.94 (br s, 3H), 7.74 (m, 2H), 7.51 (t, *J*=7.9 Hz, 1H), 7.17 (br d, *J*=1.0 Hz, 1H) 4.61 (s, 2H), 3.97-3.85 (m, 2H), 3.84-3.62 (m, 3H), 2.79-2.65 (m, 1H), 2.43-2.23 (m, 4H), 2.20-2.05 (m, 1H), 1.98 (m, 1H), 1.92-1.82 (m, 1H); LCMS (ES⁺) Method 2: *m*/*z* 516-518 (M+H)⁺, RT 2.67 min.

### Example 103: 4-(6-(7-(Aminomethyl)-7-(4-methylthiazol-2-yl)-3-azabicyclo [4.1.0] heptan-3-yl)-1H-pyrazolo[3,4-b]pyrazin-3-yl)naphthalen-1-yl sulfurofluoridate

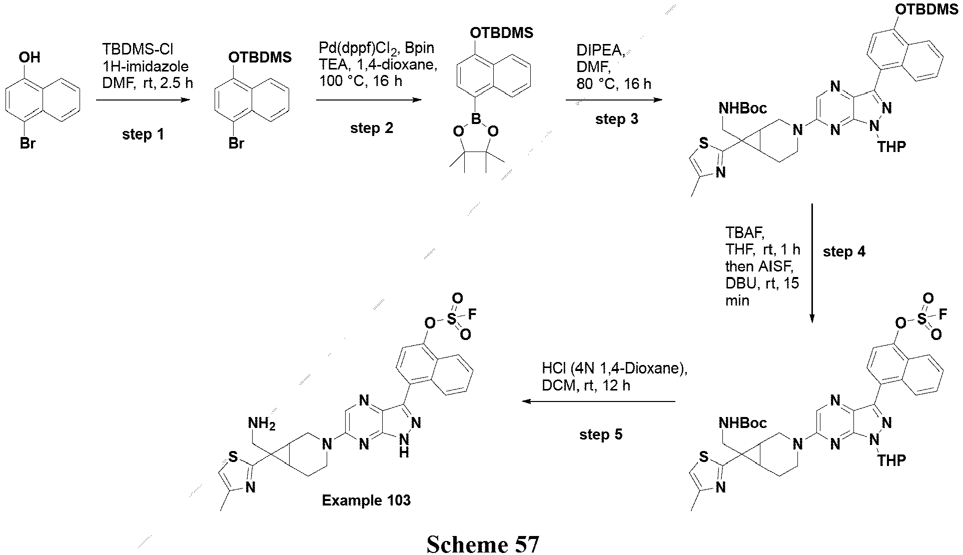

### Step 1: ((4-bromonaphthalen-1-yl)oxy) (tert-butyl)dimethylsilane

To a solution of 4-bromonaphthalen-1-ol (200 mg, 0.90 mmol) and 1*H*-imidazole (74.5 mg, 1.09 mmol) in dry DMF (2 mL, 0.025 mol) at 0 °C, TBDMS-Cl (149 mg, 0.99 mmol) was added. The reaction mixture was stirred at rt for 2.5 h, then was diluted with Et₂O (2 mL) and H₂O (2 mL). The aqueous layer was extracted again with Et₂O (3x3 mL), then combined organic layers were washed with H₂O (x2) and brine (x2), dried over Na₂SO₄, filtered and concentrated *in vacuo.* The resulting crude was purified by flash chromatography on silica gel (100% petroleum ether) to afford the title compound as white crystals (273 mg, 90%). ¹H NMR (300 MHz, CDCl₃) δ 8.16 (m, 2H), 7.62-7.44 (m, 3H), 6.71 (d, *J*=8.2 Hz, 1H), 1.07 (s, 9H), 0.26 (s, 6H)

### Step 2: tert-butyldimethyl((4-(4,4,5,5-tetramethyl-1,3,2-dioxaborolan-2-yl)naphthalen-1-yl)oxy)silane

A mixture of ((4-bromonaphthalen-1-yl)oxy)(tert-butyl)dimethylsilane (140 mg, 0.42 mmol), bis(pinacolato)diboron (211 mg, 0.83 mmol), [1,1'-bis(diphenylphosphino)ferrocene]dichloropalladium(II) complex in DCM (42 mg, 0.05 mmol) and TEA (0.23 mL, 1.66 mmol) in degassed 1,4-dioxane (1.3 mL) was stirred at 100 °C for 16 h. The black suspension was diluted with H₂O (1 mL) and DCM (1 mL), the two phases were separated and the aqueous layer was extracted again with DCM (3x3 mL). The combined organic extracts were washed with brine (x3), dried over Na₂SO₄, filtered and concentrated. The resulting crude was purified by flash chromatography on silica gel (from 0% to 5% EtOAc in petroleum ether) to afford the title compound as a yellow solid (105 mg, 66%). ¹H NMR (300 MHz, CDCl₃) δ 8.72 (br d, *J*=84 Hz, 1H), 8.19 (m, 1H), 7.94 (d, *J*=7.6 Hz, 1H), 7.55-7.38 (m, 2H) 6.83 (d, *J*=7.6 Hz, 1H), 1.39 (s, 12H), 1.07 (s, 9H), 0.27 (s, 6H); LCMS (ES⁺) Method 3: *m*/*z* 385 (M+H)⁺, RT 2.77 min.

### Step 3: tert-butyl ((3-(3-(4-((tert-butyldimethylsilyl)oxy)naphthalen-1-yl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate

*tert*-Butyl ((3-(3-iodo-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (prepared as described in step 2 of the synthesis of **Example 91;** 20 mg, 0.031 mmol) and *tert*-butyldimethyl((4-(4,4,5,5-tetramethyl-1,3,2-dioxaborolan-2-yl)naphthalen-1-yl)oxy)silane (23.6 mg, 0.061 mmol) were prepared according to the procedure described in step 2 of the synthesis of **Example 77.** The residue was purified by flash chromatography on silica gel (from 10% to 50% EtOAc in petroleum ether) to afford the title compound as a yellow oil (10 mg, 42%). LCMS (ES⁺) Method 3: *m*/*z* 782, 783 (M+H)⁺, RT 2.67 min.

### Step 4: 4-(6-(7-(((tert-butoxycarbonyl)amino)methyl)-7-(4-methylthiazol-2-yl)-3-azabicyclo[4.1.0]heptan-3-yl)-1-(tetrahydro-2H-pyran-2-yl)-1H-pyrazolo[3,4-b]pyrazin-3-yl)naphthalen-1-yl sulfurofluoridate

*tert*-Butyl ((3-(3-(4-((tert-butyldimethylsilyl)oxy)naphthalen-1-yl)-1-(tetrahydro-2H-pyran-2-yl)-1H-pyrazolo[3,4-b]pyrazin-6-yl)-7-(4-methylthiazol-2-yl)-3-azabicyclo[4.1.0]heptan-7-yl)methyl)carbamate (10 mg, 0.010 mmol) was dissolved in THF (0.5 mL) and TBAF (1M solution in THF; 0.03 mL, 0.030 mmol) was added. The resulting dark-red solution was stirred at rt for 1 h, then H₂O and saturated NH₄Cl solution were added and the mixture was extracted with EtOAc (x3). The organic layer was dried over Na₂SO₄, filtered and concentrated *in vacuo* to obtain *tert*-butyl ((3 -(3 -(4-hydroxynaphthalen-1 -yl)-1 -(tetrahydro-2H-pyran-2-yl)-1H-pyrazolo [3,4-b]pyrazin-6-yl)-7-(4-methylthiazol-2-yl)-3-azabicyclo[4.1.0]heptan-7-yl)methyl)carbamate as yellow oil (11 mg). The residue was dissolved in dry THF (0.5 mL) and 4-(acetylamino)phenyl]imidodisulfuryl difluoride (4.8 mg, 0.020 mmol), DBU (0.004 mL, 0.030 mmol) were added, then the reaction mixture was stirred at rt for 15 min. EtOAc (1 mL) and H₂O (1 mL) were added, phases were separated, and the organic layer was washed with 0.5N HCl (x3) and brine (x2). The organic phase was dried over Na₂SO₄, filtered and concentrated *in vacuo* to afford crude title compound (12 mg), which was used in the next step without further purification. LCMS (ES⁺) Method 3: *m*/*z* 750 (M+H)⁺, RT 2.20 min.

### Step 5: 4-(6-(7-(Aminomethyl)-7-(4-methylthiazol-2-yl)-3-azabicyclo[4.1.0]heptan-3-yl)-1H-pyrazolo[3,4-b]pyrazin-3-yl)naphthalen-1-yl sulfurofluoridate

4-(6-(7-(Aminomethyl)-7-(4-methylthiazol-2-yl)-3-azabicyclo[4.1.0]heptan-3-yl)-1H-pyrazolo[3,4-b]pyrazin-3-yl)naphthalen-1-yl sulfurofluoridate prepared starting from crude 4-(6-(7-(((tert-butoxycarbonyl)amino)methyl)-7-(4-methylthiazol-2-yl)-3-azabicyclo[4.1.0]heptan-3-yl)-1-(tetrahydro-2H-pyran-2-yl)-1H-pyrazolo[3,4-b]pyrazin-3-yl)naphthalen-1 -yl sulfurofluoridate (9.6 mg; 0.013 mmol) according to the procedure described in step 4 of the synthesis of **Example 91.** The residue was purified by preparative HPLC (from 15% to 65% MeCN/H₂O + 0.1% TFA) to obtain the title compound as yellow solid (TFA salt; 4.5 mg, 51% over three steps). ¹H NMR (DMSO-*d*₆+TFA) δ 8.89 (d, *J*=8.5 Hz, 1H), 8.46 (s, 1H), 8.30 (d, *J*=8.2 Hz, 1H), 8.17 (d, *J*=8.3 Hz, 1H), 8.05-7.88 (m, 4H), 7.87-7.71 (m, 2H), 7.18 (br d, *J*=1.0 Hz, 1H), 4.49-4.27 (m, 2H), 3.78 (dd, *J*=14.5 and 6.3 Hz, 1H), 3.52 (m, 1H), 3.32 (m, 1H), 3.00 (m, 1H), 2.44-2.30 (m, 4H), 2.04 (m, 1H), 1.93-1.75 (m, 2H); LCMS (ES⁺) Method 2: *m*/*z* 566 (M+H)⁺, RT 3.09 min.

### Example 104: 6-(6-(aminomethyl)-6-phenyl-3-azabicyclo[3.1.0]hexan-3-yl)-3-((2-(trifluoromethyl)pyridin-3-yl)thio)pyrazin-2-amine

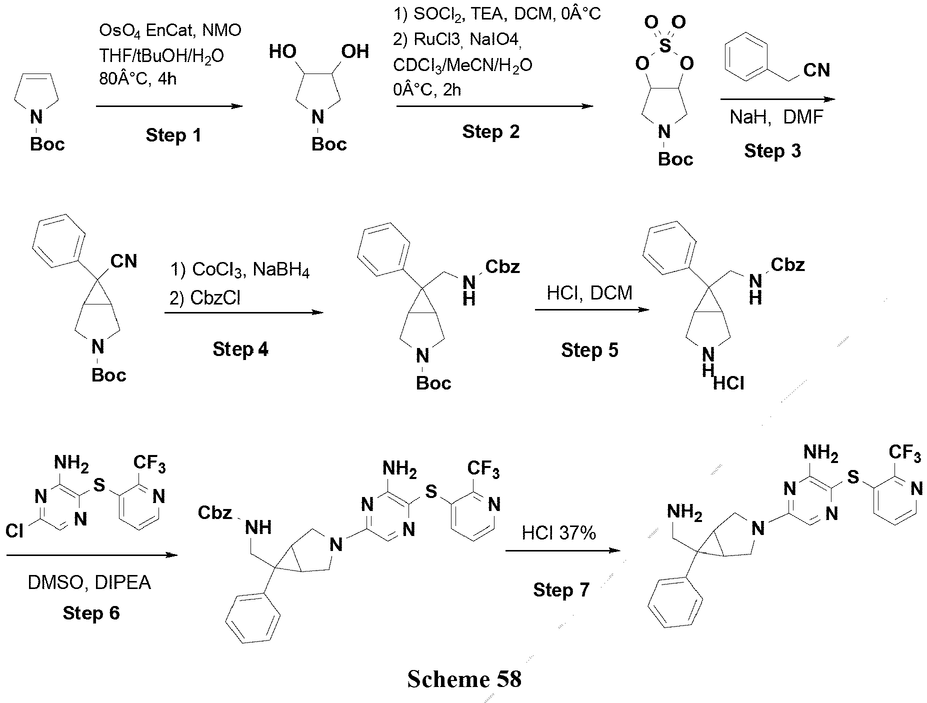

### Step 1: tert-butyl 3,4-dihydroxypyrrolidine-1-carboxylate

OsO4 En-Cat^{®} (500 mg, 0.10 mmol) was added to a solution of tert-butyl 2,5-dihydro-1H-pyrrole-1-carboxylate (450 mg, 2.66 mmol) and 4-methylmorpholine *N*-oxide (475 mg, 3.99 mmol) in THF (2.5 mL), *tert*-BuOH (1.7 mL) and H₂O (0.7 mL). The mixture was stirred at 80 °C for 4 h, then OsO4 En-Cat^{®} was filtered and washed with THF and H₂O. Solvents were removed under reduced pressure, EtOAc was added and the organic phase was washed with brine (x2), dried over Na₂SO₄, filtered and concentrated *in vacuo.* The resulting crude was purified by flash chromatography on silica gel (eluting with 50-100% of EtOAc in petroleum ether) to obtain the title compound as a colourless oil (486 mg, 90%). LCMS (ES⁺) Method 1: *m*/*z* 204 (M+H)⁺, RT 1.04 min.

### Step 2: tert-butyl tetrahydro-5H-[1,3,2]dioxathiolo[4,5-c]pyrrole-5-carboxylate 2,2-dioxide

TEA (1.33 mL, 9.56 mmol) and thionyl chloride (0.26 mL, 3.59 mmol) were added to a solution of tert-butyl 3,4-dihydroxypyrrolidine-1-carboxylate (486 mg, 2.39 mmol) in DCM (12 mL) cooled to 0°C. The mixture was stirred at this temperature for 15 min. After quenching the reaction by addition of H₂O (0.6 mL), the mixture was diluted with Et₂O and additional H₂O. The organic phase was washed with H₂O, NaHCO₃ sat. sol. and brine, dried over Na₂SO₄, filtered and concentrated *in vacuo* to obtain a dark solid (600 mg). The residue was dissolved in chloroform (7 mL), MeCN (7 mL) and H₂O (10.5 mL), then ruthenium(III) chloride (14.9 mg, 0.07 mmol) and sodium periodate (1024 mg, 4.79 mmol) were added at 0 °C. The mixture was stirred at this temperature for 2 h, then was diluted with Et₂O and washed with H₂O and brine. The organic layer was dried over Na₂SO₄, filtered and concentrated *in vacuo.* The resulting crude was purified by flash chromatography on silica gel (eluting with 40-60% of EtOAc in petroleum ether) to afford the title compound as a white solid (546 mg, 86%). LCMS (ES⁺) Method 1: *m*/*z* 266 (M+H)⁺, RT 1.75 min.

### Step 3: tert-butyl 6-cyano-6-phenyl-3-azabicyclo[3.1.0]hexane-3-carboxylate

tert-butyl tetrahydro-5H-[1,3,2]dioxathiolo[4,5-c]pyrrole-5-carboxylate 2,2-dioxide (258 mg, 0.87 mmol) and benzyl cyanide (125 mg, 1.07 mmol) were dissolved in dry DMF (2.2 mL) and the resulting solution was added dropwise over a period of 30 min to a suspension of sodium hydride (60% in mineral oil; 97 mg, 2.43 mmol) in dry DMF (1.4 mL) cooled to 0 °C under N₂ flow. The reaction mixture was stirred at 0°C for 20 min. then was quenched by addition of brine and diluted with EtOAc. The organic layer was washed with H₂O and brine, dried over Na₂SO₄, filtered and concentrated *in vacuo.* The resulting crude was purified by flash chromatography on silica gel (eluting with 10-30% of EtOAc in petroleum ether) to obtain the title compound as a white solid (255 mg, 92%). ¹H NMR (300 MHz, CDCl₃) δ 7.44-7.26 (m, 5H), 4.07-3.89 (dd, *J* = 12.0 and 23.4 Hz, 2H), 3.83-3.69 (m, 2H), 2.40-2.29 (m, 2H), 1.51 (s, 9H); LCMS (ES⁺) Method 1: *m*/*z* 285 (M+H)⁺, RT 2.08 min.

### Step 4: tert-butyl 6-((((benzyloxy)carbonyl)amino)methyl)-6-phenyl-3-azabicyclo[3.1.0]hexane-3-carboxylate

tert-butyl 6-cyano-6-phenyl-3-azabicyclo[3.1.0]hexane-3-carboxylate (100 mg, 0.35 mmol) and cobalt(II) chloride (182 mg, 1.40 mmol) were dissolved in MeOH (5 mL) in a pressure flask, and the dark solution was cooled to 0°C. Sodium borohydride (560 mg, 14.8 mmol) was added portionwise, the flask was sealed, and the reaction mixture was vigorously stirred overnight at rt. The solvent was removed *in vacuo* then the residue was dissolved in DCM (10 mL), the solution was cooled to 0 °C and benzyl chloroformate (0.062 mL, 0.40 mmol) and TEA (0.07 mL, 0.49 mmol) were sequentially added. The reaction mixture was stirred at 0 °C for 30 min. DCM and H₂O were added then the organic layer was washed with 5% citric acid solution. The organic phase was dried over Na₂SO₄, filtered and concentrated under reduced pressure. The resulting crude was purified by flash chromatography on silica gel (eluting with 0-20% of EtOAc in DCM) affording the title compound as a colorless oil (45 mg, 30%). ¹H NMR (300 MHz, CDCl₃) δ 7.35-7.06 (m, 10H), 4.95 (s, 2H), 4.54-4.35 (br s, 1H), 3.71-3.45 (m, 5H), 3.44-3.29 (m, 1H), 1.96-1.88 (m, 2H), 1.41 (s, 9H); LCMS (ES⁺) Method 1: *m*/*z* 423 (M+H)⁺, RT 2.37 min.

### Step 5: benzyl ((6-phenyl-3-azabicyclo[3.1.0]hexan-6-yl)methyl)carbamate

tert-butyl 6-((((benzyloxy)carbonyl)amino)methyl)-6-phenyl-3-azabicyclo[3.1.0]hexane-3-carboxylate (44 mg, 0.10 mmol) was dissolved in DCM (1 mL) and treated with HCl (4N in dioxane 1 mL, 4.0 mmol). The solution was stirred at RT for 1.5 h, then the solvent was removed *in vacuo* to afford the title compound that was used in the next step without further purification (42 mg). LCMS (ES⁺) Method 1: *m*/*z* 323 (M+H)⁺, RT 1.37 min.

### Step 6: benzyl ((3-(6-amino-5-((2-(trifluoromethyl)pyridin-3-yl)thio)pyrazin-2-yl)-6-phenyl-3-azabicyclo[3.7.0]hexan-6-yl)methyl)carbamate

DIPEA (0.06 mL, 0.326 mmol) was added to a solution of (**Intermediate 41;** 20 mg, 0.065 mmol) and benzyl ((6-phenyl-3-azabicyclo[3.1.0]hexan-6-yl)methyl)carbamate hydrochloride (35 mg, 0.098 mmol) in DMSO (0.5 mL). The reaction mixture was heated at 120 °C for 3.5 h then concentrated and directly purified by preparative HPLC (eluting with 35-80% H₂O/MeCN + 0.1% TFA) to afford the title compound as off-white solid (25 mg, 65%). ¹H NMR (300 MHz, DMSO-*d*₆) δ 8.50-8.42 (br d, *J*= 4.3 Hz, 1H), 7.60-7.47 (m, 1H), 7.43-7.14 (m, 12H), 6.44-5.94 (br s, 2H), 4.91 (s, 2H), 3.91-3.79 (m, 2H), 3.78-3.66 (m, 2H), 3.35-3.24 (m, 2H), 2.13 (s, 2H); LCMS (ES⁺) Method 1: *m*/*z* 593 (M+H)⁺, RT 2.40 min.

### Step 7: 6-(6-(aminomethyl)-6-phenyl-3-azabicyclo[3.1.0]hexan-3-yl)-3-((2-(trifluoromethyl)pyridin-3-yl)thio)pyrazin-2-amine

benzyl ((3-(6-amino-5-((2-(trifluoromethyl)pyridin-3-yl)thio)pyrazin-2-yl)-6-phenyl-3-azabicyclo[3.1.0]hexan-6-yl)methyl)carbamate (25 mg, 0.040 mmol) was dissolved in 37% aq. HCl (0.7 mL) and the resulting yellow solution was stirred at rt overnight. The reaction was directly purified by preparative HPLC (eluting with 10-50% H₂O/MeCN + 0.1%TFA) to afford the title compound as light-yellow solid TFA salt (20 mg, 83%). ¹H NMR (300 MHz, DMSO-*d*₆) δ 8.52-8.44 (br d, *J* = 4.29 Hz, 1H), 7.82-7.63 (br s, 3H), 7.60-7.53 (m, 1H), 7.52-7.38 (m, 4H), 7.37-7.27 (m, 3H), 6.35-6.22 (br s, 2H), 3.78 (s, 4H), 3.13-3.02 (m, 2H), 2.28 (s, 2H); LCMS (ES⁺) Method 2: *m*/*z* 459 (M+H)⁺, RT 2.48 min.

### Example 105: 4-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)-3-chloropyridin-2-amine

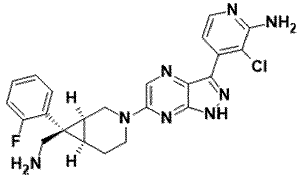

The above example was synthesized using the procedure reported for **Example 2 (Scheme 43)** using **Intermediates 51** for step 1 and **Intermediate 17** for step 2; **Example 105** was obtained as a yellow powder (TFA salt; 3.7 mg, 96% pure, 19%). ¹H NMR (400 MHz, DMSO-*d*₆) δ 13.57 (br s, 1H), 8.46 (s, 1H), 8.01 (d, *J*=5.3 Hz, 1H), 7.66 (br s, 3H), 7.49 (br t, *J*=7.8 Hz, 1H), 7.40-7.35 (m, 1H), 7.25-7.19 (m, 2H), 7.07 (d, *J*=5.0 Hz, 1H), 6.64 (br s, 2H), 4.45 (br d, *J*=14.5 Hz, 1H), 4.33 (br d, *J*=12.3 Hz, 1H), 3.75-3.70 (m, 1H), 3.15-3.05 (m, 2H), 2.98-2.90 (m, 1H), 2.36-2.31 (m, 1H), 1.84-1.75 (m, 2H), 1.57-1.52 (m, 1H); LCMS (ES⁺) Method 2: m/z 465 (M+H)⁺, RT 1.16 min.

### Example 106: ((1S,6R,7R)-7-(2,6-difluorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine

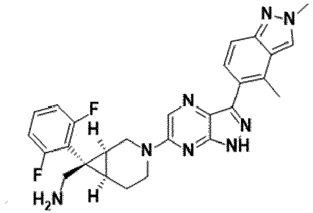

The above example was synthesized using the procedure reported for **Example 2 (Scheme 43)** using **Intermediates 52** for step 1 and **Intermediate 53** for step 2; **Example 106** was obtained as a white powder (TFA salt; 4.0 mg, 38%). ¹H NMR (400 MHz, DMSO-*d*₆) δ 13.17 (br s, 1H), 8.50 (s, 1H), 8.44 (s, 1H), 7.76-7.72 (br m, 4H), 7.50 (d, *J*=8.2 Hz, 1H), 7.47-7.40 (m, 1H), 7.12 (t, *J*=8.3 Hz, 2H), 4.49 (br d, *J*=14.0 Hz, 1H), 4.36 (br d, *J*=8.3 Hz, 1H), 4.19 (s, 3H), 3.75-3.70 (m, 1H), 3.17-3.04 (m, 2H), 2.96-2.85 (m, 1H), 2.64 (s, 3H), 2.39-2.28 (m, 1H), 1.91-1.79 (m, 2H), 1.62-1.55 (m, 1H); LCMS (ES⁺) Method 2: m/z 501 (M+H)⁺, RT 1.15 min.

### Biology

### SHP2 inhibition enzymatic assay

The assay was performed as described in YP Chen *et al,* Nature (535)2016. Assay volume of 20µL/well was assembled in 384 well black polystyrene low-binding microplates (Greiner), using the following buffer: 60 mM HEPES pH 7.2, 75 mM NaCl, 75 mM KCl, 1 mM EDTA pH 8, 0.05% tween-20, 5 mM DTT. The SHP-2 enzyme (synthetized by Origene, Met1 - Leu525, cat#TP750155) was used at a final concentration of 0.5 nM. The enzyme was activated by 500 nM IRS1 peptide (sequence: H2N-LN(pY)IDLDLV(dPEG8)LST(pY)ASINFQK-amide SEQ ID No. 1) and incubated with 75 µM DiFMUP (Sigma) as substrate.

Briefly, DMSO serially diluted testing compounds were transferred to the bottom of the assay plate. SHP2 was then added together with the IRS1 peptide. 30 minutes post incubation, the DiFMUP substrate was added to the reaction and incubated 30 min at room temperature. Finally 5µL of 160 µM bpV (Potassium bisperoxo[1,10-phenanthroline]oxovanadate [V], Sigma) were added to stop and quench the reaction. The fluorescence was detected by a microplate reader (Envision, PerkinElmer) according to the DiFMUP excitation and emission wavelength. The lower the fluorescence the higher the SHP2 inhibition.

The activity of each compound dilution was calculated as percentage of inhibition between vehicle (DMSO, 0% inhibition) and no enzyme (100% inhibition). The percentage inhibition is fitted against the compound dilutions with a four-paramenter logistic regression. The inflection point (i.e. the concetration at which half-maximal inhibiton is achieved) is the IC₅₀.

The IC₅₀ results of the compounds of the invention in the SHP2 inhibition enzymatic assay are shown in Table 2. Legend: A indicates IC₅₀ less or equal to 0.5 µM; B indicates IC₅₀ greater than 0.5 µM and lower or equal to 3 µM; C indicates IC₅₀ higher than 3 µM.

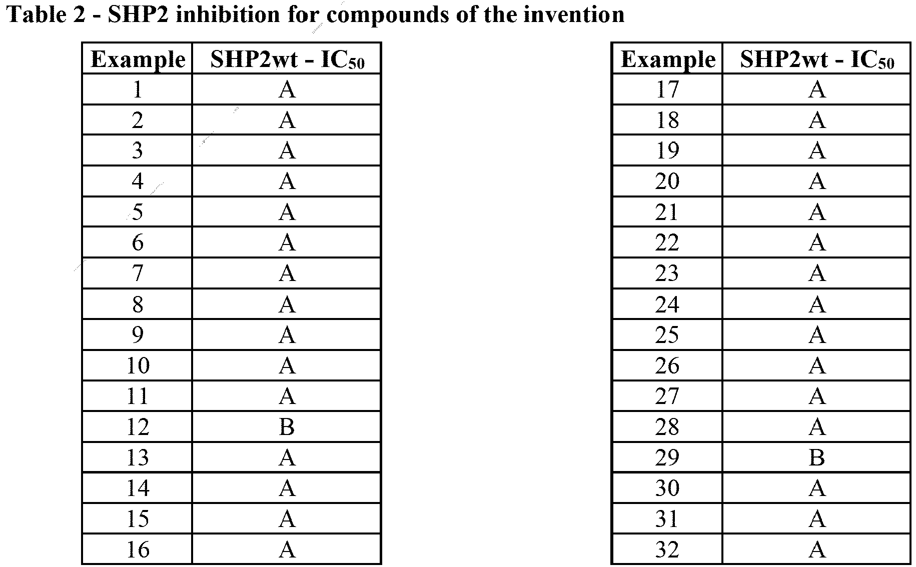

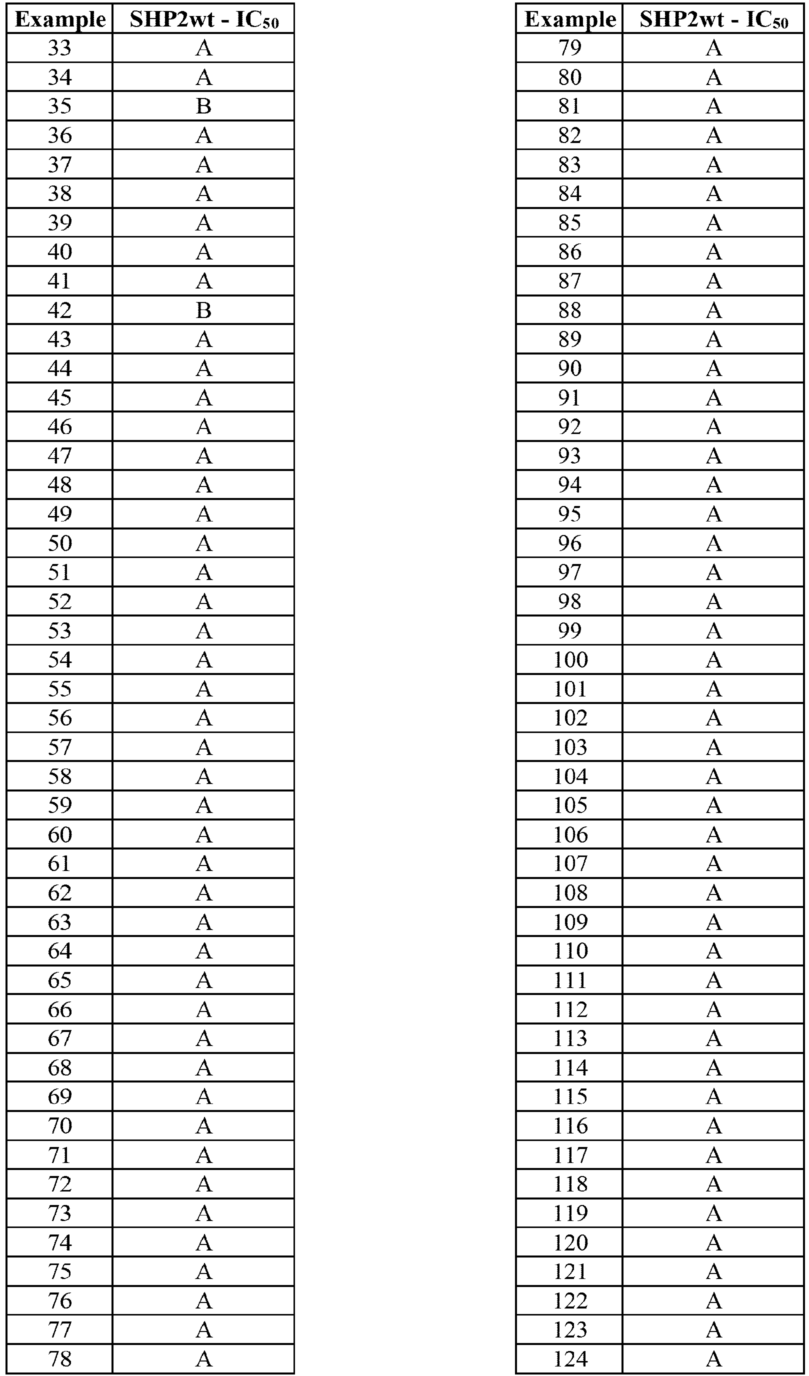

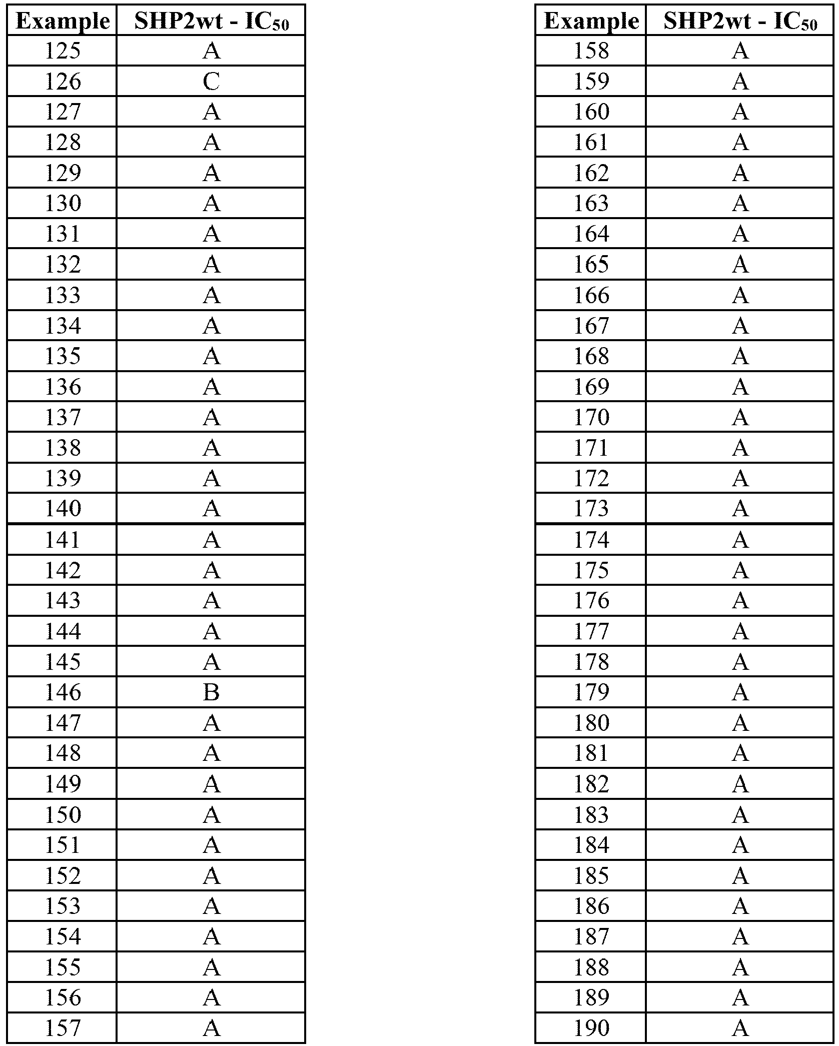

### Phospho-ERK cellular assay

ERK phosphorylation was detected using the "Advanced phospho-ERK1/2 (Thr202/Tyr204)" TR-FRET kit (Cisbio, Cat #64AERPEG/H), following the manufacturer reagents and instructions.

Briefly, 20.000/well KYSE-520 cells (DSMZ ACC 371) were plated in 6 µL RPMI-1640 (Invitrogen) growth medium, into 384 white low-volume high base TC microplates (Greiner). After an overnight incubation, cells were treated with DMSO serial diluted compounds and incubated for 2 h at 37 °C. After incubation, 2 µL/well of 4X Lysis Buffer (Cisbio 64KL1FDF), were added and incubated with cells for 30 min on gentle shaking. Finally, lysates were added with 2 µL/well of Eu cryptate (donor) and D2 (acceptor) conjugated antibodies (as provided by the Cisbio kit #64AERPEG/H) diluted 1:100 in Detection Buffer (as provided by the Cisbio kit #64AERPEG/H). Plates were then sealed and incubated at room temperature in the dark. After an overnight incubation, the TR-FRET signal was detected on a suitable reader (Envision, PerkinElmer). The lower the TR-FRET signal the higher the higher the SHP2 inhibition in cells. The activity of each compound dilution was calculated as percentage between vehicle DMSO treated cells and no cells, 0% inhibition and 100% inhibition respectively. The percentage activity is fitted against the compound dilutions with a four-paramenter logistic regression. The inflection point (i.e. the concetration at which half-maximal inhibiton is achieved) is the IC₅₀.

The IC₅₀ results of the compounds of the invention in the phospho-ERK cellular assay are shown in Table 3. Legend: "+" indicates IC₅₀ equal or higher than 5µM; "++" indicates IC₅₀ less than 5µM and higher or equal to 1µM; "+++" indicates IC₅₀ less than 1µM.

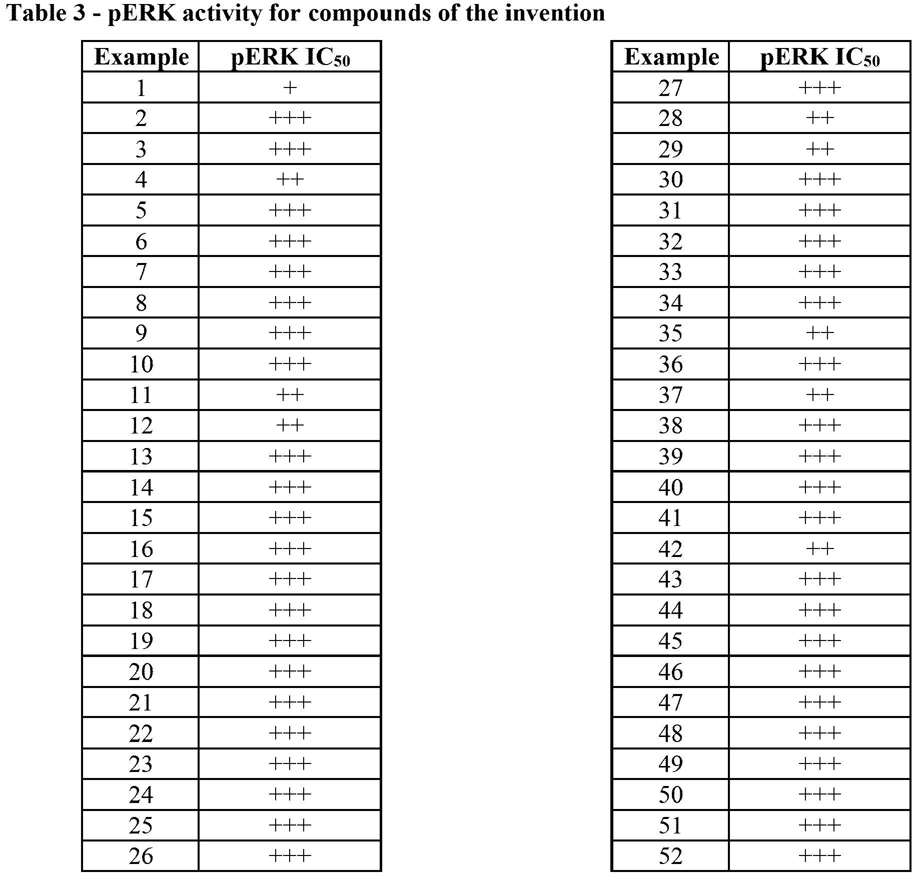

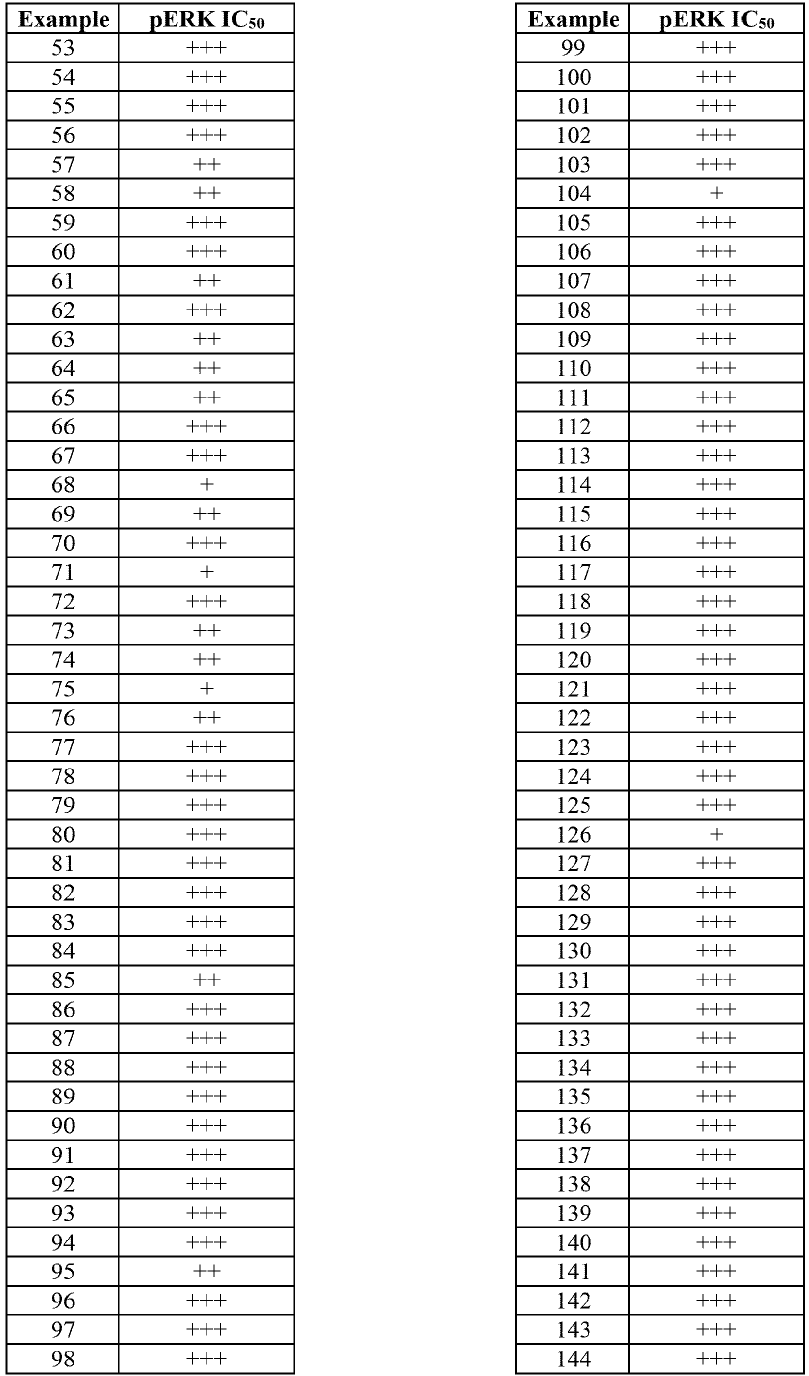

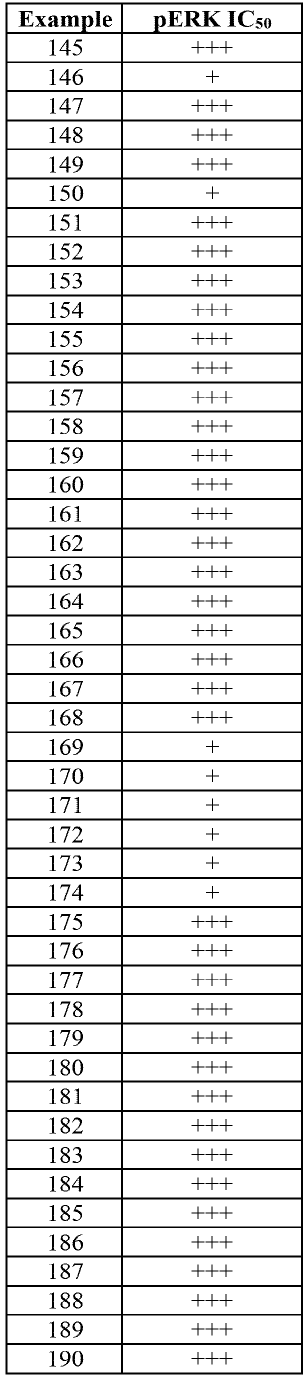

## Claims

1. A compound of Formula (I): 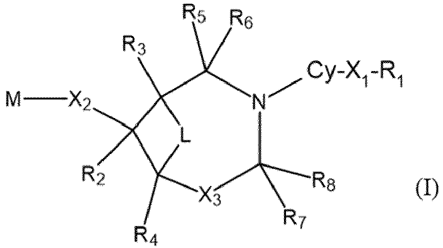 Wherein
X₁ is S, O, CR₁ₐR_{1b}, NH, N-CH₃ or a single bond;
X₂ is (CR₂ₐR_{2b})ₘ;
X₃ is (CR₃ₐR_{3b})ₙ;
M is NH₂, NH-C₁₋₆alkyl, N(C₁₋₆alkyl)₂, NHC(=O)Rₓ or NHC(=O)ORₓ wherein Rₓ is C₁₋₆alkyl, aryl, heteroaryl or C₁₋₆alkyl-aryl;
L is a bond or C₁₋₃alkyl;
Cy is a ring of general formula (A): 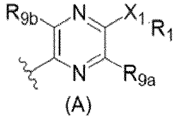 wherein
- R₉ₐ is selected from H, NH₂, C₁₋₆alkyl, halogen, cyano, C₃₋₅cycloalkyl, haloC₁₋₆alkyl, hydroxy-C₁₋₆alkyl or aminoC₁₋₆alkyl;
- R_{9b} is selected from H, C₁₋₆alkyl, halogen, haloC₁₋₆alkyl, hydroxy-C₁₋₆alkyl or aminoC₁₋₆alkyl;
or Cy is a bicyclic ring of general formula (B): 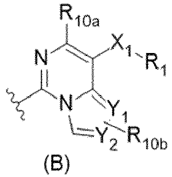 wherein
- Y₁ and Y₂ are C or N;
- R₁₀ₐ is H or C₁₋₆alkyl;
- R_{10b} is at each occurrence independently H, C₁₋₆alkyl, NH₂, CN, OH, haloC₁₋₃alkyl, C₁₋₃alkoxy, haloC₁₋₃alkoxy, or halogen;
or Cy is a bicyclic ring of general formula (C): 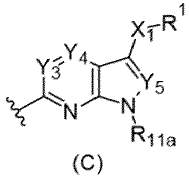 wherein
- Y₃ is C-R_{11b} or N;
- Y₄ is C-R_{11b} or N; or Y₃ is N-CH₃ and Y₄ is C(=O);
- Y₅ is C-R_{11c} or N;
- 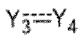 is a double bond when Y₃ is C-R_{11b} or N and when Y₄ is C-R_{11b} or N; Y₃=Y₄ is a single bond when Y₃ is N-CH₃ and Y₄ is C(=O);
- R₁₁ₐ is H or methyl;
- R_{11b} is H, C₁₋₆alkyl, hydroxyC₁₋₆alkyl;
- R_{11c} is H, C₁₋₆alkyl, halogen;
or Cy is a bicyclic ring of general formula (D): 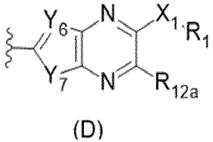 wherein
- Y₆ is N, CH or C-CH₃;
- Y₇ is N-R_{12b}, O or S;
- R₁₂a is H, C₁₋₆alkyl, halogen or cyano;
- R_{12b} is H or C₁₋₆alkyl;
or Cy is a bicyclic ring of general formula (E): 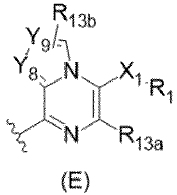 wherein
- Y₈ and Y₉ are C or N;
- R₁₃ₐ is H, C₁₋₆alkyl, halogen, NH₂ or cyano;
- R_{13b} is at each occurency independently H, CH₃ or hydroxyC₁₋₆alkyl;
R₁ is an aryl, heteroaryl, partially unsaturated aryl, partially unsaturated heteroaryl or an heterocyclic ring, each of said aryl, heteroaryl, partially unsaturated aryl, partially unsaturated heteroaryl or heterocyclic ring being optionally substituted with one or more substituents independently selected from C(O)CH₃, C(O)OCH₃, heteroaryl, aryl, OH, halogen, NH₂, NH-C₁₋₆alkyl, N(C₁₋₆alkyl)₂, C₁₋₆alkyl, C₃₋₅cycloalkyl, C₁₋₆alkyl-NH₂, C₁₋₆alkyl-NH(CH₃), C₁₋₆alkyl-NH(CH₃)₂, C₁₋₆alkyl-NHCOCH₃, C₁₋₃alkylaryl, C₁₋₃alkylheteroaryl, haloC₁₋₆alkyl, hydroxyC₁₋₆alkyl, CN, haloC₁₋₆alkoxy, C₁₋₆alkoxy, C₅₋₇heterocycloalkoxy, oxetane, OCF₃, CH₂OCH₃, OSO₂F, SO₂F and a cyclic amine selected from the group consisting of: pyrrolidine, piperidine, morpholine, thiomorpholine, piperazine, N-methylpyperazine and pyrrolidin-3-yloxy;
R₂ is H, aryl, heteroaryl, C₃₋₉cycloalkyl, partially unsaturated heteroaryl, C₁₋₃alkylaryl, C₁₋₃alkylheteroaryl, each of said aryl, heteroaryl, C₃₋₉cycloalkyl, partially unsaturated heteroaryl, C₁₋₃alkylaryl, C₁₋₃alkylheteroaryl being optionally substituted with one ore more substituents independently selected from the group consisting of: halogen, CN, C₁₋₃alkyl, C₃₋₈cycloalkyl, haloC₁₋₃alkyl, C₁₋₃alkoxy, haloC₁₋₃alkoxy, C₁₋₃alkyl-N(R_{y})₂, C(O)OC₁₋₃alkyl, C(O)C₁₋₃alkyl, OH, hydroxy-C₁₋₃alkyl, C₁₋₃alkyl-OC₁₋₃alkyl, CON(R_{y})₂, N(R_{y})₂ wherein each R_{y} is independently selected from H, methyl, ethyl and cyclopropyl;
R₁ₐ and R_{1b} are independently selected from H, C₁₋₆alkyl or R₁ₐ and R_{1b} are joined together to form together with the carbon atom to which they are linked a spiro-C₃₋₅cycloalkyl ring;
R₂ₐ and R_{2b} are independently selected from H, halogen, C₁₋₆alkyl, C₃₋₅cycloalkyl or R₃ₐ and R_{3b} are joined together to form together with the carbon atom to which they are linked a spiro-C₃₋₅cycloalkyl ring;
R₃, R₃ₐ, R_{3b}, R₄, R₅, R₆, R₇ and R₈ are at each occurrence independently selected from H, methyl or halogen;
m is 0, 1, 2 or 3;
n is 0, 1 or 2, with the proviso that if n is 0 then Cy has general structure (A), (C) or (D) or (E); or a pharmaceutically acceptable salt, tautomer, solvate, or stereoisomer thereof.

2. The compound according to claim 1 having general formula (II), (III), (IV), (V), (VI), (VII), (VIII) or (IX): 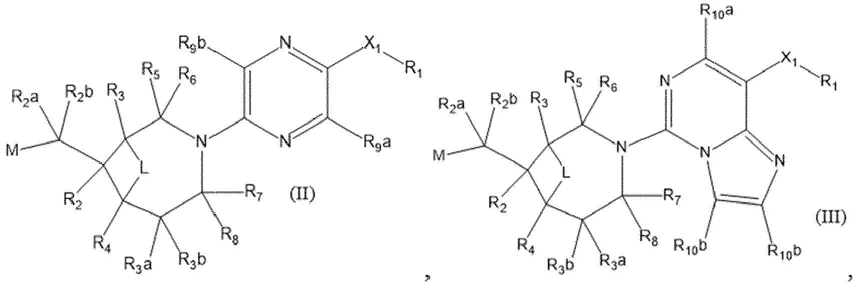 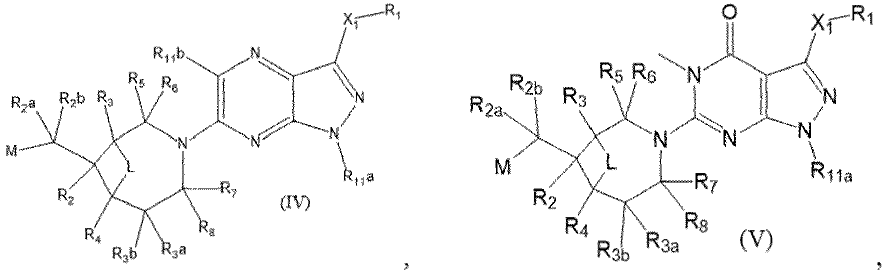 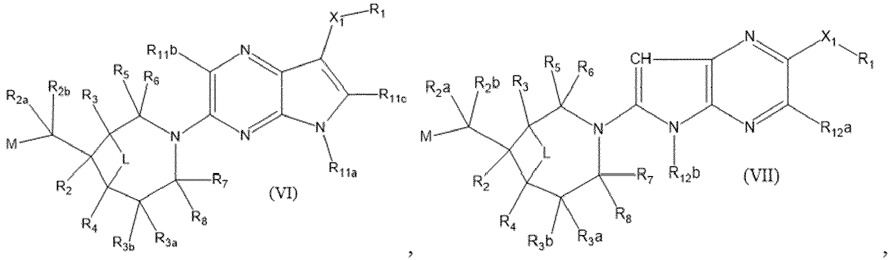 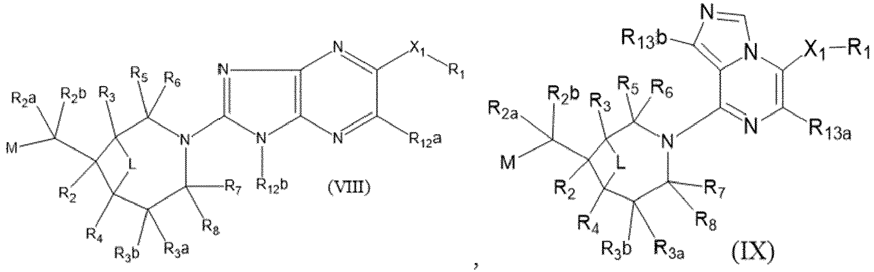 wherein: X₁, R₁, R₂, R₂ₐ, R_{2b}, R₃, R₃ₐ, R_{3b}, R₄, R₅, R₆, R₇, R₈, R₉ₐ, R_{9b}, R₁₀ₐ, R_{10b}, R₁₁ₐ, R_{11b}, R₁₂ₐ, R_{12b}, R₁₃ₐ, R_{13b}, M and L are as defined in claim 1; or a pharmaceutically acceptable salt, tautomer, solvate, or stereoisomer thereof.

3. The compound according to claim 1 having general formula (X), (XI), (XII), (XIII), (XIV), (XV) or (XVI): 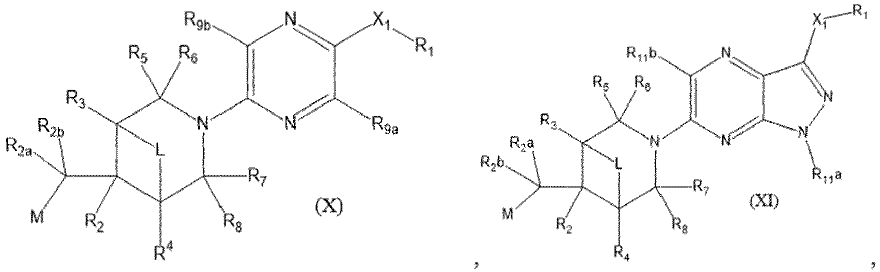 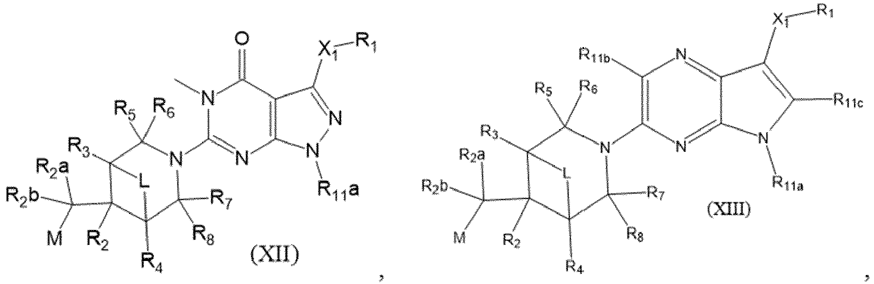 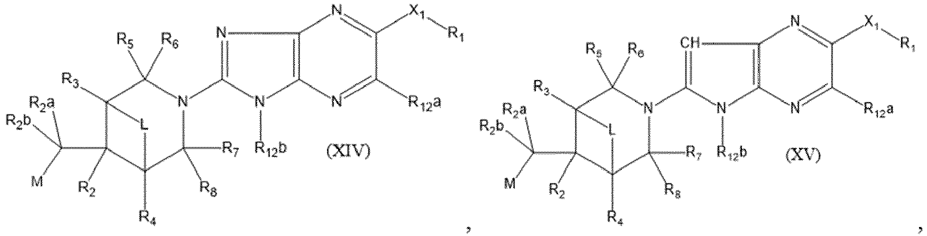 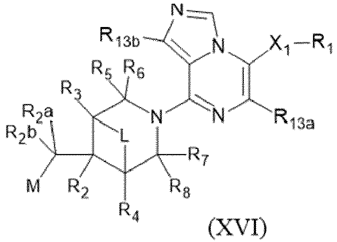 wherein: X₁, R₁, R₂, R₂ₐ, R_{2b}, R₃, R₄, R₅, R₆, R₇, R₈, R₉ₐ, R_{9b}, R₁₁ₐ, R_{11b}, R_{11c}, R₁₂ₐ, R_{12b}, R₁₃ₐ, R_{13b}, M and L are as defined in claim 1; or a pharmaceutically acceptable salt, tautomer, solvate, or stereoisomer thereof.

4. The compound according to anyone of previous claims wherein:
- L is a single bond; and/or
- X₁ is a single bond or S;
or a pharmaceutically acceptable salt, tautomer, solvate, or stereoisomer thereof.

5. The compound according to anyone of previous claims wherein M is NH₂; or a pharmaceutically acceptable salt, tautomer, solvate, or stereoisomer thereof.

6. The compound according to anyone of previous claims wherein R₂ₐ, R_{2b}, R₃, R₃ₐ, R_{3b}, R₄, R₅, R₆, R₇, R₈ are H.

7. The compound according to anyone of previous claims wherein R₁ is selected from: phenyl, napthyl, benzothiophene, quinoline, quinoxaline, isoquinoline, pyrazolo[3,4-b]pyrazine, pyridine, pyrimidine, pyrazine, thienopyridine, indole, indoline, 2,3-dihydroisoindol-1-one, indol-2-one, benzothiazole, 6a,7,8,9-tetrahydro-6H-pyrido[3,2-b]pyrrolo[1,2-d][1,4]oxazine, naphtyridine, 1,2,3,4-tetrahydro-1,5-naphthyridine, benzo[d][1,3]dioxole, 2,3-dihydrobenzo[b][1,4]dioxine, benzoxazole, thieno[3,2-b]pyridine, indazole, each being optionally substituted with one or more substituents independently selected form halogen, C₁₋₃alkyl, CN, CF₃, NH₂, OCH₃, OCF₃, CH₂OCH₃, OSO₂F, SO₂F, oxetane, C(CH₃)₂OH, CH(CH₃)OH.

8. The compound according to anyone of previous claims wherein R₂ is selected from: phenyl, pyridine, pyrimidine, pyrazine, oxazole, isoxazole, pyrrole, pyrazole triazole, oxadiazole, thiophene, thiazole, thiadiazole, imidazole, each optionally substituted with one or more substituents independently selected form halogen, C₁₋₃alkyl, CN, CF₃, NH₂, OCH₃, OH, CH₂OH, CH₂OCH₃, CO₂CH₃, CONHC₁₋₆alkyl.

9. A compound according to any one of previous claims selected from:
- [3-[7-(aminomethyl)-7-thiophen-3-yl-3-azabicyclo[4.1.0]heptan-3-yl]-6-(2,3-dichlorophenyl)-5-methylpyrazin-2-yl]methanol;
- [3-[3-(2,3-dichlorophenyl)-1H-pyrazolo[3,4-b]pyrazin-6-yl]-7-pyridin-3-yl-3-azabicyclo[4.1.0]heptan-7-yl]methanamine;
- [3-[3-(2,3-dichlorophenyl)-1H-pyrazolo[3,4-b]pyrazin-6-yl]-7-(2-fluorophenyl)-3-azabicyclo[4.1.0]heptan-7-yl]methanamine;
- [3-[3-(1-benzothiophen-7-yl)-1H-pyrazolo[3,4-b]pyrazin-6-yl]-7-(2-fluorophenyl)-3-azabicyclo[4.1.0]heptan-7-yl]methanamine;
- [7-(2-fluorophenyl)-3-(3-quinolin-5-yl-1H-pyrazolo[3,4-b]pyrazin-6-yl)-3-azabicyclo[4.1.0]heptan-7-yl]methanamine;
- [3-[3-(2,3-dichlorophenyl)-1H-pyrazolo[3,4-b]pyrazin-6-yl]-7-(3-methyl-1,2-oxazol-5-yl)-3-azabicyclo[4.1.0]heptan-7-yl]methanamine;
- [3-[3-(2,3-dichlorophenyl)-1H-pyrazolo[3,4-b]pyrazin-6-yl]-7-(3-fluorophenyl)-3-azabicyclo[4.1.0]heptan-7-yl]methanamine;
- [3-[3-(2,3-dichlorophenyl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-(3-quinolin-5-yl-1H-pyrazolo[3,4-b]pyrazin-6-yl)-3-azabicyclo[4.1.0]heptan-7-yl]methanamine;
- [3-[3-(2,3-dichlorophenyl)-1H-pyrazolo[3,4-b]pyrazin-6-yl]-7-(4-fluorophenyl)-3-azabicyclo[4.1.0]heptan-7-yl]methanamine;
- [7-(4-fluorophenyl)-3-(3-quinoxalin-6-yl-1H-pyrazolo[3,4-b]pyrazin-6-yl)-3-azabicyclo[4.1.0]heptan-7-yl]methanamine;
- [7-(4-fluorophenyl)-3-(3-quinolin-8-yl-1H-pyrazolo[3,4-b]pyrazin-6-yl)-3-azabicyclo[4.1.0]heptan-7-yl]methanamine;
- [3-[3-(2,3-dichlorophenyl)-1H-pyrazolo[3,4-b]pyrazin-6-yl]-7-(1-methyltriazol-4-yl)-3-azabicyclo[4.1.0]heptan-7-yl]methanamine;
- [3-[3-(2,3-dichlorophenyl)-1H-pyrazolo[3,4-b]pyrazin-6-yl]-7-(4-methyl-1,2-oxazol-3-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(4-methyl-1,2-oxazol-3-yl)-3-azabicyclo[4.1.0]heptan-7-yl]methanamine;
- 4-[6-[7-(aminomethyl)-7-(2-fluorophenyl)-3-azabicyclo[4.1.0]heptan-3-yl]-1H-pyrazolo[3,4-b]pyrazin-3-yl]-3-chloro-∼{N}-methylpyridin-2-amine;
- 4-[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]-3-chloro-∼{N}-methylpyridin-2-amine;
- [7-(5-methyl-1,2-oxazol-3-yl)-3-(3-thieno[3,2-b]pyridin-7-yl-1H-pyrazolo[3,4-b]pyrazin-6-yl)-3-azabicyclo[4.1.0]heptan-7-yl]methanamine;
- 4-[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]-2,3-dihydroisoindol-1-one;
- [3-[3-(1,3-benzothiazol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-[3-(8-methylquinolin-5-yl)-1H-pyrazolo[3,4-b]pyrazin-6-yl]-3-azabicyclo[4.1.0]heptan-7-yl]methanamine;
- [3-[3-(8-methoxyquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-(3-isoquinolin-5-yl-1H-pyrazolo[3,4-b]pyrazin-6-yl)-7-(5-methyl-1,2-oxazol-3-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-[3-(2-methylquinolin-5-yl)-1H-pyrazolo[3,4-b]pyrazin-6-yl]-3-azabicyclo[4.1.0]heptan-7-yl]methanamine;
- [3-[3-(2-methylindazol-4-yl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-(3-quinolin-4-yl-1H-pyrazolo[3,4-b]pyrazin-6-yl)-3-azabicyclo[4.1.0]heptan-7-yl]methanamine;
- [3-[3-(2-methoxypyridin-3-yl)-1H-pyrazolo[3,4-b]pyrazin-6-yl]-7-(5-methyl-1,2-oxazol-3-yl)-3-azabicyclo[4.1.0]heptan-7-yl]methanamine;
- (7-(5-methylisoxazol-3-yl)-3-(3-((S)-6a,7,8,9-tetrahydro-6H-pyrido[3,2-b]pyrrolo[1,2-d][1,4]oxazin-4-yl)-1H-pyrazolo[3,4-b]pyrazin-6-yl)-3-azabicyclo[4.1.0]heptan-7-yl)methanamine;
- [3-[3-(8-fluoroquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-[3-(2,3-dichloropyridin-4-yl)-1H-pyrazolo[3,4-b]pyrazin-6-yl]-7-(5-methyl-1,2-oxazol-3-yl)-3-azabicyclo[4.1.0]heptan-7-yl]methanamine;
- 5-[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]quinoline-8-carbonitrile;
- [3-[3-(8-chloroquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-[3-(8-chloroisoquinolin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [7-(5-methyl-1,2-oxazol-3-yl)-3-[3-(1,5-naphthyridin-4-yl)-1H-pyrazolo[3,4-b]pyrazin-6-yl]-3-azabicyclo[4.1.0]heptan-7-yl]methanamine;
- 4-[6-[7-(aminomethyl)-7-(5-methyl-1,2-oxazol-3-yl)-3-azabicyclo[4.1.0]heptan-3-yl]-¹H-pyrazolo[3,4-b]pyrazin-3-yl]-3-chloropyridin-2-amine;
- [(1S,6R,7S)-3-[3-(2,2-difluoro-1,3-benzodioxol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-7-(5-methyl-1,2-oxazol-3-yl)-3-[3-(1,8-naphthyridin-4-yl)-1H-pyrazolo[3,4-b]pyrazin-6-yl]-3-azabicyclo[4.1.0]heptan-7-yl]methanamine;
- [(1S,6R,7S)-3-[3-(6-fluoroquinolin-4-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(2,3-dihydro-1,4-benzodioxin-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(5-fluoroquinolin-4-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(6-chloroquinolin-4-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-7-(5-methyl-1,2-oxazol-3-yl)-3-[3-[8-(trifluoromethyl)quinolin-5-yl]-1H-pyrazolo[3,4-b]pyrazin-6-yl]-3-azabicyclo[4.1.0]heptan-7-yl]methanamine;
- [(1S,6R,7S)-3-[3-(3,4-dichloro-2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(7-fluoroquinolin-4-yl)-1H-pyrazolo[3,4-b]pyrazin-6-yl]-7-(5-methyl-1,2-oxazol-3-yl)-3-azabicyclo[4.1.0]heptan-7-yl]methanamine;
- [(1S,6R,7S)-3-[3-(4-chloro-2-methylindazol-5-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-[5-(methoxymethyl)-1,2-oxazol-3-yl]-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(5-cyclopropyl-1,2-oxazol-3-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(3-fluoropyridin-2-yl)-3-azabicyclo[4.1.0]heptan-7-yl]methanamine;
- [3-[3-(4-chloro-2-methylindazol-5-yl)-1H-pyrazolo[3,4-b]pyrazin-6-yl]-7-(5-fluoropyridin-2-yl)-3-azabicyclo[4.1.0]heptan-7-yl]methanamine;
- 4-[6-[(1S,6R,7S)-7-(aminomethyl)-7-(5-methyl-1,2-oxazol-3-yl)-3-azabicyclo[4.1.0]heptan-3-yl]-1H-pyrazolo[3,4-b]pyrazin-3-yl]-3,3-difluoro-1-methylindol-2-one;
- [3-[3-(3-methyl-1,2-benzoxazol-5-yl)-1H-pyrazolo[3,4-b]pyrazin-6-yl]-7-(4-methyl-1,2-oxazol-3-yl)-3-azabicyclo[4.1.0]heptan-7-yl]methanamine;
- 4-[[6-[7-(aminomethyl)-7-(5-methyl-1,2-oxazol-3-yl)-3-azabicyclo[4.1.0]heptan-3-yl]-1H-pyrazolo[3,4-b]pyrazin-3-yl]sulfanyl]-3-chloropyridin-2-amine;
- [7-(5-methyl-1,2-oxazol-3-yl)-3-(3-thieno[3,2-b]pyridin-7-ylsulfanyl-1H -pyrazolo[3,4-b]pyrazin-6-yl)-3-azabicyclo[4.1.0]heptan-7-yl]methanamine;
- [6-[7-(aminomethyl)-7-(3-fluorophenyl)-3-azabicyclo[4.1.0]heptan-3-yl]-3-(2,3-dichlorophenyl)-1H-pyrazolo[3,4-b]pyrazin-5-yl]methanol;
- [6-[7-(aminomethyl)-7-(3-fluorophenyl)-3-azabicyclo[4.1.0]heptan-3-yl]-3-phenyl-1H-pyrazolo[3,4-b]pyrazin-5-yl]methanol;
- [3-(2-amino-3-chloropyridin-4-yl)sulfanyl-6-[7-(aminomethyl)-7-(5-methyl-1,2-oxazol-3-yl)-3-azabicyclo[4.1.0]heptan-3-yl]-1H-pyrazolo[3,4-b]pyrazin-5-yl]methanol;
- [3-(2-amino-3-chloropyridin-4-yl)-6-[7-(aminomethyl)-7-(5-methyl-1,2-oxazol-3-yl)-3-azabicyclo[4.1.0]heptan-3-yl]-1H-pyrazolo[3,4-b]pyrazin-5-yl]methanol;
- 4-[[6-[7-(aminomethyl)-7-(2-fluorophenyl)-3-azabicyclo[4.1.0]heptan-3-yl]-5H-pyrrolo[2,3-b]pyrazin-2-yl]sulfanyl]-3-chloropyridin-2-amine;
- [3-[3-(3,4-dihydro-2H-1,5-naphthyridin-1-yl)-1H-pyrazolo[3,4-b]pyrazin-6-yl]-7-(5-methyl-1,2-oxazol-3-yl)-3-azabicyclo[4.1.0]heptan-7-yl]methanamine;
- [3-[7-(aminomethyl)-3-[3-(4-chloro-2-methylindazol-5-yl)-1H-pyrazolo[3,4-b]pyrazin-6-yl]-3-azabicyclo[4.1.0]heptan-7-yl]-1,2-oxazol-5-yl]methanol;
- 6-[7-(aminomethyl)-7-(5-methyl-1,2-oxazol-3-yl)-3-azabicyclo[4.1.0]heptan-3-yl]-3-(4-chloro-2-methylindazol-5-yl)-5-methyl-1H-pyrazolo[3,4-d]pyrimidin-4-one;
- [7-phenyl-3-[5-[2-(trifluoromethyl)pyridin-3-yl]sulfanyl-1H-imidazo[4,5-b]pyrazin-2-yl]-3-azabicyclo[4.1.0]heptan-7-yl]methanamine;
- [7-thiophen-3-yl-3-[5-[2-(trifluoromethyl)pyridin-3-yl]sulfanyl-1H-imidazo[4,5-b]pyrazin-2-yl]-3-azabicyclo[4.1.0]heptan-7-yl]methanamine;
- [7-pyridin-2-yl-3-[5-[2-(trifluoromethyl)pyridin-3-yl]thio-1H-imidazo[4,5-b]pyrazin-2-yl]-3-azabicyclo[4.1.0]heptan-7-yl]methanamine;
- [7-pyrimidin-2-yl-3-[5-[2-(trifluoromethyl)pyridin-3-yl]sulfanyl-1H-imidazo[4,5-b]pyrazin-2-yl]-3-azabicyclo[4.1.0]heptan-7-yl]methanamine;
- [7-(4-methyl-1,3-thiazol-2-yl)-3-[5-[2-(trifluoromethyl)pyridin-3-yl]thio-1H-imidazo[4,5-b]pyrazin-2-yl]-3-azabicyclo[4.1.0]heptan-7-yl]methanamine;
- [3-[5-[2-(trifluoromethyl)pyridin-3-yl]thio-1H-imidazo[4,5-b]pyrazin-2-yl]-3-azabicyclo[4.1.0]heptan-7-yl]methanamine;
- 4-[[2-[7-(aminomethyl)-7-phenyl-3-azabicyclo[4.1.0]heptan-3-yl]-1H-imidazo[4,5-b]pyrazin-5-yl]thio]-3-chloropyridin-2-amine;
- 4-[[2-[7-(aminomethyl)-7-(4-methyl-1,3-thiazol-2-yl)-3-azabicyclo[4.1.0]heptan-3-yl]-1H-imidazo[4,5-b]pyrazin-5-yl]thio]-3-chloropyridin-2-amine;
- 6-[7-(aminomethyl)-7-phenyl-3-azabicyclo[4.1.0]heptan-3-yl]-3-[(2-(trifluoromethyl)pyridin-3-yl)thio]pyrazin-2-amine;
- 6-[7-(aminomethyl)-7-pyridin-2-yl-3-azabicyclo[4.1.0]heptan-3-yl]-3-[(2-(trifluoromethyl)pyridin-3-yl)thio]pyrazin-2-amine;
- 6-(7-(aminomethyl)-7-(4-methylthiazol-2-yl)-3-azabicyclo[4.1.0]heptan-3-yl)-3-((2-(trifluoromethyl)pyridin-3-yl)thio)pyrazin-2-amine;
- 3-((2-amino-3-chloropyridin-4-yl)thio)-6-[7-(aminomethyl)-7-(4-methyl-1,3-thiazol-2-yl)-3-azabicyclo[4.1.0]heptan-3-yl]pyrazin-2-amine;
- 6-(7-(aminomethyl)-3-azabicyclo[4.1.0]heptan-3-yl)-3-((2-(trifluoromethyl)pyridin-3-yl)thio)pyrazin-2-amine;
- 4-((5-(7-(aminomethyl)-7-(4-methylthiazol-2-yl)-3-azabicyclo[4.1.0]heptan-3-yl)imidazo[1,2-c]pyrimidin-8-yl)thio)-3-chloropyridin-2-amine;
- [3-[3-(2,3-dichlorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [(1S,6R,7S)-3-[3-(2,3-dichlorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [(1S,6R,7S)-3-[3-(4-chloro-2-methylindazol-5-yl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(2,3-dichlorophenyl)-1H-pyrazolo[3,4-b]pyrazin-6-yl]-7-phenyl-3-azabicyclo[4.1.0]heptan-7-yl]methanamine;
- [3-[3-(4-chloro-2-methylindazol-5-yl)-1H-pyrazolo[3,4-b]pyrazin-6-yl]-7-phenyl-3-azabicyclo[4.1.0]heptan-7-yl]methanamine;
- [3-[3-(2,3-dichlorophenyl)-1H-pyrazolo[3,4-b]pyrazin-6-yl]-7-pyridin-2-yl-3-azabicyclo[4.1.0]heptan-7-yl]methanamine;
- [3-[3-(2,3-dichlorophenyl)-1H-pyrazolo[3,4-b]pyrazin-6-yl]-7-pyrimidin-2-yl-3-azabicyclo[4.1.0]heptan-7-yl]methanamine;
- [3-[3-(2-chlorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(3-chlorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(2,3-difluorophenyl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(2-chloro-3-methoxyphenyl)-1H-pyrazolo[3,4-b]pyrazin-6-yl]-7-(4-methyl-1,3-thiazol-2-yl)-3-azabicyclo[4.1.0]heptan-7-yl]methanamine;
- [7-(4-methyl-1,3-thiazol-2-yl)-3-(3-quinolin-5-yl-1H-pyrazolo[3,4-b]pyrazin-6-yl)-3-azabicyclo[4.1.0]heptan-7-yl]methanamine;
- [7-(4-methyl-1,3-thiazol-2-yl)-3-(3-naphthalen-1-yl-1H-pyrazolo[3,4-b]pyrazin-6-yl)-3-azabicyclo[4.1.0]heptan-7-yl]methanamine;
- [7-(4-methyl-1,3-thiazol-2-yl)-3-[3-[2-(trifluoromethyl)pyridin-3-yl]-1H-pyrazolo[3,4-b]pyrazin-6-yl]-3-azabicyclo[4.1.0]heptan-7-yl]methanamine;
- [3-[3-(1,3-benzodioxol-4-yl)-1H-pyrazolo[3,4-b]pyrazin-6-yl]-7-(4-methyl-1,3-thiazol-2-yl)-3-azabicyclo[4.1.0]heptan-7-yl]methanamine;
- [3-[3-(8-fluoroquinolin-5-yl)-1H-pyrazolo[3,4-b]pyrazin-6-yl]-7-(4-methyl-1,3-thiazol-2-yl)-3-azabicyclo[4.1.0]heptan-7-yl]methanamine;
- 4-[[6-[7-(aminomethyl)-7-(4-methyl-1,3-thiazol-2-yl)-3-azabicyclo[4.1.0]heptan-3-yl]-1H-pyrazolo[3,4-b]pyrazin-3-yl]sulfanyl]-3-chloropyridin-2-amine;
- [3-[3-(2,3-dichlorophenyl)-1H-pyrazolo[3,4-b]pyrazin-6-yl]-7-thiophen-3-yl-3-azabicyclo[4.1.0]heptan-7-yl]methanamine;
- [7-thiophen-3-yl-3-[3-[2-(trifluoromethyl)pyridin-4-yl]-1H-pyrazolo[3,4-b]pyrazin-6-yl]-3-azabicyclo[4.1.0]heptan-7-yl]methanamine;
- [7-thiophen-3-yl-3-[3-[2-(trifluoromethyl)pyridin-3-yl]-1H-pyrazolo[3,4-b]pyrazin-6-yl]-3-azabicyclo[4.1.0]heptan-7-yl]methanamine;
- [3-[3-(2,3-dichlorophenyl)-1H-pyrazolo[3,4-b]pyrazin-6-yl]-7-(2-methylpyrazol-3-yl)-3-azabicyclo[4.1.0]heptan-7-yl]methanamine;
- [3-[3-(2,3-dichlorophenyl)-1H-pyrazolo[3,4-b]pyrazin-6-yl]-7-(1-methylpyrazol-3-yl)-3-azabicyclo[4.1.0]heptan-7-yl]methanamine;
- [3-[3-(2,3-dichlorophenyl)-1H-pyrazolo[3,4-b]pyrazin-6-yl]-7-(1,2-oxazol-3-yl)-3-azabicyclo[4.1.0]heptan-7-yl]methanamine;
- [3-[3-(2,3-dichlorophenyl)-1H-pyrazolo[3,4-b]pyrazin-6-yl]-3-azabicyclo[4.1.0]heptan-7-yl]methanamine;
- [6-[7-(aminomethyl)-7-(4-methyl-1,3-thiazol-2-yl)-3-azabicyclo[4.1.0]heptan-3-yl]-3-quinolin-5-yl-1H-pyrazolo[3,4-b]pyrazin-5-yl]methanol;
- [6-[7-(aminomethyl)-7-(4-methyl-1,3-thiazol-2-yl)-3-azabicyclo[4.1.0]heptan-3-yl]-3-(2,3-dichlorophenyl)-1H-pyrazolo[3,4-b]pyrazin-5-yl]methanol;
- 2-[7-(aminomethyl)-3-[3-(4-fluorosulfonyloxynaphthalen-1-yl)-1H-pyrazolo[3,4-b]pyrazin-6-yl]-3-azabicyclo[4.1.0]heptan-7-yl]-4-methyl-1,3-thiazole;
- 6-(6-(aminomethyl)-6-phenyl-3-azabicyclo[3.1.0]hexan-3-yl)-3-((2-(trifluoromethyl)pyridin-3-yl)thio)pyrazin-2-amine;
- 4-(6-((1S,6R,7R)-7-(aminomethyl)-7-(2-fluorophenyl)-3-azabicyclo[4. 1.0]heptan-3-yl)-1H-pyrazolo[3,4-b]pyrazin-3-yl)-3-chloropyridin-2-amine;
- ((1S,6R,7R)-7-(2,6-difluorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine:
- (3-(3-(8-chloroquinolin-5-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7S)-3-(3-(7-chloro-2-methylbenzo[d]thiazol-6-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(4-chloro-2,3-dimethyl-2H-indazol-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(4-chloro-2-methyl-2H-indazol-5-yl)-1H-pyrazolo[3,4-b]pyrazin-6-yl)-7-(5-fluoropyridin-2-yl)-3-azabicyclo[4.1.0]heptan-7-yl)methanamine;
- 4-(6-(7-(aminomethyl)-7-(4-methylthiazol-2-yl)-3-azabicyclo[4.1.0]heptan-3-yl)-1H-pyrazolo[3,4-b]pyrazin-3-yl)naphthalene-1-sulfonyl fluoride;
- ((1S,6R,7S)-3-(3-(4-chloro-3-methoxy-2-methyl-2H-indazol-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(3,4-dichloro-2-methyl-2H-indazol-5-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7S)-3-(3-(7-chloro-2-methylbenzo[d]thiazol-6-yl)-1H-pyrazolo[3,4-b]pyrazin-6-yl)-7-(5-fluoropyridin-2-yl)-3-azabicyclo[4.1.0]heptan-7-yl)methanamine;
- (3-(3-(7-chloro-2-methylbenzo[d]thiazol-6-yl)-5-methyl-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(4-chloro-2-methyl-2H-indazol-5-yl)-1H-pyrazolo[3,4-b]pyrazin-6-yl)-7-(isoxazol-3-yl)-3-azabicyclo[4.1.0]heptan-7-yl)methanamine;
- ((1S,6R,7S)-3-(3-(7-chloro-2-methylbenzo[d]thiazol-6-yl)-1H-pyrazolo[3,4-b]pyrazin-6-yl)-7-(isoxazol-3-yl)-3-azabicyclo[4.1.0]heptan-7-yl)methanamine;
- ((1S,6R,7S)-3-(3-(2-chloro-3-(oxazol-2-yl)phenyl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(5-chloro-3-methoxyquinoxalin-6-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(thieno[2,3-b]pyridin-3-yl)-1H-pyrazolo[3,4-b]pyrazin-6-yl)-3-azabicyclo[4.1.0]heptan-7-yl)methanamine;
- ((1S,6R,7S)-3-(3-(4-chloropyrazolo[1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7S)-3-(3-(7-chloro-2-methylbenzo[d]thiazol-6-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7S)-3-(3-(8-fluoroquinolin-4-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(8-methoxyquinolin-4-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(8-(trifluoromethoxy)quinolin-5-yl)-1H-pyrazolo[3,4-b]pyrazin-6-yl)-3-azabicyclo[4.1.0]heptan-7-yl)methanamine;
- ((1S,6R,7S)-3-(3-(6-methoxyquinolin-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(2,4-dimethyl-2H-indazol-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(8-(methoxymethyl)quinolin-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(pyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7S)-3-(3-(7-fluoroquinolin-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7R)-7-(2-fluorophenyl)-3-(3-(pyrazolo[1,5-a]pyridin-4-yl)-1H-pyrazolo[3,4-b]pyrazin-6-yl)-3-azabicyclo[4.1.0]heptan-7-yl)methanamine;
- 4-(6-(7-(aminomethyl)-7-(4-methylthiazol-2-yl)-3-azabicyclo[4.1.0]heptan-3-yl)-1H-pyrazolo[3,4-b]pyrazin-3-yl)-2,3-dichlorophenyl sulfurofluoridate;
- 5-(6-((1S,6R,7S)-7-(aminomethyl)-7-(4-methylthiazol-2-yl)-3-azabicyclo[4.1.0]heptan-3-yl)-1H-pyrazolo[3,4-b]pyrazin-3-yl)quinolin-8-yl sulfurofluoridate;
- ((1S,6R,7S)-3-(3-(1,7-naphthyridin-5-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-3-(3-(2-methoxyquinolin-6-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(2-(trifluoromethyl)quinolin-6-yl)-1H-pyrazolo[3,4-b]pyrazin-6-yl)-3-azabicyclo[4.1.0]heptan-7-yl)methanamine;
- ((1S,6R,7S)-3-(3-(4-chloro-2-methylpyrazolo[1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine;
- ((1S,6R,7R)-7-(2-fluorophenyl)-3-(3-(8-fluoroquinolin-4-yl)-1H-pyrazolo[3,4-b]pyrazin-6-yl)-3-azabicyclo[4.1.0]heptan-7-yl)methanamine;
- ((1S,6R,7S)-3-(3-(7-methoxyquinolin-3-yl)-1H-pyrazolo[3,4-b]pyrazin-6-yl)-7-(5-methylisoxazol-3-yl)-3-azabicyclo[4.1.0]heptan-7-yl)methanamine;
- ((1S,6R,7S)-7-(5-methylisoxazol-3-yl)-3-(3-(7-(trifluoromethyl)quinolin-3-yl)-1H-pyrazolo[3,4-b]pyrazin-6-yl)-3-azabicyclo[4.1.0]heptan-7-yl)methanamine;
- ((1S,6R,7R)-7-(2,6-difluorophenyl)-3-(3-(pyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7R)-3-(3-(4-chloro-2-methyl-2H-indazol-5-yl)-1H-pyrazolo[3,4-b]pyrazin-6-yl)-7-(2,6-difluorophenyl)-3-azabicyclo[4.1.0]heptan-7-yl)methanamine;
- ((1S,6R,7S)-3-(3-(8-(difluoromethyl)quinolin-5-yl)-1H-pyrazolo[3,4-b]pyrazin-6-yl)-7-(5-methylisoxazol-3-yl)-3-azabicyclo[4.1.0]heptan-7-yl)methanamine;
- 1-(5-(6-((1S,6R,7S)-7-(aminomethyl)-7-(5-methylisoxazol-3-yl)-3-azabicyclo[4.1.0]heptan-3-yl)-1H-pyrazolo[3,4-b]pyrazin-3-yl)quinolin-8-yl)ethan-1-ol;
- ((1S,6R,7R)-7-(2-fluorophenyl)-3-(3-(8-fluoroquinolin-5-yl)-1H-pyrazolo[3,4-b]pyrazin-6-yl)-3-azabicyclo[4.1.0]heptan-7-yl)methanamine;
- 4-((8-((1S,6R,7R)-7-(aminomethyl)-7-(2-fluorophenyl)-3-azabicyclo[4.1.0]heptan-3-yl)imidazo[1,5-a]pyrazin-5-yl)thio)-3-chloropyridin-2-amine;
- ((1S,6R,7R)-7-(2-fluorophenyl)-3-(3-(pyrazolo[1,5-a]pyridin-5-yl)-1H-pyrazolo[3,4-b]pyrazin-6-yl)-3-azabicyclo[4.1.0]heptan-7-yl)methanamine;
- ((1S,6R,7S)-3-(3-(6-chloropyrazolo[1,5-a]pyridin-5-yl)-1H-pyrazolo[3,4-b]pyrazin-6-yl)-7-(4-methylthiazol-2-yl)-3-azabicyclo[4.1.0]heptan-7-yl)methanamine;
- (7-(4-chlorothiazol-2-yl)-3-(3-(8-fluoroquinolin-5-yl)-1H-pyrazolo[3,4-b]pyrazin-6-yl)-3-azabicyclo[4.1.0]heptan-7-yl)methanamine;
- 2-(5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl)propan-2-ol;
- ((1S,6R,7S)-7-(3-fluoropyridin-2-yl)-3-(3-(pyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7R)-7-(2-fluorophenyl)-3-(3-(quinolin-6-yl)-1H-pyrazolo[3,4-b]pyrazin-6-yl)-3-azabicyclo[4.1.0]heptan-7-yl)methanamine;
- ((1S,6R,7S)-7-(3-fluoropyridin-2-yl)-3-(3-(8-(trifluoromethoxy)quinolin-5-yl)-1H-pyrazolo[3,4-b]pyrazin-6-yl)-3-azabicyclo[4.1.0]heptan-7-yl)methanamine;
- ((1S,6R,7R)-3-(3-(4-chloropyrazolo[1,5-a]pyridin-5-yl)-1H-pyrazolo[3,4-b]pyrazin-6-yl)-7-(2-fluorophenyl)-3-azabicyclo[4.1.0]heptan-7-yl)methanamine;
- 1-(5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl)ethan-1-ol;
- ((1S,6R,7S)-3-(7-(8-fluoroquinolin-5-yl)-5H-pyrrolo[2,3-b]pyrazin-3-yl)-7-(4-methylthiazol-2-yl)-3-azabicyclo[4.1.0]heptan-7-yl)methanamine;
- ((1S,6R,7R)-7-(2-fluorophenyl)-3-(3-(8-isopropoxyquinolin-5-yl)-1H-pyrazolo[3,4-b]pyrazin-6-yl)-3-azabicyclo[4.1.0]heptan-7-yl)methanamine;
- ((1S,6R,7S)-3-(7-(8-fluoroquinolin-5-yl)-5H-pyrrolo[2,3-b]pyrazin-3-yl)-7-(5-methylisoxazol-3-yl)-3-azabicyclo[4.1.0]heptan-7-yl)methanamine;
- ((1S,6R,7R)-3-(3-(benzo[d]thiazol-6-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(7-(2,4-dimethyl-2H-indazol-5-yl)-5H-pyrrolo[2,3-b]pyrazin-3-yl)-7-(2-fluorophenyl)-3-azabicyclo[4.1.0]heptan-7-yl)methanamine;
- ((1S,6R,7R)-3-(3-(2,4-dimethyl-2H-indazol-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(3-(8-methoxyquinolin-5-yl)-1H-pyrazolo[3,4-b]pyrazin-6-yl)-3-azabicyclo[4.1.0]heptan-7-yl)methanamine;
- ((1S,6R,7R)-3-(3-(5-chloro-3-methoxyquinoxalin-6-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(8-cyclopropylquinolin-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(3-(imidazo[1,2-a]pyridin-5-yl)-1H-pyrazolo[3,4-b]pyrazin-6-yl)-3-azabicyclo[4.1.0]heptan-7-yl)methanamine;
- ((1S,6R,7R)-3-(3-(2-chloro-3-(pyrazin-2-yl)phenyl)-1H-pyrazolo[3,4-b]pyrazin-6-yl)-7-(2-fluorophenyl)-3-azabicyclo[4.1.0]heptan-7-yl)methanamine;
- 4-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)indolin-2-one;
- ((1S,6R,7R)-3-(3-(2,3-dihydrofuro[2,3-b]pyridin-4-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(1,7-naphthyridin-4-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(7-(8-fluoroquinolin-5-yl)-5H-pyrrolo[2,3-b]pyrazin-3-yl)-3-azabicyclo[4.1.0]heptan-7-yl)methanamine;
- ((1S,6R,7R)-3-(3-(1,6-naphthyridin-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(3-(6-methoxypyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7R)-7-(2,3-difluorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine;
- 4-(6-((1S,6R,7R)-7-(aminomethyl)-7-(2,3-difluorophenyl)-3-azabicyclo[4.1.0]heptan-3-yl)-1H-pyrazolo[3,4-b]pyrazin-3-yl)-3-chloropyridin-2-amine;
- 5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinoline-8-sulfonyl fluoride;
- ((1S,6R,7R)-3-(3-(3-chloro-2-fluoropyridin-4-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(3-chloro-2-(1H-pyrazol-1-yl)pyridin-4-yl)-1H-pyrazolo[3,4-b]pyrazin-6-yl)-7-(2-fluorophenyl)-3-azabicyclo[4.1.0]heptan-7-yl)methanamine;
- (5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl)methanol;
- ((1S,6R,7R)-3-(3-(8-(difluoromethoxy)quinolin-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-7-(2-fluorophenyl)-3-(3-(7-methylpyrazolo[1,5-a]pyridin-4-yl)-1H-pyrazolo[3,4-b]pyrazin-6-yl)-3-azabicyclo[4.1.0]heptan-7-yl)methanamine;
- ((1S,6R,7R)-7-(2-chlorophenyl)-3-(3-(2,4-dimethyl-2H-indazol-5-yl)-1H-pyrazolo[3,4-b]pyrazin-6-yl)-3-azabicyclo[4.1.0]heptan-7-yl)methanamine;
- ((1S,6R,7S)-3-(3-(2,4-dimethyl-2H-indazol-5-yl)-1H-pyrazolo[3,4-b]pyrazin-6-yl)-7-(3-fluoropyridin-2-yl)-3-azabicyclo[4.1.0]heptan-7-yl)methanamine;
- 4-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)pyrimidin-2-amine;
- 4-(6-((1S,6R,7R)-7-(aminomethyl)-7-(2-chlorophenyl)-3-azabicyclo[4.1.0]heptan-3-yl)-1H-pyrazolo[3,4-b]pyrazin-3-yl)-3-chloropyridin-2-amine;
- 5-(6-((1S,6R,7R)-7-(aminomethyl)-7-(2-fluorophenyl)-3-azabicyclo[4.1.0]heptan-3-yl)-1H-pyrazolo[3,4-b]pyrazin-3-yl)quinolin-8-yl sulfurofluoridate;
- ((1S,6R,7R)-3-(3-(8-ethoxyquinolin-5-yl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(3-bromo-2-chlorophenyl)-1H-pyrazolo[3,4-b]pyrazin-6-yl)-7-(2-fluorophenyl)-3-azabicyclo[4.1.0]heptan-7-yl)methanamine;
- ((1S,6R,7R)-3-(3-(2-chloro-3-(oxazol-2-yl)phenyl)-1H-pyrazolo[3,4-b]pyrazin-6-yl)-7-(2-fluorophenyl)-3-azabicyclo[4.1.0]heptan-7-yl)methanamine;
- (3-(3-(8-chloroquinolin-5-yl)-1H-pyrazolo[3,4-b]pyrazin-6-yl)-7-(5-methylisoxazol-3-yl)-3-azabicyclo[4.1.0]heptan-7-yl)methanamine;
- (3-(3-(4-chloro-2-methyl-2H-indazol-5-yl)-1H-pyrazolo[3,4-b]pyrazin-6-yl)-7-phenyl-3-azabicyclo[4.1.0]heptan-7-yl)methanamine;
or a pharmaceutically acceptable salt, tautomer, solvate, or stereoisomer thereof.

10. A compound or a pharmaceutically acceptable salt, tautomer, solvate, or stereoisomer thereof as defined in any one of previous claims for medical use.

11. The compound or the pharmaceutically acceptable salt, tautomer, solvate, or stereoisomer thereof according to any one of previous claims for use in inhibiting SHP2 activity.

12. The compound or the pharmaceutically acceptable salt, tautomer, solvate, or stereoisomer thereof according to any one of previous claims for use in the treatment and/or prevention of a disease or disorder mediated by the activity of SHP2.

13. The compound or the pharmaceutically acceptable salt, tautomer, solvate, or stereoisomer thereof according to any one of the previous claims for use in the treatment and/or prevention of a disease or disorder selected from the group consisting of: cancer, cardiovascular disease, immunological disorder, autoimmune disorder, fibrosis, an ocular disorder, systemic lupus erythematosus, diabetes, neutropenia and combinations thereof.

14. The compound or the pharmaceutically acceptable salt, tautomer, solvate, or stereoisomer thereof for use according to claim 12 or 13, wherein the disease or disorder is selected from the group consisting of: Noonan Syndrome, Leopard Syndrome, juvenile myelomonocytic leukemias, neuroblastoma, melanoma, head and neck squamous-cell carcinoma, acute myeloid leukemia, breast cancer, esophageal tumor, lung cancer, colon cancer, head cancer, gastric carcinoma, lymphoma, glioblastoma, gastric cancer, pancreatic cancer and combinations thereof.

15. The compound or the pharmaceutically acceptable salt, tautomer, solvate, or stereoisomer thereof for use according to claim 13 or 14 wherein any one of said cancers is a primary cancer or a cancer metastasis.

16. The compound or the pharmaceutically acceptable salt, tautomer, solvate, or stereoisomer thereof for use according to any one of claims 10-15, wherein said use is in combination with radiotherapy or with at least one further therapeutic agent, preferably said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1 H,3H)-pyrimidinedione and combinations thereof.

17. A pharmaceutical composition comprising the compound or the pharmaceutically acceptable salt, tautomer, solvate, or stereoisomer thereof as defined in anyone of claims 1-9, alone or in combination with at least one further therapeutic agent, and at least one pharmaceutically acceptable excipient, preferably said at least one further therapeutic agent is selected from the group consisting of:
(a) alkylating agents, including but not limited to carmustine, chlorambucil (LEUKERAN), cisplatin (PLATIN), carboplatin (PARAPLATIN), oxaliplatin (ELOXATIN), streptozocin (ZANOSAR), busulfan (MYLERAN), dacarbazine, ifosfamide, lomustine (CCNU), melphalan (ALKERAN), procarbazine (MATULAN), temozolomide(TEMODAR), thiotepa, and cyclophosphamide (ENDOXAN);
(b) anti-metabolites, including but not limited to cladribine (LEUSTATIN), mercaptopurine (PURINETHOL), thioguanine, pentostatin (NIPENT), cytosine arabinoside (cytarabine, ARA-C), gemcitabine (GEMZAR), fluorouracil (5-FU, CARAC), capecitabine (XELODA), leucovorin (FUSILEV), methotrexate (RHEUMATREX) and raltitrexed;
(c) antimitotics, which are often plant alkaloids and terpenoids, or derivatives thereof, including but not limited to taxanes such as docetaxel (TAXITERE) and paclitaxel (ABRAXANE, TAXOL); vinca alkaloids such as vincristine (ONCOVIN), vinblastine, vindesine, vinorelbine (NAVELBINE), and vinflunine;
(d) checkpoint inhibitors, such as anti- PD-1 or PD-L1 antibodies pembrolizumab (KEYTRUDA), nivolumab (OPDIVO), MEDI4736 and MPDL3280A; anti-CTLA-4 antibody ipilimumab (YERVOY); inhibitors that target LAG3 (lymphocyte activation gene 3 protein), KIR (killer cell immunoglobulin-like receptor), 4-1BB (tumour necrosis factor receptor superfamily member 9), TIM3 (T-cell immunoglobulin and mucin-domain containing-3) and/or OX40 (tumour necrosis factor receptor superfamily member 4);
(e) topoisomerase inhibitors, including but not limited to camptothecin (CTP), irinotecan (CAMPTOSAR), topotecan (HYCAMTIN), teniposide (VUMON) and etoposide (EPOSIN);
(f) cytotoxic antibiotics, including but not limited to actinomycin D (dactinomycin, COSMEGEN), bleomycin (BLENOXANE) doxorubicin (ADRIAMYCIN), daunorubicin (CERUBIDINE), epirubicin (ELLENCE), fludarabine (FLUDARA), idarubicin, mitomycin (MITOSOL), mitoxantrone (NOVANTRONE), plicamycin; (7) aromatase inhibitors, including but not limited to aminoglutethimide, anastrozole (ARIMIDEX), letrozole (FEMARA), vorozole (RIVIZOR) and exemestane (AROMASIN);
(g) angiogenesis inhibitors, including but not limited to genistein, sunitinib (SUTENT) and bevacizumab (AVASTIN);
(h) anti-steroids and anti-androgens such as aminoglutethimide (CYTADREN), bicalutamide (CASODEX), cyproterone, flutamide (EULEXIN) and nilutamide (NILANDRON);
(i) tyrosine kinase inhibitors, including but not limited to imatinib (GLEEVEC), erlotinib (TARCEVA), lapatininb (TYKERB), sorafenib (NEXAVAR), and axitinib (INLYTA);
(j) mTOR inhibitors such as everolimus, temsirolimus (TORISEL), and sirolimus; (12) monoclonal antibodies such as trastuzumab (HERCEPTIN) and rituximab (RITUXAN);
(k) other agents, such as amsacrine; Bacillus Calmette-Guérin (B-C-G) vaccine; buserelin (ETILAMIDE); chloroquine (ARALEN); clodronate, pamidronate, and other bisphosphonates; colchicine; demethoxyviridin; dichloroacetate; estramustine; filgrastim (NEUPOGEN); fludrocortisone (FLORINEF); goserelin (ZOLADEX); interferon; leucovorin; leuprolide (LUPRON); levamisole; lonidamine; mesna; metformin; mitotane (o,p'-DDD, LYSODREN); nocodazole; octreotide (SANDOSTATIN); perifosine; porfimer (particularly in combination with photo- and radiotherapy); suramin; tamoxifen; titanocene dichloride; tretinoin; anabolic steroids such as fluoxymesterone(HALOTESTIN); estrogens such as estradiol, diethylstilbestrol (DES), and dienestrol; progestins such as medroxyprogesterone acetate (MPA) and megestrol; testosterone; 5-fluoro-2-4(1 H,3H)-pyrimidinedione and combinations thereof.

18. The pharmaceutical composition according to claim 17 for the use as defined in any one of claims 10-15.


SEQUENCE LISTING

<110>  IRBM S.P.A.
       C.N.C.C.S. S.C.A.R.L. Collezione Nazionale dei Composti
       Chimici e Centro Screening

<120>  AZABICYCLIC SHP2 INHIBITORS

<130>  BE 140657

<160>  1

<170>  PatentIn version 3.5

<210>  1
<211>  18
<212>  PRT
<213>  Artificial Sequence

<220>
<223>  synthetic

<400>  1

Leu Asn Ile Asp Leu Asp Leu Val Leu Ser Thr Ala Ser Ile Asn Phe
1               5                   10                  15


Gln Lys